# EUROPEAN PATENT APPLICATION

(11) **EP 4 631 948 A1**
(43) Date of publication of application: **15.10.2025**
(21) Application number: 23901120.8
(22) Date of filing: 07.12.2023
(51) Int. Cl.: C07D 487/08, A61K 31/519, A61P 35/00, C07D 487/04, C07D 491/048

(54) **NOVEL TRICYCLIC COMPOUND AS KRAS G12D INHIBITOR, AND USE THEREOF**

(30) Priority: 07.12.2022 KR 20220169761
(71) Applicant: SK Biopharmaceuticals Co., Ltd., Seongnam-si, Gyeonggi-do 13494 (KR)
(72) Inventor: SONG, Ji Young, Seongnam-si, Gyeonggi-do 13494 (KR); JANG, Eunsung, Seongnam-si, Gyeonggi-do 13494 (KR); LEE, Dongho, Seongnam-si, Gyeonggi-do 13494 (KR); KIM, Minjung, Seongnam-si, Gyeonggi-do 13494 (KR); KIM, So Hyun, Seongnam-si, Gyeonggi-do 13494 (KR); YUN, Jiyeon, Seongnam-si, Gyeonggi-do 13494 (KR); JUNG, Jieun, Seongnam-si, Gyeonggi-do 13494 (KR); HONG, Beomjin, Seongnam-si, Gyeonggi-do 13494 (KR); LIM, Seona, Seongnam-si, Gyeonggi-do 13494 (KR); SHIN, Yong Je, Seongnam-si, Gyeonggi-do 13494 (KR); MOON, Mijin, Seongnam-si, Gyeonggi-do 13494 (KR)
(74) Representative: Vossius & Partner Patentanwälte Rechtsanwälte mbB
(86) International application number: PCT/KR2023/020084
(87) International publication number: WO 2024/123102

(57) **Abstract**

The present application relates to a novel tricyclic derivative compound as a KRAS G12D inhibitor, and a use thereof. The compound according to one embodiment of the present application inhibits the activity of a KRAS G12D mutant protein, and thus can be used in the prevention or treatment of diseases caused by a KRAS G12D mutation.

## Description

### [Technical Field]

The present application relates to novel tricyclic compounds, pharmaceutical compositions comprising the same, and their uses as medicaments.

### [Background Art]

Kristen rat sarcoma virus (KRAS) is a protein belonging to the RAS family (NRAS, HRAS, KRAS), which plays an important role in transmitting signals related to cell proliferation and differentiation. KRAS receives signals from cell membrane receptors and relays them to downstream signaling proteins, including PI3K, Raf, MEK, and ERK.

KRAS protein functions as a molecular switch, and becomes "activated (GTP-bound form, Turn-on) state" when GDP at the nucleotide binding site is exchanged for GTP via intrinsic nucleotide exchange, thereby transmtting signals to downstream signaling proteins. Conversely, when the GTP bound to KRAS protein is intrinsically hydrolyzed to GDP, it transitions to an "inactivated (GDP-bound form, Turn-off) state", in which downstream signal transduction no longer occurs. In actual cells, the exchange of GDP for GTP is promoted by guanine exchange factors (GEFs), and the hydrolysis of GTP to GDP is accelerated by GTPase activating proteins (GAPs).

Mutations in the KRAS gene have been identified as playing an important role in the proliferation of cancer cells in many malignant tumors, and approximately 89% of patients with KRAS-mutant cancers harbor mutations in which the glycine at codon 12 (KRAS G12) of the KRAS gene is substituted with another amino acid. KRAS G12 mutations are found in 86% of pancreatic ductal adenocarcinomas (PDAC), 41% of colorectal cancers (CRC), 32% of non-small cell lung cancers (NSCLC). Among the KRAS G12 variants, the most frequently reported are G12D (36%), G12V (23%), and G12C (14%). The KRAS G12 mutant proteins inhibit the GAP-mediated hydrolysis of GTP, thereby maintaining the activated (GTP-bound) state, which results in persistent downstream signal transduction and promotes rapid proliferation and survival of cancer cells.

Previous KRAS inhibitors were developed as competitive inhibitors targeting GTP binding to suppress the activated state of KRAS; however, many of these inhibitors failed because the intracellular concentration of GTP is significantly higher than that of the inhibitors. For this reason, the development of targeted therapies directly inhibiting KRAS was long considered unfeasible, and, as an alternative approach to suppress KRAS-mutant cancers, attempts were made to inhibit downstream signaling proteins such as PI3K, MEK, AKT, and mTOR, or to indirectly suppress the activity of KRAS-mutant cancer cells by inhibiting farnesyltransferase of KRAS by suppressing post-translational modifications of the KRAS protein, however, these approaches did not achieve satisfactory clinical effects.

Recently, with the identification of the switch-II pocket which is an allosteric binding pocket of the KRAS G12C mutant protein, sotorasib (LUMAKRAS^{™}), a selective KRAS G12C-targeting small molecule that inhibits KRAS activity by covalent binding to the cysteine residue in the GDP-bound form of KRAS G12C, was developed and received FDA approval for the treatment of non-small cell lung cancer.

However, the development of targeted therapies against the KRAS G12D mutation, which is the most prevalent among KRAS mutations, remains limited. This constitutes a significant unmet medical need in the clinic.

### [Disclosure]

### [Technical Problem]

The present inventors have identified that novel tricyclic compounds according to an embodiment of the present application exhibit inhibitory effects against KRAS G12D activity. Accordingly, an object of the present application is to provide novel tricyclic ring-based compounds represented by Chemical Formula 1, which exhibit excellent inhibitory effects on KRAS G12D activity, as well as their optical isomers, stereoisomers, isotopic variants, hydrates, solvates, or pharmaceutically acceptable salts thereof.

Another object of the present application is to provide a pharmaceutical composition comprising, as an active ingredient, the novel tricyclic ring-based compound, or an optical isomer, stereoisomer, isotopic variant, hydrate, solvate, or a pharmaceutically acceptable salt thereof.

### [Technical Solution]

According to one embodiment of the present application, a compound represented by the following Chemical Formula 1, or an optical isomer, stereoisomer, isotopic variant, hydrate, solvate, or a pharmaceutically acceptable salt thereof, is provided: 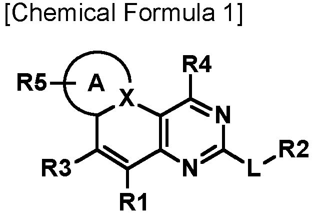 wherein,
A is 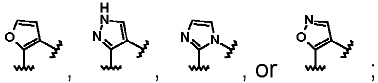
X is C or N;
R₁ is hydrogen, halogen, C₁₋₃ alkyl, or C₁₋₃ alkoxy;
L is a direct bond, O, or NR₆;
R₂ is C₁₋₆ alkyl, C₁₋₆ alkoxy, C₁₋₆ hydroxyalkyl, C₂₋₆ alkoxyalkyl, C₁₋₆ haloalkyl, Rₓ, - Z₁-Rₓ, -Z₁-R_{y}-Rₓ, -Z₁-R_{y}-Z₂-N(R₁₅)₂, -Z₁-R_{y}-Z₂-Rₓ, -N(R₁₅)₂, -Z₁-N(R₁₅)₂, -Z₁-C(O)N(R₁₅)₂, or -Z₁-OR₁₅;
Rₓ and R_{y} are each independently 3 to 10-membered cycloalkyl, 3 to 10-membered heterocyclyl, 6 to 20-membered aryl, or 6 to 20-membered heteroaryl, and may optionally be substituted with one or more of R₇;
R₇ is independently H, halogen, hydroxy, C₁₋₄ alkyl, C₂₋₄ alkenyl, C₂₋₄ alkynyl, C₁₋₄ hydroxyalkyl, C₁₋₄ haloalkyl, C₂₋₄ haloalkenyl, C₂₋₄ haloalkynyl, C₁₋₄ alkoxy, C₁₋₄ alkyl-N(R₁₆)₂, =C-(R₁₆)₂, cyano, C(O)R₁₆, C(=O)OR₁₆, C(=O)N(R₁₆)₂, -NHC(O)-6 to 20-membered aryl, -N(R₁₆)₂, (C₁₋₄ alkoxy)C₁₋₄ alkyl-, oxo, -OR₁₆, -SR₁₆, -(C₁₋₄alkyl)C(O)R₁₆, 3 to 10-membered cycloalkyl, 3 to 10-membered heterocycloalkyl, 6 to 20-membered aryl, 6 to 20-membered heteroaryl, -Z₃-3 to 10-membered cycloalkyl, -Z₃-3 to 10-membered heterocycloalkyl, -Z₃-6 to 20-membered aryl, -Z₃-6 to 20-membered heteroaryl, -Z₃-OC(O)N(R₁₆)₂, or -Z₃-OC(O)-3 to 10-membered heterocyclyl; and R₇ may be substituted with 1 to 3 of cyano, halogen, haloalkyl, amino, -OR₁₇, -SR₁₇, or -N(R₁₇)₂;
Z₁ to Z₃ are each independently C₁₋₄ alkyl; and optinally may be substituted with hydroxy, C₁₋₄ hydroxyalkyl, or 6 to 20-membered heteroaryl;
R₆, R₁₅, R₁₆, R₁₇, R₁₈, R₁₉, R₂₀ and R₂₁ are each independently H or C₁₋₃ alkyl;
R₃ is 3 to 10-membered cycloalkyl, 3 to 10-membered heterocycloalkyl, 6 to 20-membered aryl, or 6 to 20-membered heteroaryl, and may optionally be substituted with one or more of R₈;
R₈ is **H,** halogen, hydroxy, -N(R₁₈)₂, OR₁₈, **SH,** S(C₁₋₃ alkyl), S(=O)(C₁₋₆ alkyl), S(=O)₂(C₁₋₆ alkyl), C(=O)(C₁₋₆ alkyl), C(=O)OH, C(=O)N(R₁₈)₂, C₁₋₄ alkyl, C₂₋₄ alkenyl, C₂₋₄ alkynyl, C₁₋₃ haloalkyl, C₁₋₃ hydroxyalkyl, C₁₋₃ alkoxyalkyl, C₁₋₄ alkyl-N(R₁₈)₂, C₁₋₃ alkoxy, C₁₋₃ haloalkoxy, cyanoalkyl, cyano, oxo, 3 to 10-membered cycloalkyl, 3 to 10-membered heterocycloalkyl, 6 to 20-membered aryl, or 6 to 20-membered heteroaryl;
R₄ is 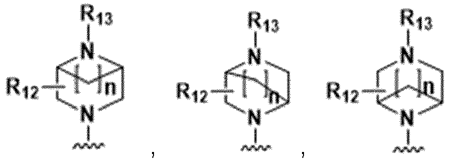 or -NH-R₉-R₁₀;
R₉ is a direct bond or C₁₋₄ alkyl;
R₁₀ is 3 to 10-membered cycloalkyl or 3 to 10-membered heterocycloalkyl, and may optionally be substituted with one or more of R₁₁;
R₁₁ is C₁₋₃ alkyl, hydroxy, N(R₁₉)₂, C₁₋₃ alkyl-N(R₁₉)₂, C₁₋₃ cyanoalkyl, or 3 to 10-membered heterocyclyl;
R₁₂ is H, C₁₋₃ alkyl, OH, -N(R₂₀)₂, -CH₂N(R₂₀)₂, cyano, cyanomethyl, or 3 to 10-membered heterocyclyl;
R₁₃ is H, or -C(=O)R₁₄;
R₁₄ is C₁₋₄ alkyl, C₂₋₄ alkenyl, C₂₋₄ alkynyl, -N(R₆)₂, -OR₆, -SR₆, 3 to 10-membered cycloalkyl, 3 to 10-membered cycloalkenyl, 3 to 10-membered cycloalkynyl, 3 to 10-membered heterocyclyl, 6 to 20-membered aryl, or 6 to 20-membered heteroaryl;
n is an integer of 1 to 4; and
R₅ is H, halogen, C₁₋₆ alkyl, C₁₋₃ halo alkyl, 3 to 6-membered cycloalkyl, 3 to 6-membered heterocycloalkyl, cyano, cyano C₁₋₃ alkyl, hydroxy, C₁₋₃ hydroxyalkyl, C(O)(NR₂₁)₂, 6 to 20-membered aryl, C₁₋₃ alkyl-3 to 6-membered cycloalkyl, C₁₋₃ alkyl-3 to 6-membered heterocycloalkyl, C₁₋₃ alkyl-6 to 20-membered aryl, or C₁₋₃ alkyl-6 to 20-membered heteroaryl.

Specifically, A may be

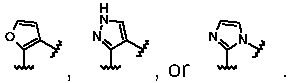

Specifically, R₁ may be hydrogen, halogen, or C₁₋₃ alkyl.

Specifically, L may be a direct bond or O.

Specifically, R₂ may be Rₓ, -Z₁-Rₓ, -Z₁-R_{y}-Rₓ, -Z₁-R_{y}-Z₂-N(R₁₅)₂, or -Z₁-R_{y}-Z₂-Rₓ.

Specifically, Rₓ and R_{y} are each independently 3 to 10-membered cycloalkyl or 3 to 10-membered heterocyclyl, and may optionally be substituted with one or more of R₇.

More specifically, Rₓ may be 3 to 10-membered heterocyclyl, R_{y} is 3 to 10-membered cycloalkyl, Rₓ and the R_{y} may be optionally substituted with one or more of R₇. More specifically, Rₓ may be 3 to 10-membered heterocycle containing 1 to 3 heteroatoms selected from the group consisting of N, O and S, or 3 to 10-membered heterocycle containing 1 to 2 heteroatoms selected from the group consisting of N and O.

In an example, Rₓ and R_{y} are each independently 3 to 5-membered cycloalkyl, azetidinyl, pyrrolidinyl, piperidinyl, morpholinyl, 2-oxa-5-azabicyclo[2.2.1]heptanyl, pyrrolizidinyl, methylenepyrrolizidinyl, tetrahydro-1'*H*,3'*H*-spiro[cyclopropane-1,2'-pyrrolizidinyl], 6-azaspiro[2.5]octanyl, quinolizidinyl, indolinyl, benzimidazolyl, azaspirooctanyl, benztriazolyl, thioxanthinyl, carbazolyl, carbolinyl, or acridinyl, and may optionally be substituted with one or more of R₇.

In a specific example, Rₓ is azetidinyl, pyrrolidinyl, piperidinyl, morpholinyl, 2-oxa-5-azabicyclo[2.2.1]heptanyl, pyrrolizidinyl, methylenepyrrolizidinyl, tetrahydro-1*'H,*3*'H-*spiro[cyclopropane-1,2'-pyrrolizidinyl], or 6-azaspiro[2.5]octanyl, and may optionally be substituted with one or more of R₇.

In a specific example, R_{y} is 3 to 5-membered cycloalkyl (e.g., cyclopropyl or cyclobutyl), and may optionally be substituted with one or more of R₇.

In an example, in Chemical Formula 1, R₂ is Rₓ, -Z₁-Rₓ, -Z₁-R_{y}-Rₓ, -Z₁-R_{y}-Z₂-N(R₁₅)₂, or -Z₁-R_{y}-Z₂-Rₓ; Rₓ is 3 to 10-membered heterocyclyl; R_{y} is 3 to 10-membered cycloalkyl; and Rₓ and R_{y} may be optionally substituted with one or more of R₇.

In a specific example, in Chemical Formula 1, R₂ is Rₓ, -Z₁-Rₓ, -Z₁-R_{y}-Rₓ, -Z₁-R_{y}-Z₂-N(R₁₅)₂, or -Z₁-R_{y}-Z₂-Rₓ; Rₓ is azetidinyl, pyrrolidinyl, piperidinyl, morpholinyl, 2-oxa-5-azabicyclo[2.2.1]heptanyl, pyrrolizidinyl, methylenepyrrolizidinyl, tetrahydro-1'*H*,3'*H-*spiro[cyclopropane-1,2'-pyrrolizidinyl], or 6-azaspiro[2.5]octanyl; Ry is 3 to 5-membered cycloalkyl; and Rₓ and R_{y} may be optionally substituted with one or more of R₇.

In a specific example, in Chemical Formula 1, R₂ is azetidinyl, Z1-pyrrolizidinyl, Z1-tetrahydro-1'*H*,3'*H*-spiro[cyclopropane-1,2'-pyrrolizidinyl], or Z₁-R_{y}-Z₂-Rₓ; R_{y} is 3 to 5-membered cycloalkyl (e.g., cycloproply or cyclobuty); and Rₓ is 3 to 10-membered heterocyclyl (e.g., pyrrolidinyl, piperidinyl, morpholinyl, 2-oxa-5-azabicyclo[2.2.1]heptanyl, or 6-azaspiro[2.5]octanyl).

Specifically, R₇ is independently H, halogen, hydroxy, C₁₋₄ alkyl, C₂₋₄ alkenyl, C₂₋₄ alkynyl, C₂₋₄ hydroxyalkyl, C₁₋₄ haloalkyl, C₂₋₄ haloalkenyl, C₁₋₄ alkoxy, C₁₋₄ alkyl-N(R₁₆)₂, =C-(R₁₆)₂, C(O)R₁₆, C(=O)OR₁₆, C(=O)N(R₁₆)₂, -NHC(O)aryl, -N(R₁₆)₂, (C₁₋₄ alkoxy)C₁₋₄ alkyl-, oxo, -OR₁₆, -SR₁₆, -(C₁₋₄ alkyl)C(O)R₁₆, 3 to 10-membered cycloalkyl, 3 to 10-membered heterocycloalkyl, 6 to 20-membered aryl, 6 to 20-membered heteroaryl, -Z₃-3 to 10-membered cycloalkyl, -Z₃-3 to 10-membered heterocycloalkyl, -Z₃-6 to 20-membered aryl, -Z₃-6 to 20-membered heteroaryl, -Z₃-OC(O)N(R₁₆)₂, or -Z₃-OC(O)-3 to 10-membered heterocyclyl; and may be substituted with 1 to 3 of cyano, halogen, haloalkyl (e.g., C₁₋₄haloalkyl), amino, -OR₁₇, -SR₁₇, or -N(R₁₇)₂.

In an example, R₇ is independently H, halogen, C₁₋₄ alkyl, C₂₋₄ alkenyl, C₂₋₄ alkynyl, C₁₋₄ haloalkyl, C₂₋₄ haloalkenyl, C₂₋₄ haloalkynyl, C₁₋₄ alkoxy, =C-(R₁₆)₂, -N(R₁₆)₂, or (C₁₋₄ alkoxy)C₁₋₄ alkyl-, and may be substituted with 1 to 3 of halogen.

In an example, R₇ is independently H, halogen, C₁₋₃ alkyl, C₂₋₄ alkenyl, C₂₋₄ alkynyl, C₁₋₃ haloalkyl, C₂₋₄ haloalkenyl, C₂₋₄ haloalkynyl, C₁₋₃ alkoxy, =C-(R₁₆)₂, -N(R₁₆)₂, or (C₁₋₃ alkoxy)C₁₋₃ alkyl-, and may be substituted with 1 to 3 of halogen.

In a specific example, R₇ is H, halogen, C₁₋₄ alkyl, =CH₂, =CF₂, =CFH, -N(R₁₆)₂, C₁₋₄ alkoxy, or (C₁₋₄ alkoxy)C₁₋₄ alkyl-.

Specifically, R₃ is 6 to 20-membered aryl, or 6 to 20-membered heteroaryl, and may optionally be substituted with one or more of R₈.

Specifically, R₃ is phenyl, biphenyl, naphthyl, toluyl, naphthalenyl, pyridinyl, anthracenyl, indenyl, indanyl, quinolinyl, isoquinolinyl, benzimidazolyl, benzothiazolyl, benzothiophenyl, benzofuranyl, or indazolyl, and may optionally be substituted with one or more of R₈.

More specifically, R₃ is phenyl, naphthyl, naphthalenyl, pyridinyl, quinolinyl, isoquinolinyl, benzothiazolyl, benzothiophenyl, benzofuranyl, or indazolyl, and may optionally be substituted with one or more of R₈.

Specifically, R₈ may be H, halogen, hydroxy, -N(R₁₈)₂, OR₆, SH, S(C₁₋₃ alkyl), S(=O)(C₁₋₆ alkyl), S(=O)₂(C₁₋₆ alkyl), C(=O)(C₁₋₆ alkyl), C(=O)OH, C(=O)N(R₁₈)₂, C₁₋₄ alkyl, C₂₋₄ alkenyl, C₂₋₄ alkynyl, C₁₋₃ haloalkyl, C₁₋₃ hydroxyalkyl, C₁₋₃ alkoxyalkyl, C₁₋₄ alkyl-N(R₁₈)₂, cyano, oxo, or 3 to 10-membered cycloalkyl.

More specifically, R₈ may be H, halogen, hydroxy, -N(R₁₈)₂, C₁₋₄ alkyl, C₂₋₄ alkenyl, C₂₋₄ alkynyl, C₁₋₃ haloalkyl, C₁₋₃ hydroxyalkyl, or cyano.

Specifically, R₁₀ is 3 to 10-membered cycloalkyl, and may optionally be substituted with one or more R₁₁.

Specifically, R₁₁ is C₁₋₃ alkyl, hydroxy, N(R₁₉)₂, C₁₋₃ alkyl-N(R₁₉)₂, or cyano C₁₋₃ alkyl.

In an example, in Chemical Formula 1, R₉ may be C₁₋₃ alkyl, R₁₀ may be 3 to 5-membered cycloalkyl, and R₁₁ may be N(R₁₉)₂.

Specifically, R₁₂ may be H.

Specifically, R₁₄ may be 3 to 10-membered heterocyclyl, 3 to 10-membered heterocyclyl containing 1 to 2 heteroatoms selected from the group consisting of N, O and S, 3 to 5-membered heterocyclyl containing 1 to 2 heteroatoms selected from the group consisting of N, O and S, for example oxirane.

Specifically, R₅ may be H or C₁₋₃ alkyl.

For example, in Chemical Formula 1, L-R₂ may be selected from the following structures. When having such structures, not only does it exhibit inhibitory activity against KRAS G12D, but it is also suitable for achieving the various aforementioned objectives of the present application.

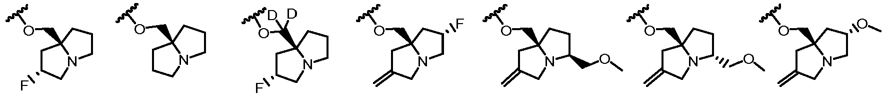

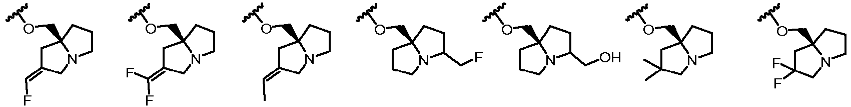

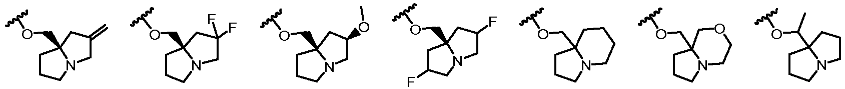

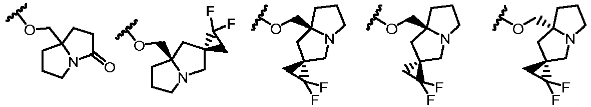

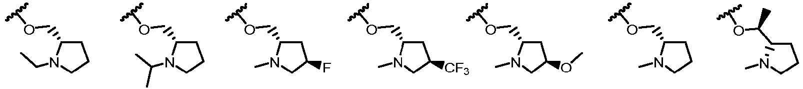

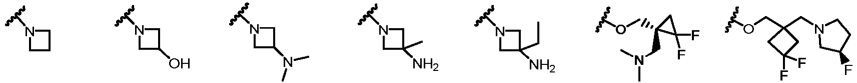

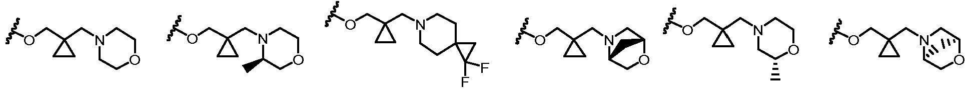

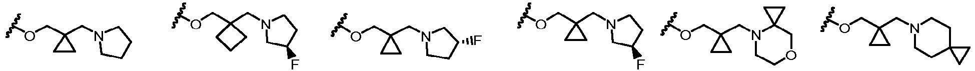

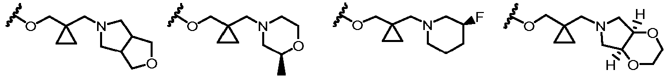

For example, in Chemical Formula 1, R₃ may be selected from the following structures. When having such structures, not only does it exhibit inhibitory activity against KRAS G12D, but it is also suitable for achieving the various aforementioned objectives of the present application.

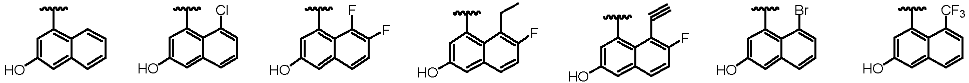

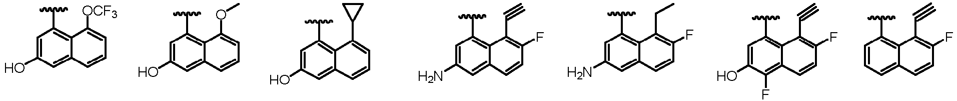

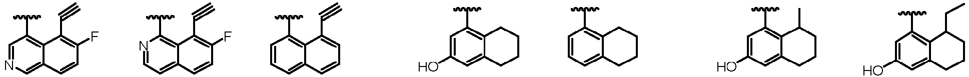

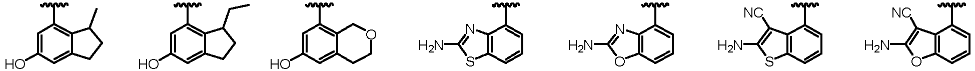

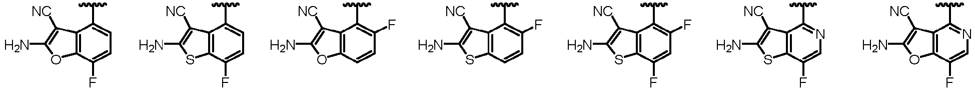

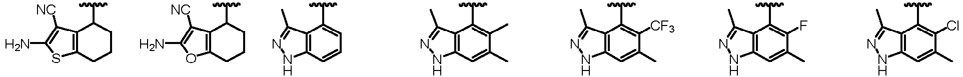

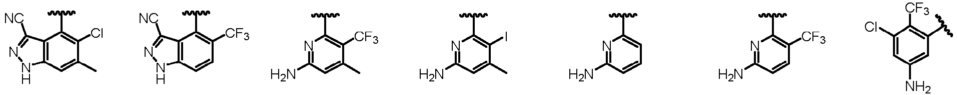

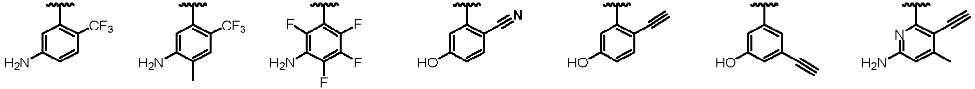

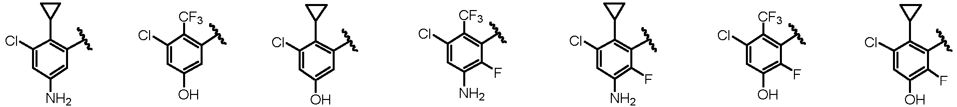

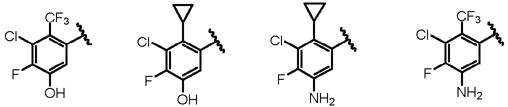

For example, in Chemical Formula 1, R₄ may be selected from the following structures. When having such structures, not only does it exhibit inhibitory activity against KRAS G12D, but it is also suitable for achieving the various aforementioned objectives of the present application.

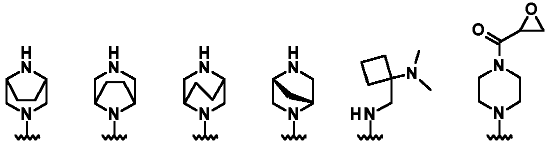

The present application provides the aforementioned compound, or an optical isomer, stereoisomer, isotopic variant, hydrate, solvate, or a pharmaceutically acceptable salt thereof.

Examples of compounds of Chemical Formula 1 according to the present application are compounds prepared in EXAMPLES 1 to 120 below. Each EXAMPLE number corresponds to the compound number. For example, the number of the final compound prepared in EXAMPLE 90 is Compound 90.

For example, representative examples of compounds of Chemical Formula 1 according to an embodiment of the present application comprise those listed in Table 1 below, but are not limited thereto.

**[Table 1]**

| EXAMPLE | Compound name |
|---|---|
| (1) | 4-(1-((1*R*,5*S*)-3,8-diazabicyclo[3.2.1]octan-3-yl)-3-(((2*R*,7a*S*)-2-fluorotetrahydro-1*H*-pyrrolizin-7a(5*H*)-yl)methoxy)imidazo[1',2':1,6]pyrido[3,2-*d*]pyrimidin-6-yl)-5-ethyl-6-fluoronaphthalen-2-ol |
| (2) | 4-(1-((1*R*,5*S*)-3,8-diazabicyclo[3.2.1]octan-3-yl)-3-(((2*R*,7a*S*)-2-fluorotetrahydro-1*H*-pyrrolizin-7a(5*H*)-yl)methoxy)imidazo[1',2':1,6]pyrido[3,2-*d*]pyrimidin-6-yl)-5-ethynyl-6-fluoronaphthalen-2-ol |
| (3) | 4-(1-((1*R*,5*S*)-3,8-diazabicyclo[3.2.1]octan-3-yl)-3-(((2*R*,7a*S*)-2-fluorotetrahydro-1*H*-pyrrolizin-7a(5*H*)-yl)methoxy)imidazo[1',2':1,6]pyrido[3,2-d]pyrimidin-6-yl)-5,6-difluoronaphthalen-2-ol |
| (4) | 4-(1-(((1-(dimethylamino)cyclobutyl)methyl)amino)-3-(((2*R*,7a*S*)-2-fluorotetrahydro-1*H*-pyrrolizin-7a(5*H*)-yl)methoxy)imidazo[1',2':1,6]pyrido[3,2-*d*]pyrimidin-6-yl)-5,6-difluoronaphthalen-2-ol. |
| (5) | 4-(1-(((1-(dimethylamino)cyclobutyl)methyl)amino)-3-(((2*R*,7a*S*)-2-fluorotetrahydro-1*H*-pyrrolizin-7a(5*H*)-yl)methoxy)imidazo[1',2':1,6]pyrido[3,2-*d*]pyrimidin-6-yl)-5-ethyl-6-fluoronaphthalen-2-ol. |
| (6) | 4-(1-(((1-(dimethylamino)cyclobutyl)methyl)amino)-3-(((2*R*,7a*S*)-2-fluorotetrahydro-1*H*-pyrrolizin-7a(5*H*)-yl)methoxy)imidazo[1',2':1,6]pyrido[3,2-*d*]pyrimidin-6-yl)-5-ethynyl-6-fluoronaphthalen-2-ol. |
| (7) | 4-(1-((1*R*,5*S*)-3,8-diazabicyclo[3.2.1]octan-3-yl)-3-((1-(morpholinomethyl)cyclopropyl)methoxy)imidazo[1',2':1,6]pyrido[3,2-*d*]pyrimidin-6-yl)-5-ethynyl-6-fluoronaphthalen-2-ol. |
| (8) | 4-(1-((1*R*,5*S*)-3,8-diazabicyclo[3.2.1]octan-3-yl)-3-((1-(((*R*)-3-methylmorpholino)methyl)cyclopropyl)methoxy)imidazo[1',2':1,6]pyrido[3 ,2-*d*]pyrimidin-6-yl)-5-ethynyl-6-fluoronaphthalen-2-ol. |
| (9) | 4-(1-((1*R*,5*S*)-3,8-diazabicyclo[3.2.1]octan-3-yl)-3-((1-((1,1-difluoro-6-azaspiro[2.5]octan-6-yl)methyl)cyclopropyl)methoxy)imidazo[1',2':1,6]pyrido[3,2-d]pyrimidin-6-yl)-5-ethynyl-6-fluoronaphthalen-2-ol. |
| (10) | 4-(3-((1-(((1*R*,4*R*)-2-oxa-5-azabicyclo[2.2.1]heptan-5-yl)methyl)cycloprppyl)methoxy)1-((1*R*,5*S*)-3,8-diazabicyclo[3.2.1]octan-3-yl)imidazo[1',2':1,6]pyrido[3,2-*d*]pyrimidin-6-yl)-5-ethynyl-6-fluoronaphthalen-2-ol. |
| (11) | 4-(1-((1*R*,5*S*)-3,8-diazabicyclo[3.2.1]octan-3-yl)-3-((1-(((*S*)-3-methylmorpholino)methyl)cyclopropyl)methoxy)imidazo[1',2':1,6]pyrido[3 ,2-*d*]pyrimidin-6-yl)-5-ethynyl-6-fluoronaphthalen-2-ol. |
| (12) | 4-(3-((1-(((1*S*,4*S*)-2-oxa-5-azabicyclo[2.2.1]heptan-5-yl)methyl)cyclopropyl)methoxy)-1-((1*R*,5*S*)-3,8-diazabicyclo[3.2.1]octan-3-yl)imidazo[1',2':1,6]pyrido[3,2-*d*]pyrimidin-6-yl)5-ethynyl-6-fluoronaphthalen-2-ol. |
| (13) | 3-(1-((1*R*,5*S*)-3,8-diazabicyclo[3.2.1]octan-3-yl)-3-(((2*R*,7a*S*)-2-fluorotetrahydro-1*H*-pyrrolizin-7a(5*H*)-yl)methoxy)imidazo[1',2':1,6]pyrido[3,2-d]pyrimidin-6-yl)-2,4,5,6-tetrafluoroaniline |
| (14) | 4-(1-((1*R*,5*S*)-3,8-diazabicyclo[3.2.1]octan-8-yl)-3-(((2R,7aS)-2-fluorotetrahydro-1*H*-pyrrolizin-7a(5*H*)-yl)methoxy)imidazo[1',2':1,6]pyrido[3,2-*d*]pyrimidin-6-yl)-5-ethynyl-6-fluoronaphthalen-2-amine. |
| (15) | 4-(1-((1*R*,5*S*)-3,8-diazabicyclo[3.2.1]octan-8-yl)-3-(((2*R*,7a*S*)-2-fluorotetrahydro-1*H*-pyrrolizin-7a(5*H*)-yl)methoxy)imidazo[1',2':1,6]pyrido[3,2-d]pyrimidin-6-yl)-7-fluorobenzo[*d*]thiazol-2-amine. |
| (16) | 4-(1-((1*R*,5*S*)-3,8-diazabicyclo[3.2.1]octan-8-yl)-3-((1-(((*R*)-3-fluoropyrrolidin-1-yl)methyl)cyclopropyl)methoxy)imidazo[1',2':1,6]pyrido[3,2-*d*]pyrimidin-6-yl)-5-ethynyl-6-fluoronaphthalen-2-ol. |
| (17) | 4-(1-((1*R*,5*S*)-3,8-diazabicyclo[3.2.1]octan-8-yl)-3-((1-(pyrrolidin-1-ylmethyl)cyclopropyl)methoxy)imidazo[1',2':1,6]pyrido[3,2-*d*]pyrimidin-6-yl)-5-ethynyl-6-fluoronaphthalen-2-ol. |
| (18) | 1-((1*R*,5*S*)-3,8-diazabicyclo[3.2.1]octan-8-yl)-3-(((2*R*,7a*S*)-2-fluorotetrahydro-1*H*-pyrrolizin-7a(5*H*)-yl)methoxy)-6-(6-methyl-5-(trifluoromethyl)-1*H*-indazol-4-yl)imidazo[1',2':1,6]pyrido[3,2-*d*]pyrimidine. |
| (19) | 4-(1-((1*R*,5*S*)-3,8-diazabicyclo[3.2.1]octan-8-yl)-3-((1-(((*S*)-3-fluoropiperidin-1-yl)methyl)cyclopropyl)methoxy)imidazo[1',2':1,6]pyrido[3,2-d]pyrimidin-6-yl)-5-ethynyl-6-fluoronaphthalen-2-ol. |
| (20) | 6-(1-((1*R*,5*S*)-3,8-diazabicyclo[3.2.1]octan-8-yl)-3-(((2*R*,7a*S*)-2-fluorotetrahydro-1*H*-pyrrolizin-7a(5*H*)-yl)methoxy)imidazo[1',2':1,6]pyrido[3,2-*d*]pyrimidin-6-yl)-4-methyl-5-(trifluoromethyl)pyridin-2-amine. |
| (21) | 4-(1-((1*R*,5*S*)-3,8-diazabicyclo[3.2.1]octan-8-yl)-3-(((*R*)-2-methylenetetrahydro-1*H*-pyrrolizin-7a(5*H*)-yl)methoxy)imidazo[1',2':1,6]pyrido[3,2-*d*]pyrimidin-6-yl)-5-ethynyl-6-fluoronaphthalen-2-ol. |
| (22) | 1-((1*R*,5*S*)-3,8-diazabicyclo[3.2.1]octan-8-yl)-6-(8-ethynyl-7-fluoronaphthalen-1-yl)-3-(((*S,Z*)-2-(fluoromethylene)tetrahydro-1*H-*pyrrolizin-7a(5*H*)-yl)methoxy)imidazo[1',2':1,6]pyrido[3,2-*d*]pyrimidine. |
| (23) | 4-(1-((1*R*,5*S*)-3,8-diazabicyclo[3.2.1]octan-8-yl)-3-(((*S*,*Z*)-2-(fluoromethylene)tetrahydro-1*H*-pyrrolizin-7a(5*H*)-yl)methoxy)imidazo[1',2':1,6]pyrido[3,2-*d*]pyrimidin-6-yl)-5-ethynyl-6-fluoronaphthalen-2-ol. |
| (24) | 4-(1-((1*R*,5*S*)-3,8-diazabicyclo[3.2.1]octan-3-yl)-3-(((2*R*,7a*S*)-2-fluorotetrahydro-1*H*-pyrrolizin-7a(5*H*)-yl)methoxy)imidazo[1',2':1,6]pyrido[3,2-*d*]pyrimidin-6-yl)-5-ethynyl-6-fluoronaphthalen-2-amine. |
| (25) | 4-(1-((1*R*,5*S*)-3,8-diazabicyclo[3.2.1]octan-3-yl)-3-(((2*R*,7a*S*)-2-fluorotetrahydro-1*H*-pyrrolizin-7a(5*H*)-yl)methoxy)imidazo[1',2':1,6]pyrido[3,2-*d*]pyrimidin-6-yl)-7-fluorobenzo[*d*]thiazol-2-amine. |
| (26) | 1-((1*R*,5*S*)-3,8-diazabicyclo[3.2.1]octan-3-yl)-3-(((2*R*,7a*S*)-2-fluorotetrahydro-1*H*-pyrrolizin-7a(5*H*)-yl)methoxy)-6-(6-methyl-5-(trifluoromethyl)-1*H*-indazol-4-yl)imidazo[1',2':1,6]pyrido[3,2-*d*]pyrimidine. |
| (27) | 1-((1*R*,5*S*)-3,8-diazabicyclo[3.2.1]octan-3-yl)-6-(8-ethynyl-7-fluoronaphthalen-1-yl)-3-(((*S*,*Z*)-2-(fluoromethylene)tetrahydro-1*H-*pyrrolizin-7a(5*H*)-yl)methoxy)imidazo[1',2':1,6]pyrido[3,2-*d*]pyrimidine. |
| (28) | 1-((1*R*,5*S*)-3,8-diazabicyclo[3.2.1]octan-3-yl)-6-(8-ethynyl-7-fluoronaphthalen-1-yl)-3-(((2*R*,7a*S*)-2-fluorotetrahydro-1*H*-pyrrolizin-7a(5*H*)-yl)methoxy)imidazo[1',2':1,6]pyrido[3,2-*d*]pyrimidine. |
| (29) | 4-(1-((1*R*,5*S*)-3,8-diazabicyclo[3.2.1]octan-3-yl)-3-(((*S*,*Z*)-2-(fluoromethylene)tetrahydro-1*H*-pyrrolizin-7a(5*H*)-yl)methoxy)imidazo[1',2':1,6]pyrido[3,2-*d*]pyrimidin-6-yl)-5-ethynyl-6-fluoronaphthalen-2-ol. |
| (30) | 4-(1-((1*R*,5*S*)-3,8-diazabicyclo[3.2.1]octan-3-yl)-3-((1-(((*R*)-3-fluoropyrrolidin-1-yl)methyl)cyclopropyl)methoxy)imidazo[1',2':1,6]pyrido[3,2-*d*]pyrimidin-6-yl)-5-ethynyl-6-fluoronaphthalen-2-ol. |
| (31) | 4-(1-((1*R*,5*S*)-3,8-diazabicyclo[3.2.1]octan-3-yl)-3-((1-(((*S*)-3-fluoropiperidin-1-yl)methyl)cyclopropyl)methoxy)imidazo[1',2':1,6]pyrido[3,2-*d*]pyrimidin-6-yl)-5-ethynyl-6-fluoronaphthalen-2-ol. |
| (32) | 4-(1-((1*R*,5*S*)-3,8-diazabicyclo[3.2.1]octan-3-yl)-3-(((2*S*,7a*R*)-2-fluoro-6-methylenetetrahydro-1*H*-pyrrolizin-7a(5*H*)-yl)methoxy)imidazo[1',2':1,6]pyrido[3,2-*d*]pyrimidin-6-yl)-5-ethynyl-6-fluoronaphthalen-2-ol. |
| (33) | 4-(1-((1*R*,5*S*)-3,8-diazabicyclo[3.2.1]octan-3-yl)-3-(((*R*)-2-methylenetetrahydro-1*H*-pyrrolizin-7a(5*H*)-yl)methoxy)imidazo[1',2':1,6]pyrido[3,2-*d*]pyrimidin-6-yl)-5-ethynyl-6-fluoronaphthalen-2-ol. |
| (34) | 6-(1-((1*R*,5*S*)-3,8-diazabicyclo[3.2.1]octan-3-yl)-3-(((2*R*,7a*S*)-2-fluorotetrahydro-1*H*-pyrrolizin-7a(5*H*)-yl)methoxy)imidazo[1',2':1,6]pyrido[3,2-*d*]pyrimidin-6-yl)-4-methyl-5-(trifluoromethyl)pyridin-2-amine. |
| (35) | 4-(1-((1*R*,5*S*)-3,8-diazabicyclo[3.2.1]octan-3-yl)-3-((1-(pyrrolidin-1-ylmethyl)cyclopropyl)methoxy)imidazo[1',2':1,6]pyrido[3,2-*d*]pyrimidin-6-yl)-5-ethynyl-6-fluoronaphthalen-2-ol. |
| (36) | 4-(1-((1*R*,5*S*)-3,8-diazabicyclo[3.2.1]octan-3-yl)-3-(((*S*,*Z*)-2-(fluoromethylene)tetrahydro-1*H*-pyrrolizin-7a(5*H*)-yl)methoxy)imidazo[1',2':1,6]pyrido[3,2-*d*]pyrimidin-6-yl)-5-ethynyl-6-fluoronaphthalen-2-amine. |
| (37) | 4-(1-((1*R*,5*S*)-3,8-diazabicyclo[3.2.1]octan-3-yl)-3-(((*S*)-2-(difluoromethylene)tetrahydro-1*H*-pyrrolizin-7a(5*H*)-yl)methoxy)imidazo[1',2':1,6]pyrido[3,2-*d*]pyrimidin-6-yl)-5-ethynyl-6-fluoronaphthalen-2-ol. |
| (38) | 4-(1-((1*R*,5*S*)-3,8-diazabicyclo[3.2.1]octan-8-yl)-3-(((*S*,*Z*)-2-(fluoromethylene)tetrahydro-1*H*-pyrrolizin-7a(5*H*)-yl)methoxy)imidazo[1',2':1,6]pyrido[3,2-*d*]pyrimidin-6-yl)-5-ethynyl-6-fluoronaphthalen-2-amine. |
| (39) | 4-(1-((1*R*,5*S*)-3,8-diazabicyclo[3.2.1]octan-8-yl)-3-(((2*S*,7a*R*)-2-fluoro-6-methylenetetrahydro-1*H*-pyrrolizin-7a(5*H*)-yl)methoxy)imidazo[1',2':1,6]pyrido[3,2-*d*]pyrimidin-6-yl)-5-ethynyl-6-fluoronaphthalen-2-ol. |
| (40) | 4-(1-((1*R*,5*S*)-3,8-diazabicyclo[3.2.1]octan-3-yl)-3-((1-(((*R*)-3-fluoropyrrolidin-1-yl)methyl)cyclobutyl)methoxy)imidazo[1',2':1,6]pyrido[3,2*-d*]pyrimidin-6-yl)-5-ethynyl-6-fluoronaphthalen-2-ol. |
| (41) | 4-(1-((1*R*,5*S*)-3,8-diazabicyclo[3.2.1]octan-3-yl)-3-(((2*R*,7a*S*)-2-fluorotetrahydro-1*H*-pyrrolizin-7a(5*H*)-yl)methoxy)-8-methylfuro[3,2-*f*]quinazolin-6-yl)naphthalen-2-ol. |
| (42) | 4-(1-((1*R*,5*S*)-3,8-diazabicyclo[3.2.1]octan-3-yl)-3-(((2*R*,7a*S*)-2-fluorotetrahydro-1*H*-pyrrolizin-7a(5*H*)-yl)methoxy)-8-methylfuro[3,2-*f*]quinazolin-6-yl)-5-ethynyl-6-fluoronaphthalen-2-ol. |
| (43) | 4-(1-((1*R*,5*S*)-3,8-diazabicyclo[3.2.1]octan-3-yl)-5-fluoro-3-(((2*R*,7a*S*)-2-fluorotetrahydro-1H-pyrrolizin-7a(5H)-yl)methoxy)-8-methylfuro[3,2-*f*]quinazolin-6-yl)naphthalen-2-ol. |
| (44) | 4-(1-((1*R*,5*S*)-3,8-diazabicyclo[3.2.1]octan-3-yl)-5-fluoro-3-(((2*R*,7a*S*)-2-fluorotetrahydro-1*H*-pyrrolizin-7a(5*H*)-yl)methoxy)-8-methylfuro[3,2-*f*]quinazolin-6-yl)-5-ethynyl-6-fluoronaphthalen-2-ol. |
| (45) | 4-(1-((1*R*,5*S*)-3,8-diazabicyclo[3.2.1]octan-3-yl)-5-fluoro-3-(((2*R*,7a*S*)-2-fluorotetrahydro-1*H*-pyrrolizin-7a(5*H*)-yl)methoxy)-8-methylfuro[3,2-*f*]quinazolin-6-yl)-5-ethyl-6-fluoronaphthalen-2-ol. |
| (46) | 4-(1-((1*R*,5*S*)-3,8-diazabicyclo[3.2.1]octan-3-yl)-5-fluoro-3-(((2*R*,7a*S*)-2-fluorotetrahydro-1*H*-pyrrolizin-7a(5*H*)-yl)methoxy)-8-methylfuro[3,2-*f*]quinazolin-6-yl)-5,6-difluoronaphthalen-2-ol. |
| (47) | 4-(1-(((1-(dimethylamino)cyclobutyl)methyl)amino)-5-fluoro-3-(((2*R*,7a*S*) 2-fluorotetrahydro-1*H*-pyrrolizin-7a(5*H*)-yl)methoxy)-8-methylfuro[3,2-*f*]quinazolin-6-yl)naphthalen-2-ol. |
| (48) | 4-(1-(((1-(dimethylamino)cyclobutyl)methyl)amino)-5-fluoro-3-(((2*R*,7a*S*) 2-fluorotetrahydro-1*H*-pyrrolizin-7a(5*H*)-yl)methoxy)-8-methylfuro[3,2-*f*]quinazolin-6-yl)-5-ethynyl-6-fluoronaphthalen-2-ol. |
| (49) | 3-(1-((1*R*,5*S*)-3,8-diazabicyclo[3.2.1]octan-3-yl)-5-fluoro-3-(((2*R*,7a*S*)-2-fluorotetrahydro-1*H*-pyrrolizin-7a(5*H*)-yl)methoxy)-8-methylfuro[3,2-*f*]quinazolin-6-yl)-4-(trifluoromethyl)aniline. |
| (50) | 6-(1-((1*R*,5*S*)-3,8-diazabicyclo[3.2.1]octan-3-yl)-5-fluoro-3-(((2*R*,7a*S*)-2-fluorotetrahydro-1*H*-pyrrolizin-7a(5*H*)-yl)methoxy)-8-methylfuro[3,2-*f*]quinazolin-6-yl)-5-(trifluoromethyl)pyridin-2-amine. |
| (51) | 1-((1*R*,5*S*)-3,8-diazabicyclo[3.2.1]octan-3-yl)-6-(5-chloro-6-methyl-1*H-*indazol-4-yl)-5-fluoro-3-(((2*R*,7a*S*)-2-fluorotetrahydro-1*H*-pyrrolizin-7a(5*H*)-yl)methoxy)-8-methylfuro[3,2-*f*]quinazoline. |
| (52) | 6-(1-((1*R*,5*S*)-3,8-diazabicyclo[3.2.1]octan-3-yl)-5-fluoro-3-(((2*R*,7a*S*)-2-fluorotetrahydro-1*H*-pyrrolizin-7a(5*H*)-yl)methoxy)-8-methylfuro[3,2-*f*]quinazolin-6-yl)-4-methylpyridin-2-amine. |
| (53) | 6-(1-((1*R*,5*S*)-3,8-diazabicyclo[3.2.1]octan-3-yl)-5-fluoro-3-(((2*R*,7a*S*)-2-fluorotetrahydro-1*H*-pyrrolizin-7a(5*H*)-yl)methoxy)-8-methylfuro[3,2-*f*]quinazolin-6-yl)-5-iodo-4-methylpyridin-2-amine. |
| (54) | 4-(1-((1*R*,5*S*)-3,8-diazabicyclo[3.21]octan-3-yl)-5-fluoro-3-(((2*R*,7a*S*)-2-fluorotetrahydro-1*H*-pyrrolizin-7a(5*H*)-yl)methoxy)-8-methylfuro[3,2-*f*]quinazolin-6-yl)benzo[*d*]thiazol-2-amine. |
| (55) | 4-(1-((1*R*,5*S*)-3,8-diazabicyclo[3.2.1]octan-3-yl)-5-fluoro-3-(((2*R*,7a*S*)-2-fluorotetrahydro-1*H*-pyrrolizin-7a(5*H*)-yl)methoxy)-8-methylfuro[3,2-*f*]quinazolin-6-yl)-7-fluorobenzo[*d*]thiazol-2-amine. |
| (56) | 3-(1-((1*R*,5*S*)-3,8-diazabicyclo[3.2.1]octan-3-yl)-5-fluoro-3-(((2*R*,7a*S*)-2-fluorotetrahydro-1*H*-pyrrolizin-7a(5*H*)-yl)methoxy)-8-methylfuro[3,2-*f*]quinazolin-6-yl)-5-methyl-4-(trifluoromethyl)aniline. |
| (57) | 4-(1-((1*R*,5*S*)-3,8-diazabicyclo[3.2.1]octan-3-yl)-5-fluoro-3-(((2*R*,7a*S*)-2-fluorotetrahydro-1*H*-pyrrolizin-7a(5*H*)-yl)methoxy)-8-methylfuro[3,2-*f*]quinazolin-6-yl)-5-ethynyl-6-fluoronaphthalen-2-amine. |
| (58) | 4-(1-((1*R*,5*S*)-3,8-diazabicyclo[3.2.1]octan-3-yl)-3-((1-((1,1-difluoro-6-azaspiro[2.5]octan-6-yl)methyl)cyclopropyl)methoxy)-5-fluoro-8-methylfuro[3,2-*f*]quinazolin-6-yl)-5-ethynyl-6-fluoronaphthalen-2-amine. |
| (59) | 4-(1-((1*R*,5*S*)-3,8-diazabicyclo[3.2.1]octan-3-yl)-3-((1-((1,1-difluoro-6-azaspiro[2.5]octan-6-yl)methyl)cyclopropyl)methoxy)-5-fluoro-8-methylfuro[3,2-*f*]quinazolin-6-yl)-5-ethynyl-6-fluoronaphthalen-2-ol. |
| (60) | 6-(1-((1*R*,5*S*)-3,8-diazabicyclo[3.2.1]octan-3-yl)-5-fluoro-3-(((2*R*,7a*S*)-2-fluorotetrahydro-1*H*-pyrrolizin-7a(5*H*)-yl)methoxy)-8-methylfuro[3,2-*f*]quinazolin-6-yl)-4-methyl-5-(trifluoromethyl)pyridin-2-amine. |
| (61) | 4-(1-((1*R*,5*S*)-3,8-diazabicyclo[3.2.1]octan-3-yl)-5-fluoro-8-methyl-3-((*S*)-1-((*S*)-1-methylpyrrolidin-2-yl)ethoxy)furo[3,2-*f*]quinazolin-6-yl)-5-ethynyl-6-fluoronaphthalen-2-ol. |
| (62) | 4-(1-((1*R*,5*S*)-3,8-diazabicyclo[3.2.1]octan-3-yl)-5-fluoro-3-(((2*R*,7a*S*)-2-fluorotetrahydro-1*H*-pyrrolizin-7a(5*H*)-yl)methoxy)-8-methylfuro[3,2-*f*]quinazolin-6-yl)-2-amino-7-fluorobenzo[*b*]thiophene-3-carbonitrile. |
| (63) | 4-(1-((1*R*,5*S*)-3,8-diazabicyclo[3.2.1]octan-3-yl)-5-fluoro-3-(((*S*,*Z*)-2-(fluoromethylene)tetrahydro-1*H*-pyrrolizin-7a(5*H*)-yl)methoxy)-8-methylfuro[3,2-*f*]quinazolin-6-yl)-2-amino-7-fluorobenzo[*b*]thiophene-3-carbonitrile. |
| (64) | 4-(1-((1*R*,5*S*)-3,8-diazabicyclo[3.2.1]octan-3-yl)-5-fluoro-3-(((*S*,*Z*)-2-(fluoromethylene)tetrahydro-1*H*-pyrrolizin-7a(5*H*)-yl)methoxy)-8-methylfuro[3,2-*f*]quinazolin-6-yl)-5-ethynyl-6-fluoronaphthalen-2-ol. |
| (65) | 4-(1-((1*R*,5*S*)-3,8-diazabicyclo[3.2.1]octan-3-yl)-5-fluoro-3-(((2*S*,7a*R*)-2-fluoro-6-methylenetetrahydro-1*H*-pyrrolizin-7a(5*H*)-yl)methoxy)-8-methylfuro[3,2-*f*]quinazolin-6-yl)-5-ethynyl-6-fluoronaphthalen-2-ol. |
| (66) | 4-(1-((1*R*,5*S*)-3,8-diazabicyclo[3.2.1]octan-3-yl)-3-(((*S*)-2-(difluoromethylene)tetrahydro-1*H*-pyrrolizin-7a(5*H*)-yl)methoxy)-5-fluoro-8-methylfuro[3,2-*f*]quinazolin-6-yl)-5-ethynyl-6-fluoronaphthalen-2-ol. |
| (67) | 4-(1-((1*R*,5*S*)-3,8-diazabicyclo[3.2.1]octan-8-yl)-5-fluoro-3-(((2*R*,7a*S*)-2-fluorotetrahydro-1*H*-pyrrolizin-7a(5*H*)-yl)methoxy)-8-methylfuro[3,2-*f*]quinazolin-6-yl)-5-ethynyl-6-fluoronaphthalen-2-amine. |
| (68) | 4-(9-((1*R*,5*S*)-3,8-diazabicyclo[3.2.1]octan-3-yl)-7-(((2*R*,7a*S*)-2-fluorotetrahydro-1*H*-pyrrolizin-7a(5*H*)-yl)methoxy)-2-methyl-2*H-*pyrazolo[4,3-*f*]quinazolin-4-yl)naphthalen-2-ol. |
| (69) | 4-(9-((1*R*,5*S*)-3,8-diazabicyclo[3.2.1]octan-3-yl)-7-(((2*R*,7a*S*)-2-fluorotetrahydro-1*H*-pyrrolizin-7a(5*H*)-yl)methoxy)-2-methyl-2*H-*pyrazolo[4,3-*f*]quinazolin-4-yl)-5-ethynyl-6-fluoronaphthalen-2-ol. |
| (70) | 4-(9-((1*R*,5*S*)-3,8-diazabicyclo[3.2.1]octan-3-yl)-7-(((2*R*,7a*S*)-2-fluorotetrahydro-1*H*-pyrrolizin-7a(5*H*)-yl)methoxy)-2-methyl-2*H-*pyrazolo[4,3-*f*]quinazolin-4-yl)-5,6-difluoronaphthalen-2-ol. |
| (71) | 4-(9-((1*R*,5*S*)-3,8-diazabicyclo[3.2.1]octan-3-yl)-7-(((2*R*,7aS*)*-2-fluorotetrahydro-1*H*-pyrrolizin-7a(5*H*)-yl)methoxy)-2-methyl-2*H-*pyrazolo[4,3-*f*]quinazolin-4-yl)-5-ethynyl-6-fluoronaphthalen-2-amine. |
| (72) | 4-(9-((1*R*,5*S*)-3,8-diazabicyclo[3.2.1]octan-3-yl)-7-(3-(dimethylamino)azetidin-1-yl)-5-fluoro-2-methyl-2*H*-pyrazolo[4,3-*f*]quinazolin-4-yl)-5-ethynyl-6-fluoronaphthalen-2-ol. |
| (73) | 4-(9-((1*R,*5*S*)-3,8-diazabicyclo[3.2.1]octan-3-yl)-7-((1-((1,1-difluoro-6-azaspiro[2.5]octan-6-yl)methyl)cyclopropyl)methoxy)-2-methyl-2*H-*pyrazolo[4,3-*f*]quinazolin-4-yl)-5-ethynyl-6-fluoronaphthalen-2-ol. |
| (74) | 4-(9-((1*R*,5*S*)-3,8-diazabicyclo[3.2.1]octan-3-yl)-7-(((*S*,*Z*)-2-(fluoromethylene)tetrahydro-1H-pyrrolizin-7a(5H)-yl)methoxy)-2-methyl-2*H*-pyrazolo[4,3*-f*]quinazolin-4-yl)-5-ethynyl-6-fluoronaphthalen-2-amine. |
| (75) | 4-(9-((1*R*,5*S*)-3,8-diazabicyclo[3.2.1]octan-3-yl)-7-(((*S*,*Z*)-2-(fluoromethylene)tetrahydro-1*H*-pyrrolizin-7a(5*H*)-yl)methoxy)-2-methyl-2H-pyrazolo[4,3-*f*]quinazolin-4-yl)-5-ethynyl-6-fluoronaphthalen-2-ol. |
| (76) | 4-(9-((1*R*,5*S*)-3,8-diazabicyclo[3.2.1]octan-3-yl)-7-((1-((1,1-difluoro-6-azaspiro[2.5]octan-6-yl)methyl)cyclopropyl)methoxy)-2-methyl-2*H-*pyrazolo[4,3-*f*]quinazolin-4-yl)-5-ethynyl-6-fluoronaphthalen-2-amine. |
| (77) | 4-(9-((1*R*,5*S*)-3,8-diazabicyclo[3.2.1]octan-3-yl)-7-(((2*R*,7a*S*)-2-fluorotetrahydro-1*H*-pyrrolizin-7a(5*H*)-yl)methoxy)-2-methyl-2*H*-pyrazolo[4,3-*f*]quinazolin-4-yl)-2-amino-7-fluorobenzo[*b*]thiophene-3-carbonitrile. |
| (78) | 9-((1*R*,5*S*)-3,8-diazabicyclo[3.2.1]octan-3-yl)-4-(8-ethynyl-7-fluoronaphthalen-1-yl)-7-(((*S*,*Z*)-2-(fluoromethylene)tetrahydro-1*H-*pyrrolizin-7a(5*H*)-yl)methoxy)-2-methyl-2*H*-pyrazolo[4,3-*f*]quinazoline |
| (79) | 4-(9-((1*R*,5*S*)-3,8-diazabicyclo[3.2.1]octan-3-yl)-7-(((2*S*,7a*R*)-2-fluoro-6-methylenetetrahydro-1*H*-pyrrolizin-7a(5*H*)-yl)methoxy)-2-methyl-2*H-*pyrazolo[4,3-*f*]quinazolin-4-yl)-5-ethynyl-6-fluoronaphthalen-2-ol. |
| (80) | 4-(9-((1*R*,5*S*)-3,8-diazabicyclo[3.2.1]octan-3-yl)-5-fluoro-7-(((2*R*,7a*S*)-2-fluorotetrahydro-1*H*-pyrrolizin-7a(5*H*)-yl)methoxy)-2-methyl-2*H-*pyrazolo[4,3-*f*]quinazolin-4-yl)-5-ethynyl-6-fluoronaphthalen-2-ol. |
| (81) | 4-(9-((1*R*,5*S*)-3,8-diazabicyclo[3.2.1]octan-3-yl)-5-fluoro-7-(((2*R*,7a*S*)-2-fluorotetrahydro-1*H*-pyrrolizin-7a(5*H*)-yl)methoxy)-2-methyl-2*H-*pyrazolo[4,3-*f*]quinazolin-4-yl)-5-ethynyl-6-fluoronaphthalen-2-amine. |
| (82) | 9-((1*R*,5*S*)-3,8-diazabicyclo[3.2.1]octan-3-yl)-4-(8-ethynyl-7-fluoronaphthalen-1-yl)-5-fluoro-7-(((2*R*,7a*S*)-2-fluorotetrahydro-1*H-*pyrrolizin-7a(5*H*)-yl)methoxy)-2-methyl-2*H*-pyrazolo[4,3-*f*]quinazoline. |
| (83) | 4-(9-((1*R*,5*S*)-3,8-diazabicyclo[3.2.1]octan-3-yl)-7-(((2*R*,7a*S*)-2-fluorotetrahydro-1*H*-pyrrolizin-7a(5*H*)-yl)methoxy)-2-methyl-2*H-*pyrazolo[4,3-*f*]quinazolin-4-yl)-5-ethyl-6-fluoronaphthalen-2-ol. |
| (84) | 4-(9-((1*R*,5*S*)-3,8-diazabicyclo[3.2.1]octan-3-yl)-7-(((*S*,*Z*)-2-(fluoromethylene)tetrahydro-1*H*-pyrrolizin-7a(5*H*)-yl)methoxy)-2-methyl-2H-pyrazolo[4,3-*f*]quinazolin-4-yl)-5,6-difluoronaphthalen-2-ol. |
| (85) | 4-(9-((1*R*,5*S*)-3,8-diazabicyclo[3.2.1]octan-3-yl)-5-fluoro-7-(((*S*,*Z*)-2-(fluoromethylene)tetrahydro-1*H*-pyrrolizin-7a(5*H*)-yl)methoxy)-2-methyl-2*H*-pyrazolo[4,3-*f*]quinazolin-4-yl)-5-ethyl-6-fluoronaphthalen-2-ol. |
| (86) | 4-(9-((1*R*,5*S*)-3,8-diazabicyclo[3.2.1]octan-3-yl)-5-fluoro-7-(((*S*,*Z*)-2-(fluoromethylene)tetrahydro-1*H*-pyrrolizin-7a(5*H*)-yl)methoxy)-2-methyl-2*H*-pyrazolo[4,3-*f*]quinazolin-4-yl)-5,6-difluoronaphthalen-2-ol. |
| (87) | 4-(9-((1*R*,5*S*)-3,8-diazabicyclo[3.2.1]octan-3-yl)-7-((1-((1,1-difluoro-6-azaspiro[2.5]octan-6-yl)methyl)cyclopropyl)methoxy)-5-fluoro-2-methyl-2*H*-pyrazolo[4,3-*f*]quinazolin-4-yl)-5-ethynyl-6-fluoronaphthalen-2-ol. |
| (88) | 4-(9-(2,5-diazabicyclo[2.2.2]octan-2-yl)-5-fluoro-7-(((2*R*,7a*S*)-2-fluorotetrahydro-1*H*-pyrrolizin-7a(5*H*)-yl)methoxy)-2-methyl-2*H-*pyrazolo[4,3-*f*]quinazolin-4-yl)-5-ethynyl-6-fluoronaphthalen-2-ol. |
| (89) | 9-((1*R*,5*S*)-3,8-diazabicyclo[3.2.1]octan-3-yl)-4-(5-chloro-3,6-dimethyl-1*H*-indazol-4-yl)-5-fluoro-7-(((2*R*,7a*S*)-2-fluorotetrahydro-1*H*-pyrrolizin-7a(5*H*)-yl)methoxy)-2-methyl-2*H*-pyrazolo[4,3-*f*]quinazoline. |
| (90) | 4-(9-((1*R*,5*S*)-3,8-diazabicyclo[3.2.1]octan-3-yl)-7-(((*S*)-2-(difluoromethylene)tetrahydro-1*H*-pyrrolizin-7a(5*H*)-yl)methoxy)-5-fluoro-2-methyl-2*H*-pyrazolo-[4,3-*f*]quinazolin-4-yl)-5-ethynyl-6-fluoronaphthalen-2-ol. |
| (91) | 4-((R)-9-((1*R*,5*S*)-3,8-diazabicyclo[3.2.1]octan-3-yl)-7-(((*S*)-2-(difluoromethylene)tetrahydro-1*H*-pyrrolizin-7a(5*H*)-yl)methoxy)-5-fluoro-2-methyl-2*H*-pyrazolo[4,3-*f*]quinazolin-4-yl)-5-ethynyl-6-fluoronaphthalen-2-ol. |
| (92) | 4-((S)-9-((1*R*,5*S*)-3,8-diazabicyclo[3.2.1]octan-3-yl)-7-(((*S*)-2-(difluoromethylene)tetrahydro-1*H*-pyrrolizin-7a(5*H*)-yl)methoxy)-5-fluoro-2-methyl-2*H*-pyrazolo[4,3-*f*]quinazolin-4-yl)-5-ethynyl-6-fluoronaphthalen-2-ol. |
| (93) | 4-(9-((1*R*,5*S*)-3,8-diazabicyclo[3.2.1]octan-3-yl)-5-fluoro-7-(((2*R*,7a*S*)-2-fluorotetrahydro-1*H*-pyrrolizin-7a(5*H*)-yl)methoxy)-2-methyl-2H-pyrazolo[4,3-*f*]quinazolin-4-yl)-5-ethyl-6-fluoronaphthalen-2-ol. |
| (94) | 4-(9-((1*R*,5*S*)-3,8-diazabicyclo[3.2.1]octan-3-yl)-5-fluoro-7-(((*S*,*Z*)-2-(fluoromethylene)tetrahydro-1*H*-pyrrolizin-7a(5*H*)-yl)methoxy)-2-methyl-2*H*-pyrazolo[4,3-*f*]quinazolin-4-yl)-5-ethynyl-6-fluoronaphthalen-2-ol. |
| (95) | 4-(9-((1*R*,5*S*)-3,8-diazabicyclo[3.2.1]octan-3-yl)-5-fluoro-7-(((2*R*,7a*S*)-2-fluorotetrahydro-1*H*-pyrrolizin-7a(5*H*)-yl)methoxy)-2-methyl-2*H-*pyrazolo[4,3-*f*]quinazolin-4-yl)-5,6-difluoronaphthalen-2-ol. |
| (96) | 4-(9-((1*R*,5*S*)-3,8-diazabicyclo[3.2.1]octan-3-yl)-5-fluoro-7-(((5*S*,7a*S*)-5-yl)methoxy)-2-methyl-2*H*-pyrazolo[4,3-*f*]quinazolin-4-yl)-5-ethynyl-6-fluoronaphthalen-2-ol. |
| (97) | 4-(9-((1*R*,5*S*)-3,8-diazabicyclo[3.2.1]octan-3-yl)-7-(((*S*)-2-(difluoromethylene)tetrahydro-1*H*-pyrrolizin-7a(5*H*)-yl)methoxy)-5-fluoro-2-methyl-2*H*-pyrazolo[4,3-*f*]quinazolin-4-yl)-5-ethyl-6-fluoronaphthalen-2-ol. |
| (98) | 4-(9-((1*R*,5*S*)-3,8-diazabicyclo[3.2.1]octan-3-yl)-5-fluoro-7-((1-(((*R*)-3-fluoropyrrolidin-1-yl)methyl)cyclobutyl)methoxy)-2-methyl-2*H-*pyrazolo[4,3-*f*]quinazolin-4-yl)-5-ethynyl-6-fluoronaphthalen-2-ol. |
| (99) | 4-(9-((1*R*,5*S*)-3,8-diazabicyclo[3.2.1]octan-3-yl)-7-(((*S*)-2-(difluoromethylene)tetrahydro-1*H*-pyrrolizin-7a(5*H*)-yl)methoxy)-5-fluoro-2-methyl-2*H*-pyrazolo[4,3-*f*]quinazolin-4-yl)-5-ethyl-6-fluoronaphthalen-2-amine. |
| (100) | 4-(9-((1*R*,5*S*)-3,8-diazabicyclo[3.2.1]octan-3-yl)-5-fluoro-7-(((2*S*,7a*R*)-2-fluoro-6-methylenetetrahydro-1*H*-pyrrolizin-7a(5*H*)-yl)methoxy)-2-methyl-2*H*-pyrazolo[4,3-*f*]quinazolin-4-yl)-5-ethynyl-6-fluoronaphthalen-2-ol. |
| (101) | 9-((1*R*,5*S*)-3,8-diazabicyclo[3.2.1]octan-3-yl)-7-(((*S*)-2-(difluoromethylene)tetrahydro-1*H*-pyrrolizin-7a(5*H*)-yl)methoxy)-4-(8-ethynyl-7-fluoronaphthalen-1-yl)-5-fluoro-2-methyl-2*H*-pyrazolo[4,3-*f*]quinazoline. |
| (102) | (4-(4-(8-ethynyl-7-fluoro-3-hydroxynaphthalen-1-yl)-5-fluoro-7-(((2*R*,7a*S*)-2-fluorotetrahydro-1*H*-pyrrolizin-7a(5*H*)-yl)methoxy)-2-methyl-2*H*-pyrazolo[4,3-*f*]quinazolin-9-yl)piperazin-1-yl)(oxiran-2-yl)methanone. |
| (103) | 4-(9-((1*R*,5*S*)-3,8-diazabicyclo[3.2.1]octan-8-yl)-5-fluoro-7-(((2*R*,7a*S*)-2-fluorotetrahydro-1*H*-pyrrolizin-7a(5*H*)-yl)methoxy)-2-methyl-2*H-*pyrazolo[4,3-*f*]quinazolin-4-yl)-5-ethynyl-6-fluoronaphthalen-2-ol. |
| (104) | 4-(9-((1*R*,5*S*)-3,8-diazabicyclo[3.2.1]octan-3-yl)-5-fluoro-7-(((2*R*,7a*S*)-2-fluorotetrahydro-1*H*-pyrrolizin-7a(5*H*)-yl)methoxy)-2-methyl-2*H-*pyrazolo[4,3-*f*]quinazolin-4-yl)-2-amino7-fluorobenzo[*b*]thiophene-3-carbonitrile |
| (105) | 4-(9-((1*R*,4*R*)-2,5-diazabicyclo[2.2.1]heptan-2-yl)-5-fluoro-7-(((2*R*,7a*S*)-2-fluorotetrahydro-1*H*-pyrrolizin-7a(5*H*)-yl)methoxy)-2-methyl-2*H-*pyrazolo[4,3-*f*]quinazolin-4-yl)-5-ethynyl-6-fluoronaphthalen-2-ol |
| (106) | 4-(1-((1*R*,5*S*)-3,8-diazabicyclo[3.2.1]octan-8-yl)-3-(((*S*)-2-(difluoromethylene)tetrahydro-1*H*-pyrrolizin-7a(5*H*)-yl)methoxy)imidazo[1',2':1,6]pyrido[3,2-*d*]pyrimidin-6-yl)-5-ethynyl-6-fluoronaphthalen-2-ol |
| (107) | 4-(1-((1*R*,5*S*)-3,8-diazabicyclo[3.2.1]octan-8-yl)-3-(((*S*)-2-(difluoromethylene)tetrahydro-1*H*-pyrrolizin-7a(5*H*)-yl)methoxy)imidazo[1',2':1,6]pyrido[3,2-*d*]pyrimidin-6-yl)-5-ethyl-6-fluoronaphthalen-2-ol |
| (108) | 4-(1-((1*R*,5*S*)-3,8-diazabicyclo[3.2.1]octan-8-yl)-3-(((*S*)-2-(difluoromethylene)tetrahydro-1*H*-pyrrolizin-7a(5*H*)-yl)methoxy)imidazo[1',2':1,6]pyrido[3,2-*d*]pyrimidin-6-yl)-5-ethynyl-6-fluoronaphthalen-2-amine |
| (109) | 4-(1-((1*R*,5*S*)-3,8-diazabicyclo[3.2.1]octan-3-yl)-3-(((*S*)-2-(difluoromethylene)tetrahydro-1*H*-pyrrolizin-7a(5*H*)-yl)methoxy)imidazo[1',2':1,6]pyrido[3,2-*d*]pyrimidin-6-yl)-5-ethyl-6-fluoronaphthalen-2-ol |
| (110) | 4-(9-((1*R*,5*S*)-3,8-diazabicyclo[3.2.1]octan-3-yl)-5-fluoro-7-(((2*R*,7a*S*)-2-fluorotetrahydro-1*H*-pyrrolizin-7a(5*H*)-yl)methoxy)-2-methyl-2*H-*pyrazolo[4,3-*f*]quinazolin-4-yl)-2-amino-5-fluorobenzo[*b*]thiophene-3-carbonitrile. |
| (111) | 4-(9-((1*R*,5*S*)-3,8-diazabicyclo[3.2.1]octan-3-yl)-5-fluoro-7-(((2*R*,7a*S*)-2-fluorotetrahydro-1*H*-pyrrolizin-7a(5*H*)-yl)methoxy)-2-methyl-2*H-*pyrazolo[4,3-*f*]quinazolin-4-yl)-2-amino-5,7-difluorobenzo[*b*]thiophene-3-carbonitrile. |
| (112) | 4-(9-((1*R*,5*S*)-3,8-diazabicyclo[3.2.1]octan-3-yl)-5-fluoro-7-(((2*R*,7a*S*)-2-fluorotetrahydro-1*H*-pyrrolizin-7a(5*H*)-yl)methoxy)-2-methyl-2*H-*pyrazolo[4,3-*f*]quinazolin-4-yl)-2-amino-7-fluorothieno[3,2-*c*]pyridine-3-carbonitrile. |
| (113) | 4-(9-((1*R*,5*S*)-3,8-diazabicyclo[3.2.1]octan-3-yl)-5-fluoro-7-(((2*R*,7a*S*)-2-fluorotetrahydro-1*H*-pyrrolizin-7a(5*H*)-yl)methoxy)-2-methyl-2*H-*pyrazolo[4,3-*f*]quinazolin-4-yl)-2-aminobenzofuran-3-carbonitrile. |
| (114) | 4-(9-((1*R*,5*S*)-3,8-diazabicyclo[3.2.1]octan-3-yl)-5-fluoro-7-(((2*R*,7a*S*)-2-fluorotetrahydro-1*H*-pyrrolizin-7a(5*H*)-yl)methoxy)-2-methyl-2*H-*pyrazolo[4,3-*f*]quinazolin-4-yl)-2-amino-7-fluorobenzofuran-3-carbonitrile. |
| (115) | 4-(9-((1*R*,5*S*)-3,8-diazabicyclo[3.2.1]octan-3-yl)-5-fluoro-7-(((2*R*,7a*S*)-2-fluorotetrahydro-1*H*-pyrrolizin-7a(5*H*)-yl)methoxy)-2-methyl-2*H-*pyrazolo[4,3-*f*]quinazolin-4-yl)-2-amino-5-fluorobenzofuran-3-carbonitrile. |
| (116) | 4-(9-((1*R*,5*S*)-3,8-diazabicyclo[3.2.1]octan-8-yl)-5-fluoro-7-(((2*R*,7a*S*)-2-fluorotetrahydro-1*H*-pyrrolizin-7a(5*H*)-yl)methoxy)-2-methyl-2*H-*pyrazolo[4,3-*f*]quinazolin-4-yl)-5-ethynyl-6-fluoronaphthalen-2-amine |
| (117) | 4-(9-((1*R*,5*S*)-3,8-diazabicyclo[3.2.1]octan-3-yl)-5-fluoro-7-(((2*R*,7a*S*)-2-methoxytetrahydro-1H-pyrrolizin-7a(5*H*)-yl)methoxy)-2-methyl-2*H-*pyrazolo[4,3-*f*]quinazolin-4-yl)-5-ethynyl-6-fluoronaphthalen-2-ol. |
| (118) | 9-((1*R*,5*S*)-3,8-diazabicyclo[3.2.1]octan-3-yl)-4-(5-ethynyl-6-fluoroisoquinolin-4-yl)-5-fluoro-7-(((2*R*,7a*S*)-2-fluorotetrahydro-1*H-*pyrrolizin-7a(5*H*)-yl)methoxy)-2-methyl-2*H*-pyrazolo[4,3-*f*]quinazoline |
| (119) | 4-(9-((1*R*,5*S*)-3,8-diazabicyclo[3.2.1]octan-3-yl)-7-(((*R*)-1-((dimethylamino)methyl)-2,2-difluorocyclopropyl)methoxy)-5-fluoro-2-methyl-2*H*-pyrazolo[4,3-*f*]quinazolin-4-yl)-5-ethynyl-6-fluoronaphthalen-2-ol. |
| (120) | 4-(9-((1*R*,5*S*)-3,8-diazabicyclo[3.2.1]octan-3-yl)-7-(((1*S*,7a'*S*)-2,2-difluorodihydro-1'*H*,3'*H*-spiro[cyclopropane-1,2'-pyrrolizin]-7a'(5'*H*)-yl)methoxy)-5-fluoro-2-methyl-2*H*-pyrazolo[4,3-*f*]quinazolin-4-yl)-5-ethynyl-6-fluoronaphthalen-2-ol. |

All technical terms used in the present application, unless otherwise defined, shall be interpreted as having the same meaning as commonly understood by those of ordinary skill in the art to which this application pertains. In addition, while exemplary methods and samples may be described herein, equivalent or similar methods and materials are also considered to fall within the scope of the present application. Furthermore, numerical values described in this specification shall be construed to include the meaning of "about," even if not explicitly stated. All publications cited in this specification are hereby incorporated by reference in their entirety.

In the Chemical Formula 1, the residues listed as R₁ to R₂₁, Rₓ, and R_{y} are used with the same meanings as generally understood by those skilled in the art.

Unless otherwise stated, the term "halogen" as used herein, when used alone or in combination with an additional term (e.g., haloalkyl) refers to fluorine, chlorine, bromine, or iodine, specifically fluorine, chlorine, but not limited thereto.

Unless otherwise stated, the term "alkoxy" as used herein means alkyloxy, for example, alkyloxy having 1 to 7 carbon atoms.

Unless otherwise stated, the term "haloalkyl" as used herein means alkyl as defined herein, wherein one or more hydrogens are replaced by the same or different halogens. Examples of haloalkyl groups include -CH₂Cl, -CH₂CF₃, -CH₂CCl₃, -CF₃, and the like, but not limited to.

Unless otherwise stated, the term "hydroxyalkyl" as used herein includes substitution of one or more hydrogen atoms in the alkyl group with one or more hydroxyl (-OH), e.g., divalent or trivalent hydroxy.

Unless otherwise stated, the term "aminoalkyl" as used herein includes substitution of one or more hydrogen atoms in an alkyl group with one or more amino (-NH₂), for example, divalent or trivalent amino.

Unless otherwise stated, the term "oxo" as used herein means a group of the formula =O (i.e., oxygen having a double bond).

Unless otherwise stated, the term "alkyl" as used herein refers to a saturated, straight-chain or branched monovalent hydrocarbon radical, unless otherwise stated. For example, the alkyl may be C₁₋₁₀ alkyl, C₁₋₈ alkyl, C₁₋₆ alkyl, C₁₋₄ alkyl, or C₁₋₃ alkyl.

Unless otherwise stated, the term " alkenyl" as used herein refers to a monovalent hydrocarbon radical containing at least one carbon-carbon double bond, unless otherwise stated, wherein each double bond may have either E- or Z-configuration. For example, the alkenyl may be C₂₋₁₀ alkenyl, C₂₋₈ alkenyl, C₂₋₆ alkenyl, or C₂₋₄ alkenyl.

Unless otherwise stated, the term " alkynyl" as used herein refers to a monovalent group derived from an unsaturated, straight-chain or branched hydrocarbon moiety having at least one carbon-carbon triple bond, unless otherwise stated. For example, the alkynyl may be C₂₋₁₀ alkynyl, C₂₋₈ alkynyl, C₂₋₆ alkynyl, or C₂₋₄ alkynyl.

These "alkyl," "alkenyl," and "alkynyl," when used alone or in combination with additional terms (e.g., haloalkyl), may be straight-chain or branched. By their definition, they mean a radical of a saturated aliphatic hydrocarbon group having 1 to 10, 1 to 9, 1 to 8, 1 to 7, 1 to 6, 1 to 5, 1 to 4, or 1 to 3 carbon atoms within the alkyl group. Common examples of alkyl groups include methyl, ethyl, n-propyl, iso-propyl, n-butyl, iso-butyl, sec-butyl, tert-butyl, n-pentyl, iso-pentyl, neo-pentyl and tert-pentyl, 1-methylpentyl, 2-methylbutyl, 1-ethylpropyl, 1,2-dimethylpropyl, n-hexyl, 3,3-dimethylbutyl and isohexyl, but are not limited to. Each of the double bond and the triple bond of the alkenyl group and the alkynyl group may be substituted at any position. Examples of alkenyl and alkynyl include ethenyl, prop-1-enyl, prop-2-enyl, but-2-enyl, 2-methylprop-2-enyl, 3-methylbut-2-enyl, hex-3-enyl, hex-4-enyl, prop-2-ynyl, but-2-ynyl, but-3-ynyl, hex-4-ynyl, or hex-5-ynyl, but are not limited to.

Unless otherwise stated herein, the terms "cycloalkyl", "cycloalkenyl", or "cycloalkynyl" as used herein mean a cyclic alkyl which may be substituted or unsubstituted and a hydrocarbon radical forming a single or fused cyclic ring having unsaturated or partially or fully saturated (e.g., from 3 to 24) carbon atoms. Specifically, the cycloalkyl, cycloalkenyl, or cycloalkynyl may have 3 to 10, 3 to 8, 3 to 6, 3 to 5, 4 to 10, 4 to 8, 4 to 6, or 4 to 5 carbon atoms. The cycloalkyl may include carbocyclyl, spirocarbocyclyl, fused carbocyclyl, bridged carbocyclyl, but not limited to.

According to one specific example of the present disclosure, the cycloalkyl may be cyclopropyl, cyclobutyl, cyclopentyl, cyclohexyl, cyclohexenyl, cycloheptyl, cyclohepsenyl, cyclooctyl, cyclooctenyl, 2,5-cyclohexadienyl, spiro[3.5]nonane, spiro[3.3]heptane, bicyclo[1.1.1]pentane, bicyclo[2.2.2]octyl, adamant-1-yl, decahydronaphthyl, oxocyclohexyl, dioxocyclohexyl, thiocyclohexyl, 2-oxobicyclo[2.2.1]hept-1-enyl, benzene, naphthalene, and may include all possible isomers thereof, but not limited to.

Unless otherwise stated herein, the term "saturated or unsaturated heterocyclyl" means a substituted or unsubstituted 3 to 24-membered hydrocarbon group comprising one or more heteroatoms selected from the group consisting of nitrogen (N), oxygen (O), and sulfur (S), for example, 1 to 8 heteroatoms, forming an unsaturated or partially or fully saturated single or fused cyclic ring. Specifically, the heterocyclyl may be 3 to 10-membered, 3 to 8-membered, 3 to 6-membered, 4 to 10-membered, 4 to 8-membered, or 4 to 6-membered hydrocarbon having 1 to 3 heteroatoms. The heterocycle includes heteroaryl, heterocyclyl, heterospirocarbocyclyl, fused heterocyclyl, bridged heterocyclyl, but not limited to.

According to one specific example of the present invention, the heterocyclyl may be pyrrolidinyl, morpholinyl, pyrrolizidinyl, quinolizidinyl, azaspirooctanyl, piperidinyl, pyrrolidinyl, imidazolinyl, piperazinyl, piperazinyl-1-oxide, morpholinyl, thiamorpholinyl, tetrahydrofuranyl, diazabicyclooctanyl, diazaspirooctanyl, tetrahydropyridinyl, dihydropyridinyl, dihydropyranyl, tetrahydropyranyl, 2-oxa-6-azaspiroheptanyl, azetidinyl, oxazepanyl, 2-oxa-5-azabicyclo[2.2.1]heptanyl, pyridyl, tetrahydrofuranyl, 8-azabicyclo[3.2.1]octanyl, 2-azaspiro[3.3]heptanyl, 2-oxa-7-azaspiro[3.4]octanyl, 2-azabicyclo[2.2.1]heptanyl, 3-oxa-8-azabicyclo[3.2.1]octanyl, pyrimidinyl, pyrazolyl, oxetane and similar groups, but not limited thereto. For example, in the case of C₂₋₁₀ heterocyclyl, the carbon number designation C₂₋₁₀ means a ring size of at least a three-membered ring including one or more heteroatoms.

The term "aryl" in this application, unless otherwise stated, represents an aromatic group which may be substituted or unsubstituted, and for example, may be 6 to 20-membered. For example, the aryl may comprise, without limitation, phenyl, biphenyl, naphthyl, toluyl, naphthalenyl, anthracenyl, indenyl, indanyl, or all possible isomers thereof.

The term "heteroaryl" in this application, unless otherwise stated, means a monocyclic or bicyclic or higher aromatic group comprising one or more heteroatoms selected from O, N and S, for example, 1 to 4, 1 to 3, or 1 to 2, and for example, may be 6 to 20-membered. For example, examples of monocyclic heteroaryls comprise thiazolyl, oxazolyl, thiophenyl, furanyl, pyrrolyl, imidazolyl, isoxazolyl, pyrazolyl, triazolyl, thiadiazolyl, tetrazolyl, oxadiazolyl, pyridinyl, pyridazinyl, pyrimidinyl, pyrazinyl, quinolinyl, isoquinolinyl, benzimidazolyl, indazolyl, 2-oxa-5-azabicyclo[2.2.1]heptanyl or similar groups, but are not limited to. For example, examples of bicyclic heteroaryl comprise pyrrolizidinyl, indolyl, indolinyl, benzothiophenyl, benzofuranyl, benzimidazolyl, benzthiazolyl, benzthiophenyl, benzoxazolyl, benzisoxazolyl, benzthiazolyl, benzthiadiazolyl, benztriazolyl, indazolyl, quinolinyl, isoquinolinyl, azaspirooctanyl, purinyl, furopyridinyl, or similar groups, but are not limited to. For example, examples of tricyclic heteroaryl comprise thioxanthinyl, carbazolyl, carbolinyl, acridinyl, or similar groups, but are not limited to.

In this application, the numerical range indicated using the term "to" refers to a range that includes the numerical values described before and after the term "to" as the lower limit and the upper limit, respectively.

The compounds of the present application may possess asymmetric carbon centers, asymmetric axes, or asymmetric planes, and thus may exist as all optical and stereoisomers including substantially pure enantiomers such as R- and S-enantiomers, as well as mixture racemates. All such isomers and mixtures thereof are within the scope of the present application. With respect to the pure enantiomers, the optical purity of such enantiomers represented by Chemical Formula 1 and pharmaceutically acceptable salts thereof is preferably 60 %ee or more, more preferably 95 %ee or more, and most preferably 98 %ee or more.

The term "ee" refers to enantiomeric excess. For example, one enantiomeric form of a particular compound is present in a mixture of enantiomeric forms in a greater amount than the other enantiomeric forms of the compound. An enantiomeric enriched form may comprise an enantiomeric mixture of a particular compound in which a single enantiomeric form has a concentration ofat least 50%, more typically at least 60%, 70%, 80%, or 90%, or even greater (e.g., >95%, >97%, >99%, >99.5%) with respect to the other enantiomeric form of the compound.

As used herein, unless otherwise specified for convenience, the compound of Chemical Formula 1 intended to include all of the compound of Chemical Formula 1, an optical isomer, a stereoisomer, an isotopic variant, a solvate, a hydrate, and a pharmaceutically acceptable salt thereof.

The term "isotopic variant" as used herein refers to a compound that contains an unusual ratio of isotopes at one or more of the atoms that make up the compound. For example, an isotopic variant of a compound may be radiolabeled, for example, the hydrogen atoms may be selected from hydrogen, deuterium and tritium, and may contain carbon-13 (¹³C), nitrogen-15 (¹⁵N), and the like.

The compound of Chemical Formula 1, or an optical isomer, a stereoisomer or an isotopic variant according to the present application may form a pharmaceutically acceptable salt. The pharmaceutically acceptable salt comprises both acid or base addition salt and a stereochemically isomeric form thereof. The salt comprises any salts that maintain the activity of the parent compound in a subject to which the salts are administered and do not cause undesirable effects, and are not particularly limited thereto. Such salts comprise inorganic and organic salts, for example, acetic acid, nitric acid, aspartic acid, sulfonic acid, sulfuric acid, maleic acid, glutamic acid, formic acid, succinic acid, phosphoric acid, phthalic acid, tannic acid, tartaric acid, hydrobromic acid, propionic acid, benzenesulfonic acid, benzoic acid, stearic acid, esylic acid, lactic acid, bicarboxylic acid, bisulfuric acid, bitartaric acid, oxalic acid, butyric acid, calcium edetate, camsylic acid, carbonic acid, chlorobenzoic acid, citric acid, edetic acid, toluenesulfonic acid, edisilic acid, esylic acid, fumaric acid, gluceptic acid, pamoic acid, gluconic acid, glycolylarsanilic acid, methylnitric acid, polygalacturonic acid, hexylisorcynic acid, malonic acid, hydrochloric acid, hydroiodic acid, hydroxynaphthoic acid, isethionic acid, lactobionic acid, mandelic acid, estolinic acid, mucic acid, naphsylic acid, muconic acid, p-nitromethanesulfonic acid, hexamic acid, pantothenic acid, monohydrogenphosphoric acid, dihydrogenphosphoric acid, salicylic acid, sulfamic acid, sulfanilic acid, methanesulfonic acid, and theoclic acid. In addition, the form of the basic salt comprises, for example, alkali and alkaline earth metal salts such ammonium salt, lithium salt, sodium salt, potassium salt, magnesium salt and calcium salt, salts with organic bases such as benzathine, N-methyl-D-glucamine, hydrabamine salts, and salts with amino acids such as arginine, lysine. In addition, the salt form may also be converted into a free form by treatment with a suitable base or acid. The term "additional salt" comprises a solvate compound thatmay be formed by the compound of Chemical Formula 1 and its salts. Such a solvate compound is, for example, a hydrate, an alcoholate.

The terms and abbreviations used herein have their original meanings unless otherwise defined.

The present application also provides a method for preparing a compound of Chemical Formula 1. Hereinafter, a method for preparing a compound of Chemical Formula 1 will be described based on an exemplary reaction scheme to aid in the understanding the present application. However, one of ordinary skill in the art may prepare the compound of Chemical Formula 1 by various methods using known compounds or compounds that can be easily prepared therefrom based on the structure of Chemical Formula 1, and all such methods should all be interpreted as being included within the scope of the present application. That is, the compound of Chemical Formula 1 may be prepared by arbitrarily combining various synthetic methods described in the present specification or disclosed in the prior art, and therefore, the following description related to the method for preparing the compound of Chemical Formula 1 merely presents exemplary methods, and the order of unit operations or the like may be selectively changed as necessary, and the scope of the preparation method of the present application is not limited thereto.

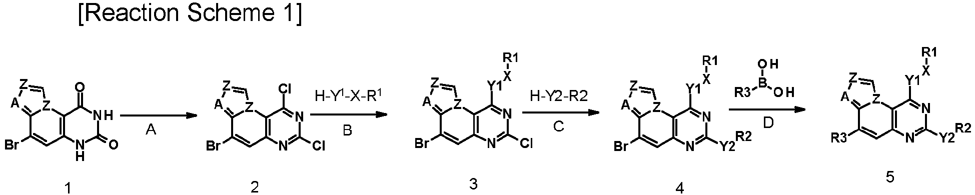

Step A: Phosphoryl trichloride and N-ethyl-N-isopropylpropan-2-amine are added to compound 1 of Reaction Scheme 1, and heat is applied to obtain compound 2.

Step B: Compound 2 of Reaction Scheme 1 is reacted with a nucleophile having the chemical formula H-Y1-X-R1 via SNAR reaction to synthesize compound 3, in the presence of a solvent such as dimethyl chloride and a base such as N-ethyl-N-isopropylpropan-2-amine.

Step C: In Reaction Scheme 1, the substituent -Y2-R2 is introduced by the 2-chlorine substituent of a nucleophilic compound having the chemical formula H-Y2-R2, by using a strong base such as sodium hydride in a nonpolar solvent such as tetrahydrofuran.

Step D: To synthesize compound 5 of Reaction Scheme 1, compound 4 and boronic acid or aryl stannan are used in Step D by Suzuki reaction or Stille reaction.

According to another aspect of the present application, a pharmaceutical composition for the prevention or treatment of a disease associated with KRAS G12D mutant protein, comprising a therapeutically effective amount of a compound represented by Chemical Formula 1, or an optical isomer, stereoisomer, racemate, isotopic variant, solvate, hydrate, or a pharmaceutically acceptable salt thereof, as an active ingredient. Specifically, the pharmaceutical composition may prevent or treat a disease associated with the KRAS G12D mutant protein by inhibiting the activity of KRAS G12D mutant protein.

The compound represented by Chemical Formula 1 according to one embodiment of the present application, or an optical isomer, stereoisomer, racemate, isotopic variant, solvate, hydrate, or a pharmaceutically acceptable salt thereof, exhibits high binding affinity to the GDP-/ GppNHp-KRAS G12D mutant protein, and may function as a KRAS G12D mutation-specific inhibitor by suppressing the phosphorylation of extracellular signal-regulated kinases (phospho-ERK, pERK) induced by KRAS G12D mutation. Accordingly, one embodiment of the present application relates to a composition for binding to KRAS G12D mutant protein, comprising a compound according to an embodiment of the present application, or an optical isomer, stereoisomer, racemate, isotopic variant, solvate, hydrate, or a pharmaceutically acceptable salt thereof. In addition, the compound of Chemical Formula 1 according to one embodiment of the present application and related forms thereof, may be used to prevent, alleviate, or treat a disease or condition associated with the KRAS G12D mutant protein, and more specifically, a disease or condition caused by the KRAS G12D mutant protein. For example, the compound of Chemical Formula 1 and related forms thereof according to the present application, as an inhibitor of KRAS G12D mutant protein, may effectively suppress the growth signaling of cancer cells caused by KRAS G12D mutation, and may be usefully employed as a pharmaceutical composition for the prevention or treatment of cancer.

According to another aspect of the present application, a pharmaceutical composition for the prevention or treatment of cancer is provided, comprising a therapeutically effective amount of a compound represented by Chemical Formula 1, or an optical isomer, stereoisomer, racemate, isotopic variant, solvate, hydrate, or a pharmaceutically acceptable salt thereof, as an active ingredient.

Further, various forms of prodrugs that are converted into the compound of Chemical Formula 1 in vivo as intended are also included within the scope of the present application. The pharmaceutical composition may further comprise one or more additives selected from the group consisting of a pharmaceutically acceptable carrier, diluent and adjuvant.

As used herein, the term "treatment" refers to the act of stopping, delaying, or alleviating the progression of a disease when administered to a subject exhibiting symptom of the disease.

As used herein, the term "prevention" refers to the act of reducing or eliminating the likelihood of developing a disease.

As used herein, the term "pharmaceutical composition" may comprise, in addition to the active compound of the present application, other chemical components such as carriers, diluents, and excipients. Accordingly, the pharmaceutical composition may optionally comprise one or more pharmaceutically acceptable carriers, diluents, excipients, or combinations thereof. Such pharmaceutical compositions facilitate the administration of the active compound into a subject. Various techniquies for administering a pharmaceutical composition comprising a compound are known in the art, including, but not limited to, oral, injectable, aerosol, parenteral, and topical administration. In addition, the pharmaceutical composition may be sterile, and may further comprise auxiliary agents such as preservatives, stabilizers, hydrating agents, emulsifying agents, salts and/or buffers for osmotic pressure control, or other therapeutically useful substances. It may also be formulated by conventional methods such as mixing, granulation, or coating.

As used herein, the term "carrier" refers to a compound that facilitates the delivery of a compound into cells or tissues. For example, dimethyl sulfoxide (DMSO) is a common carrier that facilitates the delivery of various organic compounds into cells or tissues of living organisms.

As used herein, the term "diluent" is defined as a compound that not only stabilizes the biologically active form of the subject compound, but is also diluted in water to dissolve the compound. Salts dissolved in buffers are used as diluents in the art. A commonly used buffer is phosphate buffered saline, which mimics the salt form of human body fluids. Since buffer salts can control the pH of a solution at low concentrations, it is rare for a buffer diluent to alter the biological activity of a compound.

As used herein, the term "pharmaceutically acceptable" means a property that does not impair the biological activity and physical properties of a compound.

In addition, the pharmaceutical composition may be a composition for prevention and/or treatment of a disease associated with KRAS G12D mutant protein. As used herein, the disease associated with KRAS G12D mutant protein may be, for example, cancer, and may include any other disease known to be associated with KRAS G12D mutation.

The cancer comprises hemangiosarcoma, fibrosarcoma, rhabdomyosarcoma, liposarcoma, myxoma, rhabdomyoma, fibroma, lipoma, teratoma, squamous cell carcinoma, undifferentiated small cell carcinoma, undifferentiated multicellular carcinoma, adenocarcinoma, alveolar (bronchiolar) cancer, bronchiolar adenoma, sarcoma, lymphoma, chondromatosis, mesothelioma, esophageal cancer, stomach cancer, pancreatic cancer, small intestine cancer, colon cancer, kidney cancer, bladder cancer, urethral cancer, prostate cancer, testicular cancer, liver cancer, biliary tract cancer, hepatoblastoma, hepatocellular adenoma, hemangioma, gallbladder cancer, ampullary cancer, osteosarcoma, malignant fibrous histiocytoma, chondrosarcoma, Ewing's sarcoma, malignant lymphoma (reticulocyte cell sarcoma), multiple myeloma, malignant giant cell tumor, osteochondroma, benign chondroma, chondromyxoid fibroma, chondroostoma, giant cell tumor, cranioma, cranial hemangioma, cranial granuloma, cranial xanthoma, cranial osteitis deformans, meningioma, meningiosarcoma, glioblastoma, astrocytoma, medulloblastoma, ependymoma, germinoma, oligodendroglioma, schwannoma, retinoblastoma, spinal neurofibroma, endometrial cancer, cervical cancer, ovarian cancer, blood cancer, acute lymphoblastic leukemia, chronic lymphocytic leukemia, acute myeloid leukemia, monocytic leukemia, chronic myeloid leukemia, chronic lymphocytic leukemia, mixed lineage leukemia, Hodgkin's lymphoma, non-Hodgkin's lymphoma, malignant melanoma, basal cell carcinoma, adenocarcinoma, neuroblastoma, but not limited thereto.

The pharmaceutical composition may be formulated into various oral or parenteral dosage forms. For example, it may be in the form of any oral dosage form, such as tablets, pills, hard or soft capsules, liquids, suspensions, emulsions, syrups, granules, elixirs, and the like. These oral dosage forms may comprise, in addition to the active ingredient, pharmaceutically acceptable carriers, including diluents such as lactose, dextrose, sucrose, mannitol, sorbitol, cellulose, and/or glycine, or lubricants such as silica, talc, stearic acid and magnesium or calcium salts thereof, and/or polyethylene glycol, depending on the typical composition of each dosage form.

In addition, if the oral administration formulation is a tablet, it may contain a binder such as magnesium aluminum silicate, starch paste, gelatin, tragacanth, methylcellulose, sodium carboxymethylcellulose and/or polyvinylpyrrolidine, and optionally, a disintegrating agent such as starch, agar, alginic acid or its sodium salt, an effervescent mixture and/or an absorbent, a coloring agent, a flavoring agent or a sweetener.

The pharmaceutical composition may also be formulated as a parenteral administration form, in which case it is administered by a parenteral administration method such as subcutaneous injection, intravenous injection, intramuscular injection or intrathoracic injection. In such cases, in order to formulate the pharmaceutical composition as a parenteral dosage form, the active ingredient, i.e., the compound of Chemical Formula 1 or an optical isomer, a stereoisomer, an isotope variant or a pharmaceutically acceptable salt thereof, is mixed with a stabilizer or a buffer in water to prepare a solution or suspension, and this solution or suspension may be prepared in a unit dosage form of an ampoule or a vial.

In addition, the pharmaceutical composition may be sterilized, or may further comprise auxiliary agents such as preservatives, stabilizers, hydrating agents or emulsifying agents, salts and/or buffers for osmotic pressure control, or other therapeutically useful substances, and may be formulated according to conventional methods of mixing, granulating, or coating.

The effective ingredient, i.e., the compound of the Chemical Formula 1 or a pharmaceutically acceptable salt thereof, may be included in the pharmaceutical composition for mammals including humans, in an effective amount of 0.1 to 500 mg/kg (body weight), preferably 0.5 to 100 mg/kg (body weight) per day, and this pharmaceutical composition may be administered orally or parenterally once a day or in divided doses of two or more times.

In addition, the pharmaceutical composition may be used for the treatment of cancer by comprising the compound according to one embodiment of the present application and at least one additional therapeutic agent. The compound according to one embodiment of the present application may exhibit a synergistic effect when used in combination with a different therapeutic agent. The term "synergistic" refers to a therapeutically more effective combination than the additive effect of two or more individual agents. The combination therapy provides a "synergistic" effect and proves to be "synergistic", that is, the therapeutic effect achieved when the active ingredients are used together is greater than the sum of the effects obtained when the compounds are used separately. The synergistic effect may be obtained when the active ingredients are: (1) co-formulated in a combined unit dosage form and administered or delivered simultaneously; or (2) administered as separate formulations in an alternative manner. When delivered by alternative therapy regimen, a synergistic effect may be obtained when the compounds are, for example, administered sequentially via different injections from separate syringes. In general, during alternative therapy, therapeutically effective doses of each active ingredient are administered sequentially, i.e., in a time-staggered manner. In certain embodiments, the synergistic effect is demonstrated by the lower toxicity of the combination compared to the same total dose of an equivalent amount of any single component. For example, when the compound of Chemical Formula 1 is co-administered with a different therapeutic agent, the toxicity of a 50:50 (w/w) combination of the compound of Chemical Formula 1 and the different therapeutic agent may be lower than the toxicity of 100% (w/w) of the compound of Chemical Formula 1 and/or 100% (w/w) of the different therapeutic agent, while the combination exhibits approximately the same or even greater efficacy. That is, the toxicity of the combination of the compound of Chemical Formula 1 and the different therapeutic agent is lower than that of either agent alone, while the efficacy of the combination is greater than that of the single agent. In addition, the reason for combining the compound of Chemical Formula 1 with a different therapeutic agent is not only to reduce toxicity and enhance safety, but also to achieve greater efficacy than that obtained by the single agent alone. Increased efficacy is one of the advantages of combination therapy.

The one or more therapeutic agents may include, but are not limited to, RTK/Ras-MAPK pathway-related protein inhibitor (EGFR inhibitors, FGFR inhibitors, ALK inhibitors, ROS inhibitors, MET inhibitors, RAF inhibitors, ERK inhibitors, MEK inhibitors, SHP-2 inhibitors, PI3K inhibitors, KRAS inhibitors, KRAS-G12C inhibitors, SOS1 inhibitors), a DNA-damaging agent, an EGFR antibody, or an immune-oncology therapeutic agent.

For example, the RTK/Ras-MAPK pathway inhibitors are EGFR inhibitors (erlotinib, gefitinib, afatinib, or osimertinib); FGFR inhibitors (pemigatinib or infigratinib (BGJ398)); ALK/ROS/MET inhibitors (crizotinib, cabozantinib, or foretinib); RAF inhibitors (vemurafenib, dabrafenib, or belvarafenib); ERK/MEK inhibitors (trametinib or cobimetinib); SHP-2 inhibitors (TNO155 and RMC-4630); PI3K inhibitors (AMG 511 and bupalisib); KRAS inhibitors and KRAS G12C inhibitors (sotorasib, adagrasib, and divarasib), and comprise one or more of these inhibitors, but not limited thereto.

For example, chemotherapy agents of DNA-damaging agents include alkylating agents (platinum chemotherapy: Cisplatin, Carboplatin, Oxaliplatin/ Nitrogen mustard-class drugs: Mechlorethamine (nitrogen mustard), Cyclophosphamide, Ifosfamide, Melphalan, Chlorambucil/ Nitrosourea-class drugs: Carmustine (BCNU), Lomustine (CCNU), Nimustine/ Others: Altretamine, Busulfan, Dacarbazine, Procarbazine, Temozolomide, Thiotepa, Lurbinectedin, and the like), antimetabolites (Fluorouracil (5-FU), Capecitabine, Cytarabine, Gemcitabine, Methotrexate, Mercaptopurine (6-MP), Leucovorin, Pemetrexed, and the like), Topoisomerase inhibitors (Epipodophyllotoxins: Etoposide, Teniposide/ Camptothecin: Topotecan, Irinotecan, SN-38/ Antitumor Antibiotics: Dactinomycin, Doxorubicin, Daunorubicin, Mitomycin, Bleomycin, and the like), microtubule inhibitors (Vinca alkaloids: Vinblastine, Vincristine, Vinorelbine/Taxanes: Paclitaxel, Docetaxel, and the like), hormone antagonists (Tamoxifen, Flutamide, Leuprolide, and the like), but not limited thereto.

In an example, EGFR antibody comprises cetuximab, but are not limited to.

In an example, immune-oncology therapeutic agent comprisesAMG-404, pembrolizumab, or nivolumab, but not limited thereto.

### [Advantageous Effects]

According to the present application, a tricyclic derivative compound exhibiting excellent KRAS G12D inhibitory activity, or an optical isomer, stereoisomer, or isotopic variant thereof, or a pharmaceutically acceptable salt thereof may be provided. Accordingly, such a compound, or an optical isomer, stereoisomer, or isotopic variant thereof, or a pharmaceutically acceptable salt thereof, may be effectively used for the prevention or treatment of diseases associated with KRAS G12D mutant protein, such as cancer.

In addition, the compound according to the present application, or an optical isomer, stereoisomer, or isotopic variant thereof, or a pharmaceutically acceptable salt thereof, may exhibit superior efficacy or improved pharmacokinetic properties.

### [Mode for Invention]

Hereinafter, the present application will be described in more detail through Examples. However, the Examples are only intended to exemplify the present application and the scope of the invention is not limited thereby.

### Intermediate 1: Synthesis of 6-bromo-1,3-dichloroimidazo[1',2':1,6]pyrido[3,2-d]pyrimidine.

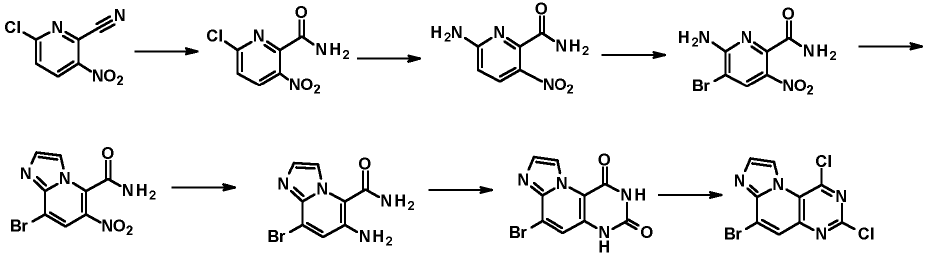

Step 1: 6-Chloro-3-nitropicolinonitrile (10 g, 54.48 mmol) was dissolved in H₂SO₄ (276 g, 2.53 mol) and heated at 70 °C for 3.5 hours. After completion of the reaction, the reaction mixture was cooled to 20 °C, and ice water (1200 mL) was added dropwise. The precipitate was filtered, washed with water, and dried in sequence, and then concentrated under reduced pressure to obtain 6-chloro-3-nitropicolinamide (9.85 g, crude compound) as a white solid. ¹H NMR (400 MHz, DMSO-*d*₆, ppm): δ 8.54 (d, *J* = 8.8 Hz, 1H), 8.29 (brs, 1H), 8.03 (brs, 1H) 7.95 (d, *J* = 8.8 Hz, 1H).

Step 2: A solution of 6-chloro-3-nitropicolinamide (7.35 g, 36.46 mmol) was dissolved in ethanol (30 mL), and a saturated 28% ammonia (27.30 g, 218.09 mmol) solution was added dropwise. The reaction was carried out at 100 °C for 48 hours in a 100 mL sealed tube. After completion of the reaction, the mixture was cooled and concentrated under reduced pressure to obtain a residue. The residue was diluted with aqueous Na₂CO₃ solution (100 mL), and the mixture was stirred at 20 °C for 1 hour, filtered, and the filter cake was washed with water (50 mL). The residue was concentrated under reduced pressure to obtain a residue. 6-amino-3-nitro-pyridine-2-carboxamide (4.85 g, crude compound) was obtained as a yellow solid. ¹H NMR (400 MHz, DMSO-*d*₆, ppm): δ 8.11 (d, *J* = 9.2 Hz, 1 H), 7.83 (brs, 1H), 7.57 (brs, 2H), 7.52 (brs, 1H), 6.50 (d, *J* = 9.2 Hz, 1 H).

Step 3: 6-amino-3-nitro-pyridine-2-carboxamide (4.85 g, 26.63 mmol) was dissolved in DMF (49 mL), then NBS (5.69 g, 31.95 mmol) was added, and the reaction mixture was stirred at 25 °C for 4 h. After the reaction was completed, most of the solvent was removed under reduced pressure, and the residue was suspended in purified water (100 mL). The solid was filtered and dried under reduced pressure. The product was stirred with petroleum ether (45 mL) and ethyl acetate (40 mL) at 20 °C for 30 min, and the solid was filtered and dried under reduced pressure. 6-amino-3-nitropicolinamide (5.6 g, 21.13 mmol, 79.37% yield) was obtained as a yellow solid. LCMS (ES-API, m/z): [M+H]⁺= 263.0; ¹H NMR (400 MHz, DMSO-*d*₆, ppm): δ 8.48 (s, 1H), 7.90 (brs, 2H), 7.65 (brs, 2H).

Step 4: 6-amino-3-nitropicolinamide (5.9 g, 22.60 mmol) was dissolved in ethanol (59 mL) and distilled water (59 mL), and 2-bromo-1,1-diethoxyethane (6.68 g, 33.90 mmol) and HBr (9.14 g, 45.21 mmol) were added dropwise, respectively, at 20 °C. The reaction mixture was heated at 100 °C for 16 h. The reaction mixture was concentrated in vacuo to remove ethanol, and the pH of the residue was adjusted to 8 with aqueous NaHCO₃ solution, the solid was precipitated, and then filtered. The filter cake was concentrated in vacuo to obtain 8-bromo-6-nitroimidazo[1,2-a]pyridine-5-carboxamide (5.28 g, 18.14 mmol, 80.26% yield) as a gray color. The results were confirmed by LCMS and HNMR. LCMS (ES-API, m/z): [M+H]⁺= 287.0; ¹H NMR (400 MHz, DMSO-*d*₆, ppm): δ 8.55 (d, *J =* 10.0 Hz, 2 H), 8.34 (d, 1H), 8.09 (d, *J* = 1.6 Hz, 1H), 7.92 (d, *J* = 1.2 Hz, 1 H).

Step 5: 8-bromo-6-nitroimidazo[1,2-a]pyridine-5-carboxamide (4.5 g, 15.79 mmol), Fe (3.53 g, 63.15 mmol) and NH₄Cl (6.76 g, 126.29 mmol) were added to ethanol (90 mL) and distilled water (18 mL), and the mixture was stirred at 85 °C for 16 h. The reaction mixture was filtered, and the filter cake was washed with 800 mL EA/MeOH (10:1). The organic phases were combined to obtain the residue. The residue was then washed with saturated sodium bicarbonate, filtered, and the filter cake was concentrated under reduced pressure to obtain the residue. 6-amino-8-bromoimidazo[1,2-a]pyridine-5-carboxamide (3.1 g, crude compound) was obtained as a green solid. LCMS (ES-API, m/z): [M+H]⁺= 254.9; ¹H NMR (400 MHz, DMSO-*d*₆, ppm): δ 8.21 - 7.61 (m, 3H), 7.57 - 7.20 (m, 2H), 6.02 - 5.40 (m, 2H).

Step 6: 6-amino-8-bromoimidazo[1,2-a]pyridine-5-carboxamide (10.47 g, 35.28 mmol) was dissolved in 1,4-dioxane (100 mL). The resulting suspension was refluxed under nitrogen at 110 °C for 16 h. After the starting material was consumed, the reaction solution was cooled to 20 °C, distilled water (20 mL) was added, and the reaction was terminated by stirring for 10 min. The mixture was filtered, and the solid was washed with ethyl acetate (200 mL), and then dried. 6-bromoimidazo[1',2':1,6]pyrido[3,2-d]pyrimidine-1,3(2H,4H)-dione (10.7 g, crude compound) was obtained as a yellow solid. LCMS (ES-API, m/z): [M+H]⁺= 283.2; ¹H NMR (400 MHz, DMSO-*d*₆, ppm): δ 12.00 - 11.95 (m, 1H), 11.91 - 11.83 (m, 1H), 9.11 (d, *J* = 0.8 Hz, 1H), 8.02 - 7.94 (m, 1H), 7.84 - 7.74 (m, 1H)

Step 7: In a 250 mL three-necked round bottom flask, POCl₃ (117.84 g, 768.50 mmol) was added dropwise to 6-bromoimidazo[1',2':1,6]pyrido[3,2-*d*]pyrimidine-1,3(2*H*,4*H*)-dione (5.4 g, 19.21 mmol) obtained in Step 6. Then, DIPEA (6.21 g, 48.03 mmol) was slowly added dropwise. The reaction mixture was filled with nitrogen and stirred at 110 °C for 20 h. After the starting material was completely consumed, the solvent and most of the POCl₃ were removed under vacuum at 40 °C, ethyl acetate (100 mL) was added, and the reaction mixture was basified to pH 7-8 with saturated Na₂CO₃ solution at 0 °C. The reaction mixture was extracted with ethyl acetate (200 mL*3), and the organic layer was dried over sodium sulfate, filtered, and concentrated. The residue was purified by silica gel chromatography. 6-bromo-1,3-dichloroimidazo[1',2':1,6]pyrido[3,2-d]pyrimidine (2.44 g, 7.24 mmol, 37.68% yield) was obtained as a yellow solid. LCMS (ES-API, m/z): [M+H]⁺= 275.0; ¹H NMR (400 MHz, DMSO-*d*₆, ppm): δ 9.28 (d, *J* = 1.2 Hz, 1 H), 9.17 - 9.12 (m, 2 H), 8.12 - 8.07 (m, 1 H), 7.95 - 7.93 (m, 1 H), 7.92 - 7.90 (m, 2 H), 7.87 - 7.84 (m, 1 H).

### Intermediate 2: Synthesis of 6-bromo-1,3-dichloro-8-methylfuro[3,2-f]quinazoline.

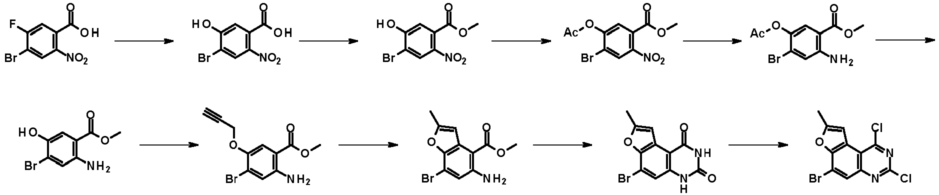

Step 1: 4-bromo-5-fluoro-2-nitrobezoic acid (50.0 g, 189 mmol) was dissolved in distilled water (600 mL), 12 M KOH (63.8 g, 1.14 mol) was added, and the reaction solution was stirred at 80 °C for one day. 1 N HCl aqueous solution was added to the reaction mixture to adjust the pH to 3, ethyl acetate was added to extract the organic layer, which was washed with saturated NaCl aqueous solution and dried over anhydrous MgSO₄. The solvent was concentrated to synthesize 4-bromo-5-hydroxy-2-nitrobenzoic acid (49.5 g, 189 mmol, crude compound). ¹H NMR (400 MHz, DMSO-*d*₆, ppm): δ 13.7 (s, 1H), 12.1 (s, 1H), 8.27 (s, 1H), 7.14 (s, 1H).

Step 2: 4-bromo-5-hydroxy-2-nitrobenzoic acid (49.5 g, 189 mmol) obtained in Step 1 was dissolved in methanol (599 mL), sulfuric acid (51.6 mL) was added at 0 °C, and the reaction solution was stirred at 70 °C for one day. Distilled water was added to the reaction mixture to terminate the reaction, and ethyl acetate was added to extract the organic layer, which was washed with a saturated NaCl aqueous solution and dried over anhydrous MgSO₄. The solvent was concentrated to synthesize methyl 4-bromo-5-hydroxy-2-nitrobenzoate (52.2 g, 189 mmol, crude compound).

Step 3: Methyl 4-bromo-5-hydroxy-2-nitrobenzoate (52.2 g, 189 mmol) obtained from Step 2 and TEA (79.0 mL, 567 mmol) were dissolved in DCM (798 mL), acetyl chloride (20.2 mL, 283 mmol) was added at 0 °C, and the reaction solution was stirred at the same temperature for 2 h. Purified water was added to the reaction mixture to quench the reaction, and the organic layer was extracted by adding DCM, and dried over anhydrous MgSO₄. The solvent was concentrated to synthesize methyl 5-acetoxy-4-bromo-2-nitrobenzoate (60.1 g, 189 mmol, crude compound). ¹H NMR (400 MHz, DMSO-*d*₆, ppm): δ 8.55 (s, 1H), 7.94 (s, 1H), 3.87 (s, 3H), 2.39 (s, 3H).

Step 4: Methyl 5-acetoxy-4-bromo-2-nitrobenzoate (60.0 g, 189 mmol) obtained from Step 3 was dissolved in ethanol (700 mL), purified water (300 mL), and acetic acid (75.5 mL, 1.32 mol), and Fe (26.3 g, 472 mmol) was slowly added. The reaction solution was stirred at room temperature for 3 hours. The reaction mixture was neutralized by adding 6 N KOH aqueous solution, the solvent was removed, and the mixture was filtered. Ethyl acetate was added to the filtrate, and the organic layer was extracted, which was washed with a saturated NaCl aqueous solution and dried over anhydrous MgSO₄. The solvent was concentrated, and the obtained material was purified by silica gel chromatography to synthesize methyl 5-acetoxy-2-amino-4-bromobenzoate (24.5 g, 85.0 mmol, 45% yield) as a solid compound. ¹H NMR (400 MHz, DMSO-*d*₆, ppm): δ 7.50 (s, 1H), 7.13 (s, 1H), 6.76 (s, 2H), 3.79 (s, 3H), 2.26 (s, 3H).

Step 5: Methyl 5-acetoxy-2-amino-4-bromobenzoate (2.28 g, 7.91 mmol) obtained from Step 4 was dissolved in MeOH (120 mL), and K₂CO₃ (2.19 g, 15.8 mmol) was added. The reaction solution was stirred at room temperature for 2 h. The reaction mixture was acidified by adding 1 N HCl aqueous solution, and the organic layer was extracted with ethyl acetate, and dried over anhydrous MgSO₄. The solvent was concentrated to synthesize methyl 2-amino-4-bromo-5-hydroxybenzoate (1.95 g, 7.91 mmol, crude compound). ¹H NMR (400 MHz, CD₃OD, ppm): δ 8.36 (d, *J* = 1.2 Hz, 1H), 8.23 (d, *J* = 1.2 Hz, 1H), 7.57 (d, *J* = 2.0 Hz, 1H), 7.44 (d, *J* = 2.0 Hz, 1H), 5.49 (s, 2H), 5.42 - 5.37 (m, 1H), 3.44 - 3.40 (m, 2H), 3.31 - 3.27 (m, 2H), 2.76 (s, 3H), 2.30 - 2.27 (m, 2H), 2.26 - 2.24 (m, 2H).

Step 6: Methyl 2-amino-4-bromo-5-hydroxybenzoate (1.95 g, 7.92 mmol) obtained from Step 5 was dissolved in DMF (50 mL), and 9.2 M 3-bromopropane (1.21 mL, 11.1 mmol) and K₂CO₃ (2.19 g, 15.8 mmol) dissolved in toluene were added sequentially, and the reaction solution was stirred at room temperature for one day. Purified water was added to the reaction mixture to quench the reaction, and ethyl acetate was added to extract the organic layer, which was washed with a saturated NaCl aqueous solution and dried over anhydrous MgSO₄. The solvent was concentrated, and the obtained material was purified by silica gel chromatography to synthesize methyl 2-amino-4-bromo-5-(prop-2-yn-1-yloxy)benzoate (1.66 g, 5.84 mmol, 74% yield) as a solid compound. ¹H NMR (400 MHz, DMSO-*d*₆, ppm): δ 7.43 (s, 1H), 7.11 (s, 1H), 6.48 (s, 2H), 4.74 (d, *J* = 2.4 Hz, 2H), 3.80 (s, 3H), 3.58 (t, *J* = 2.3 Hz, 1H).

Step 7: Methyl 2-amino-4-bromo-5-(prop-2-yn-1-yloxy)benzoate (1.66 g, 5.84 mmol) obtained from Step 6 was dissolved in N,N-diethylaniline (10 mL), CsF (1.33 g, 8.76 mmol) was added, and the reaction solution was heated to reflux and stirred for 1 h. It was diluted with ethyl acetate, washed twice with 1 N aqueous hydrochloric acid solution, and dried over anhydrous MgSO₄. The solvent was concentrated, and the obtained material was purified by silica gel chromatography to synthesize solid methyl 5-amino-7-bromo-2-methylbenzofuran-4-carboxylate (1.41 g, 4.96 mmol, 85% yield).

Step 8: Methyl 5-amino-7-bromo-2-methylbenzofuran-4-carboxylate (1.41 g, 4.96 mmol) obtained from Step 7 was dissolved in THF (50 mL), and trichloroacetyl isocyanate (0.651 mL, 5.46 mmol) was added at 0 °C, and the reaction solution was stirred at the same temperature for 30 min. The solvent was concentrated, and 7 N ammonia solution (1.69 g, 99.0 mmol) dissolved in methanol was added to the obtained substance, which was stirred at room temperature for 1 day. The solid 6-bromo-8-methylfuro[3,2-*f*[quinazoline-1,3(2*H*,4*H*)-dione (1.23 g, 4.17 mmol, 84% yield) was synthesized by filtration with methanol. ¹H NMR (400 MHz, DMSO-*d*₆, ppm): δ 10.01 (s, 2H), 7.24 (d, *J* = 1.3 Hz, 1H), 7.22 (s, 1H), 2.54 (s, 3H).

Step 9: POCl₃ (15.4 mL, 165 mmol) was added to 6-bromo-8-methylfuro[3,2-*f*]quinazoline-1,3(2*H*,4*H*)-dione (1.22 g, 4.13 mmol) obtained from Step 8, and DIPEA (1.80 mL, 10.3 mmol) was added, and the reaction solution was stirred at 110 °C for 1.5 h. The solvent was concentrated, and the reaction mixture was quenched by adding saturated NaHCO₃ solution, and the organic layer was extracted by adding ethyl acete, and dried over anhydrous MgSO₄. The solvent was concentrated, the obtained material was filtered with DCM to remove the solid, the filtrate was concentrated, and the product was purified by silica gel chromatography to synthesize solid 6-bromo-1,3-dichloro-8-methylfuro[3,2-*f*]quinazoline (40.0 mg, 0.120 mmol, 3% yield). LCMS (ES-API, m/z): [M+H]⁺= 329.9; ¹H NMR (400 MHz, DMSO-*d*₆, ppm): δ 8.23 (s, 1H), 7.70 (s, 1H), 2.68 (s, 3H).

### Intermediate 3: Synthesis of 6-bromo-1,3-dichloro-5-fluoro-8-methyl-6a,9a-dihydrofuro[3,2-f]quinazoline.

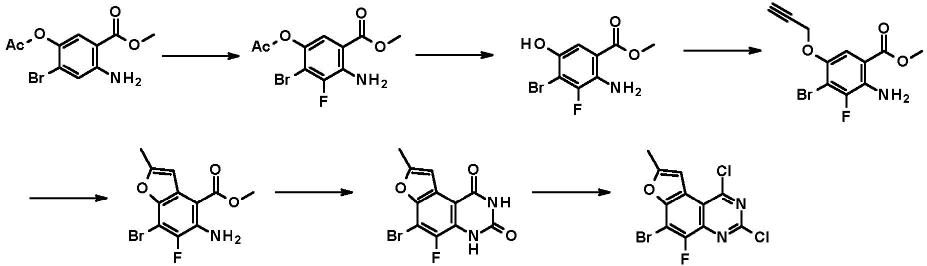

Step 1: Methyl 5-acetoxy-2-amino-4-bromobenzoate (20.0 g, 69.4 mmol) was dissolved in acetonitrile (1 L), selectfluor (27.1 g, 76.4 mmol) was added, and the reaction solution was stirred at 80 °C for one day. Saturated NaHCO₃ aqueous solution was added to the reaction mixture to adjust the pH to 8, and ethyl acetate was added to extract the organic layer, which was washed with saturated NaCl aqueous solution and dried over anhydrous MgSO₄. The solvent was concentrated, and the obtained material was purified by silica gel chromatography to synthesize solid methyl 5-acetoxy-2-amino-4-bromo-3-fluorobenzoate (5.5 g, 18.0 mmol, 26% yield). ¹H NMR (400 MHz, DMSO-*d*₆, ppm): δ 7.45 (d, *J* = 2.0 Hz, 1H), 6.74 (s, 2H), 3.83 (s, 3H), 2.29 (s, 3H).

Step 2: Methyl 5-acetoxy-2-amino-4-bromo-3-fluorobenzoate (5.50 g, 18.0 mmol) obtained from Step 1 was dissolved in methanol (275 mL), K₂CO₃ (4.97 g, 35.9 mmol) was added, and the reaction solution was stirred at room temperature for 2 h. The reaction mixture was acidified by adding 1 N HCl aqueous solution, and the organic layer was extracted with ethyl acetate and dried over anhydrous MgSO₄. The solvent was concentrated to synthesize methyl 2-amino-4-bromo-3-fluoro-5-hydroxybenzoate (4.74 g, 18.0 mmol, crude compound). ¹H NMR (400 MHz, DMSO-*d*₆, ppm): δ 9.86 (s, 1H), 7.19 (d, *J* = 2.1 Hz, 1H), 6.07 (s, 2H), 3.81 (s, 3H).

Step 3: Methyl 2-amino-4-bromo-3-fluoro-5-hydroxybenzoate (2.00 g, 7.57 mmol) obtained from Step 2 was dissolved in DMF (47.8 mL), and 9.2 M Propargyl bromide (1.15 mL, 10.6 mmol) and K₂CO₃ (2.09 g, 15.1 mmol) dissolved in toluene were added sequentially, and the reaction solution was stirred at room temperature for one day. Purified water was added to the reaction mixture to quench the reaction, and ethyl acetate was added to extract the organic layer, which was washed with saturated NaCl aqueous solution and dried over anhydrous Mg_{S}O₄. The solvent was concentrated, and the obtained material was purified by silica gel chromatography to synthesize solid methyl 2-amino-4-bromo-3-fluoro-5-(prop-2-yn-1-yloxy)benzoate (1.03 g, 3.41 mmol, 45% yield). ¹H NMR (400 MHz, DMSO-*d*₆, ppm): δ 7.34 (d, *J* = 2.0 Hz, 1H), 6.42 (s, 2H), 4.82 (s, 2H), 3.84 (s, 3H), 3.61 (t, *J* = 2.3 Hz, 1H).

Step 4: Methyl 2-amino-4-bromo-3-fluoro-5-(prop-2-yn-1-yloxy)benzoate (1.02 g, 3.38 mmol) obtained from Step 3 was dissolved in N,N-diethylaniline (10 mL), CsF (769 mg, 5.06 mmol) was added, and the reaction solution was heated to reflux and stirred for 1 h. The solution was diluted with ethyl acetate, washed twice with 1 N HCl aqueous solution, and dried over anhydrous Mg_{S}O₄. The solvent was concentrated, and the obtained material was purified by silica gel chromatography to synthesize solid methyl 5-amino-7-bromo-6-fluoro-2-methylbenzofuran-4-carboxylate (910 mg, 3.01 mmol, 89% yield). ¹H NMR (400 MHz, DMSO-*d*₆, ppm): δ 6.91 (s, 1H), 6.67 (s, 2H), 3.89 (s, 3H), 2.45 (s, 3H).

Step 5: Methyl 5-amino-7-bromo-6-fluoro-2-methylbenzofuran-4-carboxylate (910 mg, 3.01 mmol) obtained from Step 4 was dissolved in THF (30.3 mL), trichloroacetyl isocyante (0.395 mL, 3.31 mmol) was added at 0 °C, and the reaction solution was stirred at the same temperature for 30 min. The solvent was concentrated, and 7N NH₄ in methanol (8.62 mL, 60.3 mmol) was added to the obtained material, and the mixture was stirred at room temperature for 1 day. The white solid 6-bromo-5-fluoro-8-methylfuro[3,2-f]quinazoline-1,3(2*H*,4*H*)-dione (863 mg, 2.76 mmol, 91% yield) was synthesized by filtration with methanol. ¹H NMR (400 MHz, DMSO-*d*₆, ppm): δ 9.90 (s, 2H), 7.21 (s, 1H), 2.53 (s, 3H).

Step 6: POCl₃ (8.93 mL, 95.80 mmol) was added to bromo-5-fluoro-8-methylfuro[3,2-f]quinazoline-1,3(2*H*,4*H*)-dione (750 mg, 2.40 mmol) obtained from Step 5, and DIPEA (1.04 mL, 5.99 mmol) was added, and the reaction solution was stirred at 110 °C for 7 hours. Saturated NaHCO₃ solution was added to the reaction mixture to quench the reaction, and the organic layer was extracted by adding ethyl acetate, and dried over anhydrous MgSO₄. The solvent was concentrated, the obtained substance was filtered with DCM, and a white solid was obtained. The filtrate was concentrated and purified by silica gel chromatography to obtain a white solid 6-bromo-1,3-dichloro-5-fluoro-8-methyl-6a,9a-dihydrofuro[3,2-*f*]quinazoline (707 mg, 2.02 mmol, 84% yield). ¹H NMR (400 MHz, DMSO-*d*₆, ppm): δ 7.64 (s, 1H), 2.66 (s, 3H).

### Intermediate 4: Synthesis of 4-bromo-7,9-dichloro-2-methyl-2H-pyrazolo[4,3-f]quinazoline.

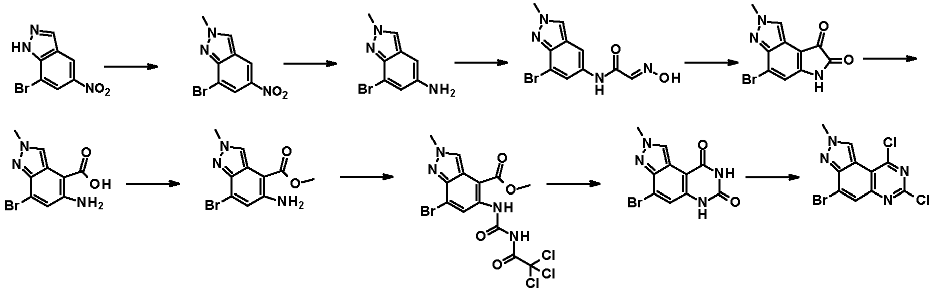

Step 1: 7-bromo-5-nitro-1H-indazole (20 g, 82.6 mmol) was dissolved in THF (200 mL), and NaH (8.3 g, 124 mmol) was added at 0 °C, and the mixture was stirred at 20 °C. After stirring at 20 °C for 30 min, Mel (35.2 g, 248 mmol) was added. The reaction mixture was stirred at 20 °C for 1 h under nitrogen. After completion of the reaction, purified water (80 mL) was added to the reaction mixture, and extracted with ethyl acetate (50 mL*3). The combined organic layers were washed with brine (40 mL*3) and dried over Na₂SO₄. The filtered solution was concentrated under reduced pressure to obtain a residue. The residue was stirred with hexane at 20 °C for 30 min, and the solid was filtered. 7-bromo-2-methyl-5-nitro-2*H*-indazole (20 g, 78.1 mmol, 94% yield) was obtained as a white solid. LCMS (ES-API, m/z): [M+H]⁺= 257.1.

Step 2: 7-bromo-2-methyl-5-nitro-2*H*-indazole (20 g, 78.1 mmol), Fe (21.8 g, 391 mmol), and NH₄Cl (5.01 g, 93.7 mmol) were added to a solution of ethanol (120 mL), THF (120 mL), and purified water (40 mL), and the mixture was stirred at 90 °C for 1 h. The reaction mixture was filtered through Celite to obtain the residue. The residue was purified by silica gel chromatography. 7-bromo-2-methyl-2*H*-indazole-5-amine (7 g, 31 mmol, 40% yield) was obtained as a yellow solid. LCMS (ES-API, m/z): [M+H]⁺= 227.1; ¹H NMR (400 MHz, CDCl₃, ppm): δ 7.73 (s, 1H), 6.85 (s,1H), 6.72 (s, 1H), 4.18 (s, 3H), 3.52 (br, 2H).

Step 3: 7-bromo-2-methyl-2*H*-indazole-5-amine (6.67 g, 29.5 mmol), chloral hydrate (9.76 g, 29.5 mmol), Na₂SO₄ (33.5 g, 236 mmol) were dissolved in purified water (70 mL), and HCl (4 mL) was slowly added dropwise. The mixture was stirred at 90 °C for 30 minutes. Then, *N*-chlorohydroxylamine (11.9 g, 177 mmol) was added and stirred at 90 °C for 1 hour. After cooling the reaction mixture to room temperature, purified water (70 mL) was added and stirred for 10 minutes. The solid was precipitated and then filtered. The filter cake was concentrated in vacuo to obtain a black solid *N*-(7-bromo-2-methyl-2*H*-indazole-5-yl)-2-(*N*-hydroxyamino)acetamide (8.77 g, 29.5 mmol, 100% yield, crude compound). LCMS (ES-API, m/z): [M+H]⁺= 298.1.

Step 4: Concentrated sulfuric acid (60 mL) was slowly added dropwise to *N*-(7-bromo-2-methyl-2*H*-indazole-5-yl-2-(*N*-hydroxyamino)acetamide (9 g, 30.3 mmol), and the reaction mixture was stirred at 90 °C for 30 min. After the reaction mixture was cooled to room temperature, ice was added, and the mixture was stirred for 30 min. The solid was precipitated and then filtered. The solid was dried to obtain black 4-bromo-2-methyl-2*H*,6*H*,7*H*,8*H*-pyrolo[3,2-e]indazole-7,8-dione (5.1 g, 18.2 mmol, 60% yield). LCMS (ES-API, m/z): [M+H]⁺= 281.1; ¹H NMR (400 MHz, CD₃OD, ppm): δ 8.30 (s, 1H), 8.14 (s, 1H), 7.74 (s, 1H), 7.61 (s, 1H), 4.24 (s, 3H).

Step 5: 4-bromo-2-methyl-2*H*,6*H*,7*H*,8*H*-pyrolo[3,2-e]indazole-7,8-dione (5.1 g, 18.2 mmol) was dissolved in 2 N NaOH aqueous solution (7.28 g, 182 mmol), and H₂O₂ (3.1 g, 91 mmol) was slowly added dropwise, followed by stirring for 1 h. Acetic acid was added to the reaction mixture at 0 °C to adjust the pH to 3, and the precipitated solid was filtered. The solid was dried to obtain black 5-amino-7-bromo-2-methyl-2H-indazole-4-carboxylic acid (3.08 g, 11.4 mmol, 63% yield). LCMS (ES-API, m/z): [M+H]⁺= 271.1; ¹H NMR (400 MHz, DMSO-*d₆*, ppm): δ 10.81 (s, 1H), 8.58 (s, 1H), 7.25 (s, 1H), 4.19 (s, 3H).

Step 6: 5-amino-7-bromo-2-methyl-2*H*-indazole-4-carboxylic acid (962 mg, 3.56 mmol) was dissolved in methanol (14 mL) and toluene (42 mL), and 2 M (diazomethyl)trimethylsilane (488 mg, 4.27 mmol) was slowly added dropwise at 0 °C. The mixture was heated to room temperature and stirred for 5 minutes. The compound was extracted with ethyl acetate (200 mL*3), and the organic layer was dried over Na₂SO₄, filtered, and concentrated. The residue was purified by silica gel chromatography. Methyl 5-amino-7-bromo-2-methyl-2*H*-indazole-4-carboxylate (283 mg, 0.996 mmol, 28% yield) was obtained. LCMS (ES-API, m/z): [M+H]⁺= 285.1; ¹H NMR (400 MHz, CD₃OD, ppm): δ 8.16 (s, 1H), 7.17 (s, 1H), 4.14 (s, 3H), 3.93 (s, 3 H).

Step 7: Methyl 5-amino-7-bromo-2-methyl-2*H*-indazole-4-carboxylate (276 mg, 0.971 mmol) was dissolved in THF (13 mL), and trichloroethanecarbonylisocyanate (220 mg, 1.17 mmol) was added dropwise at 0 °C and reacted for 5 minutes. Most of the solvent was removed under reduced pressure, and the residue was suspended in ether (10 mL). The solid was filtered and dried under reduced pressure. Methyl 7-bromo-2-methyl-5[[(2,2,2-trichloroacetyl)carbamoyl]amino]-2*H*-indazole-4-carboxylate (459 mg, 0.971 mmol, 100% yield) was obtained as a white solid. LCMS (ES-API, m/z): [M+H]⁺= 473.5.

Step 8: Methyl 7-bromo-2-methyl-5[[(2,2,2-trichloroacetyl)carbamoyl]amino]-2*H-*indazole-4-carboxylate (459 mg, 0.971 mmol) obtained in Step 7 was dissolved in 7N NH₄ in Methanol (100 mL). The resulting suspension was refluxed at 110 °C for 36 h under nitrogen. After the reaction was completed, cooled to 20 °C, the solvent was removed with nitrogen, and the mixture was filtered. The filtered solid was washed with ether (200 mL) and dried to obtain white 4-bromo-2-methyl-2*H*,6*H*,7*H*,8*H*,9*H*-pyrazolo[4,3-*f*]quinazoline-7,9-dione (287 mg, 100% yield, crude compound). LCMS (ES-API, m/z): [M+H]⁺= 296.1; ¹H NMR (400 MHz, DMSO-*d₆*, ppm): δ 8.75 (s, 1H), 7.42 (s, 1H).

Step 9: POCl₃ (5.9 g, 38.9 mmol) was added dropwise to 4-bromo-2-methyl-2*H*,6*H*,7*H*,8*H*,9*H*-pyrazolo[4,3-*f*]quinazoline-7,9-dione (287 mg, 0.973 mmol) obtained in Step 8. Then, DIPEA (377 mg, 2.92 mmol) was slowly added dropwise, and the mixture was filled with nitrogen and stirred at 110 °C for 20 h. After the reaction was completed, the solvent and most of the POCl₃ were removed under vacuum at 40 °C, ethyl acetate (100 mL) was added, and the mixture was basified to pH 7-8 with saturated Na₂CO₃ solution at 0 °C. The compound was extracted with ethyl acetate (200 mL*3), and then the organic layer was dried over Na₂SO₄, filtered, and concentrated. The residue was purified by silica gel chromatography to obtain 4-bromo-7,9-dichloro-2-methyl-2H-pyrazolo[4,3-*f*]quinazoline (124 mg, 0.374 mmol, 38% yield). LCMS (ES-API, m/z): [M+H]⁺= 333.0.

### Intermediate 5: Synthesis of 4-bromo-7,9-dichloro-5-fluoro-2-methyl-2H-pyrazolo[4,3-f]quinazoline.

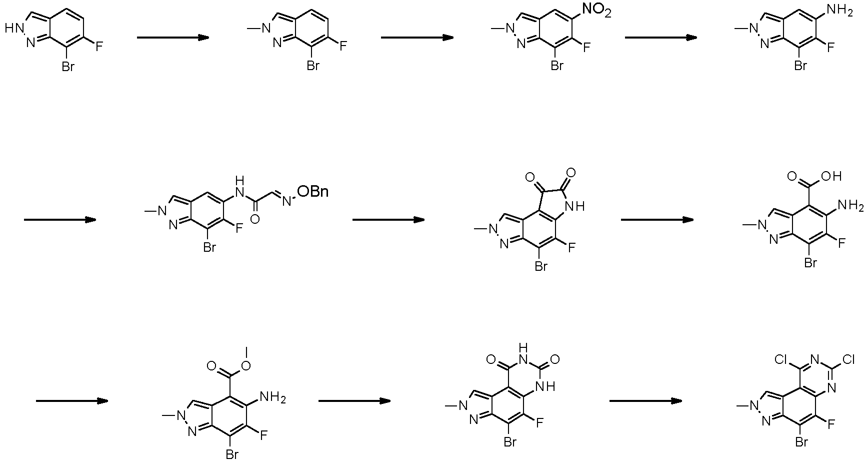

Step 1: 7-Bromo-6-fluoro-2*H*-indazole (40.0 g, 186 mmol) was dissolved in ethyl acetate (500 mL), and Me₃OBF₄ (41.3 g, 279 mmol) was added. The mixture was stirred at room temperature for 12 h. The reaction was quenched with purified water (500 mL) and extracted with ethyl acetate (500 mL*2). The extracted organic layer was washed with saturated NaCl aqueous solution, dried over anhydrous Na₂SO₄, and concentrated. The residue was purified by silica gel chromatography to obtain 7-bromo-6-fluoro-2-methyl-2*H*-indazole (37.3 g, 163 mmol, 88% yield) as a yellow solid. ¹H NMR (400 MHz, DMSO-*d*₆, ppm): δ 8.54 (s, 1H), 7.78 (dd, J = 5.2, 8.8 Hz, 1H), 7.05 (t, *J* = 9.2 Hz, 1H), 4.19 (s, 3H).

Step 2: After H₂SO₄ (370 mL) was cooled to 0 °C, 7-bromo-6-fluoro-2-methyl-2H-indazole (37.0 g, 162 mmol) was added dropwise. KNO₃ (19.8 g, 1956 mmol) was slowly added dropwise to the reaction mixture at 0 °C and stirred at room temperature for 12 h. After confirming the reaction, the reaction was quenched with cold purified water and slowly neutralized with NaHCO₃ aqueous solution at 0 °C. The organic layer was extracted with ethyl acetate (500 mL*3) and washed with saturated NaCl aqueous solution. The organic layer was dried over anhydrous Na₂SO₄ and concentrated to obtain 7-bromo-6-fluoro-2-methyl-5-nitro-2*H*-indazole (38.0 g, 139 mmol, 86% yield) as a yellow solid. ¹H NMR (400 MHz, DMSO-*d*₆, ppm): δ 8.91 (s, 1H), 8.85 (d, *J* = 7.2 Hz, 1H), 4.26 (s, 3H).

Step 3: 7-bromo-6-fluoro-2-methyl-5-nitro-2*H*-indazole (38.0 g, 139 mmol) and NH₄Cl (59.3 g, 1.11 mol) were dissolved in EtOH (400 mL) and H₂O (200 mL), and Fe (62.0 g, 1.11 mol) was added dropwise, and stirred at 80 °C for 2 h. After confirming the reaction, the produced solid was filtered, washed with EtOH, and dried. The mixture was purified by silica gel chromatography to obtain 7-bromo-6-fluoro-2-methyl-2*H*-indazol-5-amine (30.0 g, 123 mmol, 89% yield) as a yellow solid.

Step 4: 2-((benzyloxy)imino)acetic acid (34.5 g, 193 mmol) was dissolved in DMF (245 mL), and HATU (54.9 g, 144 mmol), DIPEA (99.6 g, 770 mmol), and 7-bromo-6-fluoro-2-methyl-2*H*-indazol-5-amine (23.5 g, 96.3 mmol) were added dropwise. The reaction mixture was stirred at room temperature for 2 h and the reaction was quenched with cold purified water. The organic layer was extracted with ethyl acetate (400 mL*2), washed with saturated NaCl aqueous solution, dried over anhydrous Na₂SO₄, and concentrated. The product was solidified with petroleum ether/EtOAc = 5: 1 to obtain (E)-2-((benzyloxy)imino)-N-(7-bromo-6-fluoro-2-methyl-2*H*-indazol-5-yl)acetamide (35.5 g, 87.6 mmol, 91% yield) as a yellow solid. LCMS (ES-API, m/z): [M+H]⁺= 407.0; ¹H NMR (400 MHz, DMSO-*d*₆, ppm): δ 10.10 (s, 1H), 8.54 (s, 1H), 8.12 (d, *J* = 7.2 Hz, 1H), 7.94 (s, 1H), 7.54 - 7.27 (m, 5H), 5.27 (s, 2H), 4.18 (s, 3H).

Step 5: (*E*)-2-((Benzyloxy)imino)-*N*-(7-bromo-6-fluoro-2-methyl-2*H*-indazol-5-yl)acetamide (34.0 g, 83.9 mmol) was dissolved in H₂SO₄ (680 mL) and stirred at 90 °C for 2 h. After confirming the reaction, it was cooled and the reaction was quenched with cold purified water. The organic layer was extracted with ethyl acetate (1.00 L*5) and freeze-dried. Then, it was solidified with MeOH to obtain 4-bromo-5-fluoro-2-methyl-2,6-dihydropyrrolo[3,2-e]indazole-7,8-dione (11.0 g, 36.9 mmol, 44% yield) as a red solid. LCMS (ES-API, m/z): [M+H]⁺= 320.1; ¹H NMR (400 MHz, DMSO-*d*₆, ppm): δ 11.42 (s, 1H), 8.63 (s, 1H), 4.18 (s, 3H).

Step 6: 4-bromo-5-fluoro-2-methyl-2,6-dihydropyrrolo[3,2-e]indazole-7,8-dione (10.5 g, 35.2 mmol) and NaOH (14.1 g, 352 mmol) were dissolved in 1,4-dioxane (200 mL). H₂O₂ (20.5 g, 180 mmol) was added dropwise to the reaction mixture at 0 °C and stirred at room temperature for 1 h. After confirming the reaction, the reaction was quenched with Na₂SO₃ aqueous solution (500 mL) and concentrated under reduced pressure. The pH was adjusted to 4 with 6 N HCl aqueous solution, and the produced yellow solid was filtered and dried. 5-amino-7-bromo-6-fluoro-2-methyl-2H-indazole-4-carboxylic acid (8.80 g, 30.6 mmol, 86.7% yield) was obtained as a yellow solid. LCMS (ES-API, m/z): [M+H]⁺= 287.9; ¹H NMR (400 MHz, DMSO-*d*₆, ppm): δ 8.26 (s, 1H), 4.09 (s, 3H).

Step 7: 5-Amino-7-bromo-6-fluoro-2-methyl-2*H*-indazole-4-carboxylic acid (9.00 g, 31.2 mmol) and K₂CO₃ (8.64 g, 62.5 mmol) were dissolved in DMF (90.0 mL), and CH₃I (5.10 g, 35.9 mmol) was slowly added dropwise. The reaction solution was stirred at room temperature for 2 h. After confirming the reaction, the solution was quenched with NH₄Cl aqueous solution (500 mL) and the organic layer was extracted with ethyl acetate (300 mL*3). Drying over anhydrous Na₂SO₄ and concentration were performed to obtain methyl 5-amino-7-bromo-6-fluoro-2-methyl-2*H*-indazole-4-carboxylate (10.0 g, crude mixture) as a yellow solid. LCMS (ES-API, m/z): [M+H]⁺= 303.9; ¹H NMR (400 MHz, DMSO-*d*₆, ppm): δ 8.31 (s, 1H), 7.15 (s, 2H), 4.11 (s, 3H), 3.89 (s, 3H).

Step 8: Methyl 5-amino-7-bromo-6-fluoro-2-methyl-2*H*-indazole-4-carboxylate (9.70 g, 32.1 mmol) was dissolved in THF (100 mL), and 2,2,2-trichloroacetyl isocyanate (12.1 g, 64.2 mmol) was slowly added dropwise at 0 °C. The reaction mixture was stirred at room temperature for 2 hours, and concentrated after the reaction was confirmed. The resulting white solid was dissolved in NH₃·H₂O (91.0 g, 727 mmol) and MeOH (100 mL), and stirred at 40 °C for 2 hours. The reaction solution was concentrated and solidified with MeOH (100 mL) to obtain 4-bromo-5-fluoro-2-methyl-2,6-dihydro-7*H*-pyrazolo[4,3-f]quinazoline-7,9(8H)-dione (9.10 g, 29.1 mmol, 90% yield) as a yellow solid. LCMS (ES-API, m/z): [M+H]⁺= 314.8; ¹H NMR (400 MHz, DMSO-*d*₆, ppm): δ 8.72 (s, 1H), 4.19 (s, 3H).

Step 9: 4-bromo-5-fluoro-2-methyl-2,6-dihydro-7*H*-pyrazolo[4,3-f]quinazoline-7,9(8H)-dione (8.10 g, 25.9 mmol) was dissolved in toluene (80.0 mL), and POCl₃ (39.7 g, 259 mmol) and DIPEA (7.36 g, 56.9 mmol) were slowly added dropwise. The reaction mixture was stirred at 115 °C for 40 h. After confirming the reaction, it was cooled with purified water, and the organic layer was extracted with ethyl acetate (500 mL*2). The extracted organic layer was dried over anhydrous Na₂SO₄ and concentrated to obtain 4-bromo-7,9-dichloro-5-fluoro-2-methyl-2*H*-pyrazolo[4,3-f]quinazoline (9.00 g, 25.12 mmol, 97% yield) as a yellow solid. LCMS (ES-API, m/z): [M+H]⁺= 350.8; ¹H NMR (400 MHz, CDCl₃, ppm): δ 8.80 (s, 1H), 4.37 (s, 3H).

### Intermediate 6: Synthesis of (1-(morpholinomethyl)cyclopropyl)methanol

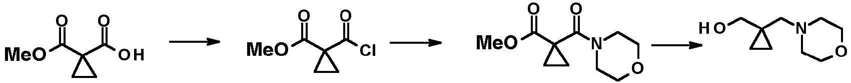

Step 1: 1-(Methoxycarbonyl)cyclopropane-1-carboxylic acid (1 g, 6.94 mmol) was dissolved in DCM (10 mL), and oxalyl chloride (1.76 g, 13.88 mmol) and DMF (50.72 mg, 694.00 µmol) were slowly added dropwise at 0 °C. The reaction mixture was stirred at room temperature under nitrogen for 3 h. Methyl 1-(chlorocarbonyl)cyclopropane-1-carboxylate (1.1 g, crude mixture) was obtained as a yellow oil without purification.

Step 2: Methyl 1-(chlorocarbonyl)cyclopropane-1-carboxylate (1.1 g, 6.77 mmol) obtained in Step 1 was dissolved in THF (22 mL), and TEA (1.37 g, 13.53 mmol) was added dropwise at 0 °C. Then, morpholine (884.22 mg, 10.15 mmol) was added, and the mixture was stirred at room temperature for 16 h. After completion of the reaction, purified water was added, and the mixture was extracted with ethyl acetate. The residue was purified by silica gel chromatography to obtain methyl 1-(morpholine-4-carbonyl)cyclopropane-1-carboxylate (650 mg, 3.05 mmol, 45% yield) as a yellow oil. ¹H NMR (400 MHz, DMSO-*d*₆, ppm): δ 3.74 (s, 3H), 3.71 - 3.63 (m, 6H), 3.53 - 3.49 (m, 2H), 1.53 - 1.48 (m, 2H), 1.36 - 1.32 (m, 2H).

Step 3: Methyl 1-(morpholine-4-carbonyl)cyclopropane-1-carboxylate (650 mg, 3.05 mmol) was dissolved in THF (13 mL), and LAH (289.25 mg, 7.62 mmol) was added dropwise at 0 °C. The reaction mixture was stirred at room temperature for 16 h under nitrogen. Purified water was slowly added to quench the reaction, and a saturated NaOH aqueous solution was added. The reaction mixture was filtered and the filtrate was concentrated. (1-(morpholinomethyl)cyclopropyl)methanol (520 mg, crude mixture) was obtained as a yellow oil. ¹H NMR (400 MHz, DMSO-*d*₆, ppm): δ 3.75 - 3.69 (m, 4H), 3.58 - 3.52 (m, 2H), 2.75 - 2.53 (m, 4H), 2.51 - 2.43 (m, 2H), 0.55 - 0.49 (m, 2H), 0.40 - 0.34 (m, 2H).

### Intermediate 7: (R)-(1-((3-methylmorpholino)methyl)cyclopropyl)methanol.

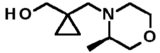

The synthesis was carried out using (*R*)-3-methylmorpholine in the same manner as Intermediate 6 to obtain (*R*)-(1-((3-methylmorpholino)methyl)cyclopropyl)methanol (843 mg, 70% yield) as a colorless oil. ¹H NMR (400 MHz, DMSO-*d₆*, ppm): δ 0.18 - 0.25 (m, 1H), 0.26 - 0.34 (m, 1H), 0.47 - 0.54 (m, 1H), 0.73 (dt, *J* = 9.2, 4.8 Hz, 1H), 1.06 (d, *J* = 6.0 Hz, 3H), 1.76 - 1.85 (m, 1H), 2.25 - 2.32 (m, 1H), 2.41 - 2.51 (m, 1H), 3.12 (br d, *J* = 11.2 Hz, 1H), 3.19 - 3.40 (m, 3H), 3.59 - 3.74 (m, 3H), 3.80 (dt, *J* = 11.6, 3.2Hz, 1H), 3.96 (br d, *J* = 11.2Hz, 1H).

### Intermediate 8: Synthesis of (1-((1,1-difluoro-6-azaspiro[2.5]octan-6-yl)methyl)cyclopropyl)methanol.

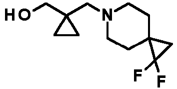

The synthesis was carried out using 1,1-difluoro-6-azaspiro[2.5]octane in the same manner as Intermediate 6 to obtain (1-((1,1-difluoro-6-azaspiro[2.5]octan-6-yl)methyl)cyclopropyl)methanol (820 mg, crude mixture) as a yellow oil. ¹H NMR (400 MHz, DMSO-*d₆*, ppm): δ 3.64 - 3.62 (m, 2H), 3.57 - 3.54 (m, 2H), 2.82 - 2.68 (m, 2H), 2.54 - 2.40 (m, 4H), 1.09 - 1.03 (m, 2H), 0.54 - 0.52 (m, 4H), 0.39 - 0.35 (m, 2H).

### Intermediate 9: Synthesis of (1-(((1R,4R)-2-oxa-5-azabicyclo[2.2.1]heptan-5-yl)methyl)cyclopropyl)methanol.

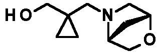

The synthesis was carried out using (1*R*,4*R*)-2-oxa-5-azabicyclo[2.2.1]heptane in the same manner as Intermediate 6 to obtain (1-(((1R,4R)-2-oxa-5-azabicyclo[2.2.1]heptan-5-yl)methyl)cyclopropyl)methanol (470 mg, crude mixture) as a yellow oil. ¹H NMR (400 MHz, CDCl₃, ppm): δ 4.41 (s, 1H), 3.95 (d, *J* = 8.0 Hz, 1H), 3.69 - 3.80 (m, 2H), 3.64 (dd, *J* = 8.0, 1.6 Hz, 1H), 3.39 (d, *J* = 11.2 Hz, 1H), 3.06 (dd, *J* = 10.4, 1.6 Hz, 1H), 2.90 (d, *J* = 12.6 Hz, 1H), 2.55 - 2.64 (m, 2H), 1.73 - 1.82 (m, 2H), 0.51 - 0.60 (m, 1H), 0.41 - 0.49 (m, 2H), 0.25 - 0.35 (m, 1H).

### Intermediate 10: Synthesis of (R)-(1-((2-methylmorpholino)methyl)cyclopropyl)methanol.

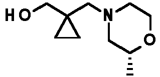

The synthesis was carried out using (*R*)-2-Methylmorpholine in the same manner as Intermediate 6 to obtain (*R*)-(1-((2-methylmorpholino)methyl)cyclopropyl)methanol (760 mg, crude mixture) as a yellow oil. ¹H NMR (400 MHz, CDCl₃, ppm): δ 5.97 - 4.68 (m, 1H), 3.92 - 3.83 (m, 1H), 3.69 - 3.59 (m, 2H), 3.57 - 3.52 (m, 2H), 3.09 - 2.95 (m, 2H), 2.51 - 2.43 (m, 2H), 2.16 - 2.04 (m, 1H), 1.82 - 1.73 (m, 1H), 1.20 - 1.13 (m, 3H), 0.55 - 0.50 (m, 2H), 0.40 - 0.34 (m, 2H).

### Intermediate 11: (1-(((1S,4S)-2-oxa-5-azabicyclo[2.2.1]heptan-5-yl)methyl)cyclopropyl) methanol.

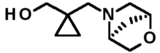

The synthesis was carried out using (1*S*,4*S*)-2-oxa-5-azabicyclo[2.2.1]heptane in the same manner as Intermediate 6 to obtain (1-(((1*S*,4*S*)-2-oxa-5-azabicyclo[2.2.1]heptan-5-yl)methyl)cyclopropyl)methanol (542 mg, crude mixture) as a yellow oil. LCMS (ES-API, m/z): [M+H]⁺= 200.2.

### Intermediate 12: (S)-(1-((3-fluoropiperidin-1-yl)methyl)cyclopropyl)methanol.

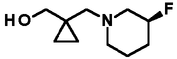

The synthesis was carried out using (S)-3-fluoropiperidine in the same manner as Intermediate 6 to obtain (*S*)-(1-((3-fluoropiperidin-1-yl)methyl)cyclopropyl)methanol (352 mg, crude mixture) as a colorless oil. LCMS (ES-API, m/z): [M+H]⁺= 174.2; ¹H NMR (400 MHz, CDCl₃ , ppm): δ 5.52 - 4.95 (m, 1H), 4.77 - 4.56 (m, 1H), 3.63 (d, *J* = 11.2 Hz, 1H), 3.46 (d, *J* = 11.2 Hz, 1H), 2.90 - 2.64 (m, 2H), 2.58 (br d, *J =* 12.4 Hz, 2H), 2.51 - 2.36 (m, 2H), 1.94 - 1.75 (m, 2H), 1.73 - 1.66 (m, 1H), 1.55 (br dd, *J* = 3.6, 8.4 Hz, 1H), 0.60 - 0.48 (m, 2H), 0.40 - 0.31 (m, 2H).

### Intermediate 13: Synthesis of (1-(pyrrolidin-1-ylmethyl)cyclopropyl)methanol.

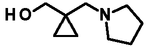

The synthesis was carried out using pyrrolidine in the same manner as Intermediate 6 to obtain (1-(pyrrolidin-1-ylmethyl)cyclopropyl)methanol (328 mg, 2.11 mmol, 67% yield) as a yellow oil. LCMS (ES-API, m/z): [M+H]⁺= 174.2; ¹H NMR (400 MHz, CDCl₃ , ppm): *δ* 6.25 - 5.82 (m, 1H), 3.56 (s, 2H), 2.65 - 2.58 (m, 6H), 1.77 (td, *J* = 3.2, 6.8 Hz, 4H), 0.52 - 0.47 (m, 2H), 0.40 - 0.34 (m, 2H).

### Intermediate 14: Synthesis of (R)-(1-((3-fluoropyrrolidin-1-yl)methyl)cyclopropyl)methanol.

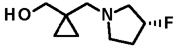

The synthesis was carried out using (*R*)-3-Fluoropyrrolidine in the same manner as Intermediate 6, obtaining (*R*)-(1-((3-fluoropyrrolidin-1-yl)methyl)cyclopropyl)methanol (837 mg, crude mixture) as a colorless oil. LCMS (ES-API, m/z): [M+H]⁺= 174.2; ¹H NMR (400 MHz, DMSO-*d₆*, ppm): δ 0.19 - 0.26 (m, 2 H), 0.33 - 0.43 (m, 2 H), 1.74 - 1.92 (m, 1 H), 1.99 - 2.19 (m, 1 H), 2.21 - 2.36 (m, 2 H), 2.39 - 2.45 (m, 1 H), 2.51 - 2.63 (m, 1 H), 2.73 - 2.90 (m, 2 H), 3.28 (br s, 1 H), 3.32 - 3.50 (m, 1 H), 4.47 (br s, 1 H), 5.01 - 5.29 (m, 1 H).

### Intermediate 15: Synthesis of (R)-(1-((3-fluoropyrrolidin-1-yl)methyl)cyclobutyl)methanol.

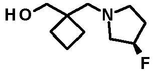

The synthesis was carried out using (*R*)-3-Fluoropyrrolidine in the same manner as Intermediate 6 to obtain (*R*)-(1-((3-fluoropyrrolidin-1-yl)methyl)cyclobutyl)methanol (1.8 g, crude mixture) as a yellow solid. ¹H NMR (400 MHz, DMSO-*d₆*, ppm): δ 5.82 - 4.99 (m, 2H), 3.80 (s, 2H), 3.03 - 2.66 (m, 5H), 2.59 - 2.47 (m, 1H), 2.18 - 1.92 (m, 3H), 1.90 - 1.77 (m, 5H).

### Intermediate 16: Synthesis of ((2S,7aR)-2-fluoro-6-methylenetetrahydro-1H-pyrrolizin-7a(5H)-yl)methanol.

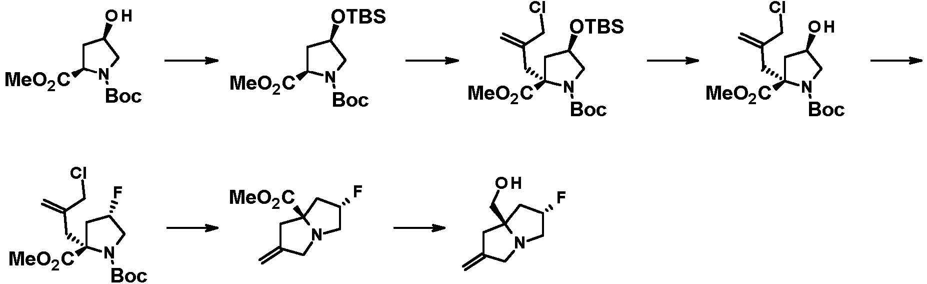

Step 1: 1-(*tert*-Butyl) 2-methyl (2*R*,4*R*)-4-hydroxypyrrolidine-1,2-dicarboxylate (2.5 g, 10.19 mmol) was dissolved in DCM (25 mL), imidazole (1.73 g, 25.48 mmol) was added, and the mixture was stirred at 0 °C for 10 min. Then, TBSCI (1.84 g, 12.23 mmol) was slowly added, and the mixture was stirred at room temperature for 12 h. After the reaction was completed, the product was purified by silica gel chromatography to obtain 1-(*tert*-butyl) 2-methyl (2*R*,4*R*)-4-((*tert*-butyldimethylsilyl)oxy)pyrrolidine-1,2-dicarboxylate (2.68 g, 7.31 mmol, 72% yield) as a colorless oil. ¹H NMR (400 MHz, DMSO-*d₆*, ppm): δ 4.38 (br dd, *J* = 3.6, 8.4 Hz, 1H), 4.32 - 4.22 (m, 1H), 3.66 - 3.56 (m, 3H), 3.55 - 3.46 (m, 1H), 3.15 - 3.05 (m, 1H), 2.42 - 2.25 (m, 1H), 1.92 - 1.82 (m, 1H), 1.43 - 1.31 (m, 9H), 0.84 - 0.79 (m, 9H), 0.08 - 0.04 (m, 6H).

Step 2: 1-(tert-Butyl) 2-methyl (2R,4R)-4-((tert-butyldimethylsilyl)oxy)pyrrolidine-1,2-dicarboxylate (2.18 g, 6.06 mmol) was dissolved in THF (21 mL), and LiHMDS (1 M, 12.13 mL) was added at -78 °C under nitrogen. The reaction mixture was stirred at -78 °C for 15 min. Then, 3-chloro-2-(chloromethyl)prop-1-ene (1.89 g, 15.16 mmol) was added to the reaction mixture and stirred at room temperature for 2 h. After completion of the reaction, the product was purified by silica gel chromatography to obtain 1-(*tert*-butyl) 2-methyl (2*R*,4*R*)-4-((*tert*-butyldimethylsilyl)oxy)-2-(2-(chloromethyl)allyl)pyrrolidine-1,2-dicarboxylate (2.62 g, 5.55 mmol, 91% yield) as a yellow oil. ¹H NMR (400 MHz, CDCl₃, ppm): δ 5.41 (d, *J = 1.2* Hz, 1H), 5.16 - 5.06 (m, 1H), 4.49 - 4.20 (m, 1H), 4.14 - 4.00 (m, 2H), 3.78 - 3.69 (m, 3H), 3.69 - 3.53 (m, 1H), 3.37 - 3.01 (m, 2H), 2.83 - 2.60 (m, 1H), 2.36 - 2.17 (m, 1H), 2.16 - 2.06 (m, 1H), 1.50 - 1.39 (m, 9H), 0.91 - 0.83 (m, 9H), 0.09 - 0.00 (m, 6H).

Step 3: 1-(*tert*-butyl) 2-methyl (2*R*,4*R*)-4-((*tert*-butyldimethylsilyl)oxy)-2-(2-(chloromethyl)allyl)pyrrolidine-1,2-dicarboxylate (4 g, 8.93 mmol) was dissolved in THF (40 mL), TBAF·3H₂O (1 M, 10.71 mL) was added, and the mixture was stirred at room temperature for 12 h. After the reaction was completed, the reaction mixture was purified by silica gel chromatography. 1-(*tert*-butyl) 2-methyl (2*R*,4*R*)-2-(2-(chloromethyl)allyl)-4-hydroxypyrrolidine-1,2-dicarboxylate (2.9 g, 7.82 mmol, 88% yield) was obtained as a colorless oil. ¹H NMR (400 MHz, CDCl₃, ppm): δ 5.42 (s, 1H), 5.06 (s, 1H), 4.17 - 4.27 (m, 1H), 4.04 - 3.93 (m, 2H), 3.92 - 3.73 (m, 4H), 3.57 (d, *J* = 10.8 Hz, 1H), 3.44 - 3.24 (m, 2H), 2.60 (t, *J =* 13.6 Hz, 1H), 2.53 - 2.37 (m, 1H), 2.15 - 2.03 (m, 2H), 1.47 (s, 9H).

Step 4: 1-(*tert*-Butyl) 2-methyl (2*R*,4*R*)-2-(2-(chloromethyl)allyl)-4-hydroxypyrrolidine-1,2-dicarboxylate (2.9 g, 8.69 mmol) was dissolved in DCM (30 mL), and BAST (2.88 g, 13.03 mmol, 2.85 mL) was slowly added dropwise at -78 °C. The reaction mixture was stirred at room temperature for 12 h. After the reaction was completed, the reaction mixture was purified by silica gel chromatography. 1-(*tert*-butyl) 2-methyl (2*R*,4*S*)-2-(2-(chloromethyl)allyl)-4-fluoropyrrolidine-1,2-dicarboxylate (1.82 g, 4.88 mmol, 56% yield) was obtained as a pale yellow oil. ¹H NMR (400 MHz, CDCl₃, ppm): δ 5.45 - 5.32 (m, 1H), 5.30 - 5.05 (m, 2H), 4.18 - 4.11 (m, 2H), 4.07 - 3.74 (m, 2H), 3.74 - 3.64 (m, 3H), 3.38 - 3.15 (m, 1H), 2.85 (d, *J* = 14.4 Hz, 1H), 2.67 - 2.23 (m, 2H), 1.46 (s, 9H).

Step 5: 1-(*tert*-Butyl) 2-methyl (2*R*,4*S*)-2-(2-(chloromethyl)allyl)-4-fluoropyrrolidine-1,2-dicarboxylate (1.8 g, 5.36 mmol) was dissolved in DCM (15 mL), and TFA (7.68 g, 67.31 mmol, 5 mL) was slowly added dropwise at 0 °C. The reaction mixture was stirred at room temperature for 2 h. After confirming that the starting material disappeared, 7 M NH₄ in MeOH was added. The reaction was quenched by adding water, and extracted with DCM. The compound was purified by silica gel chromatography to obtain methyl (2*S*,7a*R*)-2-fluoro-6-methylenetetrahydro-1*H*-pyrrolizine-7a(5H)-carboxylate (950 mg, 4.29 mmol, 80% yield) as a pale yellow oil. LCMS (ES-API, m/z): [M+H]⁺= 236.0; ¹H NMR (400 MHz, CDCl₃, ppm): δ 5.38 - 5.13 (m, 1H), 4.94 (dt, *J* = 14.8, 2.0 Hz, 2H), 3.89 (br d, *J* = 13.6 Hz, 1H), 3.73 (s, 3H), 3.56 (br d, *J* = 14.0 Hz, 1H), 3.46 - 3.27 (m, 1H), 3.22 - 3.00 (m, 2H), 2.76 (br d, *J* = 16.0 Hz, 1H), 2.64 - 2.54 (m, 1H), 2.38 - 2.21 (m, 1H).

Step 6: Methyl (2*S*,7a*R*)-2-fluoro-6-methylenetetrahydro-1*H*-pyrrolizine-7a(5*H*)-carboxylate (940 mg, 4.72 mmol) was dissolved in THF (10 mL), LAH (2.5 M, 1.89 mL) was added at 0 °C under nitrogen, and the mixture was stirred at room temperature for 2 h. When the starting material was consumed, the reaction was quenched with water and 15% aqueous NaOH. ((2*S*,7a*R*)-2-fluoro-6-methylenetetrahydro-1*H*-pyrrolizin-7a(5*H*)-yl)methanol (720 mg, 3.78 mmol, 80% yield) was obtained as a white oil. ¹H NMR (400 MHz, CDCl₃, ppm): δ 5.33 - 5.10 (m, 1 H), 4.97 - 4.85 (m, 2 H), 3.72 (br d, *J =* 14.0 Hz, 1 H), 3.53 (br d, *J* = 14.0 Hz, 1 H), 3.34 - 3.02 (m, 4 H), 2.68 (br d, *J* = 15.6 Hz, 1 H), 2.36 (dt, *J* = 15.6, 1.60 Hz, 1 H), 2.25 - 2.18 (m, 1 H), 2.17 -2.02 (m, 2 H).

### Intermediate 17: Synthesis of ((5S,7aS)-5-(methoxymethyl)-2-methylenetetrahydro-1H-pyrrolizin-7a(5H)-yl)methanol.

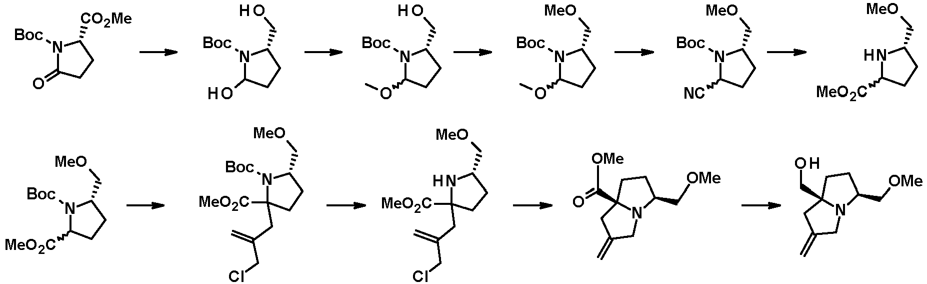

Steps 1,2: 1-(*tert*-butyl) 2-methyl (*S*)-5-oxopyrrolidine-1,2-dicarboxylate (40 g, 164.44 mmol) was dissolved in DCM (600 mL), and DIBAL-H (1 M, 500 mL) was slowly added dropwise at -68 °C, and stirred for 30 min. After that, the mixture was reacted for 2 h at room temperature. MeOH (200 mL) and HCl solution (2 M, 180 mL) were added under nitrogen at 0 °C, and stirred for 2 h at 20 °C. After completion of the reaction, 10% potassium sodium tartrate (500 mL) was added to the reaction mixture to terminate the reaction, and ethyl acetate was added to extract the organic layer, which was then dried over anhydrous MgSO₄. The reaction residue was separated by silica gel chromatography to obtain *tert*-butyl (2*S*)-2-(hydroxymethyl)-5-methoxypyrrolidine-1-carboxylate (19 g, 82.15 mmol, 50% yield) as a colorless oil. ¹H NMR (400 MHz, CDCl₃, ppm): δ 5.17 (brs, 1H), 4.03 - 4.06 (m, 1H), 3.75 - 3.77 (m, 1H), 3.52 - 3.56 (m, 1H), 3.48 (s, 1H), 3.33 (s, 3H), 1.77 - 2.05 (m, 5H), 1.49 (s, 9H).

Step 3: A solution of NaH (6.58 g, 164.44 mmol) in DMF (190 mL) was added to *tert*-butyl (2*S*)-2-(hydroxymethyl)-5-methoxypyrrolidine-1-carboxylate (19 g, 82.15 mmol) at 0 °C and stirred for 15 min. Then, Mel (23.32 g, 164.30 mmol, 10.23 mL) was added at 0 °C and stirred for 2 h at 20 °C. After completion of the reaction, purified water was added to the reaction mixture to quench the reaction, and ethyl acetate was added to extract the organic layer and dried over anhydrous MgSO₄. The product was purified by silica gel chromatography to obtain *tert*-butyl (5*S*)-2-methoxy-5-(methoxymethyl)pyrrolidine-1-carboxylate (10.7 g, 43.62 mmol, 53% yield) as a yellow oil. ¹H NMR (400 MHz, CDCl₃, ppm): δ 5.21 (brs, 1H), 3.97 (brs, 1H), 3.75 (brs, 1H), 3.38 (s, 3H), 3.30 (s, 3H), 2.07 - 2.18 (m, 1H), 1.71 - 2.01 (m, 3H), 1.49 (s, 9H).

Step 4: *tert*-Butyl (5*S*)-2-methoxy-5-(methoxymethyl)pyrrolidine-1-carboxylate (10.7 g, 43.62 mmol) was dissolved in DCM (100 mL), and TMSOTf (969.44 mg, 4.36 mmol) was added dropwise at 0 °C and stirred for 15 minutes. TMSCN (6.49 g, 65.43 mmol, 8.19 mL) was slowly added dropwise and reacted at 0 °C for 2 hours. After completion of the reaction, purified water was added to the reaction mixture to quench the reaction, and ethyl acetate was added to extract the organic layer, which was then dried over anhydrous MgSO₄. The product was purified by silica gel chromatography to obtain *tert*-butyl (5*S*)-2-cyano-5-(methoxymethyl)pyrrolidine-1-carboxylate (6.3 g, 26.22 mmol, 60% yield) as a yellow oil. ¹H NMR (400 MHz, CDCl₃, ppm): δ 4.39 - 4.59 (m, 1H), 3.89 - 4.10 (m, 1H), 3.55 - 3.63 (m, 0.5H), 3.41 - 3.52 (m, 1.3H), 3.39 (s, 1.4H), 3.33 (s, 1.5H), 2.04 - 2.17 (m, 4H), 1.51 (s, 9H).

Step 5: *tert*-butyl (5*S*)-2-cyano-5-(methoxymethyl)pyrrolidine-1-carboxylate (3 g, 12.48 mmol) was added to HCl/MeOH (2 M, 30.00 mL) and reacted at 20 °C for 12 h. After the reaction was completed, purified water was added to the reaction mixture to quench the reaction, and ethyl acetate was added to extract the organic layer, which was then dried over anhydrous MgSO₄. After removal of the solvent, methyl (5S)-5-(methoxymethyl)pyrrolidine-2-carboxylate (1.5 g, 8.66 mmol, 69% yield) was obtained as a yellow oil. ¹H NMR (400 MHz, CDCl₃, ppm): δ 3.72 - 3.85 (m, 1H), 3.72 (s, 3H), 3.37 - 3.38 (m, 1H), 3.35 (s, 3H), 3.29 - 3.33 (m, 1H), 2.01 - 2.19 (m, 2H), 1.86 - 1.91 (m, 2H), 1.47 - 1.53 (m, 1H).

Step 6: Methyl (5*S*)-5-(methoxymethyl)pyrrolidine-2-carboxylate (1.5 g, 8.66 mmol) and Boc₂O (1.89 g, 8.66 mmol) were dissolved in MeOH (30 mL) and reacted at 20 °C for 12 hours. After the reaction was completed, purified water was added to the reaction mixture to quench the reaction, and ethyl acetate was added to extract the organic layer, which was then dried over anhydrous MgSO₄. The product was purified to obtain 1-(*tert*-butyl) 2-methyl (5*S*)-5-(methoxymethyl)pyrrolidine-1,2-dicarboxylate (2 g, 7.32 mmol, 84% yield) as a yellow oil. ¹H NMR (400 MHz, CDCl₃, ppm): δ 4.30 - 4.33 (m, 0.7H), 4.25 - 4.27 (s, 1H), 3.71 - 3.73 (m, 3H), 3.38 - 3.43 (m, 1H), 3.35 (d, *J* = 12Hz, 3H), 3.29 - 3.34 (m, 0.5H), 1.88 - 2.02 (m, 4H), 1.40 - 1.47 (m, 9H).

Step 7: 1-(*tert*-Butyl) 2-methyl (5*S*)-5-(methoxymethyl)pyrrolidine-1,2-dicarboxylate (2.4 g, 8.78 mmol) was dissolved in THF (24 mL), and LiHMDS (1 M, 17.56 mL) was stirred at -68 °C for 0.5 h. (2.2 g, 17.56 mmol, 2.04 mL) was added to the reaction, and the reaction was carried out at room temperature for 12 h. After the reaction was completed, purified water was added to the reaction mixture to quench the reaction, and the organic layer was extracted by adding ethyl acetate, and dried over anhydrous MgSO₄. The product was purified to obtain 1-(*tert*-butyl) 2-methyl (5*S*)-2-(2-(chloromethyl)allyl)-5-(methoxymethyl)pyrrolidine-1,2-dicarboxylate (1.2 g, 2.95 mmol, 34% yield) as a yellow oil. ¹H NMR (400 MHz, CDCl₃, ppm): δ 5.34 - 5.43 (m, 1H), 5.05 - 5.12 (m, 1H), 4.01 - 4.11 (m, 3.4H), 3.73 (s, 3H), 3.37 (s, 3H), 3.27 - 3.35 (m, 2H), 3.11 - 3.25 (m, 1H), 2.62 - 2.77 (m, 1H), 1.81 - 2.28 (m, 4H) , 1.40 - 1.52 (m, 9H).

Step 8: 1-(*tert*-Butyl) 2-methyl (5*S*)-2-(2-(chloromethyl)allyl)-5-(methoxymethyl)pyrrolidine-1,2-dicarboxylate (600 mg, 1.66 mmol) was dissolved in DCM (6 mL), TFA (2.4 mL) was added at 0 °C, and the mixture was reacted at room temperature for 2 h. After the reaction was completed, the mixture was concentrated to obtain methyl (5*S*)-2-(2-(chloromethyl)allyl)-5-(methoxymethyl)pyrrolidine-2-carboxylate (434 mg, crude mixture) as a brown oil. LCMS (ES-API, m/z): [M+H]⁺= 490.2.

Step 9: Methyl (5*S*)-2-(2-(chloromethyl)allyl)-5-(methoxymethyl)pyrrolidine-2-carboxylate (434 mg, 1.66 mmol) and K₂CO₃ (458.32 mg, 3.32 mmol) were dissolved in MeOH (6 mL) and stirred at room temperature for 3 h. After completion of the reaction, purified water was added to the reaction mixture to quench the reaction, and ethyl acetate was added to extract the organic layer, which was then dried over anhydrous MgSO₄. The product was purified to obtain methyl (5*S*,7a*S*)-5-(methoxymethyl)-2-methylenetetrahydro-1*H*-pyrrolizine-7a(5*H*)-carboxylate (180 mg, 798.99 µmol, 48% yield) as a yellow oil. LCMS (ES-API, m/z): [M+H]⁺= 226.0; ¹H NMR (400 MHz, DMSO-*d*₆, ppm): δ 4.88 (br d, *J =* 10.4 Hz, 2H), 3.58 (s, 3H), 3.53 (br d, *J* = 14.8 Hz, 1H), 3.31 - 3.28 (m, 1H), 3.27 - 3.24 (m, 1H), 3.23 (s, 3H), 3.21 - 3.16 (m, 1H), 2.78 (s, 2H), 2.39 (br dd, *J =* 1.2, 16.0 Hz, 1H), 2.32 - 2.24 (m, 1H), 1.99 - 1.88 (m, 1H), 1.76 - 1.66 (m, 1H), 1.44 (br d, *J* = 9.2 Hz, 1H).

Step 10: Methyl (5S,7aS)-5-(methoxymethyl)-2-methylenetetrahydro-1*H-*pyrrolizine-7a(5*H*)-carboxylate (234 mg, 1.04 mmol) was dissolved in THF (2.5 mL), LiAlH4 (2.5 M, 415.48 µL) was added at 0 °C, and the mixture was stirred at room temperature for 1 h. After the reaction was completed, purified water was added to the reaction mixture to quench the reaction, and ethyl acetate was added to extract the organic layer, which was dried over anhydrous MgSO₄. After removal of the solvent, ((5*S*,7a*S*)-5-(methoxymethyl)-2-methylenetetrahydro-1*H*-pyrrolizin-7a(5*H*)-yl)methanol (190 mg, 963.13 µmol, 93% yield) was obtained as a yellow oil.

### Intermediate 18: Synthesis of 6-fluoro-4-methyl-5-((triisopropylsilyl)ethynyl)naphthalen-2-amine.

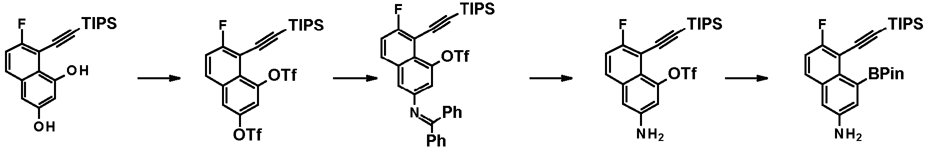

Step 1: 7-Fluoro-8-((triisopropylsilyl)ethynyl)naphthalene-1,3-diol (2.5 g, 6.97 mmol) was dissolved in DCM (17.5 mL), and then diethylamine (3.60 g, 27.89 mmol) and Tf₂O (4.13 g, 14.64 mmol) were added dropwise at 0 °C. The reaction mixture was stirred under nitrogen at 0 °C for 3 h. The reaction was quenched by adding purified water, and the reaction mixture was extracted with DCM. Purification was performed by by silica gel chromatography to obtain 7-fluoro-8-((triisopropylsilyl)ethynyl)naphthalene-1,3-diyl bis(trifluoromethanesulfonate) (3.95 g, 6.34 mmol, 91 % yield) as a yellow solid. ¹H NMR (400 MHz, DMSO-*d*₆, ppm): δ 8.55 (d, *J* = 2.4 Hz, 1H), 8.42 - 8.33 (m, 1H), 8.16 - 8.08 (m, 1H), 7.91 - 7.82 (m, 1H), 1.20 - 1.16 (m, 3H), 1.15 - 1.07 (m, 18H).

Step 2: 7-fluoro-8-((triisopropylsilyl)ethynyl)naphthalene-1,3-diyl bis(trifluoromethanesulfonate (4.6 g, 7.39 mmol) and diphenylmethaneimine (1.34 g, 7.39 mmol, 1.24 mL) obtained in Step 1 were dissolved in toluene (46 mL) and purged with nitrogen three times. Then, the mixture was stirred at 100 °C for 12 h under nitrogen. After completion of the reaction, purified water was added and extracted several times with ethyl acetate. Silica gel chromatography was performed to obtain 3-((diphenylmethylene)amino)-7-fluoro-8-((triisopropylsilyl)ethynyl)naphthalen-1-yl trifluoromethanesulfonate (3.5 g, 5.35 mmol, 72% yield) as a brown solid. ¹H NMR (400 MHz, DMSO-*d*₆, ppm): δ 7.96 - 7.90 (m, 1H), 7.75 - 7.67 (m, 3H), 7.54 (br s, 4H), 7.41 - 7.39 (m, 1H), 7.32 - 7.28 (m, 3H), 7.23 - 7.19 (m, 2H), 1.13 (br s, 21H).

Step 3: 3-((diphenylmethylene)amino)-7-fluoro-8-((triisopropylsilyl)ethynyl)naphthalen-1-yl trifluoromethanesulfonate (3 g, 4.59 mmol) obtained in Step 2 was dissolved in THF (30 mL), and HCl (1 M, 30.00 mL) was added dropwise. The reaction mixture was stirred at room temperature for 2 h. After the reaction was completed, the product was purified by silica gel chromatography to obtain 3-amino-7-fluoro-8-((triisopropylsilyl)ethynyl)naphthalen-1-yl trifluoromethanesulfonate (1.78 g, 3.35 mmol, 73 % yield). LCMS (ES-API, m/z): [M+H]⁺= 490.2; ¹H NMR (400 MHz, DMSO-*d*₆, ppm): δ 7.81 - 7.72 (m, 1H), 7.45 - 7.34 (m, 1H), 7.23 - 7.15 (m, 1H), 7.05 - 6.96 (m, 1H), 6.01 - 5.90 (m, 2H), 1.18 - 1.08 (m, 21H).

Step 4: 3-amino-7-fluoro-8-((triisopropylsilyl)ethynyl)naphthalen-1-yl trifluoromethanesulfonate (800 mg, 1.63 mmol) obtained in Step 3 was dissolved in toluene together with BPD (829.87 mg, 3.27 mmol), potassium acetate (320.72 mg, 3.27 mmol), and Pd(dppf)Cl₂ (119.56 mg, 163.40 µmol). Then, nitrogen purge was performed three times. The reaction mixture was stirred at 110 °C for 12 h under nitrogen. After silica gel chromatography purification, 6-fluoro-4-methyl-5-((triisopropylsilyl)ethynyl)naphthalen-2-amine (360 mg, 671.26 µmol, 41% yield) was obtained as a yellow solid. LCMS (ES-API, m/z): [M+H]⁺= 468.5; ¹H NMR (400 MHz, DMSO-*d*₆, ppm): *δ* 7.63 (dd, *J* = 6.0, 9.0 Hz, 1H), 7.33 - 7.24 (m, 1H), 7.23 - 7.19 (m, 1H), 6.91 - 6.85 (m, 1H), 5.54 - 5.44 (m, 2H), 1.36 - 1.29 (m, 12H), 1.13 - 1.07 (m, 21H).

### Intermediate 19: Synthesis of (2-((tert-butoxycarbonyl)amino)-7-fluorobenzo[b]thiophen-4-yl)boronic acid.

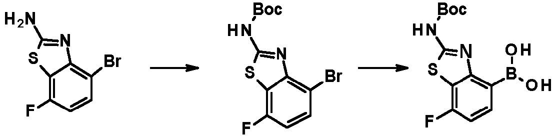

Step 1: 4-Bromo-7-fluorobenzo[*d*]thiazol-2-amine (450 mg, 1.82 mmol) was dissolved in THF (9 mL), then Boc₂O (476.97 mg, 2.19 mmol), diethylamine (353.07 mg, 2.73 mmol), and DMAP (4.45 mg, 36.42 µmol) were added, and the mixture was purged with nitrogen three times. The reaction mixture was stirred for 2 h, concentrated, and purified by silica gel chromatography to obtain *tert*-butyl (4-bromo-7-fluorobenzo[*d*]thiazol-2-yl)carbamate (500 mg, 1.40 mmol, 77% yield) as a white solid. LCMS (ES-API, m/z): [M+H]⁺= 313.2; ¹H NMR (400 MHz, CDCl₃, ppm): δ 8.99 - 8.52 (m, 1H), 7.55 (dd, *J* = 4.8, 8.6 Hz, 1H), 6.91 (t, *J* = 8.6 Hz, 1H), 1.56 (s, 8H), 1.50 - 1.47 (m, 1H).

Step 2: The compound *tert*-butyl (4-bromo-7-fluorobenzo[d]thiazol-2-yl)carbamate (250 mg, 720.04 µmol) obtained in Step 1 was dissolved in THF (2 mL), and n-BuLi (2.5 M in THF, 351.38 µL) was added dropwise at -15 °C and stirred for 30 min. Triisopropyl borate (338.55 mg, 1.80 mmol) was added at -65 °C and stirred for 30 min, then MeLi (1.6 M, 540.03 µL) was added at -65 °C and nitrogen purged 3 times. The reaction mixture was reacted at 0 °C for 1 h under nitrogen. After the reaction was completed, NH₄Cl solution and ethyl acetate were added to extract the compound. Na₂SO₄ was added to dry water and the solvent was removed under reduced pressure to obtain (2-((*tert*-butoxycarbonyl)amino)-7-fluorobenzo[b]thiophen-4-yl)boronic acid (220 mg, crude mixture) as a white solid. LCMS (ES-API, m/z): [M+H]⁺= 256.9; ¹H NMR (400 MHz, CDCl₃, ppm): δ 7.94 (dd, *J* = 6.0, 8.1 Hz, 1H), 7.06 (dd, *J* = 8.1, 9.5 Hz, 1H), 1.68 (s, 9H).

### Intermediate 20: Synthesis of tert-butyl (4-(4,4,5,5-tetramethyl-1,3,2-dioxaborolan-2-yl)benzo[d]thiazol-2-yl)carbamate.

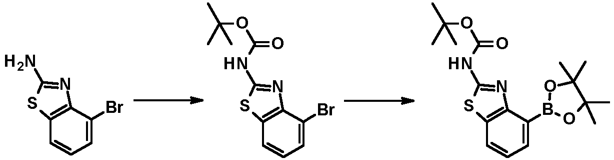

Step 1: 4-Bromobenzo[*d*]thiazol-2-amine (1 g, 4.36 mmol) was dissolved in THF (4 mL) and DCM (6 mL), and the temperature was lowered to 0 °C. At 0 °C, di-*tert*-butyl dicarbonate (1.1 mL, 4.8 mmol), TEA (1.16 mL, 8.73 mmol), and DMAP (53.3 mg, 436 µmol) were added, and stirred for 2 h. After the starting material was completely consumed, the reaction was quenched by adding water, and the organic layer was extracted with ethyl acetate. After drying over anhydrous Na₂SO₄, filtered and concentrated. The residue was purified by silica gel chromatography (10% ethyl acetate/hexane) to obtain *tert*-butyl (4-bromobenzo[*d*]thiazol-2-yl)carbamate (1.2 g, 3.65 mmol, 84% yield). LCMS (ES-API, m/z): [M+H]⁺= 330.2.

Step 2: *tert*-butyl (4-bromobenzo[*d*]thiazol-2-yl)carbamate (1.2 g, 3.65 mmol) obtained in Step 1 was dissolved in 1,4-dioxane (10 mL), then potassium acetate (1.07 g, 10.9 mmol), 4,4,5,5-tetramethyl-2-(4,4,5,5-tetramethyl-1,3,2-dioxaborolan-2-yl)-1,3,2-dioxaborolane (1.85 g, 7.29 mmol), [1,1'-Bis(diphenylphosphino)ferrocene]dichloropalladium(II)·DCM complex (267 mg, 365 µmol) were added, and the temperature was raised to 100 °C and stirred for 24 h. After the reaction was completed, the reaction temperature was lowered to room temperature. After removing the palladium catalyst by Celite filter, the organic layer was extracted with ethyl acetate. After drying over anhydrous Na₂SO₄, it was concentrated under reduced pressure. The residue was purified by silica gel chromatography (10% ethyl acetate/hexane) to obtain *tert*-butyl (4-(4,4,5,5-tetramethyl-1,3,2-dioxaborolan-2-yl)benzo[*d*]thiazol-2-yl)carbamate (1.2 g, 3.19 mmol, 88% yield). LCMS (ES-API, m/z): [M+H]⁺= 376.7.

### Intermediate 21: Synthesis of (6-methyl-1-(tetrahydro-2H-pyran-2-yl)-5-(trifluoromethyl)-1H-indazol-4-yl)boronic acid.

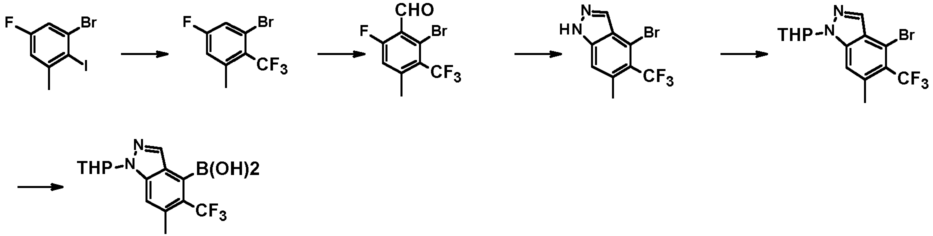

Step 1: 1-Bromo-5-fluoro-2-iodo-3-methylbenzene (3 g, 9.53 mmol) was dissolved in DMF (60 mL), and Cul (10.89 g, 57.16 mmol) and methyl 2,2-difluoro-2-fluorosulfonyl-acetate (10.98 g, 57.16 mmol, 7.27 mL) were added. The reaction mixture was stirred at 60 °C for 12 h. After the reaction was completed, purified water (60 mL) was added dropwise to the reaction mixture, and ethyl acetate (60 mL*3) was added to extract the organic layer, which was washed with saturated NaCl aqueous solution (150 mL*2), and then dried over anhydrous MgSO₄. The obtained reaction residue was purified by silica gel chromatography to obtain 1-bromo-5-fluoro-3-methyl-2-(trifluoromethyl)benzene (1.1 g, 4.28 mmol, 45% yield). ¹H NMR (400 MHz, CDCl₃, ppm): δ 7.33 (dd, *J* = 2.4, 7.6 Hz, 1H), 6.95 (dd, *J =* 2.4, 8.8 Hz, 1H), 2.54 (q, *J* = 3.6 Hz, 3H).

Step 2: 1-Bromo-5-fluoro-3-methyl-2-(trifluoromethyl)benzene (1.7 g, 6.61 mmol) was dissolved in THF (34 mL), and LDA (2 M, 6.61 mL) was slowly added dropwise under nitrogen at -60 °C and stirred for 30 min. DMF (1.45 g, 19.84 mmol, 1.53 mL) was added dropwise to the reaction mixture and stirred at -60 °C for 1.5 h. After the reaction was completed, saturated NH₄Cl aqueous solution (40 mL) was added dropwise to the reaction mixture, and ethyl acetate (40 mL*3) was added to extract the organic layer, washed with saturated NaCl aqueous solution (100 mL*2), and dried over anhydrous MgSO₄. The obtained reaction residue was purified by silica gel chromatography to obtain 2-bromo-6-fluoro-4-methyl-3-(trifluoromethyl)benzaldehyde (860 mg, 3.02 mmol, 46% yield) as a yellow oil. ¹H NMR (400 MHz, CDCl₃, ppm): δ 10.35 (s, 1H), 7.08 (d, *J* = 10.5 Hz, 1H), 2.61 (q, *J* = 4.0 Hz, 3H).

Step 3: 2-Bromo-6-fluoro-4-methyl-3-(trifluoromethyl)benzaldehyde (860 mg, 3.02 mmol) and NH₂NH₂·H₂O (4.43 g, 86.72 mmol, 4.29 mL) were dissolved in DMSO (9 mL), and then nitrogen was purged three times. The reaction mixture was reacted at 60 °C for 2 h. After the reaction was completed, purified water (20 mL) was added dropwise to the reaction mixture, and ethyl acetate (20 mL*3) was added to extract the organic layer, which was washed with saturated NaCl aqueous solution (30 mL*2), and dried over anhydrous MgSO₄. The obtained reaction residue was purified by silica gel chromatography to obtain 4-bromo-6-methyl-5-(trifluoromethyl)-1H-indazole (618 mg, 2.21 mmol, 73% yield) as a yellow oil. LCMS (ES-API, m/z): [M+H]⁺= 278.9; ¹H NMR (400 MHz, CDCl₃, ppm): δ 8.20 (s, 1H), 7.34 (s, 1H), 2.66 (q, *J* = 3.2 Hz, 3H).

Step 4: 4-Bromo-6-methyl-5-(trifluoromethyl)-1H-indazole (618 mg, 2.21 mmol) was dissolved in DCM (12 mL), then TsOH·H₂O (42.13 mg, 221.46 µmol) was added sequentially, and a solution of DHP (745.14 mg, 8.86 mmol) in acetonitrile (3 mL) was added, and the mixture was stirred at room temperature for 16 hours. After the reaction was completed, purified water (60 mL) was added dropwise to the reaction mixture, and ethyl acetate (20 mL*3) was added to extract the organic layer, which was washed with a saturated NaCl aqueous solution (30 mL*2), and then dried over anhydrous MgSO₄. The obtained reaction residue was purified by silica gel chromatography to obtain 4-bromo-6-methyl-1-(tetrahydro-2*H*-pyran-2-yl)-5-(trifluoromethyl)-1*H*-indazole (800 mg, 2.20 mmol, 99% yield) as a pale yellow oil. ¹H NMR (400 MHz, CDCl₃, ppm): δ 8.11 (s, 1H), 7.43 (s, 1H), 5.69 (dd, *J* = 2.8, 8.8 Hz, 1H), 4.05 - 3.96 (m, 1H), 3.81 - 3.70 (m, 1H), 2.67 (q, *J* = 3.2 Hz, 3H), 2.57 - 2.46 (m, 1H), 2.23 - 2.12 (m, 1H), 1.78 - 1.67 (m, 3H), 1.59 - 1.54 (m, 1H).

Step 5: Hypoboric acid (185.14 mg, 2.07 mmol), 4-bromo-6-methyl-1-(tetrahydro-2*H*-pyran-2-yl)-5-(trifluoromethyl)-1*H*-indazole (250 mg, 688.38 µmol), cataCXium Pd G4 (50.20 mg, 68.84 µmol) were dissolved in MeOH (3 mL), and DIEA (266.90 mg, 2.07 mmol) was slowly added dropwise under nitrogen. After degassing with nitrogen for 10 minutes, it was stirred at 60 °C for 40 minutes. After completion of the reaction, the solvent was removed under reduced pressure to obtain (6-methyl-1-(tetrahydro-2*H*-pyran-2-yl)-5-(trifluoromethyl)-1*H*-indazol-4-yl)boronic acid (210 mg, crude mixture) as a black solid. LCMS (ES-API, m/z): [M+H]⁺ = 245.1.

### Intermediate 22: Synthesis of (5-chloro-3,6-dimethyl-1-(tetrahydro-2H-pyran-2-yl)-1H-indazol-4-yl)boronic acid.

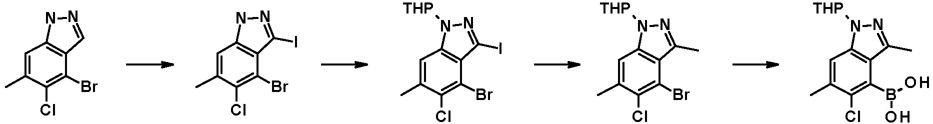

Step 1: 4-Bromo-5-chloro-6-methyl-1*H*-indazole (2 g, 8.15 mmol) was dissolved in MeOH (25 mL), and then NaOH (4 M, 13.24 mL) and I₂ (2.48 g, 9.78 mmol, 1.97 mL) were added dropwise at 0 °C. It was stirred at room temperature for 2 h under nitrogen. After completion of the reaction, purified water was added to the reaction mixture to quench the reaction, and ethyl acetate was added to extract the organic layer, which was then dried over anhydrous MgSO₄. It was purified by silica gel chromatography to obtain 4-bromo-5-chloro-3-iodo-6-methyl-1*H*-indazole (2.9 g, 7.73 mmol, 95% yield) as a white solid. LCMS (ES-API, m/z): [M+H]⁺= 372.8; ¹H NMR (400 MHz, DMSO-*d*₆, ppm): δ 14.24 - 13.32 (m, 1H), 7.60 (s, 1H), 2.50 (s, 3H).

Step 2: 4-Bromo-5-chloro-3-iodo-6-methyl-1*H*-indazole (200 mg, 538.50 µmol), p-TsOH (4.64 mg, 26.93 µmol), DHP (90.59 mg, 1.08 mmol) were dissolved in DCM (2.5 mL), degassed and purged with nitrogen three times, and stirred at room temperature under nitrogen for 2 h. After completion of the reaction, purified water was added to the reaction mixture to quench the reaction, and the organic layer was extracted by adding ethyl acetate and dried over anhydrous MgSO₄. Trituration with ethyl acetate was performed to obtain 4-bromo-5-chloro-3-iodo-6-methyl-1-(tetrahydro-2*H*-pyran-2-yl)-1*H-*indazole (120 mg, 252.90 µmol, 47% yield) as a white solid. LCMS (ES-API, m/z): [M+H]⁺= 454.9; ¹H NMR (400 MHz, DMSO-*d*₆, ppm): δ 7.88 (d, *J* = 0.6 Hz, 1H), 5.83 (dd, *J* = 2.5, 9.8 Hz, 1H), 3.93 - 3.82 (m, 1H), 3.74 (td, *J* = 6.9, 11.5 Hz, 1H), 2.54 (s, 3H), 2.41 - 2.21 (m, 1H), 2.10 - 1.90 (m, 2H), 1.81 - 1.64 (m, 1H), 1.64 - 1.49 (m, 2H).

Step 3: 4-Bromo-5-chloro-3-iodo-6-methyl-1-(tetrahydro-2*H*-pyran-2-yl)-1H-indazole (1 g, 2.20 mmol) and Pd(dppf)Cl₂·CH₂Cl₂ (89.64 mg, 109.77 µmol) were dissolved in DMF (10 mL), dimethylzinc (1 M, 5.49 mL) was added under nitrogen, and stirred at 80 °C for 12 hours. After the reaction was completed, purified water was added to the reaction mixture to quench the reaction, and the organic layer was extracted by adding ethyl acetate and dried over anhydrous MgSO₄. The reaction residue was purified using silica gel chromatography to obtain 4-bromo-5-chloro-3,6-dimethyl-1-(tetrahydro-2*H*-pyran-2-yl)-1*H*-indazole (320 mg, 931.19 µmol, 42% yield) as a white solid. LCMS (ES-API, m/z): [M+H]⁺= 260.8; ¹H NMR (400 MHz, CDCl₃, ppm): δ 7.34 (s, 1H), 5.55 (dd, *J* = 2.4, 9.6 Hz, 1H), 4.10 - 3.99 (m, 1H), 3.73 (dt, *J* = 2.4, 11.2 Hz, 1H), 2.74 (s, 3H), 2.55 (s, 3H), 2.54 - 2.46 (m, 1H), 2.20 - 2.10 (m, 1H), 2.01 (br dd, *J* = 3.2, 12.8 Hz, 1H), 1.82 - 1.70 (m, 2H), 1.69 - 1.62 (m, 1H).

Step 4: 4-bromo-5-chloro-3,6-dimethyl-1-(tetrahydro-2*H*-pyran-2-yl)-1H-indazole (320 mg, 651.83 µmol), hypoboric acid (175.31 mg, 1.96 mmol), DIEA (252.73 mg, 1.96 mmol), cataCXium Pd G4 (47.54 mg, 65.18 µmol) were dissolved in MeOH (4 mL), degassed and purged with nitrogen three times, then stirred at 60 °C for 40 minutes. After the reaction was completed, purified water was added to the reaction mixture to quench the reaction, and ethyl acetate was added to extract the organic layer, which was then dried over anhydrous MgSO₄. The reaction residue was purified by silica gel chromatography to obtain (5-chloro-3,6-dimethyl-1-(tetrahydro-2*H*-pyran-2-yl)-1H-indazol-4-yl)boronic acid (200 mg, 582.04 µmol, 89% yield) as a black solid. LCMS (ES-API, m/z): [M+H]⁺= 309.0; ¹H NMR (400 MHz, CDCl₃ , ppm): δ 7.36 (s, 1H), 5.55 (dd, *J =* 2.4, 9.7 Hz, 1H), 5.33 - 5.12 (m, 1H), 4.09 - 4.02 (m, 1H), 3.77 - 3.72 (m, 1H), 3.70 (s, 1H), 2.53 (s, 3H), 2.50 - 2.49 (m, 3H), 2.18 - 2.10 (m, 1H), 2.01 (br d, *J* = 13.2 Hz, 2H), 1.81 - 1.71 (m, 3H).

### Intermediate 23: Synthesis of N,N-bis(4-methoxybenzyl)-4-methyl-6-(tributylstannyl)-5-(trifluoromethyl)pyridin-2-amine.

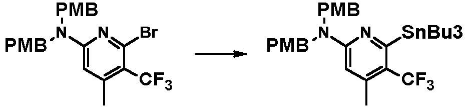

Step 1: 6-bromo-*N*,*N*-bis(4-methoxybenzyl)-4-methyl-5-(trifluoromethyl)pyridin-2-amine (110.67 mg, 190.78 **µ**mol) and Su₂Bu₆ (110.67 mg, 190.78 **µ**mol) were dissolved in 1,4-dioxane (1 mL), and Pd₂(dba)₃ (5.55 mg, 6.06 **µ**mol), tricyclohexylphosphane (3.40 mg, 12.11 **µ**mol), and LiCl (12.84 mg, 302.83 **µ**mol) were added. The reaction mixture was stirred at 110 °C for 16 h. After completion of the reaction, silica gel chloromatography purification was performed to obtain *N,N*-bis(4-methoxybenzyl)-4-methyl-6-(tributylstannyl)-5-(trifluoromethyl)pyridin-2-amine (25 mg, 32.25 µmol, 53.24% yield) as a colorless oil. LCMS (ES-API, m/z): [M+H]⁺= 705.2; ¹H NMR (400 MHz, DMSO-*d*₆, ppm): δ 0.76 (t, *J* = 7.2 Hz, 9H), 0.87 (br t, *J* = 7.2 Hz, 2H), 0.96 - 1.03 (m, 5H), 1.14 - 1.22 (m, 6H), 1.39 - 1.47 (m, 5H), 2.24 (s, 3H), 3.71 (s, 6H), 4.72 (br s, 4H), 6.48 (s, 1H), 6.86 (d, *J* = 8.4 Hz, 4H), 7.12 (d, *J* = 8.4 Hz, 4H).

### Intermediate 24: Synthesis of N,N-bis(4-methoxybenzyl)-6-(tributylstannyl)-5-(trifluoromethyl)pyridin-2-amine.

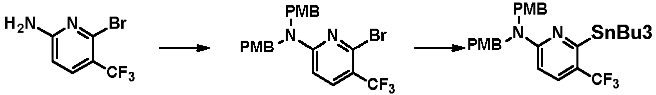

Step 1: 6-Bromo-5-(trifluoromethyl)pyridin-2-amine (700 mg, 2.9 mmol) was dissolved in DMF (10 mL), and the temperature was lowered to 0 °C. Sodium hydride (290 mg, 7.26 mmol) was added and stirred at 0 °C for 30 minutes. After 30 minutes, 4-methoxybenzyl chloride (1.07 mL, 7.26 mmol) was added to the reaction mixture, and stirred at room temperature for 4 hours. After completion of the reaction, the temperature was lowered to 0 °C, water was slowly added to quench the reaction, and the organic layer was extracted with ethyl acetate. After drying over anhydrous MgSO₄, concentration under reduced pressure was performed. The residue was purified by silica gel chromatography to obtain 6-bromo-*N,N*-bis(4-methoxybenzyl)-5-(trifluoromethyl)pyridin-2-amine (1.1 g, 2.29 mmol, 79% yield). LCMS (ES-API, m/z): [M+H]⁺= 481.3.

Step 2: 6-Bromo-*N,N*-bis(4-methoxybenzyl)-5-(trifluoromethyl)pyridin-2-amine (1.1 g, 2.29 mmol) obtained in Step 1 was dissolved in 1,4-dioxane (10 mL), then tricyclohexylphosphane (128 mg, 457 µmol), bis(tributylstannane) (3.48 mL, 6.86 mmol), Tris(dibenzylideneacetone)dipalladium(0) (209 mg, 229 µmol), and lithium chloride (484 mg, 11.4 mmol) were added, the temperature was raised to 110 °C and stirred for 5 h. After completion of the reaction, the temperature was lowered to room temperature. After removing the palladium catalyst using a Celite filter, the organic layer was extracted with ethyl acetate. The organic layer was dried over anhydrous Na₂SO₄ and concentrated under reduced pressure. The residue was purified by silica gel chromatography to obtain *N,N*-bis(4-methoxybenzyl)-6-(tributylstannyl)-5-(trifluoromethyl)pyridin-2-amine (160 mg, 231 mmol, 10% yield). LCMS (ES-API, m/z): [M+H]⁺= 692.5.

### Intermediate 25: Synthesis of N,N-bis(4-methoxybenzyl)-6-(4,4,5,5-tetramethyl-1,3,2-dioxaborolan-2-yl)-5-(trifluoromethyl)pyridin-2-amine.

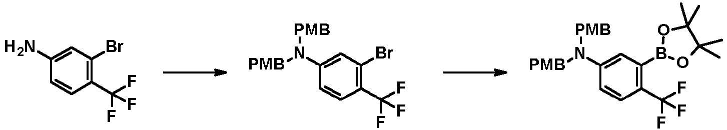

Step 1: 3-Bromo-4-(trifluoromethyl)aniline (500 mg, 2.08 mmol) was dissolved in DMF (10 mL), and the temperature was lowered to 0 °C. NaH (416 mg, 10.41 mmol) was added at 0 °C and the mixture was stirred for 30 minutes. After 30 minutes, 4-methoxybenzyl chloride (1.4 mL, 10.41 mmol) was added to the reaction mixture, and the mixture was stirred at room temperature for 24 hours. After the reaction was completed, the reaction temperature was lowered to 0 °C, and water was slowly added to quench the reaction, and the organic layer was extracted with ethyl acetate. After drying over anhydrous Na₂SO₄, concentration was performed under reduced pressure. The residue was purified by silica gel chromatography (5% ethyl acetate/hexane) to obtain 3-bromo-N,N-bis(4-methoxybenzyl)-4-(trifluoromethyl)aniline (816 mg, 1.7 mmol, 85% yield). LCMS (ES-API, m/z): [M+H]⁺= 481.3.

Step 2: 3-Bromo-*N,N*-bis(4-methoxybenzyl)-4-(trifluoromethyl)aniline (160 mg, 333 µmol) obtained in Step 1 was dissolved in 1,4-dioxane (2 mL). Then, potassium acetate (98.1 mg, 999 µmol), 4,4,5,5-tetramethyl-2-(4,4,5,5-tetramethyl-1,3,2-dioxaborolan-2-yl)-1,3,2-dioxaborolane (102 mg, 400 µmol), [1,1'-Bis(diphenylphosphino)ferrocene]dichloropalladium(II), complex with dichloromethane (24.4 mg, 33.3 µmol) were added and the temperature was raised to 90 °C and stirred for 24 h. After the starting material was completely consumed, the reaction temperature was lowered to room temperature. After removing the palladium catalyst by Celite filter, the organic layer was extracted with ethyl acetate. After drying over anhydrous MgSO₄, concentration was performed under reduced pressure. The residue was purified by silica gel chromatography to obtain *N,N*-bis(4-methoxybenzyl)-6-(4,4,5,5-tetramethyl-1,3,2-dioxaborolan-2-yl)-5-(trifluoromethyl)pyridin-2-amine (175 mg, 333 mmol, 100% yield). LCMS (ES-API, m/z): [M+H]⁺= 528.4

### Intermediate 26: Synthesis of 2,3,4,5-tetrafluoro-6-(4,4,5,5-tetramethyl-1,3,2-dioxaborolan-2-yl)aniline.

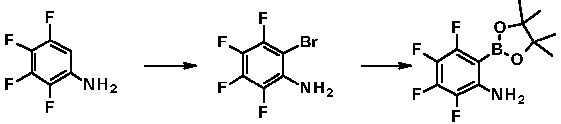

Step 1: 2,3,4,5-Tetrafluoroaniline (5 g, 30.3 mmol) was dissolved in acetonitrile (20 mL), NBS (5.93 mg, 33.3 mmol) was added at room temperature, and the mixture was stirred at 60 °C for 2 hours. The reaction temperature was lowered to room temperature, and the reaction was quenched with a saturated sodium thiosulfate aqueous solution, followed by extraction with ethyl acetate. Silica gel chromatography purification was performed to obtain 2-bromo-3,4,5,6-tetrafluoroaniline (5.5 g, 22.5 mmol, 74% yield) as a brown solid. ¹H NMR (400 MHz, CDCl₃, ppm): δ 4.27 - 4.15 (m, 2H).

Step 2: 2-Bromo-3,4,5,6-tetrafluoroaniline (0.5 g, 2.05 mmol) obtained in Step 1, B₂pin₂ (1.30 g, 5.12 mmol), Pd(dppf)Cl₂ (149.95 mg, 204.93 µmol), potassium acetate (402.25 mg, 4.10 mmol) were added to 1,4-dioxane (5 mL), and performed nitrogen purged three times. Then, the mixture was reacted at 100 °C for 2 h under nitrogen. After silica gel chromatography purification, 2,3,4,5-tetrafluoro-6-(4,4,5,5-tetramethyl-1,3,2-dioxaborolan-2-yl)aniline (500 mg, 628.59 µmol, 31% yield) was obtained as a white solid. LCMS (ES-API, m/z): [M+H]⁺= 210.0; ¹H NMR (400 MHz, DMSO-*d*₆, ppm): δ 1.19 - 1.15 (m, 12H).

### EXAMPLE 1: Synthesis of 4-(1-((1R,5S)-3,8-diazabicyclo[3.2.1]octan-3-yl)-3-(((2R,7aS)-2-fluorotetrahydro-1H-pyrrolizin-7a(5H)-yl)methoxy)imidazo[1',2':1,6]pyrido[3,2-d]pyrimidin-6-yl)-5-ethyl-6-fluoronaphthalen-2-ol.

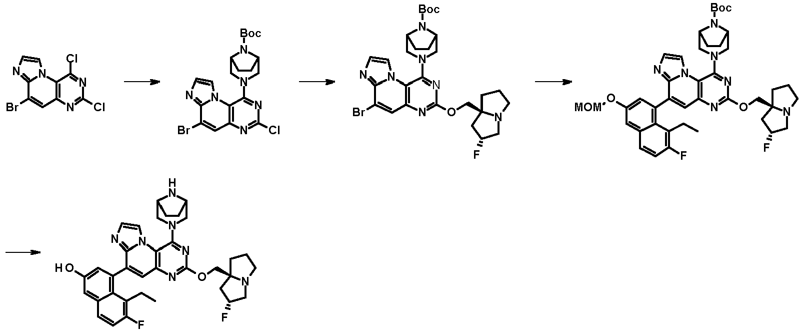

Step 1: 6-bromo-1,3-dichloroimidazo[1',2':1,6]pyrido[3,2-d]pyrimidine (2.4 g, 7.55 mmol) was dissolved in DCM (48 mL), and then *N*,*N*-diisopropylamine (1.46 g, 11.32 mmol) and *tert*-butyl (1*R*,5*S*)-3,8-diazabicyclo[3.2.1]octane-8-carboxylate (1.92 g, 9.06 mmol) were added, and the reaction mixture was stirred at 25 °C for 1 h. The reaction mixture was diluted with purified water (80 mL) and extracted with DCM (50 mL*3). The organic matter was washed with NaCl aqueous solution (50 mL*2), dried over Na₂SO₄, filtered and concentrated to obtain the residue. The product was stirred with petroleum ether (50 mL) and EA (20 mL) at 25 °C for 30 min, then the solid was filtered and dried. *tert*-Butyl (1*R*,5*S*)-3-(6-bromo-3-chloroimidazo[1',2':1,6]pyrido[3,2-*d*]pyrimidin-1-yl)-3,8-diazabicyclo[3.2.1]octane-8-carboxylate (3.44 g, 0.63 mmol, 88% yield) was obtained as a yellow solid. LCMS (ES-API, m/z): [M+H]⁺= 494.1; ¹H NMR (400 MHz, DMSO-*d*_{6,} ppm): δ 8.28 (s, 1H), 7.82 - 7.69 (m, 2H), 4.17 - 4.09 (m, 2H), 3.84 - 3.70 (m, 2H), 3.65 - 3.51 (m, 2H), 1.71 - 1.60 (m, 2H), 1.58 - 1.49 (m, 2H), 1.48 - 1.43 (m, 9H).

Step 2: *tert*-butyl (1*R*,5*S*)-3-(6-bromo-3-chloroimidazo[1',2':1,6]pyrido[3,2-d]pyrimidin-1-yl)-3,8-diazabicyclo[3.2.1]octane-8-carboxylate (2.44 g, 4.94 mmol) obtained in Step 1 was dissolved in THF (45 mL), and then NaH (395.28 mg, 9.88 mmol) was added at 0 °C. The reaction mixture was heated at room temperature for 30 min, and then a mixture of ((2*R*,7a*S*)-2-fluorotetrahydro-1*H*-pyrrolizin-7a(5*H*)-yl)methanol (865.35 mg, 5.44 mmol) dissolved in THF (5 mL) was added. The reaction mixture was stirred at room temperature for 16 h under nitrogen. Purified water was added to the reaction mixture, and extraction was performed with ethyl acetate (50 mL*3). The combined organic layers were washed with NaCl aqueous solution (40 mL*2) and dried over Na₂SO₄. The filtered solution was concentrated under reduced pressure to obtain the residue. The residue was purified by RP-HPLC (0.1% FA condition). *tert*-Butyl (1*R*,5*S*)-3-(4-bromo-7-(((2*R*,7a*S*)-2-fluorotetrahydro-1*H*-pyrrolizin-7a(5*H*)-yl)methoxy)imidazo[1,2-*a*][1,5]naphthyridin-9-yl)-3,8-diazabicyclo[3.2.1]octane-8-carboxylate (920 mg, 1.58 mmol, 32% yield) was obtained as a yellow solid. LCMS (ES-API, m/z): [M+H]⁺= 572.5; ¹H NMR (400 MHz, DMSO-*d*₆, ppm): δ ppm 8.19 (s, 1 H), 7.69 - 7.65 (m, 1 H), 7.62 - 7.55 (m, 1 H), 5.39 - 5.16 (m, 1 H), 4.17 - 4.08 (m, 3 H), 3.83 - 3.69 (m, 2 H), 3.62 - 3.49 (m, 2 H), 3.12 - 2.98 (m, 4 H), 2.79 - 2.87 (m, 1 H), 2.10 - 2.15 (m, 1 H), 2.03 - 2.06 (m, 1 H), 1.73 - 1.88 (m, 4 H), 1.54 - 1.69 (m, 4 H), 1.42 - 1.48 (m, 9 H).

Step 3: *tert*-butyl (1*R*,5*S*)-3-(4-bromo-7-(((2*R*,7a*S*)-2-fluorotetrahydro-1*H*-pyrrolizin-7a(5*H*)-yl)methoxy)imidazo[1,2-*a*][1,5]naphthyridin-9-yl)-3,8-diazabicyclo[3.2.1]octane-8-carboxylate (470 mg, 821.57 µmol) obtained in Step 2, 2-(8-ethyl-7-fluoro-3-(methoxymethoxy)naphthalen-1-yl)-4,4,5,5-tetramethyl-1,3,2-dioxaborolane (355.14 mg, 985.89 µmol), cataCXium A Pd G3 (29.92 mg, 41.08 µmol) and Cs₂CO₃ (535.37 mg, 1.64 mmol) was added to ethanol (9.4 mL) and purified water (0.9 mL), and degassing and nitrogen purging were repeated three times. And then, the reaction mixture was stirred under nitrogen at 80 °C for 16 h. After the starting material was completely consumed, the reaction mixture was concentrated under reduced pressure, and the residue was diluted with water (10 mL), and the compound was extracted with ethyl acetate (5 mL*2). The organic layer was washed with saturated NaCl aqueous solution (5 mL*2), dried over anhydrous Na₂SO₄, and concentrated under reduced pressure. The residue was purified by prep-TLC. *tert*-Butyl (1*R*,5*S*)-3-(6-(8-ethyl-7-fluoro-3-(methoxymethoxy)naphthalen-1-yl)-3-(((2*R*,7a*S*)-2-fluorotetrahydro-1*H*-pyrrolizin-7a(5*H*)-yl)methoxy)imidazo[1',2':1,6]pyrido[3,2-*d*]pyrimidin-1-yl)-3,8-diazabicyclo[3.2.1]octane-8-carboxylate (105 mg, 128.84 µmol, 16% yield) was obtained as a yellow solid.

LCMS (ES-API, m/z): [M+H]⁺= 770.4; ¹H NMR (400 MHz, DMSO-*d*₆, ppm): δ 8.23 - 8.11 (m, 1H), 7.93 - 7.84 (m, 1H), 7.68 - 7.61 (m, 1H), 7.60 - 7.34 (m, 3H), 7.27 - 7.14 (m, 1H), 5.34 (s, 3H), 4.23 - 4.11 (m, 2H), 4.09 - 3.95 (m, 2H), 3.95 - 3.90 (m, 1H), 3.76 - 3.51 (m, 2H), 3.43 (s, 3H), 3.06 - 3.14 (m, 2H), 2.98 - 3.05 (m, 1H), 2.80 - 2.90 (m, 1H), 1.95 - 2.23 (m, 6H), 1.66 - 1.93 (m, 7H), 1.41 - 1.50 (m, 9H), 0.54 (br t, *J* = 6.4 Hz, 3H).

Step 4: *tert*-butyl (1*R*,5*S*)-3-(6-(8-ethyl-7-fluoro-3-(methoxymethoxy)naphthalen-1-yl)-3-(((2*R*,7a*S*)-2-fluorotetrahydro-1*H*-pyrrolizin-7a(5*H*)-yl)methoxy)imidazo[1',2':1,6]pyrido[3,2-*d*]pyrimidin-1-yl)-3,8-diazabicyclo[3.2.1]octane-8-carboxylate (65 mg, 84.43 µmol) was dissolved in DCM (1.3 mL), and 4 N HCl in ethyl acetate solution (650 µL) was added. The reaction mixture was stirred at 25 °C for 1 h. After the starting material was completely consumed, the reaction mixture was concentrated under reduced pressure, and the residue was diluted with saturated NaHCO₃ solution (10 mL), and the compound was extracted with ethyl acetate (5 mL*2). The organic layer was washed with saturated NaCl aqueous solution, dried over anhydrous Na₂SO₄, and concentrated. The residue was purified by Prep-HPLC. 4-(1-((1*R*,5*S*)-3,8-diazabicyclo[3.2.1]octan-3-yl)-3-(((2*R*,7a*S*)-2-fluorotetrahydro-1*H-*pyrrolizin-7a(5*H*)-yl)methoxy)imidazo[1',2':1,6]pyrido[3,2-*d*]pyrimidin-6-yl)-5-ethyl-6-fluoronaphthalen-2-ol (25.72 mg, 41.10 µmol, 49% yield) was obtained as an off-white solid. LCMS (ES-API, m/z): [M+H]⁺= 626.3; ¹H NMR (400 MHz, DMSO-*d*₆, ppm): δ 9.86 (s, 1H), 8.11 - 7.99 (m, 1H), 7.75 (d, *J =* 2.4 Hz, 1H), 7.68 - 7.52 (m, 1H), 7.51 - 7.42 (m, 1H), 7.42 - 7.24 (m, 3H), 7.08 - 7.02 (m, 1H), 5.42 - 5.17 (m, 1H), 4.12 (s, 3H), 3.78 - 3.59 (m, 1H), 3.57 - 3.38 (m, 3H), 3.15 - 3.07 (m, 2H), 3.02 (s, 1H), 2.88 - 2.79 (m, 1H), 2.44 - 2.37 (m, 1H), 2.24 - 2.08 (m, 2H), 2.09 - 1.96 (m, 3H), 1.92 - 1.67 (m, 5H), 1.59 - 1.45 (m, 1H), 1.42 - 1.15 (m, 1H), 0.50 (t, *J* = 6.8 Hz, 3H).

### EXAMPLE 2: Synthesis of 4-(1-((1R,5S)-3,8-diazabicyclo[3.2.1]octan-3-yl)-3-(((2R,7aS)-2-fluorotetrahydro-1H-pyrrolizin-7a(5H)-yl)methoxy)imidazo[1',2':1,6]pyrido[3,2-d]pyrimidin-6-yl)-5-ethynyl-6-fluoronaphthalen-2-ol

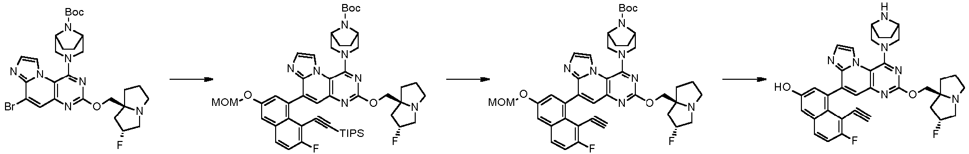

Step 1: *tert*-Butyl (1*R*,5*S*)-3-(6-bromo-3-(((2*R*,7a*S*)-2-fluorotetrahydro-1H-pyrrolizin-7a(5*H*)-yl)methoxy)imidazo[1',2':1,6]pyrido[3,2-d]pyrimidin-1-yl)-3,8-diazabicyclo[3.2.1]octane-8-carboxylate (150 mg, 243.30 µmol), ((2-Fluoro-6-(methoxymethoxy)-8-(4,4,5,5-tetramethyl-1,3,2-dioxaborolan-2-yl)naphthalen-1-yl)ethynyl)triisopropylsilane (149.64 mg, 291.96 µmol), cataCXium A Pd G3 (8.86 mg, 12.16 µmol) and Cs₂CO₃ (158.54 mg, 486.60 µmol) were added to ethanol (3 mL) and water (0.3 mL), and degassing and nitrogen purging were repeated three times. And then, the mixture was stirred under nitrogen at 80 °C for 16 h. After completion of the reaction, the reaction mixture was concentrated under reduced pressure to remove ethanol, and the crude residue was diluted with purified water (15 mL) and extracted with ethyl acetate (10 mL*2). The organic layer was washed with a saturated NaCl aqueous solution, dried over anhydrous Na₂SO₄, and concentrated under reduced pressure. The residue was purified by prep-TLC. *tert*-butyl (1*R*,5*S*)-3-(6-(7-fluoro-3-(methoxymethoxy)-8-((triisopropylsilyl)ethynyl)naphthalen-1-yl)-3-(((2*R*,7a*S*)-2-fluorotetrahydro-1H-pyrrolizin-7a(5*H*)-yl)methoxy)imidazo[1',2':1,6]pyrido[3,2-*d*]pyrimidin-1-yl)-3,8-diazabicyclo[3.2.1]octane-8-carboxylate (120 mg, 126.59 µmol, 52% yield) was obtained as a yellow solid. LCMS (ES-API, m/z): [M+H]⁺= 922.5; ¹H NMR (400 MHz, DMSO-*d*₆, ppm): δ 8.22 - 7.97 (m, 2H), 7.77 - 7. 63 (m, 1H), 7.59 - 7. 47 (m, 2H), 7.46 - 7. 30 (m, 2H), 5.36 (s, 3H), 4.41 (brs, 1H), 4.32 - 4. 21 (m, 1H), 4.18- 3.99 (m, 2H), 3.97 - 3.78 (m, 1H), 3.43 (s, 3H), 3.14 - 3. 07 (m, 2H), 3.07 - 3.00 (m, 1H), 2.93 - 2. 79 (m, 2H), 2.31 - 2. 23 (m, 1H), 2.22 - 1.95 (m, 5H), 1.95 - 1. 62 (m, 6H), 1.46 (brs, 9H), 0.83 - 0.62 (m, 18H), 0.46 - 0.26 (m, 3H).

Step 2: *tert*-butyl (1*R*,5*S*)-3-(6-(7-fluoro-3-(methoxymethoxy)-8-((triisopropylsilyl)ethynyl)naphthalen-1-yl)-3-(((2*R*,7a*S*)-2-fluorotetrahydro-1H-pyrrolizin-7a(5*H*)-yl)methoxy)imidazo[1',2':1,6]pyrido[3,2-*d*]pyrimidin-1-yl)-3,8-diazabicyclo[3.2.1]octane-8-carboxylate (120 mg, 130.13 µmol) was dissolved in DMF (1.2 mL), and CsF (296.50 mg, 1.95 mmol, 71.97 µL) was added. The reaction mixture was stirred at 40 °C for 16 h. The reaction mixture was diluted with water (15 mL) and extracted with ethyl acetate (10 mL*3). The organic matter was washed with saturated NaCl aqueous solution (10 mL*2), dried over anhydrous Na₂SO₄, filtered, and concentrated to obtain the residue. The residue was purified by prep-TLC. *tert*-Butyl (1*R*,5*S*)-3-(6-(8-ethynyl-7-fluoro-3-(methoxymethoxy)naphthalen-1-yl)-3-(((2*R*,7a*S*)-2-fluorotetrahydro-1H-pyrrolizin-7a(5*H*)-yl)methoxy)imidazo[1',2':1,6]pyrido[3,2-*d*]pyrimidin-1-yl)-3,8-diazabicyclo[3.2.1]octane-8-carboxylate (70 mg, 91.17 µmol, 70% yield) was obtained as a yellow solid. LCMS (ES-API, m/z): [M+H]⁺= 766.5; ¹H NMR (400 MHz, CDCl₃, ppm): δ 8.75 - 8. 36 (m, 1H), 7.85 (dd, *J* = 9.05, 5.75 Hz, 1H), 7.78 - 7. 61 (m, 1H), 7.56 (s, 1H), 7.51 (br d, *J* = 13.2 Hz, 1H), 7.40 (d, *J* = 2.0 Hz, 1H), 7.32 - 7. 26 (m, 2H), 5.51 - 5. 13 (m, 3H), 4.60 - 4. 11 (m, 4H), 3.66 - 3. 58 (m, 1H), 3.55 (s, 3H), 3.52 - 3. 46 (m, 2H), 3.38 - 3. 19 (m, 3H), 2.12 - 3. 99 (m, 1H), 2.63 - 2. 51 (m, 1H), 2.40 - 2. 09 (m, 5H), 2.06- 1.81 (m, 5H), 1.57- 1.48 (m, 9H).

Step 3: *tert*-butyl (1*R*,5*S*)-3-(6-(8-ethynyl-7-fluoro-3-(methoxymethoxy)naphthalen-1-yl)-3-(((2*R*,7a*S*)-2-fluorotetrahydro-1*H*-pyrrolizin-7a(5*H*)-yl)methoxy)imidazo[1',2':1,6]pyrido[3,2-*d*]pyrimidin-1-yl)-3,8-diazabicyclo[3.2.1]octane-8-carboxylate (70 mg, 91.40 µmol) was dissolved in ACN (0.7 mL), 4N HCl in dioxane (700 µL) was added at 0 °C under nitrogen, and stirred for 1 h. After the starting material was completely consumed, the reaction mixture was concentrated under reduced pressure, and the residue was diluted with saturated sodium bicarbonate (10 mL), and the compound was extracted with ethyl acetate (5 mL*4). The organic layer was washed with brine, dried over anhydrous Na₂SO₄, and concentrated under reduced pressure. The residue was collected and purified by Prep-HPLC. 4-(1-((1*R*,5*S*)-3,8-diazabicyclo[3.2.1]octan-3-yl)-3-(((2*R*,7a*S*)-2-fluorotetrahydro-1*H*-pyrrolizin-7a(5*H*)-yl)methoxy)imidazo[1',2':1,6]pyrido[3,2-d]pyrimidin-6-yl)-5-ethynyl-6-fluoronaphthalen-2-ol (27.37 mg, 43.79 umol, 48% yield) was obtained as a white solid. LCMS (ES-API, m/z): [M+H]⁺= 622.4; ¹H NMR (400 MHz, DMSO-*d*₆, ppm): δ 10.33 - 9.92 (m, 1H), 8.55 - 8.36 (m, 1H), 7.99 - 7.93 (m, 1H), 7.61 - 7.47 (m, 1H), 7.47 - 7.34 (m, 3H), 7.33 - 7.26 (m, 1H), 7.20 (br s, 1H), 5.40 - 5.17 (m, 1H), 4.17 - 4.08 (m, 1H), 4.06 - 3.84 (m, 2H), 3.70 - 3.54 (m, 2H), 3.54 - 3.41 (m, 3H), 3.15 - 3.05 (m, 2H), 3.04 - 3.00 (m, 1H), 2.99 - 2.88 (m, 1H), 2.84 (br d, *J* = 7.2 Hz, 1H), 2.20 - 1.98 (m, 4H), 1.93 - 1.68 (m, 5H), 1.57 - 1.42 (m, 1H).

### EXAMPLE 3: Synthesis of 4-(1-((1R,5S)-3,8-diazabicyclo[3.2.1]octan-3-yl)-3-(((2R,7aS)-2-fluorotetrahydro-1H-pyrrolizin-7a(5H)-yl)methoxy)imidazo[1',2':1,6]pyrido[3,2-d]pyrimidin-6-yl)-5,6-difluoronaphthalen-2-ol.

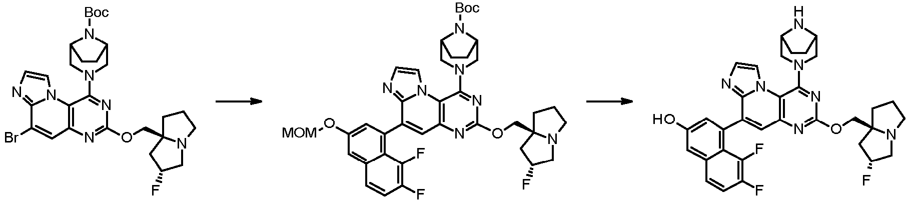

Step 1: *tert*-butyl (1*R*,5*S*)-3-(6-bromo-3-(((2*R*,7a*S*)-2-fluorotetrahydro-1*H*-pyrrolizin-7a(5*H*)-yl)methoxy)imidazo[1',2':1,6]pyrido[3,2-d]pyrimidin-1-yl)-3,8-diazabicyclo[3.2.1]octane-8-carboxylate (150 mg, 262.20 µmol), 2-(7,8-difluoro-3-(methoxymethoxy)naphthalen-1-yl)-4,4,5,5-tetramethyl-1,3,2-dioxaborolane (110.18 mg, 314.65 µmol), PdCl₂(dtbpf) (17.09 mg, 26.22 µmol), K₃PO₄ (166.97 mg, 786.61 µmol) was added to 1,4-dioxane (2.4 mL) and water (0.6 mL), and degassing and nitrogen purging were repeated three times. And then, the mixture was stirred at 80 °C for 1 h under nitrogen. After the starting material was completely consumed, the reaction mixture was concentrated under reduced pressure, and the residue was diluted with water (10 mL), and the compound was extracted with ethyl acetate (5 mL*2). The organic layer was washed with saturated NaCl aqueous solution (5 mL*2), dried over anhydrous Na₂SO₄, and concentrated under reduced pressure. The residue was purified by prep-TLC (DCM:MeOH=10:1) to obtain *tert*-butyl (1*R*,5*S*)-3-(6-(7,8-difluoro-3-(methoxymethoxy)naphthalen-1-yl)-3-(((2*R*,7a*S*)-2-fluorotetrahydro-1*H*-pyrrolizin-7a(5*H*)-yl)methoxy)imidazo[1',2':1,6]pyrido[3,2-*d*]pyrimidin-1-yl)-3,8-diazabicyclo[3.2.1]octane-8-carboxylate (150 mg, 195.70 µmol, 75% yield) as a yellow solid. LCMS (ES-API, m/z): [M+H]⁺= 760.2; ¹H NMR (400 MHz, DMSO-*d*₆, ppm): δ 7.68 (s, 1H), 7.57 - 7.34 (m, 4H), 7.27 - 7.16 (m, 2H), 5.33 - 5.12 (m, 3H), 4.69 (br d, *J = 2.4* Hz, 2H), 4.46 - 4.10 (m, 4H), 3.62 (s, 1H), 3.46 - 3.42 (m, 3H), 3.39 (s, 3H), 3.37 - 3.28 (m, 1H), 3.26 - 3.15 (m, 2H), 3.13 - 3.06 (m, 1H), 2.97 - 2.86 (m, 1H), 2.27 - 2.04 (m, 4H), 1.94 - 1.83 (m, 3H), 1.47 - 1.37 (m, 9H).

Step 2: *tert*-butyl (1*R*,5*S*)-3-(6-(7,8-difluoro-3-(methoxymethoxy)naphthalen-1-yl)-3-(((2*R*,7a*S*)-2-fluorotetrahydro-1*H*-pyrrolizin-7a(5*H*)-yl)methoxy)imidazo[1',2':1,6]pyrido[3,2-d]pyrimidin-1-yl)-3,8-diazabicyclo[3.2.1]octane-8-carboxylate (130 mg, 171.09 µumol) was dissolved in ACN (1.3 mL), HCl in dioxane (4 M, 1.30 mL) was added at 0 °C, and stirred at 0 °C for 1 h. After the starting material was completely consumed, the reaction mixture was concentrated under reduced pressure. The residue was diluted with saturated NaHCO₃ solution (15 mL), and the compound was extracted with ethyl acetate (10 mL*3). The organic layer was washed with aqueous NaCl solution, dried over anhydrous Na₂SO₄, and concentrated under reduced pressure. The residue was collected and purified by RP-HPLC to obtain 4-(1-((1*R*,5*S*)-3,8-diazabicyclo[3.2.1]octan-3-yl)-3-(((2*R*,7a*S*)-2-fluorotetrahydro-1*H*-pyrrolizin-7a(5*H*)-yl)methoxy)imidazo[1',2':1,6]pyrido[3,2-*d*]pyrimidin-6-yl)-5,6-difluoronaphthalen-2-ol (14.11 mg, 22.48 µmol, 13% yield) as a yellow-green solid. LCMS (ES-API, m/z): [M+H]⁺= 616.3; ¹H NMR (400 MHz, DMSO-*d*₆, ppm): δ 10.21 (s, 1H), 8.07 - 7.96 (m, 1H), 7.74 - 7.59 (m, 1H), 7.58 - 7.42 (m, 2H), 7.42 - 7.32 (m, 2H), 7.21 (s, 1H), 5.39 - 5.18 (m, 1H), 4.12 (br d, *J* = 9.2 Hz, 1H), 4.03 (br s, 1H), 3.97 - 3.83 (m, 1H), 3.72 - 3.60 (m, 1H), 3.59 - 3.42 (m, 3H), 3.18 - 2.91 (m, 4H), 2.88 - 2.78 (m, 1H), 2.20 - 1.95 (m, 4H), 1.92 - 1.60 (m, 5H), 1.58 - 1.36 (m, 2H).

### EXAMPLE 4: Synthesis of 4-(1-(((1-(dimethylamino)cyclobutyl)methyl)amino)-3-(((2R,7aS)-2-fluorotetrahydro-1H-pyrrolizin-7a(5H)-yl)methoxy)imidazo[1',2':1,6]pyrido[3,2-d]pyrimidin-6-yl)-5-ethyl-6-fluoronaphthalen-2-ol.

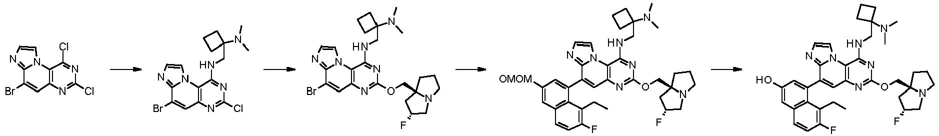

Step 1: 6-Bromo-1,3-dichloroimidazo[1',2':1,6]pyrido[3,2-d]pyrimidine (2 g, 6.29 mmol) was dissolved in DCM (40 mL), and DIPEA (1.22 g, 9.44 mmol) and 1-(aminomethyl)-*N,N*-dimethylcyclobutan-1-amine (967.79 mg, 7.55 mmol) were added sequentially. The reaction mixture was stirred at 25 °C for 1 h. The reaction mixture was diluted with water (80 mL) and extracted with DCM (50 mL*2). The organic matter was washed with saturated NaCl aqueous solution (50 mL*2), dried over anhydrous Na₂SO₄, filtered, and concentrated to obtain the residue. The product was stirred with petroleum ether (15 mL) and ethyl acetate (45 mL) at 25 °C for 30 min, filtered, and dried. 6-Bromo-3-chloro-*N-*((1-(dimethylamino)cyclobutyl)methyl)imidazo[1',2':1,6]pyrido[3,2-*d*]pyrimidin-1-amine (2.2 g, 5.33 mmol, 85% yield) was obtained as an off-white solid. LCMS (ES-API, m/z): [M+H]⁺= 365.0; ¹H NMR (400 MHz, DMSO-*d*₆, ppm): δ 8.57 - 8.48 (m, 1H), 7.90 (s, 1H), 7.77 - 7.75 (m, 1H), 7.74 - 7.64 (m, 1H), 3.79 (s, 2H), 2.27 (s, 6H), 2.21 - 2.10 (m, 2H), 1.91 - 1.65 (m, 4H).

Step 2: 6-bromo-3-chloro-*N*-((1-(dimethylamino)cyclobutyl)methyl)imidazo[1',2':1,6]pyrido[3,2-*d*]pyrimidin-1-amine (1.3 g, 3.56 mmol) and ((2*R*,7a*S*)-2-fluorotetrahydro-1*H*-pyrrolizin-7a(5*H*)-yl)methanol (2.27 g, 14.24 mmol) were dissolved in THF (26 mL), and then NaH (1.42 g, 35.59 mmol) was added at 0 °C. The reaction mixture was warmed to 25 °C and stirred for 10 min, and then the reaction mixture was stirred at 70 °C under nitrogen for 16 h. The mixture was poured into a saturated NH₄Cl aqueous solution (100 mL) and extracted with ethyl acetate (50 mL*3). The combined organic layers were washed with saturated NaCl aqueous solution (50 mL*3), dried, and concentrated under reduced pressure to obtain the product. The residue was purified by silica gel chromatography and then purified using Prep-HPLC. 6-Bromo-*N*-((1-(dimethylamino)cyclobutyl)methyl)-3-(((2*R*,7a*S*)-2-fluorotetrahydro-1*H-*pyrrolizin-7a(5*H*)-yl)methoxy)imidazo[1',2':1,6]pyrido[3,2-*d*]pyrimidin-1-amine (720 mg, 1.39 mmol, 39% yield) was obtained as a yellow solid. LCMS (ES-API, m/z): [M+H]⁺= 488.2; ¹H NMR (400 MHz, DMSO-*d*₆, ppm): δ 8.42 (s, 1H), 7.83 (s, 1H), 7.66 - 7.54 (m, 1H), 7.40 - 7.20 (m, 1H), 5.38 - 5.14 (m, 1H), 4.15 - 3.95 (m, 2H), 3.85 - 3.72 (m, 2H), 3.15 - 3.05 (m, 2H), 3.04 - 2.98 (m, 1H), 2.88 - 2.78 (m, 1H), 2.29 - 2.22 (m, 6H), 2.19 - 2.11 (m, 3H), 2.06 - 2.02 (m, 1H), 2.01 - 1.94 (m, 1H), 1.88 - 1.64 (m, 7H).

Step 3: 6-bromo-*N*-((1-(dimethylamino)cyclobutyl)methyl)-3-(((2*R*,7a*S*)-2-fluorotetrahydro-1*H*-pyrrolizin-7a(5*H*)-yl)methoxy)imidazo[1',2':1,6]pyrido[3,2-*d*]pyrimidin-1-amine (150 mg, 307.38 µmol), 2-(8-Ethyl-7-fluoro-3-(methoxymethoxy)naphthalen-1-yl)-4,4,5,5-tetramethyl-1,3,2-dioxaborolane (132.87 mg, 368.85 µmol), PdCl₂(dtbpf) (20.03 mg, 30.74 µmol), K₃PO₄ (195.74 mg, 922.13 µmol) was dissolved in dioxane (2.4 mL) and water (0.6 mL), purged with nitrogen three times, and the mixture was stirred under nitrogen at 80 °C for 1.5 h. After completion of the reaction, the reaction mixture was diluted with water (20 mL), and the compound was extracted with ethyl acetate (15 mL*3). The organic layer was washed with saturated NaCl aqueous solution (15 mL), dried over anhydrous Na₂SO₄, and concentrated under reduced pressure. The residue was purified by silica gel chromatography to obtain N-((1-(dimethylamino)cyclobutyl)methyl)-6-(8-ethyl-7-fluoro-3-(methoxymethoxy)naphthalen-1-yl)-3-(((2*R*,7a*S*)-2-fluorotetrahydro-1*H*-pyrrolizin-7a(5*H*)-yl)methoxy)imidazo[1',2':1,6]pyrido[3,2-*d*]pyrimidin-1-amine (190 mg, 260.98 µmol, 85% yield) as a yellow solid. LCMS (ES-API, m/z): [M+H]⁺= 488.2; ¹H NMR (400 MHz, DMSO-*d*₆, ppm): δ 8.15 (s, 1H), 7.69 - 7.66 (m, 2H), 7.52 - 7.51 (m, 2H), 7.27 - 7.23 (m, 1H), 7.21 - 7.18 (m, 1H), 6.98 - 6.94 (m, 1H), 5.30 - 5.26 (m, 3H), 4.31 - 4.30 (m, 1H), 4.13 - 4.12 (m, 1H), 3.85 - 3.84 (m, 2H), 3.51 (s, 3H), 3.29 - 3.21 (m, 3H), 3.04 - 2.96 (m, 1H), 2.46-2.42 (m, 2H), 2.41 (s, 6H), 2.23 - 2.21 (m, 2H), 2.16 - 2.12 (m, 2H), 1.98 - 1.61 (m, 8H), 0.7 (t, *J* = 7.2 Hz, 3H).

Step 4: *N*-((1-(dimethylamino)cyclobutyl)methyl)-6-(8-ethyl-7-fluoro-3-(methoxymethoxy)naphthalen-1-yl)-3-(((2*R*,7a*S*)-2-fluorotetrahydro-1*H*-pyrrolizin-7a(5*H*)-yl)methoxy)imidazo[1',2':1,6]pyrido[3,2-*d*]pyrimidin-1-amine (100 mg, 145.81 µmol) obtained in Step 3 was dissolved in ACN (1 mL), HCl in dioxane (4 M, 0.5 mL) was added at 0 °C, and the mixture was stirred for 1 h. After completion of the reaction, the reaction mixture was concentrated under reduced pressure, and the residue was diluted with saturated NaHCO₃ solution (20 mL) and the compound was extracted with ethyl acetate (10 mL*3). The organic layer was washed with saturated NaCl aqueous solution, dried over anhydrous Na₂SO₄, and concentrated under reduced pressure. The residue was collected and purified by Prep-HPLC. 4-(1-(((1-(dimethylamino)cyclobutyl)methyl)amino)-3-(((2*R*,7a*S*)-2-fluorotetrahydro-1*H*-pyrrolizin-7a(5*H*)-yl)methoxy)imidazo[1',2':1,6]pyrido[3,2-d]pyrimidin-6-yl)-5-ethyl-6-fluoronaphthalen-2-ol (30.44 mg, 46.40 µmol, 32% yield) was obtained as a white solid. LCMS (ES-API, m/z): [M+H]⁺= 488.2; ¹H NMR (400 MHz, DMSO-*d*₆, ppm): δ 9.86 (br s, 1H), 8.39 (s, 1H), 7.75 (dd, *J* = 6.0, 9.2 Hz, 1H), 7.63 (s, 1H), 7.41 (s, 1H), 7.38 - 7.26 (m, 3H), 6.99 (d, *J* = 2.4 Hz, 1H), 5.40 - 5.17 (m, 1H), 4.16 - 4.01 (m, 2H), 3.92 (brs, 1H), 3.75 (brs, 1H), 3.16 - 2.97 (m, 3H), 2.88 - 2.79 (m, 1H), 2.47 - 2.40 (m, 1H), 2.29 (s, 6H), 2.24 - 2.11 (m, 3H), 2.10 - 1.96 (m, 3H), 1.93 - 1.67 (m, 7H), 0.60 (t, *J* = 7.2 Hz, 3H).

### EXAMPLE 5: Synthesis of 4-(1-(((1-(dimethylamino)cyclobutyl)methyl)amino)-3-(((2R,7aS)-2-fluorotetrahydro-1H-pyrrolizin-7a(5H)-yl)methoxy)imidazo[1',2':1,6]pyrido[3,2-d]pyrimidin-6-yl)-5,6-difluoronaphthalen-2-ol.

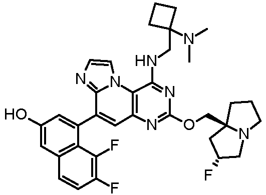

Instead of 2-(8-Ethyl-7-fluoro-3-(methoxymethoxy)naphthalen-1-yl)-4,4,5,5-tetramethyl-1,3,2-dioxaborolane, 2-(7,8-difluoro-3-(methoxymethoxy)naphthalen-1 -yl)-4,4,5,5-tetramethyl-1,3,2-dioxaborolane was used, and the synthesis was performed in the same manner as in EXAMPLE 4. 4-(1-(((1-(dimethylamino)cyclobutyl)methyl)amino)-3-(((2*R*,7a*S*)-2-fluorotetrahydro-1*H*-pyrrolizin-7a(5*H*)-yl)methoxy)imidazo[1',2':1,6]pyrido[3,2-*d*]pyrimidin-6-yl)-5,6-difluoronaphthalen-2-ol (46.13 mg, 72.23 µmol, 49% yield) was obtained as a gray solid.

LCMS (ES-API, m/z): [M+H]⁺= 632.5; ¹H NMR (400 MHz, DMSO-*d*₆, ppm): δ 10.34 - 10.07 (m, 1H), 8.45 - 8.26 (m, 1H), 7.75 - 7.67 (m, 1H), 7.64 - 7.60 (m, 1H), 7.57 - 7.48 (m, 1H), 7.40 - 7.29 (m, 3H), 7.23 - 7.10 (m, 1H), 5.42 - 5.14 (m, 1H), 4.15 - 4.01 (m, 2H), 3.96 - 3.83 (m, 1H), 3.81 - 3.71 (m, 1H), 3.13 - 2.98 (m, 3H), 2.89 - 2.77 (m, 1H), 2.32 - 2.26 (m, 6H), 2.25 - 2.12 (m, 3H), 2.10 - 1.97 (m, 2H), 1.91 - 1.68 (m, 7H).

### EXAMPLE 6: Synthesis of 4-(1-(((1-(dimethylamino)cyclobutyl)methyl)amino)-3-(((2R,7aS)-2-fluorotetrahydro-1H-pyrrolizin-7a(5H)-yl)methoxy)imidazo[1',2':1,6]pyrido[3,2-d]pyrimidin-6-yl)-5-ethynyl-6-fluoronaphthalen-2-ol.

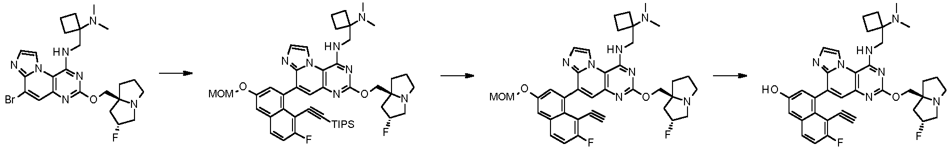

Step 1: 6-bromo-*N*-((1-(dimethylamino)cyclobutyl)methyl)-3-(((2*R*,7a*S*)-2-fluorotetrahydro-1*H*-pyrrolizin-7a(5*H*)-yl)methoxy)imidazo[1',2':1,6]pyrido[3,2-d]pyrimidin-1-amine (150 mg, 307.38 µmol), ((2-Fluoro-6-(methoxymethoxy)-8-(4,4,5,5-tetramethyl-1,3,2-dioxaborolan-2-yl)naphthalen-1-yl)ethynyl)triisopropylsilane (189.05 mg, 368.85 µmol), PdCl₂(dtbpf) (20.03 mg, 30.70 µmol), K₃PO₄ (195.74 mg, 922.13 µmol) was added to dioxane (2.4 mL) and water (0.6 mL), and the mixture was purged with nitrogen three times. The reaction mixture was stirred under nitrogen at 80 °C for 1.5 h. After completion of the reaction, the reaction mixture was diluted with water (20 mL), and the compound was extracted with ethyl acetate (15 mL*3). The organic layer was washed with saturated NaCl aqueous solution (15 mL), dried over anhydrous Na₂SO₄, and concentrated under reduced pressure. The residue was purified by silica gel chromatography. *N*-((1-(dimethylamino)cyclobutyl)methyl)-6-(7-fluoro-3-(methoxymethoxy)-8-((triisopropylsilyl)ethynyl)naphthalen-1-yl)-3-(((2*R*,7a*S*)-2-fluorotetrahydro-1*H*-pyrrolizin-7a(5*H*)-yl)methoxy)imidazo[1',2':1,6]pyrido[3,2-d]pyrimidin-1-amine (175 mg, 197.88 µmol, 64% yield) was obtained as a yellow solid. LCMS (ES-API, m/z): [M+H]⁺= 838.6; ¹H NMR (400 MHz, DMSO-*d*₆, ppm): δ 8.14 - 8.05 (m, 1H), 7.83 - 7.74 (m, 1H), 7.71 - 7.65 (m, 1H), 7.54 - 7.48 (m, 2H), 7.31 - 7.29 (m, 1H), 7.27 - 7.23 (m, 1H), 6.98 - 6.86 (m, 1H), 5.29 (s, 3H), 4.38 - 4.26 (m, 1H), 4.21 - 4.10 (m, 1H), 3.94 - 3.74 (m, 2H), 3.52 (s, 3H), 3.38 - 3.16 (m, 3H), 3.09 - 2.93 (m, 1H), 2.45 (br d, *J* = 10.8 Hz, 2H), 2.37 (s, 6H), 2.30 - 2.20 (m, 2H), 2.01 - 1.79 (m, 8H), 0.79 - 0.74 (m, 18H), 0.51 (quin, *J* = 7.2 Hz, 3H).

Step 2: *N*-((1-(dimethylamino)cyclobutyl)methyl)-6-(7-fluoro-3-(methoxymethoxy)-8-((triisopropylsilyl)ethynyl)naphthalen-1-yl)-3-(((2*R*,7a*S*)-2-fluorotetrahydro-1*H*-pyrrolizin-7a(5*H*)-yl)methoxy)imidazo[1',2':1,6]pyrido[3,2-d]pyrimidin-1-amine (145 mg, 173.01 µmol) obtained in Step 1 was dissolvd in DMF (1.5 mL), and then CsF (394.20 mg, 2.60 mmol) was added, and the mixture was stirred at 40 °C for 16 h. The reaction mixture was diluted with water (25 mL) and extracted with ethyl acetate (15 mL*3). The organic matter was washed with saturated NaCl aqueous solution (15 mL*2), dried over anhydrous Na₂SO₄, filtered and concentrated to obtain the residue. The residue was purified by prep-TLC to obtain *N*-((1-(dimethylamino)cyclobutyl)methyl)-6-(8-ethynyl-7-fluoro-3-(methoxymethoxy)naphthalen-1-yl)-3-(((2*R*,7a*S*)-2-fluorotetrahydro-1*H-*pyrrolizin-7a(5*H*)-yl)methoxy)imidazo[1',2':1,6]pyrido[3,2-*d*]pyrimidin-1-amine (90 mg, 128.62 µmol, 74% yield) as a yellow solid.

LCMS (ES-API, m/z): [M+H]⁺= 838.6; ¹H NMR (400 MHz, CDCl₃, ppm): δ 8.20 - 8.09 (m, 1H), 7.88 - 7.78 (m, 1H), 7.71 - 7.66 (m, 1H), 7.58 - 7.52 (m, 1H), 7.49 - 7.45 (m, 1H), 7.39 - 7.34 (m, 1H), 7.29 - 7.28 (m, 1H), 7.26 - 7.24 (m, 1H), 7.07 - 6.92 (m, 1H), 5.41 - 5.36 (m, 1H), 5.31 - 5.29 (m, 1H), 5.28 - 5.22 (m, 1H), 4.37 - 4.27 (m, 1H), 4.25 - 4.10 (m, 1H), 3.85 (br d, *J* = 2.4 Hz, 2H), 3.54 (s, 3H), 3.40 - 3.19 (m, 3H), 3.02 (br s, 1H), 2.62 - 2.56 (m, 1H), 2.48 - 2.40 (m, 2H), 2.36 (s, 6H), 2.27 (br d, *J* = 12.0 Hz, 2H), 2.04 - 1.78 (m, 8H).

Step 3: *N*-((1-(dimethylamino)cyclobutyl)methyl)-6-(8-ethynyl-7-fluoro-3-(methoxymethoxy)naphthalen-1-yl)-3-(((2*R*,7a*S*)-2-fluorotetrahydro-1*H*-pyrrolizin-7a(5*H*)-yl)methoxy)imidazo[1',2':1,6]pyrido[3,2-*d*]pyrimidin-1-amine (80 mg, 117.34 µmol) obtained in Step 2 was dissolved in acetonitrile (0.8 mL) and DCM (0.8 mL), and then HCl in dioxane (4 M, 800 µL) was added at 0 °C, and sttired for 1 h. After the starting material was completely consumed, the reaction mixture was concentrated under reduced pressure, and the residue was diluted with saturated NaHCO₃ solution (15 mL), and the compound was extracted with ethyl acetate (10 mL*3). The organic layer was washed with saturated NaCl aqueous solution (10 mL), dried over anhydrous Na₂SO₄, and concentrated under reduced pressure. The residue was purified by Prep-HPLC to obtain 4-(1-(((1-(dimethylamino)cyclobutyl)methyl)amino)-3-(((2*R*,7a*S*)-2-fluorotetrahydro-1*H-*pyrrolizin-7a(5*H*)-yl)methoxy)imidazo[1',2':1,6]pyrido[3,2-*d*]pyrimidin-6-yl)-5-ethynyl-6-fluoronaphthalen-2-ol (56.98 mg, 88.14 µmol, 75% yield) as an off-white solid.

LCMS (ES-API, m/z): [M+H]⁺= 838.6; ¹H NMR (400 MHz, DMSO-*d*₆, ppm): δ 10.34 - 9.88 (m, 1H), 8.32 (s, 1H), 7.95 (dd, *J* = 6.0, 9.2 Hz, 1H), 7.58 (s, 1H), 7.42 (t, *J* = 9.0 Hz, 1H), 7.36 (d, *J* = 2.4 Hz, 1H), 7.30 (s, 1H), 7.26 (br s, 1H), 7.17 (d, *J* = 2.0 Hz, 1H), 5.44 - 5.16 (m, 1H), 4.15 - 4.07 (m, 1H), 4.05 - 3.91 (m, 2H), 3.76 - 3.63 (m, 2H), 3.16 - 2.98 (m, 3H), 2.84 (br d, *J* = 6.0 Hz, 1H), 2.29 (s, 6H), 2.22 (br d, *J* = 9.6 Hz, 2H), 2.14 (br d, *J =* 5.6 Hz, 1H), 2.10 - 1.96 (m, 2H), 1.92 - 1.67 (m, 7H).

### EXAMPLE 7: Synthesis of 4-(1-((1R,5S)-3,8-diazabicyclo[3.2.1]octan-3-yl)-3-((1-(morpholinomethyl)cyclopropyl)methoxy)imidazo[1',2':1,6]pyrido[3,2-d]pyrimidin-6-yl)-5-ethynyl-6-fluoronaphthalen-2-ol.

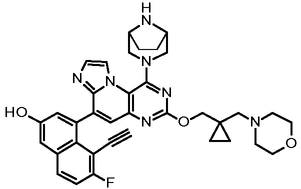

The compound was synthesized in the same manner as in EXAMPLE 2 using Intermediate 6. 4-(1-((1*R*,5*S*)-3,8-diazabicyclo[3.2.1]octan-3-yl)-3-((1-(morpholinomethyl)cyclopropyl)methoxy)imidazo[1',2':1,6]pyrido[3,2-d]pyrimidin-6-yl)-5-ethynyl-6-fluoronaphthalen-2-ol (35 mg, 55.22 µmol, 43% yield) was obtained as a yellow solid.

LCMS (ES-API, m/z): [M+H]⁺= 634.3; ¹H NMR (400 MHz, DMSO-*d*₆, ppm): δ 10.27 - 10.03 (m, 1H), 8.57 - 8.39 (m, 1H), 8.02 - 7.92 (m, 1H), 7.61 - 7.51 (m, 1H), 7.48 - 7.36 (m, 3H), 7.32 - 7.14 (m, 2H), 4.29 (s, 2H), 3.71 - 3.63 (m, 1H), 3.62 - 3.57 (m, 1H), 3.54 (br s, 4H), 3.51 - 3.48 (m, 1H), 3.47 - 3.42 (m, 1H), 3.25 - 3.13 (m, 1H), 3.03 - 2.88 (m, 1H), 2.45 - 2.29 (m, 6H), 2.26 - 2.05 (m, 1H), 1.98 - 1.68 (m, 2H), 1.64 - 1.35 (m, 2H), 0.71 - 0.60 (m, 2H), 0.47 - 0.39 (m, 2H).

### EXAMPLE 8: Synthesis of 4-(1-((1R,5S)-3,8-diazabicyclo[3.2.1]octan-3-yl)-3-((1-(((R)-3-methylmorpholino)methyl)cyclopropyl)methoxy)imidazo[1',2':1,6]pyrido[3,2-d]pyrimidin-6-yl)-5-ethynyl-6-fluoronaphthalen-2-ol.

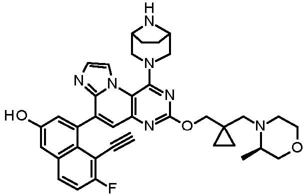

The compound was synthesize in the same manner as in EXAMPLE 2 using Intermediate 7. 4-(1-((1*R*,5*S*)-3,8-diazabicyclo[3.2.1]octan-3-yl)-3-((1-(((*R*)-3-methylmorpholino)methyl)cyclopropyl)methoxy)imidazo[1',2':1,6]pyrido[3,2-*d*]pyrimidin-6-yl)-5-ethynyl-6-fluoronaphthalen-2-ol (81.41 mg, 122.92 µmol, 51% yield) was obtained as a white solid.

LCMS (ES-API, m/z): [M+H]⁺= 648.4; ¹H NMR (400 MHz, DMSO-*d*₆, ppm): δ 0.31 - 0.39 (m, 1H), 0.46 - 0.61 (m, 2H), 0.62 - 0.73 (m, 1H), 0.84 (dd, *J =* 6.0, 3.2Hz, 3H), 1.36 - 1.66 (m, 3H), 1.68 - 1.93 (m, 2H), 2.03 - 2.19 (m, 2H), 2.21 - 2.35 (m, 2H), 2.83 - 3.08 (m, 3H), 3.39 - 3.55 (m, 6H), 3.61 - 3.72 (m, 2H), 3.79 - 4.11 (m, 2H), 4.45 - 4.73 (m, 1H), 7.15 - 7.24 (m, 1H), 7.26 - 7.43 (m, 3H), 7.43 - 7.59 (m, 1H), 7.96 (dd, *J* = 8.8, 6.0Hz, 2H), 9.87 - 10.22 (m, 1H).

### EXAMPLE 9: Synthesis of 4-(1-((1R,5S)-3,8-diazabicyclo[3.2.1]octan-3-yl)-3-((1-((1,1-difluoro-6-azaspiro[2.5]octan-6-yl)methyl)cyclopropyl)methoxy)imidazo[1',2':1,6]pyrido[3,2-d]pyrimidin-6-yl)-5-ethynyl-6-fluoronaphthalen-2-ol.

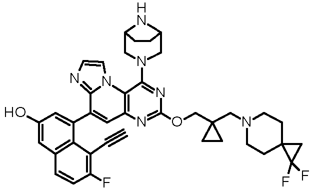

The compound was synthesized in the same manner as in EXAMPLE 2 using Intermediate 8. 4-(1-((1*R*,5*S*)-3,8-diazabicyclo[3.2.1]octan-3-yl)-3-((1-((1,1-difluoro-6-azaspiro[2.5]octan-6-yl)methyl)cyclopropyl)methoxy)imidazo[1',2':1,6]pyrido[3,2-d]pyrimidin-6-yl)-5-ethynyl-6-fluoronaphthalen-2-ol (66.62 mg, 94.29 µmol, 53% yield) was obtained as an off-white solid.

LCMS (ES-API, m/z): [M+H]⁺= 694.4; ¹H NMR (400 MHz, DMSO-*d*₆, ppm): δ 10.25 - 10.00 (m, 1H), 8.52 - 8.40 (m, 1H), 8.05 - 7.90 (m, 1H), 7.57 - 7.51 (m, 1H), 7.46 - 7.35 (m, 3H), 7.32 - 7.26 (m, 1H), 7.24 - 7.17 (m, 1H), 4.35 - 4.24 (m, 2H), 4.06 - 3.92 (m, 1H), 3.72 - 3.40 (m, 4H), 3.01 - 2.87 (m, 1H), 2.47 - 2.38 (m, 4H), 2.38 - 2.33 (m, 2H), 2.27 - 2.04 (m, 1H), 1.92 - 1.71 (m, 2H), 1.64 - 1.41 (m, 6H), 1.24 - 1.12 (m, 2H), 0.71 - 0.60 (m, 2H), 0.43 (br s, 2H).

### EXAMPLE 10: Synthesis of 4-(3-((1-(((1R,4R)-2-oxa-5-azabicyclo[2.2.1]heptan-5-yl)methyl)cyclopropyl)methoxy)-1-((1R,5S)-3,8-diazabicyclo[3.2.1]octan-3-yl)imidazo[1',2':1,6]pyrido[3,2-d]pyrimidin-6-yl)-5-ethynyl-6-fluoronaphthalen-2-ol.

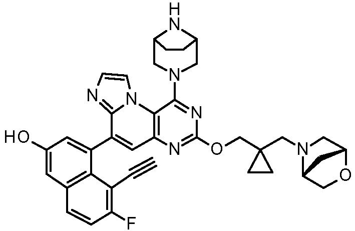

The compound was synthesized in the same manner as in EXAMPLE 2 using Intermediate 9. 4-(3-((1-(((1*R*,4*R*)-2-oxa-5-azabicyclo[2.2.1]heptan-5-yl)methyl)cyclopropyl)methoxy)-1-((1*R*,5*S*)-3,8-diazabicyclo[3.2.1]octan-3-yl)imidazo[1',2':1,6]pyrido[3,2-*d*]pyrimidin-6-yl)-5-ethynyl-6-fluoronaphthalen-2-ol (39 mg, 59.19 µmol, 31% yield) was obtained as a yellow solid.

LCMS (ES-API, m/z): [M+H]⁺= 646.2; ¹H NMR (400 MHz, DMSO-*d*₆, ppm): δ 10.21 - 10.02 (m, 1H), 8.51 - 8.40 (m, 1H), 8.02 - 7.90 (m, 2H), 7.58 - 7.50 (m, 1H), 7.47 - 7.36 (m, 3H), 7.30 - 7.25 (m, 1H), 7.24 - 7.16 (m, 1H), 4.38 - 4.28 (m, 2H), 4.27 - 4.17 (m, 1H), 3.84 - 3.74 (m, 1H), 3.58 (br s, 1H), 3.53 - 3.45 (m, 4H), 3.44 - 3.39 (m, 2H), 2.89 (s, 1H), 2.88 - 2.82 (m, 1H), 2.65 - 2.57 (m, 2H), 2.44 - 2.36 (m, 1H), 2.24 - 2.05 (m, 1H), 1.94 - 1.70 (m, 2H), 1.70 - 1.63 (m, 1H), 1.57 - 1.46 (m, 2H), 0.62 - 0.55 (m, 2H), 0.53 - 0.45 (m, 1H), 0.44 - 0.38 (m, 1H).

### EXAMPLE 11: Synthesis of 4-(1-((1R,5S)-3,8-diazabicyclo[3.2.1]octan-3-yl)-3-((1-(((R)-2-methylmorpholino)methyl)cyclopropyl)methoxy)imidazo[1',2':1,6]pyrido[3,2-d]pyrimidin-6-yl)-5-ethynyl-6-fluoronaphthalen-2-ol.

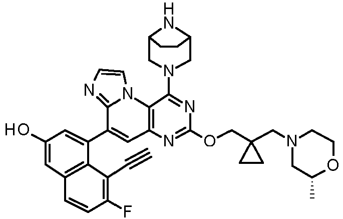

The compound was synthesized in the same manner as in EXAMPLE 2 using Intermediate 10. 4-(1-((1*R*,5*S*)-3,8-diazabicyclo[3.2.1]octan-3-yl)-3-((1-(((*R*)-2-methylmorpholino)methyl)cyclopropyl)methoxy)imidazo[1',2':1,6]pyrido[3,2-*d*]pyrimidin-6-yl)-5-ethynyl-6-fluoronaphthalen-2-ol (15 mg, 22.93 µmol, 20% yield) was obtained as a yellow solid.

LCMS (ES-API, m/z): [M+H]⁺= 648.5; ¹H NMR (400 MHz, DMSO-*d*₆, ppm): δ 10.23 - 9.97 (m, 1H), 8.54 - 8.37 (m, 1H), 8.03 - 7.89 (m, 1H), 7.58 - 7.15 (m, 5H), 4.36 - 4.21 (m, 2H), 4.05 - 3.93 (m, 1H), 3.84 - 3.61 (m, 2H), 3.60 - 3.52 (m, 1H), 3.51 - 3.35 (m, 5H), 2.99 - 2.72 (m, 2H), 2.43 - 2.23 (m, 4H), 2.16 - 2.06 (m, 1H), 2.03 - 1.89 (m, 1H), 1.88 - 1.75 (m, 1H), 1.73 - 1.59 (m, 1H), 1.58 - 1.39 (m, 2H), 1.05 - 0.95 (m, 3H), 0.71 - 0.59 (m, 2H), 0.46 - 0.38 (m, 2H).

### EXAMPLE 12: Synthesis of 4-(3-((1-(((1S,4S)-2-oxa-5-azabicyclo[2.2.1]heptan-5-yl)methyl)cyclopropyl)methoxy)-1-((1R,5S)-3,8-diazabicyclo[3.2.1]octan-3-yl)imidazo[1',2':1,6]pyrido[3,2-d]pyrimidin-6-yl)-5-ethynyl-6-fluoronaphthalen-2-ol.

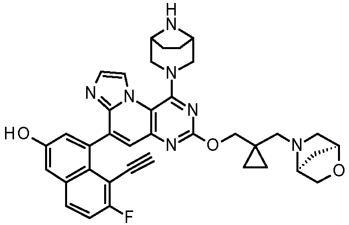

The compound was synthesize in the same manner as in EXAMPLE 2 using Intermediate 11. 4-(3-((1-(((1*S*,4*S*)-2-oxa-5-azabicyclo[2.2.1]heptan-5-yl)methyl)cyclopropyl)methoxy)-1-((1*R*,5*S*)-3,8-diazabicyclo[3.2.1]octan-3-yl)imidazo[1',2':1,6]pyrido[3,2-*d*]pyrimidin-6-yl)-5-ethynyl-6-fluoronaphthalen-2-ol (62 mg, 91.22 µmol, 40% yield) was obtained as a yellow solid.

LCMS (ES-API, m/z): [M+H]⁺= 646.5; ¹H NMR (400 MHz, DMSO-*d*₆, ppm): δ 10.16 - 10.03 (m, 1H), 8.50 - 8.40 (m, 1H), 8.02 - 7.90 (m, 1H), 7.58 - 7.48 (m, 1H), 7.46 - 7.41 (m, 1H), 7.41 - 7.35 (m, 2H), 7.32 - 7.25 (m, 1H), 7.24 - 7.16 (m, 1H), 4.40 - 4.27 (m, 2H), 4.26 - 4.19 (m, 1H), 4.07 - 3.90 (m, 1H), 3.82 - 3.74 (m, 1H), 3.68 - 3.55 (m, 2H), 3.53 - 3.38 (m, 5H), 2.99 - 2.89 (m, 1H), 2.88 - 2.79 (m, 1H), 2.69 - 2.60 (m, 2H), 2.45 - 2.35 (m, 2H), 2.27 - 2.04 (m, 1H), 1.85 - 1.65 (m, 2H), 1.57 - 1.43 (m, 2H), 0.67 - 0.54 (m, 2H), 0.53 - 0.36 (m, 2H).

### EXAMPLE 13: Synthesis of 3-(1-((1R,5S)-3,8-diazabicyclo[3.2.1]octan-3-yl)-3-(((2R,7aS)-2-fluorotetrahydro-1H-pyrrolizin-7a(5H)-yl)methoxy)imidazo[1',2':1,6]pyrido[3,2-d]pyrimidin-6-yl)-2,4,5,6-tetrafluoroaniline.

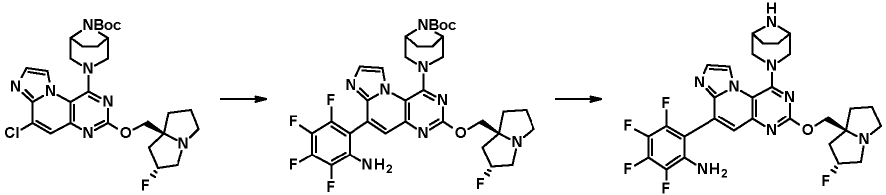

Step 1: In Step 1 of EXAMPLE 2, the copound was synthesized in the same manner using Intermediate 26 instead of ((2-Fluoro-6-(methoxymethoxy)-8-(4,4,5,5-tetramethyl-1,3,2-dioxaborolan-2-yl)naphthalen-1-yl)ethynyl)triisopropylsilane. *tert*-butyl (1*R*,5*S*)-3-(6-(2-amino-3,4,5,6-tetrafluorophenyl)-3-(((2*R*,7a*S*)-2-fluorotetrahydro-1*H*-pyrrolizin-7a(5*H*)-yl)methoxy)imidazo[1',2':1,6]pyrido[3,2-*d*]pyrimidin-1-yl)-3,8-diazabicyclo[3.2.1]octane-8-carboxylate (75 mg, 103.82 µmol, 27% yield) was obtained as a yellow solid.

LCMS (ES-API, m/z): [M+H]⁺= 701.4; ¹H NMR (400 MHz, CDCl₃, ppm): δ 8.68 - 8.53 (m, 1H), 7.83 - 7.72 (m, 1H), 7.69 - 7.63 (m, 1H), 7.59 - 7.49 (m, 1H), 5.39 - 5.21 (m, 2H), 4.36 - 4.28 (m, 1H), 4.23 (br d, *J* = 7.4 Hz, 3H), 4.17 - 4.09 (m, 1H), 3.88 - 3.54 (m, 2H), 3.45 - 3.36 (m, 1H), 3.26 (br s, 2H), 3.19 (br s, 1H), 3.05 - 2.95 (m, 1H), 2.37 - 2.11 (m, 5H), 2.02 - 1.80 (m, 7H), 1.55 - 1.47 (m, 9H).

Step 2: *tert*-butyl (1*R*,5*S*)-3-(6-(2-amino-3,4,5,6-tetrafluorophenyl)-3-(((2*R*,7a*S*)-2-fluorotetrahydro-1*H*-pyrrolizin-7a(5*H*)-yl)methoxy)imidazo[1',2':1,6]pyrido[3,2-d]pyrimidin-1-yl)-3,8-diazabicyclo[3.2.1]octane-8-carboxylate (75.00 mg, 107.04 µmol) obtained in Step 1 was dissolved in acetonitrile, and HCl in dioxane (4 M, 0.8 mL) was added dropwise. The reaction mixture was stirred at 0 °C for 1 h. Saturated NaHCO₃ aqueous solution was added, and then extracted with ethyl acetate. The crude compound was purified by prep-HPLC to obtain 3-(1-((1*R*,5*S*)-3,8-diazabicyclo[3.2.1]octan-3-yl)-3-(((2*R*,7a*S*)-2-fluorotetrahydro-1*H*-pyrrolizin-7a(5*H*)-yl)methoxy)imidazo[1',2':1,6]pyrido[3,2-*d*]pyrimidin-6-yl)-2,4,5,6-tetrafluoroaniline (15 mg, 24.98 µmol, 23% yield) as a white solid.

LCMS (ES-API, m/z): [M+H]⁺= 601.3; ¹H NMR (400 MHz, CDCl₃, ppm): δ 8.64 - 8.47 (m, 1H), 8.09 - 7.93 (m, 1H), 7.75 - 7.51 (m, 1H), 7.49 - 7.26 (m, 1H), 5.61 - 5.42 (m, 2H), 5.40 - 5.17 (m, 1H), 4.16 - 4.08 (m, 1H), 4.07 - 3.97 (m, 1H), 3.59 - 3.44 (m, 4H), 3.13 - 3.05 (m, 2H), 3.04 - 3.00 (m, 1H), 2.88 - 2.79 (m, 1H), 2.21 - 1.93 (m, 4H), 1.91 - 1.70 (m, 4H), 1.64 - 1.43 (m, 2H), 1.42 - 1.09 (m, 2H).

### EXAMPLE 14: Synthesis of 4-(1-((1R,5S)-3,8-diazabicyclo[3.2.1]octan-8-yl)-3-(((2R,7aS)-2-fluorotetrahydro-1H-pyrrolizin-7a(5H)-yl)methoxy)imidazo[1',2':1,6]pyrido[3,2-d]pyrimidin-6-yl)-5-ethynyl-6-fluoronaphthalen-2-amine.

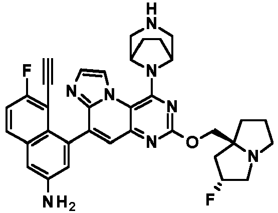

The compound was synthesized in the same manner as in EXAMPLE 2 using *tert*-Butyl (1*R*,5*S*)-3,8-diazabicyclo[3.2.1]octane-3-carboxylate and intermediate 18. 4-(1-((1*R,5S)-*3,8-Diazabicyclo[3.2.1]octan-8-yl)-3-(((2*R*,7a*S*)-2-fluorotetrahydro-1*H*-pyrrolizin-7a(5*H*)-yl)methoxy)imidazo[1',2':1,6]pyrido[3,2-*d*]pyrimidin-6-yl)-5-ethynyl-6-fluoronaphthalen-2-amine (12.71 mg, 20.21 µmol, 15% yield) was obtained as a yellow solid.

LCMS (ES-API, m/z): [M+H]⁺= 621.5; ¹H NMR (400 MHz, CDCl₃, ppm): δ 8.78 - 8.59 (m, 1H), 7.80 - 7.69 (m, 1H), 7.48 (s, 1H), 7.35 - 7.26 (m, 2H), 7.10 - 7.00 (m, 2H), 5.67 - 5.53 (m, 2H), 5.40 - 5.16 (m, 1H), 4.22 (br dd, *J* = 4.4, 6.4 Hz, 1H), 4.14 - 4.07 (m, 1H), 4.05 - 3.97 (m, 1H), 3.91 (br s, 1H), 3.56 - 3.38 (m, 1H), 3.31 - 3.19 (m, 2H), 3.18 - 3.04 (m, 3H), 3.02 (s, 1H), 2.88 - 2.80 (m, 1H), 2.80 - 2.70 (m, 1H), 2.18 - 2.12 (m, 1H), 2.11 - 1.93 (m, 4H), 1.92 - 1.66 (m, 5H).

### EXAMPLE 15: Synthesis of 4-(1-((1R,5S)-3,8-diazabicyclo[3.2.1]octan-8-yl)-3-(((2R,7aS)-2-fluorotetrahydro-1H-pyrrolizin-7a(5H)-yl)methoxy)imidazo[1',2':1,6]pyrido[3,2-d]pyrimidin-6-yl)-7-fluorobenzo[d]thiazol-2-amine.

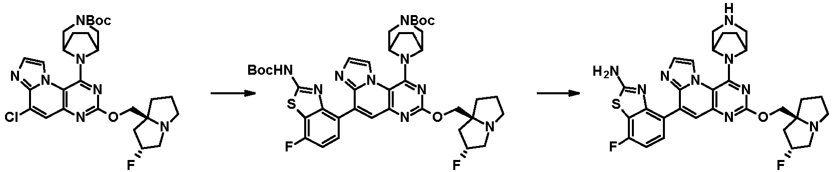

Step 1: (2-((*tert*-Butoxycarbonyl)amino)-7-fluorobenzo[*b*]thiophen-4-yl)boronic acid (220 mg, 563.88 µmol) and *tert*-butyl (1*R*,5*S*)-8-(6-chloro-3-(((2*R*,7a*S*)-2-fluorotetrahydro-1*H-*pyrrolizin-7a(5*H*)-yl)methoxy)imidazo[1',2':1,6]pyrido[3,2-*d*]pyrimidin-1-yl)-3,8-diazabicyclo[3.2.1]octane-3-carboxylate (280 mg, 489.45 µmol) was dissolved in 1,4-dioxane (5 mL) and water (1 mL), then K₃PO₄ (311.68 mg, 1.47 mmol) and Pd(amphos)Cl₂ (31.90 mg, 48.94 µmol) were added and nitrogen purged three times. And then, the reaction mixture was stirred at 80 °C for 12 hours. After completion of the reaction, purification was performed by silica gel chromatography to obtain tert-butyl (1*R*,5*S*)-8-(6-(2-((*tert*-butoxycarbonyl)amino)-7-fluorobenzo[*d*]thiazol-4-yl)-3-(((2*R*,7a*S*)-2-fluorotetrahydro-1*H*-pyrrolizin-7a(5*H*)-yl)methoxy)imidazo[1',2':1,6]pyrido[3,2-*d*]pyrimidin-1-yl)-3,8-diazabicyclo[3.2.1]octane-3-carboxylate (280 mg, 318.67 µmol, 65% yield).

LCMS (ES-API, m/z): [M+H]⁺= 804.7; ¹H NMR (400 MHz, DMSO-*d*₆, ppm): δ 12.23 (br s, 1H), 9.05 - 8.51 (m, 1H), 8.17 (dd, *J* = 5.8, 8.6 Hz, 1H), 7.92 (s, 1H), 7.67 (s, 1H), 7.33 (t, *J =* 8.8 Hz, 1H), 5.39 -5.19 (m, 1H), 4.28 (br d, *J* = 11.0 Hz, 2H), 4.17 - 4.12 (m, 1H), 4.06 (d, *J* = 9.8 Hz, 1H), 3.19 - 2.99 (m, 4H), 2.88 - 2.78 (m, 1H), 2.21 - 2.00 (m, 4H), 1.93 - 1.66 (m, 7H), 1.53 - 1.50 (m, 9H), 1.43 (br s, 9H).

Step 2: *tert*-butyl (1*R*,5*S*)-8-(6-(2-((*tert*-butoxycarbonyl)amino)-7-fluorobenzo[*d*]thiazol-4-yl)-3-(((2*R*,7a*S*)-2-fluorotetrahydro-1*H*-pyrrolizin-7a(5*H*)-yl)methoxy)imidazo[1',2':1,6]pyrido[3,2-*d*]pyrimidin-1-yl)-3,8-diazabicyclo[3.2.1]octane-3-carboxylate (227.62 mg, 323.42 µmol) obtained in Step 1 was dissolved in ACN, HCl in dioxane (4 M, 0.5 mL) was added and stirred at 0 °C for 2 h. After completion of the reaction, the compound was purified by Prep-HPLC to obtain 4-(1-((1*R*,5*S*)-3,8-diazabicyclo[3.2.1]octan-8-yl)-3-(((2*R*,7a*S*)-2-fluorotetrahydro-1*H*-pyrrolizin-7a(5*H*)-yl)methoxy)imidazo[1',2':1,6]pyrido[3,2-*d*]pyrimidin-6-yl)-7-fluorobenzo[*d*]thiazol-2-amine (80 mg, 127.62 µmol, 39% yield) as a yellow solid.

LCMS (ES-API, m/z): M+H⁺= 604.3; ¹H NMR (400 MHz, DMSO-*d*₆, ppm): *δ* 8.93 - 8.61 (m, 1H), 8.07 - 7.96 (m, 3H), 7.92 (s, 1H), 7.67 (s, 1H), 7.08 (t, *J* = 8.9 Hz, 1H), 5.42 - 5.13 (m, 1H), 4.29 - 4.05 (m, 3H), 4.04 - 3.96 (m, 1H), 3.31 - 2.94 (m, 6H), 2.89 - 2.70 (m, 2H), 2.26 - 1.95 (m, 5H), 1.94 - 1.58 (m, 6H).

### EXAMPLE 16: Synthesis of 4-(1-((1R,5S)-3,8-diazabicyclo[3.2.1]octan-8-yl)-3-((1-(((R)-3-fluoropyrrolidin-1-yl)methyl)cyclopropyl)methoxy)imidazo[1',2':1,6]pyrido[3,2-d]pyrimidin-6-yl)-5-ethynyl-6-fluoronaphthalen-2-ol.

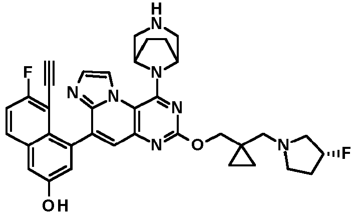

The compound was synthesized in the same manner as in EXAMPLE 2 using Intermediate 14. 4-(1-((1*R*,5*S*)-3,8-diazabicyclo[3.2.1]octan-8-yl)-3-((1-(((*R*)-3-fluoropyrrolidin-1-yl)methyl)cyclopropyl)methoxy)imidazo[1',2':1,6]pyrido[3,2-*d*]pyrimidin-6-yl)-5-ethynyl-6-fluoronaphthalen-2-ol (70.42 mg, 108.56 µmol, 56% yield) was obtained as a yellow solid.

LCMS (ES-API, m/z): [M+H]⁺= 636.4; ¹H NMR (400 MHz, DMSO-*d*₆, ppm): δ 0.45 (s, 2 H), 0.56 - 0.67 (m, 2 H), 0.68 - 0.88 (m, 1 H), 1.11 - 1.31 (m, 2 H), 1.74 - 1.93 (m, 3 H), 2.01 - 2.18 (m, 2 H), 2.28 - 2.34 (m, 1 H), 2.38 (br dd, J = 12.0, 9.07 Hz, 1 H), 2.57 (br d, *J* = 4.4 Hz, 1 H), 2.60 - 2.68 (m, 1 H), 2.73 - 2.82 (m, 2 H), 2.87 (br d, *J* = 11.2 Hz, 1 H), 3.08 - 3.28 (m, 2 H), 3.52 - 3.64 (m, 1 H), 3.83 - 3.98 (m, 1 H), 4.07 - 4.26 (m, 2 H), 4.27 - 4.33 (m, 1 H), 5.03 - 5.30 (m, 1 H), 7.21 (d, *J* = 2.4 Hz, 1 H), 7.35 (s, 1 H), 7.37 (d, *J =* 2.4 Hz, 1 H), 7.42 (t, J = 9.2 Hz, 1 H), 7.47 (s, 1 H), 7.96 (dd, *J* = 9.2, 6.00 Hz, 1 H), 8.30 - 8.93 (m, 1 H), 9.79 - 10.39 (m, 1 H).

### EXAMPLE 17: Synthesis of 4-(1-((1R,5S)-3,8-diazabicyclo[3.2.1]octan-8-yl)-3-((1-(pyrrolidin-1-ylmethyl)cyclopropyl)methoxy)imidazo[1',2':1,6]pyrido[3,2-d]pyrimidin-6-yl)-5-ethynyl-6-fluoronaphthalen-2-ol.

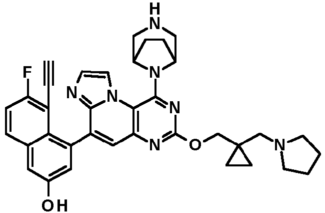

The compound was synthesized in the same manner as in EXAMPLE 2 using Intermediate 13. 4-(1-((1*R*,5*S*)-3,8-diazabicyclo[3.2.1]octan-8-yl)-3-((1-(pyrrolidin-1-ylmethyl)cyclopropyl)methoxy)imidazo[1',2':1,6]pyrido[3,2-*d*]pyrimidin-6-yl)-5-ethynyl-6-fluoronaphthalen-2-ol (20 mg, 32.05 µmol, 19% yield) was obtained as a pale yellow solid. LCMS (ES-API, m/z): [M+H]⁺= 618.3; ¹H NMR (400 MHz, DMSO-*d*₆, ppm): δ 10.18 - 10.08 (m, 1H), 8.78 - 8.60 (m, 1H), 7.96 (dd, *J* = 6..0, 9.2 Hz, 1H), 7.46 (br d, *J* = 9.2 Hz, 1H), 7.44 - 7.39 (m, 1H), 7.37 (d, *J* = 2.4 Hz, 1H), 7.35 (s, 1H), 7.21 (d, *J* = 2.4 Hz, 1H), 4.25 (s, 3H), 4.01 - 3.78 (m, 1H), 3.67 - 3.48 (m, 1H), 3.27 (br d, *J* = 12.4 Hz, 2H), 2.81 - 2.70 (m, 1H), 2.47 - 2.37 (m, 8H), 1.91 - 1.78 (m, 2H), 1.65 (br s, 5H), 0.62 - 0.59 (m, 2H), 0.45 - 0.41 (m, 2H).

### EXAMPLE 18: Synthesis of 1-((1R,5S)-3,8-diazabicyclo[3.2.1]octan-8-yl)-3-(((2R,7aS)-2-fluorotetrahydro-1H-pyrrolizin-7a(5H)-yl)methoxy)-6-(6-methyl-5-(trifluoromethyl)-1H-indazol-4-yl)imidazo[1',2':1,6]pyrido[3,2-d]pyrimidine.

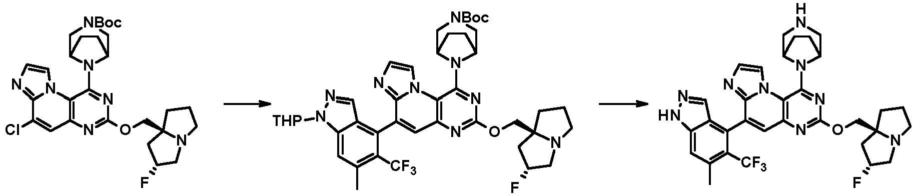

Step 1: Intermediate 21 (210 mg, 640.06 µmol) and tert-butyl (1*R*,5*S*)-8-(6-chloro-3-(((2*R*,7a*S*)-2-fluorotetrahydro-1*H*-pyrrolizin-7a(5*H*)-yl)methoxy)imidazo[1',2':1,6]pyrido[3,2-d]pyrimidin-1-yl)-3,8-diazabicyclo[3.2.1]octane-3-carboxylate (347.85 mg, 608.06 µmol) were dissolved in 1,4-dioxane (2.5 mL) and water (0.5 mL), and then Cs₂CO₃ (625.63 mg, 1.92 mmol) and cataCXium A Pd G4 (46.68 mg, 64.01 µmol) was added. The reaction mixture was stirred at 90 °C for two hours under nitrogen. After completion of the reaction, purification was performed by Prep-HPLC to obtain *tert*-butyl (1*R*,5*S*)-8-(3-(((2*R*,7a*S*)-2-fluorotetrahydro-1*H*-pyrrolizin-7a(5*H*)-yl)methoxy)-6-(6-methyl-1-(tetrahydro-2*H*-pyran-2-yl)-5-(trifluoromethyl)-1*H*-indazol-4-yl)imidazo[1',2':1,6]pyrido[3,2-*d*]pyrimidin-1-yl)-3,8-diazabicyclo[3.2.1]octane-3-carboxylate (279 mg, 309.66 µmol, 48% yield) as a yellow solid.

LCMS (ES-API, m/z): [M+H]⁺= 820.5 ; ¹H NMR (400 MHz, CDCD₃, ppm): *δ* 8.70 (br s, 1H), 7.67 (s, 1H), 7.63 - 7.59 (m, 1H), 7.54 (br s, 1H), 7.42 (d, *J* = 7.2 Hz, 1H), 5.82 - 5.66 (m, 1H), 5.42 - 5.16 (m, 1H), 4.63 - 4.37 (m, 1H), 4.34 - 4.17 (m, 2H), 4.10 - 3.99 (m, 2H), 3.97 - 3.88 (m, 1H), 3.85 - 3.68 (m, 2H), 3.61 - 3.37 (m, 2H), 3.32 - 3.11 (m, 3H), 3.07 - 2.95 (m, 1H), 2.73 (br s, 3H), 2.61 - 2.42 (m, 1H), 2.35 - 2.10 (m, 5H), 2.02 - 1.93 (m, 3H), 1.91 - 1.66 (m, 7H), 1.53 - 1.45 (m, 9H).

Step 2: tert-butyl (1*R*,5*S*)-8-(3-(((2*R*,7a*S*)-2-fluorotetrahydro-1*H*-pyrrolizin-7a(5*H*)-yl)methoxy)-6-(6-methyl-1-(tetrahydro-2*H*-pyran-2-yl)-5-(trifluoromethyl)-1*H*-indazol-4-yl)imidazo[1',2':1,6]pyrido[3,2-*d*]pyrimidin-1-yl)-3,8-diazabicyclo[3.2.1]octane-3-carboxylate (100 mg, 121.97 µmol) obtained in Step 1 was dissolved in DCM (1 mL), and then TFA (767.50 mg, 6.73 mmol) was added. The reaction mixture was reacted at room temperature for 1 h. After completion of the reaction, the reaction compound was purified by Prep-HPLC. 1-((1*R*,5*S*)-3,8-diazabicyclo[3.2.1]octan-8-yl)-3-(((2*R*,7a*S*)-2-fluorotetrahydro-1*H*-pyrrolizin-7a(5*H*)-yl)methoxy)-6-(6-methyl-5-(trifluoromethyl)-1*H-*indazol-4-yl)imidazo[1',2':1,6]pyrido[3,2-d]pyrimidine (25.17 mg, 38.80 µmol, 32% yield) was obtained as a white solid.

LCMS (ES-API, m/z): [M+H]⁺= 636.3; ¹H NMR (400 MHz, DMSO-*d*₆, ppm): δ 13.52 - 13.32 (m, 1H), 9.05 - 8.48 (m, 1H), 7.74 - 7.63 (m, 2H), 7.59 - 7.51 (m, 1H), 7.34 (s, 1H), 5.37 - 5.18 (m, 1H), 4.17 (br s, 1H), 4.10 (s, 1H), 4.07 - 4.01 (m, 1H), 3.28 (s, 3H), 3.09 (br dd, *J* = 2.0, 8.0 Hz, 2H), 3.05 - 3.00 (m, 1H), 2.87 - 2.74 (m, 2H), 2.66 (br d, *J* = 1.6 Hz, 3H), 2.47 - 2.41 (m, 2H), 2.21 - 2.08 (m, 2H), 2.06 (br s, 3H), 1.90 - 1.70 (m, 5H).

### EXAMPLE 19: Synthesis of 4-(1-((1R,5S)-3,8-diazabicyclo[3.2.1]octan-8-yl)-3-((1-(((S)-3-fluoropiperidin-1-yl)methyl)cyclopropyl)methoxy)imidazo[1',2':1,6]pyrido[3,2-d]pyrimidin-6-yl)-5-ethynyl-6-fluoronaphthalen-2-ol.

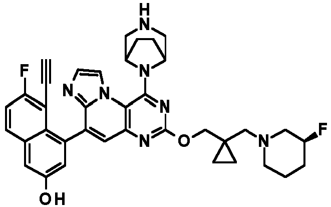

The compound was synthesized in the same manner as in EXAMPLE 2 using Intermediate 12. 4-(1-((1*R*,5*S*)-3,8-diazabicyclo[3.2.1]octan-8-yl)-3-((1-(((*S*)-3-fluoropiperidin-1-yl)methyl)cyclopropyl)methoxy)imidazo[1',2':1,6]pyrido[3,2-*d*]pyrimidin-6-yl)-5-ethynyl-6-fluoronaphthalen-2-ol (23 mg, 35.05 µmol, 28% yield) was obtained as a yellow solid.

LCMS (ES-API, m/z): [M+H]⁺= 650.5; ¹H NMR (400 MHz, DMSO-*d*₆, ppm): δ 10.18 - 10.04 (m, 1H), 8.82 - 8.58 (m, 1H), 7.96 (dd, *J* = 6.0, 9.2 Hz, 1H), 7.48 (s, 1H), 7.42 (t, *J* = 9.2 Hz, 1H), 7.39 - 7.33 (m, 2H), 7.22 (d, *J* = 2.4 Hz, 1H), 4.69 - 4.48 (m, 1H), 4.46 - 4.04 (m, 4H), 4.03 - 3.75 (m, 1H), 3.72 - 3.38 (m, 2H), 3.29 - 3.13 (m, 2H), 2.96 - 2.69 (m, 3H), 2.36 (br s, 3H), 2.28 - 2.15 (m, 2H), 2.13 - 1.94 (m, 1H), 1.93 - 1.73 (m, 3H), 1.71 - 1.62 (m, 1H), 1.54 - 1.34 (m, 2H), 0.65 (s, 2H), 0.43 (s, 2H).

### EXAMPLE 20: Synthesis of 6-(1-((1R,5S)-3,8-diazabicyclo[3.2.1]octan-8-yl)-3-(((2R,7aS)-2-fluorotetrahydro-1H-pyrrolizin-7a(5H)-yl)methoxy)imidazo[1',2':1,6]pyrido[3,2-d]pyrimidin-6-yl)-4-methyl-5-(trifluoromethyl)pyridin-2-amine.

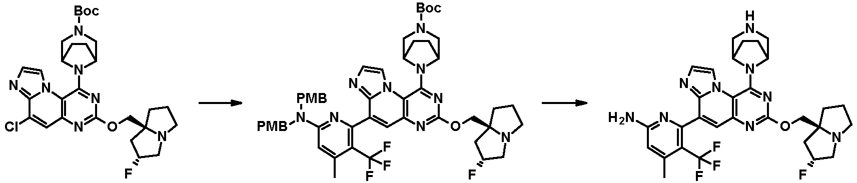

Step 1: *tert*-Butyl (1*R*,5*S*)-3-(6-chloro-3-(((2*R*,7a*S*)-2-fluorotetrahydro-1*H*-pyrrolizin-7a(5*H*)-yl)methoxy)imidazo[1',2':1,6]pyrido[3,2-*d*]pyrimidin-1-yl)-3,8-diazabicyclo[3.2.1]octane-8-carboxylate (62.28 mg, 108.86 µmol), intermediate 23 (64 mg, 90.72 µmol), Cul (4.32 mg, 22.68 µmol), LiCl (9.61 mg, 226.80 µmol), cataCXium Pd G4 (16.54 mg, 22.68 µmol) were dissolved simultaneously in 1,4-dioxane (2 mL) and then purged with nitrogen three times. Then, the reaction mixture was stirred at 110 °C for 12 h. After completion of the reaction, purification was performed by silica gel chromatography to obtain *tert*-butyl (1*R*,5*S*)-8-(6-(6-(bis(4-methoxybenzyl)amino)-4-methyl-3-(trifluoromethyl)pyridin-2-yl)-3-(((2*R*,7a*S*)-2-fluorotetrahydro-1*H*-pyrrolizin-7a(5*H*)-yl)methoxy)imidazo[1',2':1,6]pyrido[3,2-*d*]pyrimidin-1-yl)-3,8-diazabicyclo[3.2.1]octane-3-carboxylate (31 mg, 29.31 µmol, 32% yield) as a yellow solid. LCMS (ES-API, m/z): [M+H]⁺= 952.4; ¹H NMR (400 MHz, DMSO-*d*₆, ppm): δ 1.41 (br s, 9 H), 1.56 - 1.62 (m, 6 H), 1.69 (br s, 4 H), 1.89 - 1.94 (m, 4 H), 2.39 (br s, 3 H), 2.77 - 2.88 (m, 2 H), 3.05 - 3.13 (m, 2 H), 3.73 (s, 6 H), 4.02 - 4.14 (m, 2 H), 4.16 - 4.32 (m, 2 H), 4.59 - 4.75 (m, 4 H), 5.13 - 5.39 (m, 1 H), 6.79 (s, 1 H), 6.87 (br d, *J* = 8.0 Hz, 4 H), 7.19 (d, *J* = 8.4 Hz, 5 H), 7.26 - 7.29 (m, 1 H), 7.64 - 7.69 (m, 1 H).

Step 3: tert-butyl (1*R*,5*S*)-8-(6-(6-(bis(4-methoxybenzyl)amino)-4-methyl-3-(trifluoromethyl)pyridin-2-yl)-3-(((2*R*,7a*S*)-2-fluorotetrahydro-1*H*-pyrrolizin-7a(5*H*)-yl)methoxy)imidazo[1',2':1,6]pyrido[3,2-*d*]pyrimidin-1-yl)-3,8-diazabicyclo[3.2.1]octane-3-carboxylate (24 mg, 25.21 µmol) obtained in Step 2 was dissolved in dichloroethane (0.24 mL), and then TFA (368.40 mg, 3.23 mmol) was added. The reaction mixture was stirred at 60 °C for 1 h. The reaction was quenched by adding saturated NaHCO₃ solution, and extracted with ethyl acetate. The reaction mixture was purified by Prep-HPLC to obtain 6-(1-((1*R*,5*S*)-3,8-diazabicyclo[3.2.1]octan-8-yl)-3-(((2*R*,7a*S*)-2-fluorotetrahydro-1*H-*pyrrolizin-7a(5*H*)-yl)methoxy)imidazo[1',2':1,6]pyrido[3,2-*d*]pyrimidin-6-yl)-4-methyl-5-(trifluoromethyl)pyridin-2-amine (3.61 mg, 4.93 µmol, 20% yield).

LCMS (ES-API, m/z): [M+H]⁺= 612.4; ¹H NMR (400 MHz, DMSO-*d*₆, ppm): δ 1.19 - 1.28 (m, 2 H), 1.70 - 1.87 (m, 4 H), 1.96 - 2.08 (m, 3 H), 2.09 - 2.24 (m, 2 H), 2.37 (s, 3 H), 2.71 - 2.86 (m, 2 H), 3.01 (s, 1 H), 3.08 (br d, *J* = 6.4 Hz, 2 H), 3.13 - 3.23 (m, 2 H), 3.23 - 3.28 (m, 2 H), 3.92 - 4.27 (m, 4 H), 5.11 - 5.42 (m, 1 H), 6.50 (s, 1 H), 6.73 (s, 2 H), 7.18 (s, 1 H), 7.59 (s, 1 H).

### EXAMPLE 21: Synthesis of 4-(1-((1R,5S)-3,8-diazabicyclo[3.2.1]octan-8-yl)-3-(((R)-2-methylenetetrahydro-1H-pyrrolizin-7a(5H)-yl)methoxy)imidazo[1',2':1,6]pyrido[3,2-d]pyrimidin-6-yl)-5-ethynyl-6-fluoronaphthalen-2-ol.

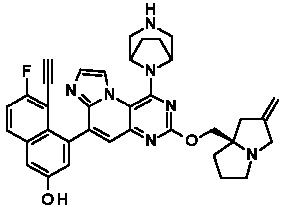

The compound was synthesized in the same manner as in EXAMPLE 2 using (*R*)-(2-Methylenetetrahydro-1*H*-pyrrolizin-7a(5*H*)-yl)methanol. 4-(1-((1*R*,5*S*)-3,8-Diazabicyclo[3.2.1]octan-8-yl)-3-(((*R*)-2-methylenetetrahydro-1*H*-pyrrolizin-7a(5*H*)-yl)methoxy)imidazo[1',2':1,6]pyrido[3,2-*d*]pyrimidin-6-yl)-5-ethynyl-6-fluoronaphthalen-2-ol (32.76 mg, 122.23 µmol, 36% yield) was obtained as a yellow solid.

LCMS (ES-API, m/z): [M+H]⁺= 616.3; ¹H NMR (400 MHz, MeOD, ppm): δ 8.87 - 8.62 (m, 1H), 7.82 (dd, *J* = 5.8, 9.1 Hz, 1H), 7.51 (s, 1H), 7.41 (s, 1H), 7.32 (d, *J* = 2.5 Hz, 1H), 7.29 - 7.21 (m, 2H), 5.00 (s, 2H), 4.53 - 4.23 (m, 4H), 3.78 (br d, *J* = 14.4 Hz, 1H), 3.55 - 3.34 (m, 2H), 3.25 - 3.15 (m, 1H), 3.09 - 2.58 (m, 6H), 2.51 (br d, *J* = 15.9 Hz, 1H), 2.26 - 1.80 (m, 8H), 1.36 - 1.21 (m, 2H).

### EXAMPLE 22: Synthesis of 1-((1R,5S)-3,8-diazabicyclo[3.2.1]octan-8-yl)-6-(8-ethynyl-7-fluoronaphthalen-1-yl)-3-(((S,Z)-2-(fluoromethylene)tetrahydro-1H-pyrrolizin-7a(5H)-yl)methoxy)imidazo[1',2':1,6]pyrido[3,2-d]pyrimidine.

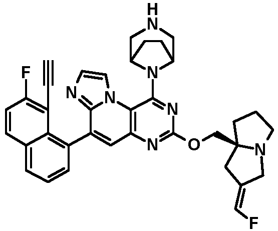

The compound was synthesized in the same manner as in EXAMPLE 2 using ((2-Fluoro-8-(4,4,5,5-tetramethyl-1,3,2-dioxaborolan-2-yl)naphthalen-1-yl)ethynyl)triisopropylsilane and (*S*,*Z*)-(2-(fluoromethylene)tetrahydro-1*H*-pyrrolizin-7a(5*H*)-yl)methanol. 1-((1*R*,5*S*)-3,8-diazabicyclo[3.2.1]octan-8-yl)-6-(8-ethynyl-7-fluoronaphthalen-1-yl)-3-(((*S*,*Z*)-2-(fluoromethylene)tetrahydro-1*H*-pyrrolizin-7a(5*H*)-yl)methoxy)imidazo[1',2':1,6]pyrido[3,2-*d*]pyrimidine (56.5 mg, 90.56 µmol, 50% yield) was obtained as a yellow solid.

LCMS (ES-API, m/z): [M+H]⁺= 618.3; ¹H NMR (400 MHz, DMSO-*d*₆, ppm): δ 1.67 - 1.73 (m, 1 H), 1.74 - 1.83 (m, 2 H), 1.83 - 1.92 (m, 2 H), 1.92 - 2.07 (m, 2 H), 2.08 - 2.27 (m, 1 H), 2.28 - 2.39 (m, 1 H), 2.52 - 2.65 (m, 3 H), 2.66 - 2.90 (m, 2 H), 3.01 (dt, *J* = 9.6, 4.91 Hz, 1 H), 3.08 - 3.32 (m, 3 H), 3.71 (br d, *J* = 14.8 Hz, 2 H), 3.93 (br dd, *J* = 2.8, 1.38 Hz, 1 H), 3.99 - 4.11 (m, 2 H), 4.20 (br d, *J* = 2.4 Hz, 1 H), 6.63 - 6.90 (m, 1 H), 7.39 (s, 1 H), 7.47 (br s, 1 H), 7.57 (t, *J* = 9.2 Hz, 1 H), 7.64 - 7.72 (m, 2 H), 8.15 - 8.24 (m, 2 H), 8.59 - 8.79 (m, 1 H).

### EXAMPLE 23: Synthesis of 4-(1-((1R,5S)-3,8-diazabicyclo[3.2.1]octan-8-yl)-3-(((S,Z)-2-(fluoromethylene)tetrahydro-1H-pyrrolizin-7a(5H)-yl)methoxy)imidazo[1',2':1,6]pyrido[3,2-d]pyrimidin-6-yl)-5-ethynyl-6-fluoronaphthalen-2-ol.

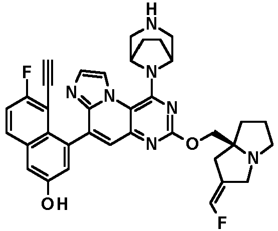

The compound was synthesized in the same manner as in EXAMPLE 2 using (*S,Z*)-(2-(fluoromethylene)tetrahydro-1*H*-pyrrolizin-7a(5*H*)-yl)methanol. 4-(1-((1*R,*5*S*)-3,8-diazabicyclo[3.2.1]octan-8-yl)-3-(((*S,Z*)-2-(fluoromethylene)tetrahydro-1*H*-pyrrolizin-7a(5H)-yl)methoxy)imidazo[1',2':1,6]pyrido[3,2-*d*]pyrimidin-6-yl)-5-ethynyl-6-fluoronaphthalen-2-ol (8 mg, 12.27 µmol, 13% yield) was obtained as a white solid. LCMS (ES-API, m/z): [M+H]⁺= 634.3; ¹H NMR (400 MHz, DMSO-*d*₆, ppm): δ 10.12 (brs, 1H), 8.86 - 8.56 (m, 1H), 7.97 (dd, *J* = 6.0, 9.2 Hz, 1H), 7.49 (s, 1H), 7.43 (t, *J* = 9.2 Hz, 1H), 7.40 - 7.33 (m, 2H), 7.22 (d, *J* = 2.0 Hz, 1H), 7.01 (t, *J* = 7.6 Hz, 1H), 6.90 - 6.63 (m, 1H), 5.61 (d, *J* = 7.6 Hz, 1H), 5.28 (br s, 2H), 4.20 (br s, 1H), 4.10 - 4.09 (m, 1H), 4.11 - 4.01 (m, 2H), 3.72 (br d, *J* = 15.2 Hz, 1H), 3.27 (br s, 3H), 3.11 - 2.90 (m, 2H), 2.85 - 2.70 (m, 1H), 2.63 - 2.53 (m, 3H), 2.40 - 2.30 (m, 1H), 2.12 - 1.94 (m, 2H), 1.92 - 1.68 (m, 5H).

### EXAMPLE 24: Synthesis of 4-(1-((1R,5S)-3,8-diazabicyclo[3.2.1]octan-3-yl)-3-(((2R,7aS)-2-fluorotetrahydro-1H-pyrrolizin-7a(5H)-yl)methoxy)imidazo[1',2':1,6]pyrido[3,2-d]pyrimidin-6-yl)-5-ethynyl-6-fluoronaphthalen-2-amine.

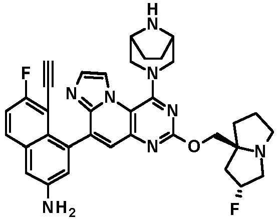

The compound was synthesized in the same manner as in EXAMPLE 2 using intermediate 18 instead of ((2-Fluoro-6-(methoxymethoxy)-8-(4,4,5,5-tetramethyl-1,3,2-dioxaborolan-2-yl)naphthalen-1-yl)ethynyl)triisopropylsilane. 4-(1-((1*R*,5*S*)-3,8-diazabicyclo[3.2.1]octan-3-yl)-3-(((2*R*,7aS)-2-fluorotetrahydro-1*H*-pyrrolizin-7a(5*H*)-yl)methoxy)imidazo[1',2':1,6]pyrido[3,2-*d*]pyrimidin-6-yl)-5-ethynyl-6-fluoronaphthalen-2-amine (38 mg, 59.51 µmol, 47% yield) was obtained as a yellow solid.

LCMS (ES-API, m/z): [M+H]⁺= 621.3; ¹H NMR (400 MHz, DMSO-*d*₆, ppm): δ 8.46 (s , 0.44H), 7.96 (s , 0.45H), 7.74 - 7.78 (m, 1H), 7.30 (d, *J* = 9.2Hz, 2H), 7.24 - 7.28 (m, 2H), 7.04 - 7.07 (m, 2H), 5.59 (s, 2H), 5.36 (d, *J* = 55.6Hz, 1H), 4.01 - 4.13 (m, 2H), 3.33 - 3.59 (m , 4H), 2.98 - 3.15 (m, 4H), 2.78 - 2.88 (m, 1H), 1.95 - 2.23 (m, 5H), 1.78 - 1.84 (m, 5H), 1.53 (brs, 1H).

### EXAMPLE 25: Synthesis of 4-(1-((1R,5S)-3,8-diazabicyclo[3.2.1]octan-3-yl)-3-(((2R,7aS)-2-fluorotetrahydro-1H-pyrrolizin-7a(5H)-yl)methoxy)imidazo[1',2':1,6]pyrido[3,2-d]pyrimidin-6-yl)-7-fluorobenzo[d]thiazol-2-amine.

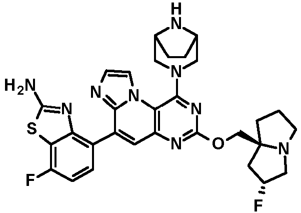

The compound was synthesized in the same manner as in EXAMPLE 15 using tert-Butyl (1*R*,5*S*)-3,8-diazabicyclo[3.2.1]octane-8-carboxylate. 4-(1-((1*R*,5*S*)-3,8-Diazabicyclo[3.2.1]octan-3-yl)-3-(((2*R*,7a*S*)-2-fluorotetrahydro-1*H*-pyrrolizin-7a(5*H*)-yl)methoxy)imidazo[1',2':1,6]pyrido[3,2-*d*]pyrimidin-6-yl)-7-fluorobenzo[d]thiazol-2-amine (71.61 mg, 118.61 µmol, 53% yield) was obtained as a yellow solid.

LCMS (ES-API, m/z): [M+H]⁺= 604.3; ¹H NMR (400 MHz, DMSO-*d*₆, ppm): δ 8.53 (br d, *J* = 2.8 Hz, 1H), 8.07 - 7.94 (m, 4H), 7.93 - 7.55 (m, 2H), 7.06 (t, *J* = 8.8 Hz, 1H), 5.40 - 5.19 (m, 1H), 4.15 - 3.97 (m, 2H), 3.74 - 3.62 (m, 1H), 3.54 (br d, *J* = 13.2 Hz, 2H), 3.39 (br s, 1H), 3.15 - 2.99 (m, 4H), 2.89 - 2.80 (m, 1H), 2.21 - 1.96 (m, 4H), 1.91 - 1.72 (m, 4H), 1.65 - 1.41 (m, 3H).

### EXAMPLE 26: Synthesis of 1-((1R,5S)-3,8-diazabicyclo[3.2.1]octan-3-yl)-3-(((2R,7aS)-2-fluorotetrahydro-1H-pyrrolizin-7a(5H)-yl)methoxy)-6-(6-methyl-5-(trifluoromethyl)-1H-indazol-4-yl)imidazo[1',2':1,6]pyrido[3,2-d]pyrimidine.

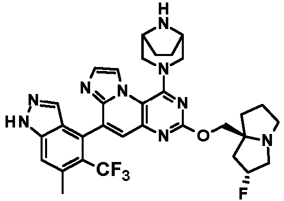

The compound was synthesized in the same manner as in EXAMPLE 18 using *tert*-Butyl (1*R*,5*S*)-3,8-diazabicyclo[3.2.1]octane-8-carboxylate. 1-((1*R*,5*S*)-3,8-Diazabicyclo[3.2.1]octan-3-yl)-3-(((2*R*,7a*S*)-2-fluorotetrahydro-1*H*-pyrrolizin-7a(5*H*)-yl)methoxy)-6-(6-methyl-5-(trifluoromethyl)-1*H*-indazol-4-yl)imidazo[1 ',2': 1 ,6]pyrido[3,2-d]pyrimidine (25.36 mg, 39.89 µmol, 23% yield) was obtained as a white solid.

LCMS (ES-API, m/z): [M+H]⁺= 636.3; ¹H NMR (400 MHz, DMSO-*d*₆, ppm): δ 13.44 (br s, 1H), 8.58 (br s, 1H), 8.02 (br s, 1H), 7.74 - 7.63 (m, 2H), 7.51 - 7.24 (m, 2H), 5.41 - 5.15 (m, 1H), 4.18 - 3.97 (m, 2H), 3.81 - 3.37 (m, 5H), 3.16 - 3.00 (m, 4H), 2.89 - 2.78 (m, 1H), 2.66 (br d, *J* = 1.6 Hz, 3H), 2.23 - 1.95 (m, 4H), 1.94 - 1.71 (m, 4H), 1.63 - 1.47 (m, 2H), 1.29 - 1.21 (m, 1H).

### EXAMPLE 27: Synthesis of 1-((1R,5S)-3,8-diazabicyclo[3.2.1]octan-3-yl)-6-(8-ethynyl-7-fluoronaphthalen-1-yl)-3-(((S,Z)-2-(fluoromethylene)tetrahydro-1H-pyrrolizin-7a(5H)-yl)methoxy)imidazo[1',2':1,6]pyrido[3,2-d]pyrimidine.

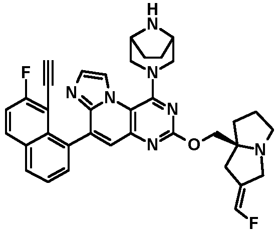

The compound was synthesized in the same manner as in EXAMPLE 2 using ((2-Fluoro-8-(4,4,5,5-tetramethyl-1,3,2-dioxaborolan-2-yl)naphthalen-1-yl)ethynyl)triisopropylsilane and (*S,Z*)-(2-(fluoromethylene)tetrahydro-1*H*-pyrrolizin-7a(5*H*)-yl)methanol. 1-((1*R*,5*S*)-3,8-Diazabicyclo[3.2.1]octan-3-yl)-6-(8-ethynyl-7-fluoronaphthalen-1-yl)-3-(((*S,Z*)-2-(fluoromethylene)tetrahydro-1*H*-pyrrolizin-7a(5*H*)-yl)methoxy)imidazo[1',2':1,6]pyrido[3,2-*d*]pyrimidine (28 mg, 44.88 µmol, 40 % yield) was obtained as a white solid.

LCMS (ES-API, m/z): [M+H]⁺= 618.3; ¹H NMR (400 MHz, DMSO-*d*₆, ppm): δ 8.45 (br d, *J* = 1.5 Hz, 1H), 8.26 - 8.12 (m, 2H), 7.97 (br s, 1H), 7.68 (br s, 2H), 7.60 - 7.54 (m, 1H), 7.41 (br d, *J* = 7.5 Hz, 2H), 6.89 - 6.61 (m, 1H), 4.12 - 3.99 (m, 2H), 3.97 - 3.86 (m, 1H), 3.77 - 3.63 (m, 2H), 3.61 - 3.47 (m, 2H), 3.43 (br s, 2H), 3.09 - 2.91 (m, 2H), 2.63 - 2.52 (m, 3H), 2.34 (br d, *J* = 14.4 Hz, 1H), 2.24 - 2.07 (m, 1H), 2.02 - 1.94 (m, 1H), 1.93 - 1.62 (m, 5H), 1.55 - 1.42 (m, 1H).

### EXAMPLE 28: Synthesis of 1-((1R,5S)-3,8-diazabicyclo[3.2.1]octan-3-yl)-6-(8-ethynyl-7-fluoronaphthalen-1-yl)-3-(((2R,7aS)-2-fluorotetrahydro-1H-pyrrolizin-7a(5H)-yl)methoxy)imidazo[1',2':1,6]pyrido[3,2-d]pyrimidine.

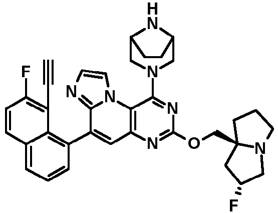

The compound was synthesized in the same manner as in EXAMPLE 2 using ((2-Fluoro-8-(4,4,5,5-tetramethyl-1,3,2-dioxaborolan-2-yl)naphthalen-1-yl)ethynyl)triisopropylsilane. 1-((1*R*,5*S*)-3,8-Diazabicyclo[3.2.1]octan-3-yl)-6-(8-ethynyl-7-fluoronaphthalen-1-yl)-3-(((2*R*,7a*S*)-2-fluorotetrahydro-1*H*-pyrrolizin-7a(5*H*)-yl)methoxy)imidazo[1',2':1,6]pyrido[3,2-*d*]pyrimidine (42.24 mg, 69.25 µmol, 49% yield) was obtained as a yellow solid.

LCMS (ES-API, m/z): [M+H]⁺= 606.4; ¹H NMR (400 MHz, DMSO-*d*₆, ppm): δ 8.4 (br s, 1H), 8.25 - 8.13 (m, 2H), 7.97 (br s, 1H), 7.67 (br d, *J* = 4.8 Hz, 2H), 7.60 - 7.51 (m, 1H), 7.51 - 7.26 (m, 2H), 5.38 - 5.20 (m, 1H), 4.17 - 3.98 (m, 2H), 3.98 - 3.70 (m, 1H), 3.70 - 3.37 (m, 4H), 3.31 - 3.24 (m, 2H), 3.20 - 2.90 (m, 4H), 2.89 - 2.79 (m, 1H), 2.25 - 1.96 (m, 4H), 1.92 - 1.40 (m, 6H).

### EXAMPLE 29: Synthesis of 4-(1-((1R,5S)-3,8-diazabicyclo[3.2.1]octan-3-yl)-3-(((S,Z)-2-(fluoromethylene)tetrahydro-1H-pyrrolizin-7a(5H)-yl)methoxy)imidazo[1',2':1,6]pyrido[3,2-d]pyrimidin-6-yl)-5-ethynyl-6-fluoronaphthalen-2-ol.

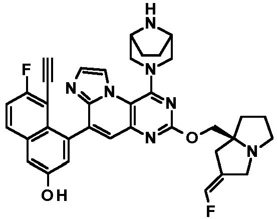

The compound was synthesized in the same manner as in EXAMPLE 2 using (*S,Z*)-(2-(fluoromethylene)tetrahydro-1*H*-pyrrolizin-7a(5*H*)-yl)methanol. 4-(1-((1*R*,5*S*)-3,8-Diazabicyclo[3.2.1]octan-3-yl)-3-(((*S,Z*)-2-(fluoromethylene)tetrahydro-1*H*-pyrrolizin-7a(5H)-yl)methoxy)imidazo[1',2':1,6]pyrido[3,2-*d*]pyrimidin-6-yl)-5-ethynyl-6-fluoronaphthalen-2-ol (50 mg, 78.11 µmol, 25% yield) was obtained as a white solid. LCMS (ES-API, m/z): [M+H]⁺= 634.2; ¹H NMR (400 MHz, DMSO-*d*₆, ppm): δ 10.40 - 9.90 (m, 1H), 8.45 (br s, 1H), 7.96 (dd, *J* = 6.0, 9.1 Hz, 1H), 7.54 (br s, 1H), 7.50 - 7.25 (m, 4H), 7.21 (br s, 1H), 6.94 - 6.56 (m, 1H), 4.11 - 3.90 (m, 2H), 3.74 - 3.41 (m, 5H), 3.29 - 3.23 (m, 3H), 3.05 - 2.91 (m, 2H), 2.74 - 2.52 (m, 2H), 2.34 (br d, *J* = 15.1 Hz, 2H), 2.11 (br d, *J* = 1.1 Hz, 1H), 2.02 - 1.66 (m, 6H), 1.60 - 1.31 (m, 2H).

### EXAMPLE 30: Synthesis of 4-(1-((1R,5S)-3,8-diazabicyclo[3.2.1]octan-3-yl)-3-((1-(((R)-3-fluoropyrrolidin-1-yl)methyl)cyclopropyl)methoxy)imidazo[1',2':1,6]pyrido[3,2-d]pyrimidin-6-yl)-5-ethynyl-6-fluoronaphthalen-2-ol.

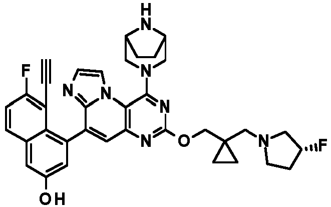

The compound was synthesized in the same manner as in EXAMPLE 2 using Intermediate 14. 4-(1-((1R,5S)-3,8-Diazabicyclo[3.2.1]octan-3-yl)-3-((1-(((R)-3-fluoropyrrolidin-1-yl)methyl)cyclopropyl)methoxy)imidazo[1',2':1,6]pyrido[3,2-d]pyrimidin-6-yl)-5-ethynyl-6-fluoronaphthalen-2-ol (22 mg, 33.92 µmol, 33% yield) was obtained as a white solid.

LCMS (ES-API, m/z): [M+H]⁺= 636.4; ¹H NMR (400 MHz, DMSO-*d*₆, ppm): δ 10.28 - 9.95 (m, 1H), 8.46 (br s, 1H), 7.97 (br dd, *J* = 6.1, 8.9 Hz, 1H), 7.54 (br d, *J* = 1.0 Hz, 1H), 7.47 - 7.35 (m, 3H), 7.33 - 7.26 (m, 1H), 7.21 (br s, 1H), 5.10 (br s, 1H), 4.39 - 4.13 (m, 2H), 4.04 - 3.92 (m, 1H), 3.75 - 3.54 (m, 2H), 3.51 - 3.43 (m, 2H), 2.90 - 2.78 (m, 2H), 2.68 - 2.60 (m, 1H), 2.43 - 2.27 (m, 3H), 2.26 - 1.96 (m, 3H), 1.93 - 1.69 (m, 3H), 1.55 - 1.41 (m, 1H), 1.31 - 1.22 (m, 1H), 0.64 (br s, 2H), 0.46 (br s, 2H).

### EXAMPLE 31: Synthesis of 4-(1-((1R,5S)-3,8-diazabicyclo[3.2.1]octan-3-yl)-3-((1-(((S)-3-fluoropiperidin-1-yl)methyl)cyclopropyl)methoxy)imidazo[1',2':1,6]pyrido[3,2-d]pyrimidin-6-yl)-5-ethynyl-6-fluoronaphthalen-2-ol.

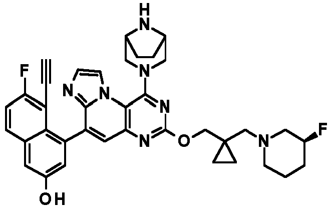

The compound was synthesized in the same manner as in EXAMPLE 2 using Intermediate 12. 4-(1-((1*R*,5*S*)-3,8-Diazabicyclo[3.2.1]octan-3-yl)-3-((1-(((*S*)-3-fluoropiperidin-1-yl)methyl)cyclopropyl)methoxy)imidazo[1',2':1,6]pyrido[3,2-*d*]pyrimidin-6-yl)-5-ethynyl-6-fluoronaphthalen-2-ol (22.27 mg, 33.86 µmol, 30% yield) was obtained as a yellow solid.

LCMS (ES-API, m/z): [M+H]⁺= 650.4; ¹H NMR (400 MHz, DMSO-*d*₆, ppm): δ 10.1 (s , 0.93H), 8.44 (s , 0.45H), 7.94 - 7.98 (m, 1H), 7.53 (s, 0.48H), 7.36 - 7.44 (m, 3H), 7.20 (d, J = 30.8Hz, 1.6H), 4.52 (d, *J* = 47.6Hz, 2H), 4.22 - 4.31 (m, 2H), 3.97 (brs, 0.48H), 3.47 - 3.65 (m , 4H), 2.63 - 3.03 (m, 2H), 2.22 - 2.36 (m, 6H), 1.60 - 1.88 (m, 4H), 1.33 - 1.58 (m, 4H), 0.65 (s, 2H), 0.42 (s, 2H).

### EXAMPLE 32: Synthesis of 4-(1-((1R,5S)-3,8-diazabicyclo[3.2.1]octan-3-yl)-3-(((2S,7aR)-2-fluoro-6-methylenetetrahydro-1H-pyrrolizin-7a(5H)-yl)methoxy)imidazo[1',2':1,6]pyrido[3,2-d]pyrimidin-6-yl)-5-ethynyl-6-fluoronaphthalen-2-ol.

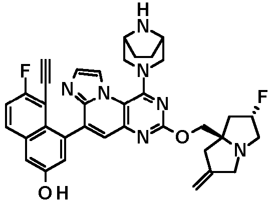

The compound was synthesized in the same manner as in EXAMPLE 2 using Intermediate 16. 4-(1-((1*R*,5*S*)-3,8-Diazabicyclo[3.2.1]octan-3-yl)-3-(((2*S*,7a*R*)-2-fluoro-6-methylenetetrahydro-1*H*-pyrrolizin-7a(5*H*)-yl)methoxy)imidazo[1',2':1,6]pyrido[3,2-*d*]pyrimidin-6-yl)-5-ethynyl-6-fluoronaphthalen-2-ol was obtained as a yellow solid. LCMS (ES-API, m/z): [M+H]⁺= 634.4; ¹H NMR (400 MHz, DMSO-*d*₆, ppm): δ 10.83 - 9.19 (m, 1H), 8.45 (br s, 1H), 7.95 (dd, *J* = 6.0, 9.2 Hz, 1H), 7.59 - 7.25 (m, 4H), 7.20 (br s, 1H), 5.46 - 5.19 (m, 1H), 4.92 (br s, 2H), 4.15 - 3.79 (m, 2H), 3.74 - 3.55 (m, 4H), 3.30 - 3.11 (m, 5H), 3.09 - 2.90 (m, 2H), 2.62 (br s, 2H), 2.27 - 2.05 (m, 3H), 1.94 - 1.62 (m, 2H), 1.60 - 1.20 (m, 2H).

### EXAMPLE 33: Synthesis of 4-(1-((1R,5S)-3,8-diazabicyclo[3.2.1]octan-3-yl)-3-(((R)-2-methylenetetrahydro-1H-pyrrolizin-7a(5H)-yl)methoxy)imidazo[1',2':1,6]pyrido[3,2-d]pyrimidin-6-yl)-5-ethynyl-6-fluoronaphthalen-2-ol.

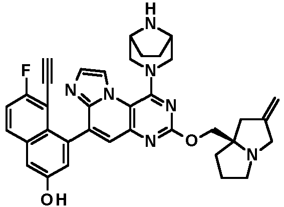

The compound was synthesized in the same manner as in EXAMPLE 2 using (*R*)-(2-methylenetetrahydro-1*H*-pyrrolizin-7a(5*H*)-yl)methanol instead of ((2*R*,7a*S*)-2-fluorotetrahydro-1*H*-pyrrolizin-7a(5*H*)-yl)methanol. 4-(1-((1*R,*5*S*)-3,8-Diazabicyclo[3.2.1]octan-3-yl)-3-(((*R*)-2-methylenetetrahydro-1*H*-pyrrolizin-7a(5H)-yl)methoxy)imidazo[1',2':1,6]pyrido[3,2-*d*]pyrimidin-6-yl)-5-ethynyl-6-fluoronaphthalen-2-ol (15 mg, 48.73 µmol, 37% yield) was obtained as a white solid.

LCMS (ES-API, m/z): [M+H]⁺= 616.3; ¹H NMR (400 MHz, DMSO-*d*₆, ppm): δ 10.36 - 9.87 (m, 1H), 8.45 (br s, 1H), 7.96 (dd, *J* = 6.0, 9.1 Hz, 1H), 7.54 (br s, 1H), 7.46 - 7.25 (m, 4H), 7.20 (br s, 1H), 4.90 (br s, 2H), 4.07 - 3.93 (m, 2H), 3.72 - 3.40 (m, 6H), 3.20 (br d, *J* = 13.9 Hz, 2H), 3.05 - 2.89 (m, 2H), 2.69 - 2.53 (m, 2H), 2.37 (br d, *J* = 15.6 Hz, 1H), 2.25 - 2.05 (m, 1H), 1.98 (ddd, *J* = 4.4, 7.1, 11.7 Hz, 1H), 1.93 - 1.64 (m, 5H), 1.60 - 1.22 (m, 2H).

### EXAMPLE 34: Synthesis of 6-(1-((1R,5S)-3,8-diazabicyclo[3.2.1]octan-3-yl)-3-(((2R,7aS)-2-fluorotetrahydro-1H-pyrrolizin-7a(5H)-yl)methoxy)imidazo[1',2':1,6]pyrido[3,2-d]pyrimidin-6-yl)-4-methyl-5-(trifluoromethyl)pyridin-2-amine.

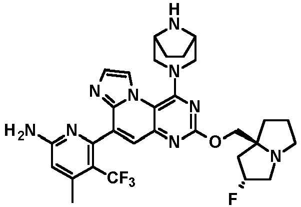

The compound was synthesized in the same manner as in EXAMPLE 20 using tert-butyl (1*R*,5*S*)-3,8-diazabicyclo[3.2.1]octane-8-carboxylate. 6-(1-((1*R,*5*S*)-3,8-diazabicyclo[3.2.1]octan-3-yl)-3-(((2*R*,7a*S*)-2-fluorotetrahydro-1*H*-pyrrolizin-7a(5*H*)-yl)methoxy)imidazo[1',2':1,6]pyrido[3,2-*d*]pyrimidin-6-yl)-4-methyl-5-(trifluoromethyl)pyridin-2-amine (5.11 mg, 11.44 µmol, 14.5% yield) was obtained as an off-white solid.

LCMS (ES-API, m/z): [M+H]⁺= 616.3; ¹H NMR (400 MHz, DMSO-*d*₆, ppm): δ 8.49 (br d, *J* = 1.2 Hz, 1H), 7.98 (br s, 1H), 7.75 - 7.46 (m, 1H), 7.27 - 7.07 (m, 1H), 6.70 (br s, 2H), 6.50 (s, 1H), 5.39 - 5.14 (m, 1H), 4.16 - 3.93 (m, 2H), 3.76 - 3.44 (m, 4H), 3.25 - 3.17 (m, 1H), 3.14 - 2.94 (m, 4H), 2.90 - 2.75 (m, 1H), 2.37 (br d, *J* = 1.6 Hz, 3H), 2.21 - 1.94 (m, 4H), 1.90 - 1.69 (m, 4H), 1.63 - 1.18 (m, 3H).

### EXAMPLE 35: Synthesis of 4-(1-((1R,5S)-3,8-diazabicyclo[3.2.1]octan-3-yl)-3-((1-(pyrrolidin-1-ylmethyl)cyclopropyl)methoxy)imidazo[1',2':1,6]pyrido[3,2-d]pyrimidin-6-yl)-5-ethynyl-6-fluoronaphthalen-2-ol.

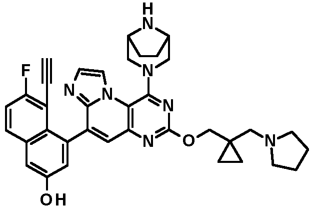

The compound was synthesized in the same manner as in EXAMPLE 2 using Intermediate 13. 4-(1-((1*R*,5*S*)-3,8-diazabicyclo[3.2.1]octan-3-yl)-3-((1-(pyrrolidin-1-ylmethyl)cyclopropyl)methoxy)imidazo[1',2':1,6]pyrido[3,2-*d*]pyrimidin-6-yl)-5-ethynyl-6-fluoronaphthalen-2-ol (95.78 mg, 148.54 µmol, 42% yield) was obtained as a yellow solid. LCMS (ES-API, m/z): [M+H]⁺= 618.3; ¹H NMR (400 MHz, DMSO-*d*₆, ppm): δ 9.81 - 10.39 (m, 1 H), 8.44 (br s, 1 H), 7.96 (dd, *J* = 9.2, 6.00 Hz, 1 H), 7.26 - 7.57 (m, 4 H), 7.21 (br s, 1 H), 4.26 (s, 2 H), 3.89 - 4.08 (m, 1 H), 3.54 - 3.75 (m, 2 H), 3.38 - 3.53 (m, 4 H), 2.82 - 3.06 (m, 1 H), 2.38 - 2.47 (m, 6 H), 1.70 - 2.26 (m, 3 H), 1.21 - 1.57 (m, 2 H), 0.61 (br s, 2 H), 0.43 (s, 2 H).

### EXAMPLE 36: Synthesis of 4-(1-((1R,5S)-3,8-diazabicyclo[3.2.1]octan-3-yl)-3-(((S,Z)-2-(fluoromethylene)tetrahydro-1H-pyrrolizin-7a(5H)-yl)methoxy)imidazo[1',2':1,6]pyrido[3,2-d]pyrimidin-6-yl)-5-ethynyl-6-fluoronaphthalen-2-amine.

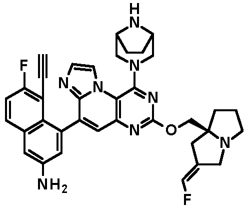

The compound was synthesized in the same manner as in EXAMPLE 2 using Intermediate 18 and (*S,Z*)-(2-(fluoromethylene)tetrahydro-1*H*-pyrrolizin-7a(5*H*)-yl)methanol. 4-(1-((1*R*,5*S*)-3,8-Diazabicyclo[3.2.1]octan-3-yl)-3-(((*S,Z*)-2-(fluoromethylene)tetrahydro-1*H*-pyrrolizin-7a(5*H*)-yl)methoxy)imidazo[1',2':1,6]pyrido[3,2-*d*]pyrimidin-6-yl)-5-ethynyl-6-fluoronaphthalen-2-amine (36.17 mg, 56.71 µmol, 28% yield) was obtained as a yellow solid.

LCMS (ES-API, m/z): [M+H]⁺= 633.4; ¹H NMR (400 MHz, DMSO-*d*₆, ppm): δ 8.45 (br s, 1H), 7.96 (br s, 1H), 7.81 - 7.72 (m, 1H), 7.59 - 7.39 (m, 1H), 7.37 - 7.23 (m, 2H), 7.12 - 7.01 (m, 2H), 6.91 - 6.63 (m, 1H), 5.59 (br s, 2H), 4.14 - 3.95 (m, 2H), 3.71 (d, *J* = 14.8 Hz, 1H), 3.67 - 3.38 (m, 4H), 3.28 (br s, 2H), 3.08 - 2.88 (m, 2H), 2.62 - 2.54 (m, 2H), 2.34 (br d, *J* = 14.8 Hz, 1H), 2.25 - 2.04 (m, 1H), 2.02 - 1.93 (m, 1H), 1.93 - 1.67 (m, 4H), 1.62 - 1.34 (m, 2H).

### EXAMPLE 37: Synthesis of 4-(1-((1R,5S)-3,8-diazabicyclo[3.2.1]octan-3-yl)-3-(((S)-2-(difluoromethylene)tetrahydro-1H-pyrrolizin-7a(5H)-yl)methoxy)imidazo[1',2':1,6]pyrido[3,2-d]pyrimidin-6-yl)-5-ethynyl-6-fluoronaphthalen-2-ol.

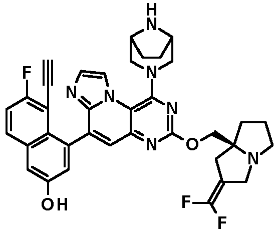

The compound was synthesized in the same manner as in EXAMPLE 2 using (7a*R*)-2-(difluoromethylene)tetrahydro-1*H*-pyrrolizine-7a(5*H*)-methanol. 4-(1-((1*R*,5*S*)-3,8-Diazabicyclo[3.2.1]octan-3-yl)-3-(((*S*)-2-(difluoromethylene)tetrahydro-1*H*-pyrrolizin-7a(5*H*)-yl)methoxy)imidazo[1',2':1,6]pyrido[3,2-*d*]pyrimidin-6-yl)-5-ethynyl-6-fluoronaphthalen-2-ol (79.66 mg, 122.23 µmol, 36% yield) was obtained as a yellow solid. LCMS (ES-API, m/z): [M+H]⁺= 652.2; ¹H NMR (400 MHz, DMSO-*d*₆, ppm): δ 10.25 - 9.94 (m, 1H), 8.45 (br s, 1H), 7.96 (dd, *J* = 6.0, 9.2 Hz, 1H), 7.58 - 7.26 (m, 4H), 7.20 (br s, 1H), 4.19 - 4.06 (m, 2H), 4.04 - 3.88 (m, 1H), 3.71 - 3.42 (m, 5H), 3.09 - 2.85 (m, 2H), 2.70 - 2.52 (m, 3H), 2.42 (br d, *J* = 16.0 Hz, 1H), 2.27 - 2.06 (m, 1H), 2.04 - 1.68 (m, 6H), 1.59 - 1.35 (m, 2H).

### EXAMPLE 38: Synthesis of 4-(1-((1R,5S)-3,8-diazabicyclo[3.2.1]octan-8-yl)-3-(((S,Z)-2-(fluoromethylene)tetrahydro-1H-pyrrolizin-7a(5H)-yl)methoxy)imidazo[1',2':1,6]pyrido[3,2-d]pyrimidin-6-yl)-5-ethynyl-6-fluoronaphthalen-2-amine.

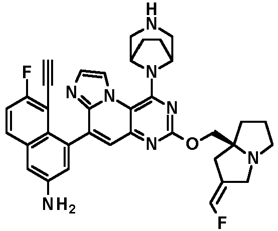

The compound was synthesized in the same manner as in EXAMPLE 2 using (*S,Z*)-(2-(fluoromethylene)tetrahydro-1*H*-pyrrolizin-7a(5H)-yl)methanol and intermediate 18. 4-(1-((1*R*,5*S*)-3,8-diazabicyclo[3.2.1]octan-8-yl)-3-(((*S,Z*)-2-(fluoromethylene)tetrahydro-1*H-*pyrrolizin-7a(5H)-yl)methoxy)imidazo[1',2':1,6]pyrido[3,2-*d*]pyrimidin-6-yl)-5-ethynyl-6-fluoronaphthalen-2-amine (8 mg, 12.39 µmol, 18% yield) was obtained as a yellow solid. LCMS (ES-API, m/z): [M+H]⁺= 633.4; ¹H NMR (400 MHz, DMSO-*d*₆, ppm): δ 8.76 - 8.55 (m, 1H), 7.68 (dd, *J* = 5.6, 9.2 Hz, 1H), 7.58 (s, 1H), 7.46 (s, 1H), 7.19 (t, *J* = 8.9 Hz, 1H), 7.15 - 7.08 (m, 2H), 6.66 - 6.38 (m, 1H), 4.64 - 4.43 (m, 1H), 4.35 - 3.99 (m, 3H), 3.98 - 3.83 (m, 3H), 3.53 - 3.31 (m, 2H), 3.28 - 3.14 (m, 1H), 2.99 - 2.84 (m, 2H), 2.82 - 2.72 (m, 1H), 2.71 - 2.58 (m, 2H), 2.58 - 2.45 (m, 1H), 2.37 (br d, *J* = 13.2 Hz, 2H), 2.26 - 2.11 (m, 2H), 1.95 (br s, 3H), 1.83 (ddd, *J* = 4.8, 8.2, 12.4 Hz, 1H).

### EXAMPLE 39: Synthesis of 4-(1-((1R,5S)-3,8-diazabicyclo[3.2.1]octan-8-yl)-3-(((2S,7aR)-2-fluoro-6-methylenetetrahydro-1H-pyrrolizin-7a(5H)-yl)methoxy)imidazo[1',2':1,6]pyrido[3,2-d]pyrimidin-6-yl)-5-ethynyl-6-fluoronaphthalen-2-ol.

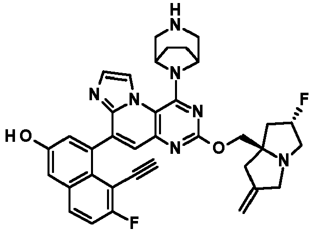

The compound was synthesized in the same manner as in EXAMPLE 2 using *tert*-Butyl (1*R*,5*S*)-3,8-diazabicyclo[3.2.1]octane-3-carboxylate and intermediate 16. 4-(1-((1*R*,5*S)-*3,8-Diazabicyclo[3.2.1]octan-8-yl)-3-(((2*S*,7a*R*)-2-fluoro-6-methylenetetrahydro-1*H-*pyrrolizin-7a(5*H*)-yl)methoxy)imidazo[1',2':1,6]pyrido[3,2-*d*]pyrimidin-6-yl)-5-ethynyl-6-fluoronaphthalen-2-ol (45.18 mg, 70.58 µmol, 69% yield) was obtained as a white solid. LCMS (ES-API, m/z): [M+H]⁺= 634.4; ¹H NMR (400 MHz, DMSO-*d*₆, ppm): δ 10.20 - 10.06 (m, 1H), 8.78 - 8.61 (m, 1H), 7.96 (dd, *J* = 6.0, 9.2 Hz, 1H), 7.71 (br s, 1H), 7.52 - 7.46 (m, 1H), 7.43 (t, *J* = 9.2 Hz, 1H), 7.39 - 7.30 (m, 2H), 7.21 (s, 1H), 5.44 - 5.21 (m, 1H), 4.92 (br s, 2H), 4.34 - 4.12 (m, 1H), 4.09 - 4.00 (m, 2H), 3.98 - 3.79 (m, 1H), 3.68 (br d, *J* = 14.0 Hz, 1H), 3.48 (br d, *J* = 13.6 Hz, 2H), 3.29 - 3.12 (m, 3H), 3.11 - 2.96 (m, 1H), 2.89 - 2.66 (m, 2H), 2.63 (br s, 2H), 2.25 - 2.09 (m, 3H), 2.01 - 1.62 (m, 3H).

### EXAMPLE 40: Synthesis of 4-(1-((1R,5S)-3,8-diazabicyclo[3.2.1]octan-3-yl)-3-((1-(((R)-3-fluoropyrrolidin-1-yl)methyl)cyclobutyl)methoxy)imidazo[1',2':1,6]pyrido[3,2-d]pyrimidin-6-yl)-5-ethynyl-6-fluoronaphthalen-2-ol.

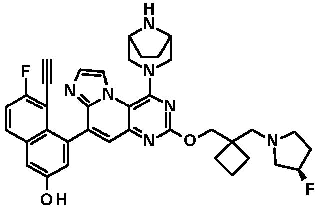

The compound was synthesized in the same manner as in EXAMPLE 2 using Intermediate 15. 4-(1-((1*R*,5*S*)-3,8-diazabicyclo[3.2.1]octan-3-yl)-3-((1-(((*R*)-3-fluoropyrrolidin-1-yl)methyl)cyclobutyl)methoxy)imidazo[1',2':1,6]pyrido[3,2-*d*]pyrimidin-6-yl)-5-ethynyl-6-fluoronaphthalen-2-ol (132.88 mg, 201.04 µmol, 65% yield) was obtained as a yellow solid.

LCMS (ES-API, m/z): [M+H]⁺= 650.4; ¹H NMR (400 MHz, DMSO-*d*₆, ppm): δ 10.36 - 9.95 (m, 1H), 8.56 - 7.88 (m, 2H), 7.43 (s, 2H), 7.41 - 7.29 (m, 2H), 7.21 (br d, *J* = 5.2 Hz, 1H), 5.25 - 5.01 (m, 1H), 4.44 (br s, 2H), 4.05 - 3.90 (m, 1H), 3.72 - 3.63 (m, 1H), 3.59 (br s, 1H), 3.55 - 3.39 (m, 3H), 3.30 - 3.13 (m, 1H), 3.04 - 2.90 (m, 1H), 2.83 - 2.70 (m, 2H), 2.70 - 2.57 (m, 3H), 2.33 (br d, *J* = 2.0 Hz, 1H), 2.27 - 1.97 (m, 3H), 1.97 - 1.89 (m, 3H), 1.89 - 1.79 (m, 4H), 1.79 - 1.70 (m, 1H), 1.58 - 1.43 (m, 1H).

### EXAMPLE 41: Synthesis of 4-(1-((1R,5S)-3,8-diazabicyclo[3.2.1]octan-3-yl)-3-(((2R,7aS)-2-fluorotetrahydro-1H-pyrrolizin-7a(5H)-yl)methoxy)-8-methylfuro[3,2-f]quinazolin-6-yl)naphthalen-2-ol.

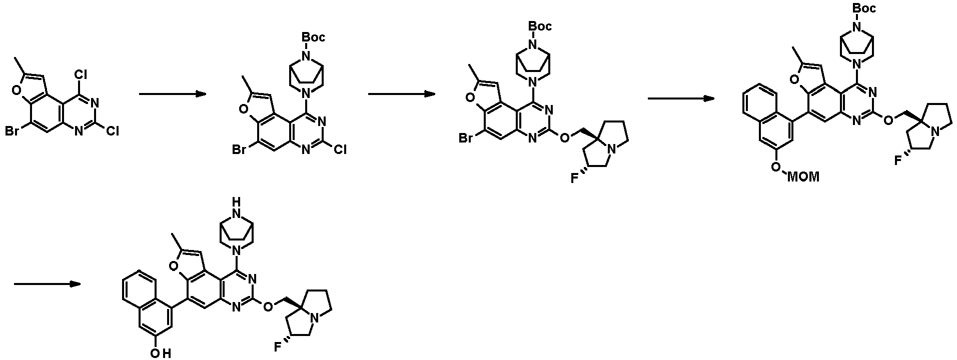

Step 1: 6-Bromo-1,3-dichloro-8-methylfuro[3,2-*f*]quinazoline (40.0 mg, 0.120 mmol) was dissolved in DCM (3 mL), and *tert*-butyl (1*R*,5*S*)-3,8-diazabicyclo[3.2.1]octane-8-carboxylate (30.7 mg, 0.145 mmol) and DIPEA (0.064 mL, 0.361 mmol) were added sequentially at 0 °C, and the reaction solution was stirred at the same temperature for 30 min. Purified water was added to the reaction mixture to quench the reaction, and the organic layer was extracted by adding ethyl acetate, and dried over anhydrous MgSO₄. The solvent was concentrated and the obtained material was purified by silica gel chromatography to obtain solid *tert*-butyl (1*R*,5*S*)-3-(6-bromo-3-chloro-8-methylfuro[3,2-*f*]quinazolin-1-yl)-3,8-diazabicyclo[3.2.1]octane-8-carboxylate (50 mg, 0.098 mmol, 81.7% yield). LCMS (ES-API, m/z): [M+H]⁺= 510.1.

Step 2: ((2*R*,7a*S*)-2-fluorotetrahydro-1*H*-pyrrolizin-7a(5*H*)-yl)methanol (33.0 mg, 0.207 mmol) was dissolved in THF (10 mL), NaH (78.8 mg, 0.207 mmol) was added and the mixture was stirred for 30 min, and then *tert*-butyl (1*R*,5*S*)-3-(6-bromo-3-chloro-8-methylfuro[3,2-*f*]quinazolin-1-yl)-3,8-diazabicyclo[3.2.1]octane-8-carboxylate (50.0 mg, 0.098 mmol) synthesized in Step 1 was slowly added to the solution in THF (1 mL), and the reaction solution was stirred at room temperature for 7 h. Purified water was added to the reaction mixture to quench the reaction, and ethyl acetate was added to extract the organic layer, which was then dried over anhydrous MgSO₄. The solvent was concentrated and the obtained material was purified by silica gel chromatography to obtain solid tert-butyl (1*R*,5*S*)-3-(6-bromo-3-(((2*R*,7a*S*)-2-fluorotetrahydro-1*H*-pyrrolizin-7a(5*H*)-yl)methoxy)-8-methylfuro[3,2-*f*]quinazolin-1-yl)-3,8-diazabicyclo[3.2.1]octane-8-carboxylate (51 mg, 0.081 mmol, 39% yield). LCMS (ES-API, m/z): [M+H]⁺= 631.6.

Step 3: tert-butyl (1*R*,5*S*)-3-(6-bromo-3-(((2*R*,7a*S*)-2-fluorotetrahydro-1*H*-pyrrolizin-7a(5*H*)-yl)methoxy)-8-methylfuro[3,2-*f*]quinazolin-1-yl)-3,8-diazabicyclo[3.2.1]octane-8-carboxylate (28.0 mg, 0.044 mmol) obtained from Step 2, 2-(3-(methoxymethoxy)naphtalene-1-yl)-4,4,5,5-tetramethyl-1,3,2-dioxaborolane (27.9 mg, 0.089 mmol), and K₃PO₄ (28.3 mg, 0.133 mmol) were dissolved in ethanol (1.50 mL), DMF (0.30 mL), and purified water (0.15 mL), and cataCXium A Pd G3 (4.84 mg, 0.007 mmol) was added and the reaction solution was stirred at 80 °C for 5 min. Purified water was added to the reaction mixture to quench the reaction, and ethyl acetate was added to extract the organic layer, which was then dried over anhydrous MgSO₄. The solvent was concentrated and the obtained material was purified by silica gel chromatography to obtain solid *tert*-butyl (1*R*,5*S*)-3-(3-(((2*R*,7a*S*)-2-fluorotetrahydro-1*H*-pyrrolizin-7a(5*H*)-yl)methoxy)-6-(3-(methoxymethoxy)naphthalen-1-yl)-8-methylfuro[3,2-*f*]quinazolin-1-yl)-3,8-diazabicyclo[3.2.1]octane-8-carboxylate (30.0 mg, 0.041 mmol, 93% yield). LCMS (ES-API, m/z): [M+H]⁺= 738.9.

Step 4: *tert*-butyl (1*R*,5*S*)-3-(3-(((2*R*,7a*S*)-2-fluorotetrahydro-1*H*-pyrrolizin-7a(5*H*)-yl)methoxy)-6-(3-(methoxymethoxy)naphthalen-1-yl)-8-methylfuro[3,2-*f*]quinazolin-1-yl)-3,8-diazabicyclo[3.2.1]octane-8-carboxylate (30.0 mg, 0.041 mmol) obtained from Step 3 was dissolved in DCM (1 mL), and 4 N hydrochloric acid solution in 1,4-dioxane (0.50 mL) was added at 0 °C, and the reaction solution was stirred at room temperature for 30 min. The solvent was concentrated, purified using Prep-HPLC, and then lyophilized to obtain 4-(1-((1*R*,5*S*)-3,8-diazabicyclo[3.2.1]octan-3-yl)-3-(((2*R*,7a*S*)-2-fluorotetrahydro-1*H-*pyrrolizin-7a(5*H*)-yl)methoxy)-8-methylfuro[3,2-*f*]quinazolin-6-yl)naphthalen-2-ol (17.0 mg, 0.029 mmol, 70% yield).

LCMS (ES-API, m/z): [M+H]⁺= 594.8; ¹H NMR (400 MHz, DMSO-*d*₆, ppm): δ 7.84 (d, *J* = 8.3 Hz, 1H), 7.50 - 7.43 (m, 2H), 7.40 (d, *J* = 8.5 Hz, 1H), 7.32 (d, *J =* 2.4 Hz, 1H), 7.24 - 7.17 (m, 2H), 6.88 (s, 1H), 5.66 (s, 0.5H), 5.53 (s, 0.5H), 4.61 (s, 2H), 4.37 - 4.23 (m, 3H), 4.15 (s, 2H), 3.95 - 3.88 (m, 4H), 3.37 - 3.27 (m, 2H), 2.43 (s, 3H), 2.25 - 2.13 (m, 4H), 2.11 - 1.86 (m, 6H).

### EXAMPLE 42: Synthesis of 4-(1-((1R,5S)-3,8-diazabicyclo[3.2.1]octan-3-yl)-3-(((2R,7aS)-2-fluorotetrahydro-1H-pyrrolizin-7a(5H)-yl)methoxy)-8-methylfuro[3,2-f]quinazolin-6-yl)-5-ethynyl-6-fluoronaphthalen-2-ol.

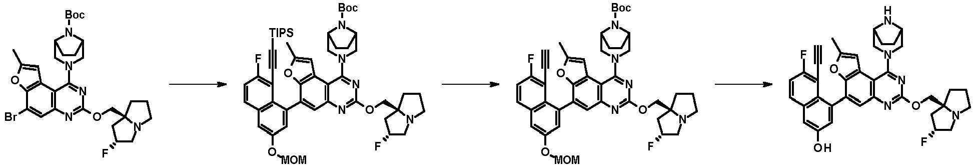

Step 1: tert-Butyl (1*R*,5*S*)-3-(6-bromo-3-(((2*R*,7a*S*)-2-fluorotetrahydro-1*H*-pyrrolizin-7a(5*H*)-yl)methoxy)-8-methylfuro[3,2-f]quinazolin-1-yl)-3,8-diazabicyclo[3.2.1]octane-8-carboxylate (51.0 mg, 0.081 mmol), ((2-Fluoro-6-(methoxymethoxy)-8-(4,4,5,5-tetramethyl-1,3,2-dioxaborolan-2-yl)naphthalen-1-yl)ethynyl)triisopropylsilane (62.2 mg, 0.121 mmol), and K₃PO₄ (51.5 mg, 0.243 mmol) were dissolved in ethanol (1.50 mL), DMF (0.30 mL), and purified water (0.15 mL), cataCXium A Pd G3 (8.82 mg, 0.012 mmol) was added and the reaction solution was stirred at 80 °C for 1 hour. Purified water was added to the reaction mixture to quench the reaction and the organic layer was extracted by adding ethyl acetate, and dried over anhydrous MgSO₄. The solvent was concentrated, and the obtained material was purified by silica gel chromatography to obtain *tert*-butyl (1*R*,5*S*)-3-(6-(7-fluoro-3-(methoxymethoxy)-8-((triisopropylsilyl)ethynyl)naphthalen-1-yl)-3-(((2*R*,7a*S*)-2-fluorotetrahydro-1*H*-pyrrolizin-7a(5*H*)-yl)methoxy)-8-methylfuro[3,2-*f*]quinazolin-1-yl)-3,8-diazabicyclo[3.2.1]octane-8-carboxylate (62.0 mg, 0.076 mmol, 81% yield) as a solid compound. LCMS (ES-API, m/z): [M+H]⁺= 937.4.

Step 2: *tert*-butyl (1*R*,5*S*)-3-(6-(7-fluoro-3-(methoxymethoxy)-8-((triisopropylsilyl)ethynyl)naphthalen-1-yl)-3-(((2*R*,7a*S*)-2-fluorotetrahydro-1*H*-pyrrolizin-7a(5*H*)-yl)methoxy)-8-methylfuro[3,2-*f*]quinazolin-1-yl)-3,8-diazabicyclo[3.2.1]octane-8-carboxylate (62.0 mg, 0.066 mmol) obtained from Step 1 was dissolved in THF (0.5 mL), and 1 M TBAF (0.13 mL, 0.132 mmol) dissolved in THF was added at 0 °C and the reaction solution was stirred at room temperature for 20 min. Purified water was added to the reaction mixture to quench the reaction, ethyl acetate was added to extract the organic layer, and dried over anhydrous MgSO₄. The solvent was concentrated and the obtained material was purified by silica gel chromatography to obtain *tert*-butyl (1*R*,5*S*)-3-(6-(8-ethynyl-7-fluoro-3-(methoxymethoxy)naphthalen-1-yl)-3-(((2*R*,7a*S*)-2-fluorotetrahydro-1*H*-pyrrolizin-7a(5*H*)-yl)methoxy)-8-methylfuro[3,2-*f*]quinazolin-1-yl)-3,8-diazabicyclo[3.2.1]octane-8-carboxylate (50.0 mg, 0.064 mmol, 97% yield) as a solid compound. LCMS (ES-API, m/z): [M+H]⁺= 780.9.

Step 3: *tert*-butyl (1*R*,5*S*)-3-(6-(8-ethynyl-7-fluoro-3-(methoxymethoxy)naphthalen-1-yl)-3-(((2*R*,7a*S*)-2-fluorotetrahydro-1*H*-pyrrolizin-7a(5*H*)-yl)methoxy)-8-methylfuro[3,2-*f*]quinazolin-1-yl)-3,8-diazabicyclo[3.2.1]octane-8-carboxylate (50.0 mg, 0.064 mmol) obtained from Step 2 was dissolved in DCM (1 mL), and 4 N HCl solution dissolved in 1,4-dioxane (0.30 mL) was added at 0 °C, and the reaction solution was stirred at room temperature for 30 min. The solvent was concentrated and purified using Prep-HPLC, and then lyophilized to obtain 4-(1-((1*R*,5*S*)-3,8-diazabicyclo[3.2.1]octan-3-yl)-3-(((2*R*,7a*S*)-2-fluorotetrahydro-1*H*-pyrrolizin-7a(5*H*)-yl)methoxy)-8-methylfuro[3,2-*f*]quinazolin-6-yl)-5-ethynyl-6-fluoronaphthalen-2-ol (38.0 mg, 0.051 mmol, 79% yield). LCMS (ES-API, m/z): [M+H]⁺= 636.9; ¹H NMR (400 MHz, DMSO-*d*₆, ppm): δ 8.00 (dd, *J* = 9.2, 6.0 Hz, 1H), 7.52 - 7.35 (m, 3H), 7.19 (d, *J* = 2.5 Hz, 1H), 6.82 (s, 1H), 5.66 (s, 0.5H), 5.53 (s, 0.5H), 4.64 (s, 2H), 4.36 - 4.24 (m, 1H), 4.22 - 4.10 (m, 3H), 3.97 - 3.82 (m, 4H), 3.35 - 3.26 (m, 2H), 2.41 (s, 3H), 2.36 - 2.30 (m, 2H), 2.27 - 2.13 (m, 3H), 2.11 - 2.04 (m, 1H), 2.04 -1.86 (m, 5H).

### EXAMPLE 43: Synthesis of 4-(1-((1R,5S)-3,8-diazabicyclo[3.2.1]octan-3-yl)-5-fluoro-3-(((2R,7aS)-2-fluorotetrahydro-1H-pyrrolizin-7a(5H)-yl)methoxy)-8-methylfuro[3,2-f]quinazolin-6-yl)naphthalen-2-ol.

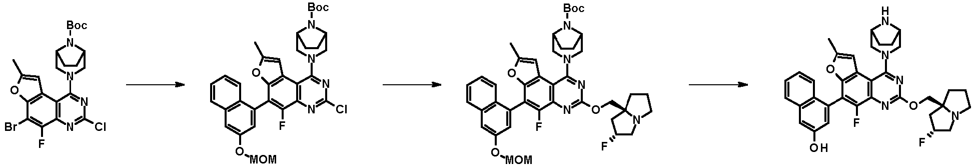

Step 1: *tert*-Butyl (1*R*,5*S*)-3-(6-bromo-3-chloro-5-fluoro-8-methylfuro[3,2-*f*]quinazolin-1-yl)-3,8-diazabicyclo[3.2.1]octane-8-carboxylate (60.0 mg, 0.114 mmol), 2-[3-(methoxymethoxy)naphtalene-1-yl-4,4,5,5-tetramethyl-1,3,2-dioxaborolane (53.8 mg, 0.171 mmol), Cs₂CO₃ (112 mg, 0.342 mmol) were dissolved in toluene (1.00 mL) and purified water (0.25 mL), and cataCXium A Pd G3 (8.3 mg, 0.011 mmol) was added and the reaction solution was stirred at 100 °C for 2 h. Purified water was added to the reaction mixture to quench the reaction, and ethyl acetate was added to extract the organic layer, which was then dried over anhydrous MgSO₄. The solvent was concentrated, and the obtained material was purified by silica gel chromatography to synthesize solid tert-butyl (1*R*,5*S*)-3-(3-chloro-5-fluoro-6-(3-(methoxymethoxy)naphthalen-1-yl)-8-methylfuro[3,2-*f*]quinazolin-1-yl)-3,8-diazabicyclo[3.2.1]octane-8-carboxylate (20.0 mg, 0.032 mmol, 28% yield). LCMS (ES-API, m/z): [M+H]⁺= 634.3.

Step 2: ((2*R*,7a*S*)-2-fluorotetrahydro-1*H*-pyrrolizin-7a(5*H*)-yl)methanol (50.3 mg, 0.316 mmol) was dissolved in THF (3.00 mL), and NaH (12.6 mg, 0.316 mmol) was added and the mixture was stirred for 30 min. tert-butyl (1*R*,5*S*)-3-(3-chloro-5-fluoro-6-(3-(methoxymethoxy)naphthalen-1-yl)-8-methylfuro[3,2-*f*]quinazolin-1-yl)-3,8-diazabicyclo[3.2.1]octane-8-carboxylate (20.0 mg, 0.032 mmol) obtained from Step 1 was added to the reaction solution, and the reaction solution was stirred at 70 °C for one day. Purified water was added to the reaction mixture to quench the reaction, ethyl acetate was added to extract the organic layer, and dried over anhydrous MgSO₄. The solvent was concentrated, and the obtained material was purified by silica gel chromatography to synthesize solid *tert*-butyl (1*R*,5*S*)-3-(5-fluoro-3-(((2*R*,7a*S*)-2-fluorotetrahydro-1*H-*pyrrolizin-7a(5*H*)-yl)methoxy)-6-(3-(methoxymethoxy)naphthalen-1-yl)-8-methylfuro[3,2-*f*]quinazolin-1-yl)-3,8-diazabicyclo[3.2.1]octane-8-carboxylate (15.0 mg, 0.020 mmol, 63% yield). LCMS (ES-API, m/z): [M+H]⁺= 756.6.

Step 3: *tert*-butyl (1*R*,5*S*)-3-(5-fluoro-3-(((2*R*,7a*S*)-2-fluorotetrahydro-1*H*-pyrrolizin-7a(5*H*)-yl)methoxy)-6-(3-(methoxymethoxy)naphthalen-1-yl)-8-methylfuro[3,2-f]quinazolin-1-yl)-3,8-diazabicyclo[3.2.1]octane-8-carboxylate (23.0 mg, 0.030 mmol) obtained from Step 2 was dissolved in DCM (3.00 mL), and a 4 N hydrochloric acid solution (0.2 mL) dissolved in 1,4-dioxane was added at 0 °C, and the reaction solution was stirred at room temperature for 3 h. The solvent was concentrated, purified using Prep-HPLC, and then lyophilized to obtain 4-(1-((1*R*,5*S*)-3,8-diazabicyclo[3.2.1]octan-3-yl)-5-fluoro-3-(((2*R*,7a*S*)-2-fluorotetrahydro-1*H*-pyrrolizin-7a(5*H*)-yl)methoxy)-8-methylfuro[3,2-f]quinazolin-6-yl)naphthalen-2-ol (15.0 mg, 0.025 mmol, 65% yield). LCMS (ES-API, m/z): [M+H]⁺= 612.8; ¹H NMR (400 MHz, MeOD) δ=7.69 (d, *J* = 8.3 Hz, 1H), 7.33 (ddd, *J* = 8.1, 6.7, 1.2 Hz, 1H), 7.28 - 7.17 (m, 2H), 7.15 - 7.02 (m, 2H), 6.77 (d, *J* = 1.2 Hz, 1H), 5.54 (t, *J* = 3.7 Hz, 1, 0.5H), 5.41 (s, 0.5H), 4.64 (d, *J* = 1.6 Hz, 2H), 4.49 (t, *J* = 13.3 Hz, 2H), 4.08 (s, 2H), 4.02 - 3.71 (m, 5H), 3.37 (td, *J* = 10.7, 6.1 Hz, 1H), 3.25 (s, 1H), 2.77 - 2.18 (m, 9H), 2.18 - 1.85 (m, 6H).

### EXAMPLE 44: Synthesis of 4-(1-((1R,5S)-3,8-diazabicyclo[3.2.1]octan-3-yl)-5-fluoro-3-(((2R,7aS)-2-fluorotetrahydro-1H-pyrrolizin-7a(5H)-yl)methoxy)-8-methylfuro[3,2-f]quinazolin-6-yl)-5-ethynyl-6-fluoronaphthalen-2-ol.

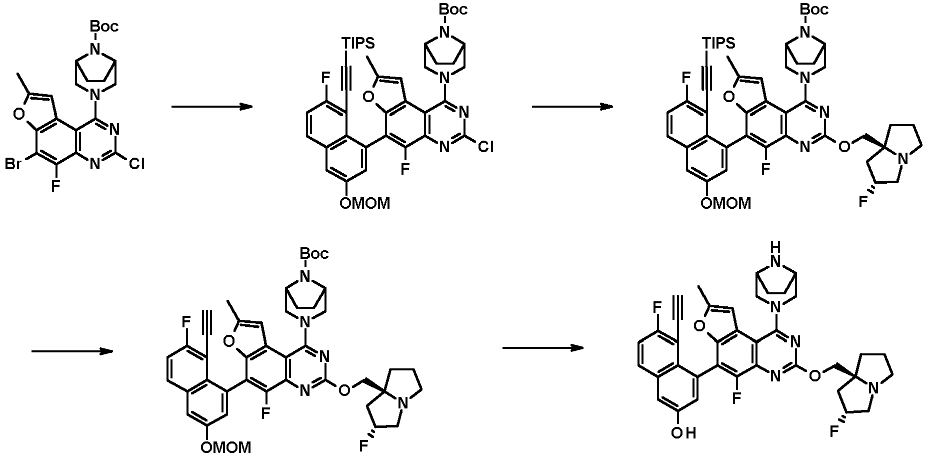

Step 1: *tert*-Butyl (1*R*,5*S*)-3-(6-bromo-3-chloro-5-fluoro-8-methylfuro[3,2-*f*]quinazolin-1-yl)-3,8-diazabicyclo[3.2.1]octane-8-carboxylate (1.19 g, 2.26 mmol), ((2-fluoro-6-(methoxymethoxy)-8-(4,4,5,5-tetramethyl-1,3,2-dioxaborolan-2-yl)naphthalen-1-yl)ethynyl)triisopropylsilane (1.74 g, 3.39 mmol), and Cs₂CO₃ (2.21 g, 6.79 mmol) were dissolved in toluene (34.0 mL) and purified water (8.5 mL), and cataCXium A Pd G3 (165 mg, 0.226 mmol) was added and the reaction solution was stirred at 100 °C for one day. Purified water was added to the reaction mixture to quench the reaction, ethyl acetate was added to extract the organic layer, and dried over anhydrous MgSO₄. The solvent was concentrated and the obtained material was purified by silica gel chromatography. The obtained material was concentrated, dissolved in hexane, and the solid formed by ultrasonic treatment was filtered. The obtained solid was washed with hexane to obtain *tert*-butyl (1*R*,5*S*)-3-(3-chloro-5-fluoro-6-(7-fluoro-3-methyl-8-((triisopropylsilyl)ethynyl)naphthalen-1-yl)-8-methylfuro[3,2-*f*]quinazolin-1-yl)-3,8-diazabicyclo[3.2.1]octane-8-carboxylate (640 mg, 0.770 mmol, 34% yield) as a solid compound.

LCMS (ES-API, m/z): [M+H]⁺= 831.4; ¹H NMR (400 MHz, CD₃OD_{,} ppm): δ 8.04 - 7.98 (m, 1H), 7.72 (d, *J* = 2.6 Hz, 1H), 7.44 (t, *J* = 8.9 Hz, 1H), 7.33 (d, *J* = 2.6 Hz, 1H), 6.88 (s, 1H), 5.38 (s, 2H), 4.66 - 4.62 (m, 1H), 4.45 - 4.42 (m, 1H), 4.29 - 4.26 (m, 1H), 4.04 - 3.99 (m, 1H), 3.75 - 3.71 (m, 1H), 3.54 (s, 3H), 2.45 (s, 3H), 1.91 - 1.82 (m, 2H), 1.56 (s, 9H), 1.37 - 1.29 (m, 3H), 0.80 (dd, *J* = 16.0, 7.5 Hz, 18H), 0.39 (p, *J* = 7.5 Hz, 3H).

Step 3: ((2*R*,7a*S*)-2-fluorotetrahydro-1*H*-pyrrolizin-7a(5*H*)-yl)methanol (1.05 g, 6.61 mmol) was dissolved in THF (29 mL), and NaH (265 mg, 6.61 mmol) was added. The mixture was stirred for 30 minutes, followed by slow addition of a THF solution (22.0 mL) of *tert*-butyl (1*R*,5*S*)-3-(3-chloro-5-fluoro-6-(7-fluoro-3-methyl-8-((triisopropylsilyl)ethynyl)naphthalen-1-yl)-8-methylfuro[3,2-*f*]quinazolin-1-yl)-3,8-diazabicyclo[3.2.1]octane-8-carboxylate (550 mg, 0.661 mmol) obtained from Step 2, and the reaction solution was stirred at room temperature for 3 hours. Purified water was added to the reaction mixture to quench the reaction, ethyl acetate was added to extract the organic layer, and dried over anhydrous MgSO₄. The solvent was concentrated and the obtained material was purified by silica gel chromatography to obtain *tert*-butyl (1*R*,5*S*)-3-(5-fluoro-6-(7-fluoro-3-methyl-8-((triisopropylsilyl)ethynyl)naphthalen-1-yl)-3-(((2*R*,7a*S*)-2-fluorotetrahydro-1*H*-pyrrolizin-7a(5*H*)-yl)methoxy)-8-methylfuro[3,2-*f*]quinazolin-1-yl)-3,8-diazabicyclo[3.2.1]octane-8-carboxylate (500 mg, 0.524 mmol, 79% yield) as a solid compound.

LCMS (ES-API, m/z): [M+H]⁺= 954.5; ¹H NMR (400 MHz, CD₃OD, ppm): δ 7.98 (dd, *J =* 9.1, 5.7 Hz, 1H), 7.68 (d, *J* = 2.6 Hz, 1H), 7.41 (t, *J* = 8.9 Hz, 1H), 7.30 (t, *J* = 2.8 Hz, 1H), 6.82 (s, 1H), 5.47 - 5.40 (m, 0.5H), 5.36 (s, 2H), 5.32 - 5.27 (m, 0.5H), 4.84 - 4.70 (m, 1H), 4.43 - 4.23 (m, 4H), 3.99 - 3.87 (m, 1H), 3.82 - 3.71 (m, 1H), 3.53 (s, 3H), 3.50 - 3.41 (m, 1H), 3.41 - 3.22 (m, 2H), 3.14 - 3.04 (m, 1H), 2.42 (s, 3H), 2.38 - 2.16 (m, 4H), 2.13 - 2.02 (m, 3H), 1.99 - 1.83 (m, 3H), 1.55 (s, 9H), 1.47 - 1.37 (m, 1H), 0.99 - 0.89 (m, 1H), 0.86 - 0.72 (m, 18H), 0.48 - 0.42 (m, 3H).

Step 4: *tert*-butyl (1*R*,5*S*)-3-(5-fluoro-6-(7-fluoro-3-methyl-8-((triisopropylsilyl)ethynyl)naphthalen-1-yl)-3-(((2*R*,7a*S*)-2-fluorotetrahydro-1*H*-pyrrolizin-7a(5*H*)-yl)methoxy)-8-methylfuro[3,2-*f*]quinazolin-1-yl)-3,8-diazabicyclo[3.2.1]octane-8-carboxylate (500 mg, 0.524 mmol) obtained from Step 3 was dissolved in THF (17.5 mL), 1 M TBAF in THF (1.05 mL, 1.05 mmol) was added at 0 °C and the reaction solution was stirred at room temperature for 20 min. Purified water was added to the reaction mixture to quench the reaction, ethyl acetate was added to extract the organic layer, and dried over anhydrous MgSO₄. The solvent was concentrated and the obtained material was purified by silica gel chromatography to obtain *tert*-butyl (1*R*,5*S*)-3-(6-(8-ethynyl-7-fluoro-3-methylnaphthalen-1-yl)-5-fluoro-3-(((2*R*,7a*S*)-2-fluorotetrahydro-1*H*-pyrrolizin-7a(5*H*)-yl)methoxy)-8-methylfuro[3,2-*f*]quinazolin-1-yl)-3,8-diazabicyclo[3.2.1]octane-8-carboxylate (280 mg, 0.351 mmol, 67% yield) as a solid compound.

LCMS (ES-API, m/z): [M+H]⁺= 798.3; ¹H NMR (400 MHz, CD₃OD, ppm): δ 7.99 (dd, *J* = 9.2, 5.7 Hz, 1H), 7.70 (d, *J* = 2.6 Hz, 1H), 7.42 - 7.34 (m, 2H), 6.81 (d, *J* = 1.2 Hz, 1H), 5.45 - 5.40 (m, 1H), 5.38 (s, 2H), 5.32 - 5.26 (m, 1H), 4.54 - 4.27 (m, 5H), 4.27 - 4.09 (m, 1H), 3.81 - 3.69 (m, 1H), 3.69 - 3.59 (m, 1H), 3.55 (s, 3H), 3.33 (qd, *J* = 3.3, 2.2 Hz, 2H), 3.14 - 3.04 (m, 1H), 2.95 - 2.88 (m, 1H), 2.42 (s, 3H), 2.38 - 2.16 (m, 3H), 2.11 - 2.00 (m, 3H), 2.00 - 1.82 (m, 4H), 1.78 - 1.69 (m, 1H), 1.55 (s, 9H).

Step 5: *tert*-butyl (1*R*,5*S*)-3-(6-(8-ethynyl-7-fluoro-3-methylnaphthalen-1-yl)-5-fluoro-3-(((2*R*,7a*S*)-2-fluorotetrahydro-1*H*-pyrrolizin-7a(5*H*)-yl)methoxy)-8-methylfuro[3,2-*f*]quinazolin-1-yl)-3,8-diazabicyclo[3.2.1]octane-8-carboxylate (20 mg, 0.025 mmol) obtained from Step 4 was dissolved in DCM (3 mL), 4 N HCl in 1,4-dioxane (0.06 mL) was added at 0 °C, and the reaction solution was stirred at room temperature for 1 h. The solvent was concentrated, purified using Prep-HPLC, and then lyophilized to obtain 4-(1-((1*R*,5*S*)-3,8-diazabicyclo[3.2.1]octan-3-yl)-5-fluoro-3-(((2*R*,7a*S*)-2-fluorotetrahydro-1*H-*pyrrolizin-7a(5*H*)-yl)methoxy)-8-methylfuro[3,2-*f*]quinazolin-6-yl)-5-ethynyl-6-fluoronaphthalen-2-ol (6.2 mg, 0.008 mmol, 32% yield).

LCMS (ES-API, m/z): [M+H]⁺= 654.3; ¹H NMR (400 MHz, CD₃OD, ppm): δ 7.91 (dd, *J* = 9.2, 5.7 Hz, 1H), 7.41 (d, *J* = 2.6 Hz, 1H), 7.35 (t, *J* = 8.9 Hz, 1H), 7.22 (d, *J* = 2.6 Hz, 1H), 6.85 (s, 1H), 5.66 - 5.61 (m, 1H), 5.52 - 5.48 (m, 1H), 4.82 - 4.66 (m, 3H), 4.44 - 4.35 (m, 1H), 4.27 - 4.22 (m, 1H), 4.18 - 4.14 (m, 1H), 4.09 - 3.97 (m, 2H), 3.97 - 3.84 (m, 3H), 3.54 - 3.44 (m, 1H), 2.77 - 2.58 (m, 3H), 2.47 (s, 3H), 2.43 - 2.22 (m, 3H), 2.20 - 1.95 (m, 4H).

### EXAMPLE 45: Synthesis of 4-(1-((1R,5S)-3,8-diazabicyclo[3.2.1]octan-3-yl)-5-fluoro-3-(((2R,7aS)-2-fluorotetrahydro-1H-pyrrolizin-7a(5H)-yl)methoxy)-8-methylfuro[3,2-f]quinazolin-6-yl)-5-ethyl-6-fluoronaphthalen-2-ol.

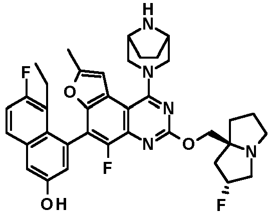

The compound was synthesized in the same manner as in EXAMPLE 43 using 2-(8-Ethyl-7-fluoro-3-(methoxymethoxy)naphthalen-1-yl)-4,4,5,5-tetramethyl-1,3,2-dioxaborolane. The product was purified by Prep-HPLC and lyophilized to synthesize 4-(1-((1*R*,5*S*)-3,8-diazabicyclo[3.2.1]octan-3-yl)-5-fluoro-3-(((2*R*,7a*S*)-2-fluorotetrahydro-1*H*-pyrrolizin-7a(5*H*)-yl)methoxy)-8-methylfuro[3,2-*f*]quinazolin-6-yl)-5-ethyl-6-fluoronaphthalen-2-ol (10 mg, 61% yield).

LCMS (ES-API, m/z): [M+H]⁺= 657.29; ¹H NMR (400 MHz, CD₃OD, ppm): δ 7.74 (dd, *J* = 9.0, 5.9 Hz, 1H), 7.36 (d, *J* = 2.7 Hz, 1H), 7.29 (t, *J =* 9.4 Hz, 1H), 7.05 (d, *J* = 2.6 Hz, 1H), 6.89 (s, 1H), 5.66 (s, 0.5H), 5.53 (s, 0.5H), 4.80 - 4.68 (m, 2H), 4.63 (s, 1H), 4.48 (s, 1H), 4.21 (d, *J* = 23.7 Hz, 2H), 4.05 - 3.87 (m, 4H), 2.64 (d, *J* = 20.5 Hz, 1H), 2.46 (s, 3H), 2.38 (t, *J* = 8.7 Hz, 2H), 2.14 (d, *J* = 48.8 Hz, 5H), 0.59 (t, *J* = 7.3 Hz, 2H).

### EXAMPLE 46: Synthesis of 4-(1-((1R,5S)-3,8-diazabicyclo[3.2.1]octan-3-yl)-5-fluoro-3-(((2R,7aS)-2-fluorotetrahydro-1H-pyrrolizin-7a(5H)-yl)methoxy)-8-methylfuro[3,2-f]quinazolin-6-yl)-5,6-difluoronaphthalen-2-ol.

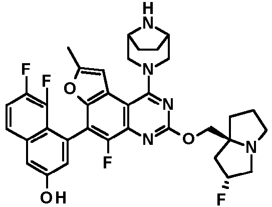

The compound was synthesized in the same manner as in EXAMPLE 43 using 2-(7,8-Difluoro-3-(methoxymethoxy)naphthalen-1-yl)-4,4,5,5-tetramethyl-1,3,2-dioxaborolane. The compound was purified by Prep-HPLC and lyophilized to obtain 4-(1-((1*R*,5*S*)-3,8-diazabicyclo[3.2.1]octan-3-yl)-5-fluoro-3-(((2*R*,7a*S*)-2-fluorotetrahydro-1*H*-pyrrolizin-7a(5*H*)-yl)methoxy)-8-methylfuro[3,2-*f*]quinazolin-6-yl)-5,6-difluoronaphthalen-2-ol (14 mg, 57% yield).

LCMS (ES-API, m/z): [M+H]⁺= 647.25; ¹H NMR (400 MHz, CD₃OD, ppm): δ 7.74 (dd, *J* = 9.0, 5.9 Hz, 1H), 7.36 (d, *J* = 2.7 Hz, 1H), 7.29 (t, *J =* 9.4 Hz, 1H), 7.05 (d, *J* = 2.6 Hz, 1H), 6.89 (s, 1H), 5.66 (s, 0.5H), 5.53 (s, 0.5H), 4.79 - 4.69 (m, 2H), 4.64 (d, *J* = 13.2 Hz, 1H), 4.47 (m, 1H), 4.21 (d, *J* = 23.4 Hz, 2H), 3.96 (m, 4H), 3.56 - 3.42 (m, 1H), 2.87 - 2.55 (m, 3H), 2.46 (s, 3H), 2.39 (q, *J* = 8.5 Hz, 3H), 2.14 (d, J = 49.5 Hz, 6H), 0.59 (t, *J* = 7.3 Hz, 3H).

### EXAMPLE 47: Synthesis of 4-(1-(((1-(dimethylamino)cyclobutyl)methyl)amino)-5-fluoro-3-(((2R,7aS)-2-fluorotetrahydro-1H-pyrrolizin-7a(5H)-yl)methoxy)-8-methylfuro[3,2-f]quinazolin-6-yl)naphthalen-2-ol.

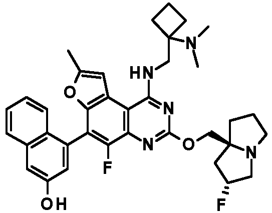

The compound was synthesized in the same manner as in EXAMPLE 43 using 1-(Aminomethyl)-*N,N*-dimethyl-cyclobutanamine. The product was purified by RP-HPLC and lyophilized to obtain 4-(1-(((1-(dimethylamino)cyclobutyl)methyl)amino)-5-fluoro-3-(((2*R*,7a*S*)-2-fluorotetrahydro-1*H*-pyrrolizin-7a(5H)-yl)methoxy)-8-methylfuro[3,2-*f*]quinazolin-6-yl)naphthalen-2-ol (14 mg, 75% yield).

LCMS (ES-API, m/z): [M+H]⁺= 627.30; ¹H NMR (400 MHz, CD₃OD, ppm): δ 7.81 (d, *J* = 8.3 Hz, 1H), 7.46 (t, *J* = 7.5 Hz, 1H), 7.39 - 7.32 (m, 2H), 7.30 - 7.15 (m, 3H) 5.61 (s, 0.5H), 5.48 (s, 0.5H), 4.85 - 4.67 (m, 7H), 4.53 - 4.31 (m, 2H), 4.08 - 3.77 (m, 3H), 3.46 (t, *J* = 8.6 Hz, 1H), 2.72 - 2.30 (m, 11H), 2.13 (p, *J* = 9.0 Hz, 2H), 2.02 (d, *J* = 6.5 Hz, 1H).

### EXAMPLE 48: Synthesis of 4-(1-(((1-(dimethylamino)cyclobutyl)methyl)amino)-5-fluoro-3-(((2R,7aS)-2-fluorotetrahydro-1H-pyrrolizin-7a(5H)-yl)methoxy)-8-methylfuro[3,2-f]quinazolin-6-yl)-5-ethynyl-6-fluoronaphthalen-2-ol.

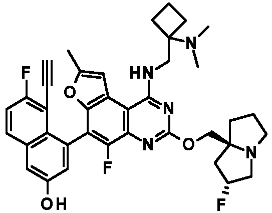

The compound was synthesized in the same manner as in EXAMPLE 44 using 1-(Aminomethyl)-*N,N*-dimethyl-cyclobutanamine. The compound was purified by Prep-HPLC and lyophilized to obtain 4-(1-(((1-(dimethylamino)cyclobutyl)methyl)amino)-5-fluoro-3-(((2*R*,7a*S*)-2-fluorotetrahydro-1*H*-pyrrolizin-7a(5*H*)-yl)methoxy)-8-methylfuro[3,2-*f*]quinazolin-6-yl)-5-ethynyl-6-fluoronaphthalen-2-ol (9 mg, 75% yield). LCMS (ES-API, m/z): [M+H]⁺= 669.29; ¹H NMR (400 MHz, CD₃OD, ppm): δ 7.92 (dd, *J* = 9.1, 5.8 Hz, 1H), 7.42 (d, *J* = 2.6 Hz, 1H), 7.40 - 7.30 (m, 2H), 7.22 (t, *J* = 2.2 Hz, 1H), 5.59 (s, 0.5H), 5.46 (s, 0.5H), 4.76 (qd, *J* = 13.3, 6.8 Hz, 5H), 4.41 (dt, *J* = 64.6, 15.7 Hz, 3H), 3.89 (d, *J* = 15.0 Hz, 3H), 3.57 - 3.43 (m, 2H), 3.02 (s, 5H), 2.97 (dd, *J* = 15.2, 1.1 Hz, 1H), 2.84 (s, 1H), 2.68 (s, 1H), 2.64 (s, 1H), 2.51 (s, 7H), 2.36 (s, 3H), 2.19 - 1.97 (m, 4H).

### EXAMPLE 49: Synthesis of 3-(1-((1R,5S)-3,8-diazabicyclo[3.2.1]octan-3-yl)-5-fluoro-3-(((2R,7aS)-2-fluorotetrahydro-1H-pyrrolizin-7a(5H)-yl)methoxy)-8-methylfuro[3,2-f]quinazolin-6-yl)-4-(trifluoromethyl)aniline.

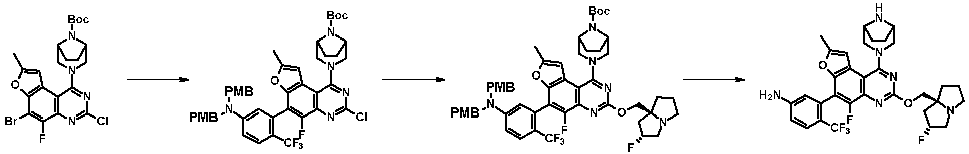

Step 1: *tert*-Butyl (1*R*,5*S*)-3-(6-bromo-3-chloro-5-fluoro-8-methylfuro[3,2-*f*]quinazolin-1-yl)-3,8-diazabicyclo[3.2.1]octane-8-carboxylate (150 mg, 285 µmol), *N,N*-bis(4-methoxybenzyl)-3-(4,4,5,5-tetramethyl-1,3,2-dioxaborolan-2-yl)-4-(trifluoromethyl)aniline (228 mg, 428 µmol), cataCXium A Pd G3 (20.8 mg, 28.5 µmol), and Cs₂CO₃ (279 mg, 856 µmol) were added to toluene (2 mL) and water (0.5 mL), followed by degassing and nitrogen purging three times. And then, the reaction mixture was stirred under nitrogen at 110 °C for 5 h. After the starting material was completely consumed, the reaction temperature was lowered to room temperature, and the palladium catalyst was removed through a Celite filter, and the organic layer was extracted with ethyl acetate. After drying over anhydrous Na₂SO₄, concentration under reduced pressure was performed. The residue was purified by silica gel chromatography to obtain tert-butyl (1*R*,5*S*)-3-(6-(5-(bis(4-methoxybenzyl)amino)-2-(trifluoromethyl)phenyl)-3-chloro-5-fluoro-8-methylfuro[3,2-*f*]quinazolin-1-yl)-3,8-diazabicyclo[3.2.1]octane-8-carboxylate (70 mg, 82.7 µmol, 29% yield). LCMS (ES-API, m/z): [M+H]⁺= 946.3.

Step 2: The compound was synthesized in the same manner as in EXAMPLE 43 to obtain tert-butyl (1*R*,5*S*)-3-(6-(5-(bis(4-methoxybenzyl)amino)-2-(trifluoromethyl)phenyl)-5-fluoro-3-(((2*R*,7a*S*)-2-fluorotetrahydro-1*H*-pyrrolizin-7a(5*H*)-yl)methoxy)-8-methylfuro[3,2-*f*]quinazolin-1-yl)-3,8-diazabicyclo[3.2.1]octane-8-carboxylate (50 mg, 51.6 µmol, 62% yield). LCMS (ES-API, m/z): [M+H]⁺= 970.1.

Step 3: To *tert*-butyl (1*R*,5*S*)-3-(6-(5-(bis(4-methoxybenzyl)amino)-2-(trifluoromethyl)phenyl)-5-fluoro-3-(((2*R*,7a*S*)-2-fluorotetrahydro-1*H*-pyrrolizin-7a(5*H*)-yl)methoxy)-8-methylfuro[3,2-*f*]quinazolin-1-yl)-3,8-diazabicyclo[3.2.1]octane-8-carboxylate (40 mg, 41.3 µmol) obtained in Step 3, TFA (4 mL) was added and stirred at room temperature for 9 h. After the starting material was completely consumed, the mixture was concentrated under reduced pressure. The residue was purified by silica gel chromatography to obtain 3-(1-((1*R*,5*S*)-3,8-diazabicyclo[3.2.1]octan-3-yl)-5-fluoro-3-(((2*R*,7a*S*)-2-fluorotetrahydro-1*H*-pyrrolizin-7a(5*H*)-yl)methoxy)-8-methylfuro[3,2-*f*]quinazolin-6-yl)-4-(trifluoromethyl)aniline (19 mg, 25.9 µmol, 73% yield).

LCMS (ES-API, m/z): [M+H]⁺= 629.6; ¹H NMR (400 MHz, MeOD) δ 7.55 (d, *J* = 8.7 Hz, 1H), 6.89 - 6.79 (m, 1H), 6.77 (d, *J =* 1.2 Hz, 1H), 6.65 (d, *J* = 2.3 Hz, 1H), 5.45 (s, 0.5H), 5.25 (s, 0.5H), 4.32 (d, *J* = 10.3 Hz, 2H), 4.24 (dd, *J* = 10.3, 2.6 Hz, 2H), 3.64 (q, *J* = 12.7 Hz, 2H), 3.54 (s, 2H), 3.31 - 3.13 (m, 3H), 3.03 (td, J = 9.6, 5.5 Hz, 1H), 2.46 (s, 4H), 2.35 - 2.13 (m, 3H), 2.01 (dq, *J* = 12.0, 6.5 Hz, 2H), 1.77 (s, 2H), 1.31 (s, 1H).

### EXAMPLE 50: Synthesis of 6-(1-((1R,5S)-3,8-diazabicyclo[3.2.1]octan-3-yl)-5-fluoro-3-(((2R,7aS)-2-fluorotetrahydro-1H-pyrrolizin-7a(5H)-yl)methoxy)-8-methylfuro[3,2-f]quinazolin-6-yl)-5-(trifluoromethyl)pyridin-2-amine.

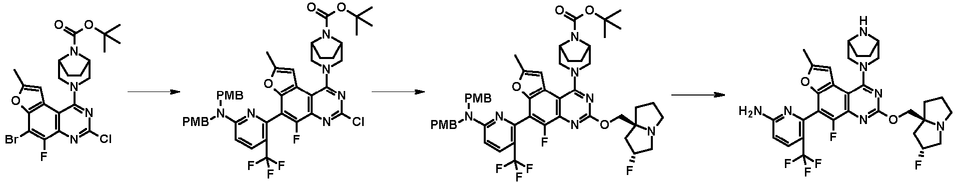

Step 1: *tert*-Butyl (1*R*,5*S*)-3-(6-bromo-3-chloro-5-fluoro-8-methylfuro[3,2-f]quinazolin-1-yl)-3,8-diazabicyclo[3.2.1]octane-8-carboxylate (80 mg, 152 µmol), *N,N*-bis(4-methoxybenzyl)-6-(tributylstannyl)-5-(trifluoromethyl)pyridin-2-amine (126 mg, 183 µmol), tetrakis(triphenylphosphine)palladium(0) (35.2 mg, 30.4 µmol), and lithium chloride (19.3 mg, 456 µmol), copper(I) iodide (5.8 mg, 30.4 µmol) were added to 1.4-dioxane (2 mL), followed by degassing and nitrogen purging three times. Then, the reaction mixture was stirred at 130 °C for 8 h under nitrogen. After the starting material was completely consumed, the reaction temperature was lowered to room temperature, and the palladium catalyst was removed through a Celite filter, and the organic layer was extracted with ethyl acetate. After drying over anhydrous Na₂SO₄, the organic layer was concentrated under reduced pressure. The residue was purified by silica gel chromatography to obtain *tert-*butyl (1*R*,5*S*)-3-(6-(6-(bis(4-methoxybenzyl)amino)-3-(trifluoromethyl)pyridin-2-yl)-3-chloro-5-fluoro-8-methylfuro[3,2-*f*]quinazolin-1-yl)-3,8-diazabicyclo[3.2.1]octane-8-carboxylate (40 mg, 47.2 µmol, 31% yield). LCMS (ES-API, m/z): [M+H]⁺= 848.3.

Step 2: The compound was synthesized in the same manner as in EXAMPLE 43 to obtain *tert*-butyl (1*R*,5*S*)-3-(6-(6-(bis(4-methoxybenzyl)amino)-3-(trifluoromethyl)pyridin-2-yl)-5-fluoro-3-(((2*R*,7a*S*)-2-fluorotetrahydro-1*H*-pyrrolizin-7a(5*H*)-yl)methoxy)-8-methylfuro[3,2-*f*]quinazolin-1-yl)-3,8-diazabicyclo[3.2.1]octane-8-carboxylate (30 mg, 30.9 µmol, 37% yield). LCMS (ES-API, m/z): [M+H]⁺= 971.1.

Step 3: To *tert*-butyl (1*R*,5*S*)-3-(6-(6-(bis(4-methoxybenzyl)amino)-3-(trifluoromethyl)pyridin-2-yl)-5-fluoro-3-(((2*R*,7a*S*)-2-fluorotetrahydro-1*H*-pyrrolizin-7a(5*H*)-yl)methoxy)-8-methylfuro[3,2-*f*]quinazolin-1-yl)-3,8-diazabicyclo[3.2.1]octane-8-carboxylate (20 mg, 41.3 µmol) obtained in Step 2, TFA (2 mL) was added and stirred at 40 °C for 9 h. After the starting material was completely consumed, the mixture was concentrated under reduced pressure. The residue was purified by Prep-HPLC (Column: Waters Xbridge C18 150*50mm* 10um; Mobile phase: [water(0.1% TFA)-acetonitrile]; B%: 30%) to obtain 6-(1-((1*R*,5*S*)-3,8-diazabicyclo[3.2.1]octan-3-yl)-5-fluoro-3-(((2*R*,7a*S*)-2-fluorotetrahydro-1*H*-pyrrolizin-7a(5*H*)-yl)methoxy)-8-methylfuro[3,2-*f*]quinazolin-6-yl)-5-(trifluoromethyl)pyridin-2-amine (7.5 mg, 11.9 µmol, 58% yield). LCMS (ES-API, m/z): [M+H]⁺= 630.6; ¹H NMR (400 MHz, MeOD) δ 7.94 (d, *J* = 8.9 Hz, 1H), 6.87 (d, *J* = 1.5 Hz, 1H), 5.67 (s, 0.5H), 5.54 (s, 0.5H), 4.79 - 4.68 (m, 2H), 4.56 (d, *J* = 15.5 Hz, 1H), 4.48 (s, 1H), 4.19 (s, 2H), 4.14 - 3.80 (m, 5H), 3.50 (td, *J* = 11.0, 6.1 Hz, 1H), 2.84 - 2.55 (m, 3H), 2.52 (s, 4H), 2.38 (dq, *J =* 11.4, 6.4 Hz, 2H), 2.08 (d, J= 8.3 Hz, 5H).

### EXAMPLE 51: Synthesis of 1-((1R,5S)-3,8-diazabicyclo[3.2.1]octan-3-yl)-6-(5-chloro-6-methyl-1H-indazol-4-yl)-5-fluoro-3-(((2R,7aS)-2-fluorotetrahydro-1H-pyrrolizin-7a(5H)-yl)methoxy)-8-methylfuro[3,2-f]quinazoline.

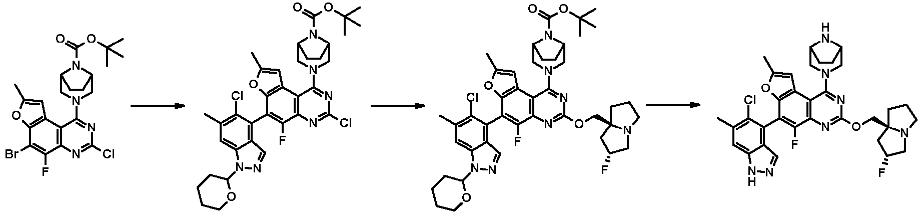

Step 1: *tert*-Butyl (1*R*,5*S*)-3-(6-bromo-3-chloro-5-fluoro-8-methylfuro[3,2-*f*]quinazolin-1-yl)-3,8-diazabicyclo[3.2.1]octane-8-carboxylate (100 mg, 190 µmol), 5-chloro-6-methyl-1-(tetrahydro-2*H*-pyran-2-yl)-4-(4,4,5,5-tetramethyl-1,3,2-dioxaborolan-2-yl)-1*H*-indazole (107 mg, 285 µmol), Tetrakis(triphenylphosphine)palladium(0) (22 mg, 19 µmol) and Potassium phosphate tribasic (121 mg, 571 µmol) was added to 1,4-dioxane (2 mL) and water (0.5 mL), and degassing and nitrogen purging were repeated three times. Then, the reaction mixture was stirred at 95 °C for 30 min under nitrogen. After the starting material was completely consumed, water was added to the reaction mixture to quench the reaction, and the compound was extracted with ethyl acetate. The organic layer was dried over anhydrous Na₂SO₄ and concentrated under reduced pressure. The residue was purified by silica gel chromatography to obtain tert-butyl (1*R*,5*S*)-3-(3-chloro-6-(5-chloro-6-methyl-1-(tetrahydro-2*H*-pyran-2-yl)-1*H*-indazol-4-yl)-5-fluoro-8-methylfuro[3,2-*f*]quinazolin-1-yl)-3,8-diazabicyclo[3.2.1]octane-8-carboxylate (50 mg, 86.3 µmol, 45% yield). LCMS (ES-API, m/z): [M+H]⁺= 696.6.

Step 2: The compound was synthesized in the same manner as in Step 2 of EXAMPLE 43 to obtain tert-butyl (1*R*,5*S*)-3-(6-(5-chloro-6-methyl-1-(tetrahydro-2*H*-pyran-2-yl)-1*H-*indazol-4-yl)-5-fluoro-3-(((2*R*,7a*S*)-2-fluorotetrahydro-1*H*-pyrrolizin-7a(5*H*)-yl)methoxy)-8-methylfuro[3,2-*f*]quinazolin-1-yl)-3,8-diazabicyclo[3.2.1]octane-8-carboxylate (25 mg, 30.5 µmol, 10% yield). LCMS (ES-API, m/z): [M+H]⁺= 819.4.

Step 3: *tert*-butyl (1*R*,5*S*)-3-(6-(5-chloro-6-methyl-1-(tetrahydro-2*H*-pyran-2-yl)-1*H-*indazol-4-yl)-5-fluoro-3-(((2*R*,7a*S*)-2-fluorotetrahydro-1*H*-pyrrolizin-7a(5*H*)-yl)methoxy)-8-methylfuro[3,2-*f*]quinazolin-1-yl)-3,8-diazabicyclo[3.2.1]octane-8-carboxylate (25 mg, 30.5 µmol) obtained in Step 2 was dissolved in DCM (1 mL), and 4.0 M HCl in 1,4-dioxane (2 mL) was added. The mixture was stirred at room temperature for 1 h. After confirming the complete consumption of the starting material, the reaction mixture was concentrated under reduced pressure. The residue was purified by Prep-HPLC (Column: Waters Xbridge C18 150*50mm* 10um; Mobile phase: [water(0.1% TFA)-acetonitrile]; B%: 25%) to obtain 1-((1*R*,5*S*)-3,8-diazabicyclo[3.2.1]octan-3-yl)-6-(5-chloro-6-methyl-1*H*-indazol-4-yl)-5-fluoro-3-(((2*R*,7a*S*)-2-fluorotetrahydro-1*H*-pyrrolizin-7a(5*H*)-yl)methoxy)-8-methylfuro[3,2-*f*]quinazoline (6 mg, 9.46 µmol, 31% yield).

LCMS (ES-API, m/z): [M+H]⁺= 630.6; ¹H NMR (400 MHz, MeOD) δ 7.72 (s, 1H), 7.53 (s, 1H), 6.90 (s, 1H), 5.66 (s, 0.5H), 5.53 (s, 0.5H), 4.75 (d, *J* = 2.8 Hz, 3H), 4.60 (t, *J* = 16.6 Hz, 2H), 4.20 (s, 2H), 4.13 - 3.84 (m, 7H), 2.90 - 2.57 (m, 7H), 2.47 (s, 5H), 2.38 (dq, J = 12.2, 6.4 Hz, 3H), 2.28 - 1.92 (m, 8H).

### EXAMPLE 52: Synthesis of 6-(1-((1R,5S)-3,8-diazabicyclo[3.2.1]octan-3-yl)-5-fluoro-3-(((2R,7aS)-2-fluorotetrahydro-1H-pyrrolizin-7a(5H)-yl)methoxy)-8-methylfuro[3,2-f]quinazolin-6-yl)-4-methylpyridin-2-amine.

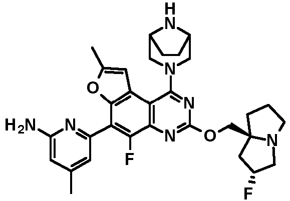

The compound was synthesized in the same manner as in EXAMPLE 43 to obtain 6-(1-((1*R*,5*S*)-3,8-diazabicyclo[3.2.1]octan-3-yl)-5-fluoro-3-(((2*R*,7a*S*)-2-fluorotetrahydro-1*H-*pyrrolizin-7a(5*H*)-yl)methoxy)-8-methylfuro[3,2-*f*]quinazolin-6-yl)-4-methylpyridin-2-amine (23 mg, 42% yield) as a yellow solid.

LCMS (ES-API, m/z): [M+H]⁺= 690.7; ¹H NMR (400 MHz, MeOD) δ 7.19 (s, 1H), 6.98 (d, *J* = 1.2 Hz, 1H), 6.91 (d, *J* = 1.2 Hz, 1H), 5.69 (s, 0.5H), 5.56 (s, 0.5H), 4.73 (s, 2H), 4.50 (t, *J* = 13.1 Hz, 3H), 4.18 (s, 2H), 3.95 (d, *J =* 15.3 Hz, 5H), 2.68 (s, 1H), 2.63 (s, 1H), 2.60 (s, 3H), 2.54 (d, *J* = 1.0 Hz, 4H), 2.48 (s, 1H), 2.39 (s, 2H), 2.22 (s, 1H), 2.06 (s, 4H), 1.33 (d, *J* = 17.7 Hz, 2H).

### EXAMPLE 53: Synthesis of 6-(1-((1R,5S)-3,8-diazabicyclo[3.2.1]octan-3-yl)-5-fluoro-3-(((2R,7aS)-2-fluorotetrahydro-1H-pyrrolizin-7a(5H)-yl)methoxy)-8-methylfuro[3,2-f]quinazolin-6-yl)-5-iodo-4-methylpyridin-2-amine.

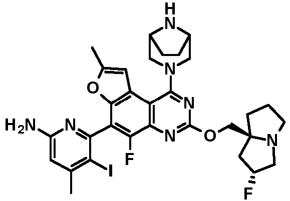

The compound was synthesized in the same manner as in EXAMPLE 43 to obtain 6-(1-((1*R*,5*S*)-3,8-diazabicyclo[3.2.1]octan-3-yl)-5-fluoro-3-(((2*R*,7a*S*)-2-fluorotetrahydro-1*H-*pyrrolizin-7a(5*H*)-yl)methoxy)-8-methylfuro[3,2-*f*]quinazolin-6-yl)-5-iodo-4-methylpyridin-2-amine (12 mg, 23% yield) as a yellow solid.

LCMS (ES-API, m/z): [M+H]⁺= 816.6; ¹H NMR (400 MHz, MeOD) δ 6.86 (s, 1H), 6.78 (s, 1H), 5.69 (s, 0.5H), 5.56 (s, 0.5H), 4.73 (s, 2H), 4.50 (t, *J* = 13.1 Hz, 3H), 4.18 (s, 2H), 3.95 (d, *J* = 15.3 Hz, 5H), 2.68 (s, 1H), 2.63 (s, 1H), 2.60 (s, 3H), 2.54 (d, *J =* 1.0 Hz, 4H), 2.48 (s, 1H), 2.39 (s, 2H), 2.22 (s, 1H), 2.06 (s, 4H), 1.33 (d, *J* = 17.7 Hz, 2H).

### EXAMPLE 54: Synthesis of 4-(1-((1R,5S)-3,8-diazabicyclo[3.2.1]octan-3-yl)-5-fluoro-3-(((2R,7aS)-2-fluorotetrahydro-1H-pyrrolizin-7a(5H)-yl)methoxy)-8-methylfuro[3,2-f]quinazolin-6-yl)benzo[d]thiazol-2-amine.

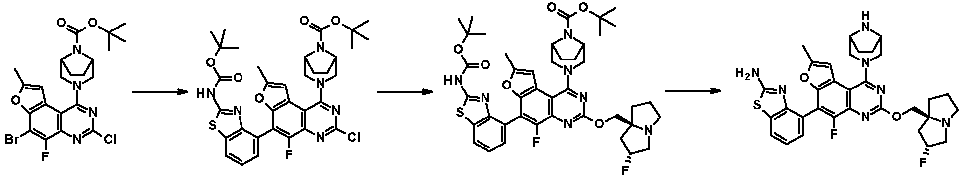

Step 1: *tert*-Butyl (1*R*,5*S*)-3-(6-bromo-3-chloro-5-fluoro-8-methylfuro[3,2-*f*]quinazolin-1-yl)-3,8-diazabicyclo[3.2.1]octane-8-carboxylate (100 mg, 190 µmol), intermediate 20 (107 mg, 285 µmol), [1,1'-Bis(di-*tert*-butylphosphino)ferrocene]dichloropalladium(II) (24.8 mg, 38 µmol), and potassium phosphate tribasic (121 mg, 571 µmol) were added to 1,4-dioxane (2 mL) and water (0.5 mL), followed by degassing and nitrogen purging three times. Then, the reaction mixture was stirred under nitrogen at 90 °C for 24 h. After the starting material was completely consumed, the palladium catalyst was removed by Celite filter. The reaction mixture was diluted with water, and the compound was extracted with ethyl acetate. The organic layer was dried over anhydrous Na₂SO₄ and concentrated under reduced pressure. The residue was purified by silica gel chromatography (15% ethyl acetate/hexane) to obtain *tert*-butyl (1*R*,5*S*)-3-(6-(2-((*tert-*butoxycarbonyl)amino)benzo[*d*]thiazol-4-yl)-3-chloro-5-fluoro-8-methylfuro[3,2-*f*]quinazolin-1-yl)-3,8-diazabicyclo[3.2.1]octane-8-carboxylate (26 mg, 37.4 µmol, 20% yield). LCMS (ES-API, m/z): [M+H]⁺= 696.2.

Step 2: ((2*R*,7a*S*)-2-fluorotetrahydro-1*H*-pyrrolizin-7a(5*H*)-yl)methanol (40.1 mg, 252 µmol) and sodium hydride (10.1 mg, 252 µmol) were added to THF (2 mL) and stirred at room temperature for 30 min. After 30 min, *tert*-butyl (1*R*,5*S*)-3-(6-(2-((*tert-*butoxycarbonyl)amino)benzo[d]thiazol-4-yl)-3-chloro-5-fluoro-8-methylfuro[3,2-*f*]quinazolin-1-yl)-3,8-diazabicyclo[3.2.1]octane-8-carboxylate (35 mg, 50.3 µmol) obtained in Step 1 was added to the reaction mixture at room temperature and stirred for 1 h. After the starting material was completely consumed, water was slowly added to quench the reaction and the organic layer was extracted with ethyl acetate. After drying over anhydrous sodium sulfate, concentration under reduced pressure was performed. The residue was purified by silica gel chromatography (5% methanol/DCM) to obtain *tert-*butyl (1*R*,5*S*)-3-(6-(2-((*tert*-butoxycarbonyl)amino)benzo[*d*]thiazol-4-yl)-5-fluoro-3-(((2*R*,7a*S*)-2-fluorotetrahydro-1*H*-pyrrolizin-7a(5*H*)-yl)methoxy)-8-methylfuro[3,2-*f*]quinazolin-1-yl)-3,8-diazabicyclo[3.2.1]octane-8-carboxylate (20 mg, 24.5 µmol, 49% yield). LCMS (ES-API, m/z): [M+H]⁺= 833.4

Step 3: *tert*-butyl (1*R*,5*S*)-3-(6-(2-((tert-butoxycarbonyl)amino)benzo[d]thiazol-4-yl)-5-fluoro-3-(((2*R*,7a*S*)-2-fluorotetrahydro-1*H*-pyrrolizin-7a(5*H*)-yl)methoxy)-8-methylfuro[3,2-*f*]quinazolin-1-yl)-3,8-diazabicyclo[3.2.1]octane-8-carboxylate (20 mg, 24.5 µmol) obtained in Step 2 was dissolved in DCM (1 mL), 4.0 M HCl in 1,4-dioxane (2 mL) was added, and the mixture was stirred at 50 °C for 1 h. After the starting material was completely consumed, the reaction mixture was concentrated under reduced pressure. The residue was purified by Prep-HPLC (Column: Waters Xbridge C18 150*50mm* 10um; Mobile phase: [water(0.1% TFA)-acetonitrile]; B%: 35%) to obtain 4-(1-((1*R*,5*S*)-3,8-diazabicyclo[3.2.1]octan-3-yl)-5-fluoro-3-(((2*R*,7a*S*)-2-fluorotetrahydro-1*H*-pyrrolizin-7a(5*H*)-yl)methoxy)-8-methylfuro[3,2-*f*]quinazolin-6-yl)benzo[*d*]thiazol-2-amine (13 mg, 21.04 µmol, 86% yield).

LCMS (ES-API, m/z): [M+H]⁺= 618.7; ¹H NMR (400 MHz, MeOD) δ 7.85 (dd, *J* = 7.9, 1.3 Hz, 1H), 7.45 (d, *J* = 7.5 Hz, 1H), 7.33 (t, *J* = 7.8 Hz, 1H), 6.85 (d, *J* = 1.2 Hz, 1H), 5.65 (s, 0.5H), 5.52 (s, 0.5H), 4.85 - 4.66 (m, 3H), 4.63 (s, 1H), 4.49 (d, *J* = 14.5 Hz, 1H), 4.19 (s, 2H), 4.12 - 3.77 (m, 4H), 2.79 - 2.53 (m, 3H), 2.51 (s, 2H), 2.44 - 2.31 (m, 2H), 2.12 (d, *J* = 44.9 Hz, 4H).

### EXAMPLE 55: Synthesis of 4-(1-((1R,5S)-3,8-diazabicyclo[3.2.1]octan-3-yl)-5-fluoro-3-(((2R,7aS)-2-fluorotetrahydro-1H-pyrrolizin-7a(5H)-yl)methoxy)-8-methylfuro[3,2-f]quinazolin-6-yl)-7-fluorobenzo[d]thiazol-2-amine.

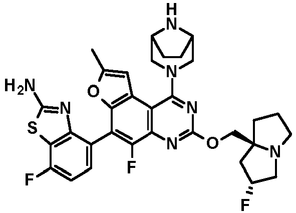

The compound was synthesized in the same manner as in EXAMPLE 54 using Intermediate 19. Lyophilization was performed to obtain 4-(1-((1*R*,5*S*)-3,8-diazabicyclo[3.2.1]octan-3-yl)-5-fluoro-3-(((2*R*,7a*S*)-2-fluorotetrahydro-1*H*-pyrrolizin-7a(5*H*)-yl)methoxy)-8-methylfuro[3,2-*f*]quinazolin-6-yl)-7-fluorobenzo[*d*]thiazol-2-amine (26 mg, 31.4 µmol, 83% yield).

LCMS (ES-API, m/z): [M+H]⁺= 636.7; ¹H NMR (400 MHz, MeOD) δ 7.47 (dd, *J* = 8.5, 5.4 Hz, 1H), 7.11 (t, *J* = 8.8 Hz, 1H), 6.85 (d, *J* = 1.3 Hz, 1H), 5.66 (s, 0.5H), 5.53 (s, 0.5H), 4.75 (t, *J* = 5.3 Hz, 2H), 4.72 - 4.57 (m, 2H), 4.49 (d, *J* = 14.4 Hz, 1H), 4.19 (s, 2H), 4.14 - 3.82 (m, 6H), 2.78 - 2.59 (m, 3H), 2.59 - 2.45 (m, 4H), 2.38 (dq, *J* = 11.7, 6.5 Hz, 3H), 2.22 - 2.13 (m, 2H), 2.04 (d, *J* = 9.6 Hz, 4H).

### EXAMPLE 56: Synthesis of 3-(1-((1R,5S)-3,8-diazabicyclo[3.2.1]octan-3-yl)-5-fluoro-3-(((2R,7aS)-2-fluorotetrahydro-1H-pyrrolizin-7a(5H)-yl)methoxy)-8-methylfuro[3,2-f]quinazolin-6-yl)-5-methyl-4-(trifluoromethyl)aniline.

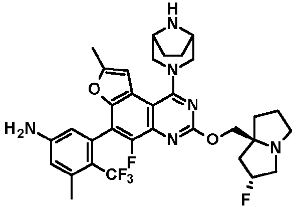

The compound was synthesized in the same manner as in EXAMPLE 51 using *N,N*-Bis(4-methoxybenzyl)-3-methyl-5-(4,4,5,5-tetramethyl-1,3,2-dioxaborolan-2-yl)-4-(trifluoromethyl)aniline. Purification was performed by Prep-HPLC to obtain 3-(1-((1*R*,5*S*)-3,8-diazabicyclo[3.2.1]octan-3-yl)-5-fluoro-3-(((2*R*,7a*S*)-2-fluorotetrahydro-1*H-*pyrrolizin-7a(5*H*)-yl)methoxy)-8-methylfuro[3,2-*f*]quinazolin-6-yl)-5-methyl-4-(trifluoromethyl)aniline (3.3 mg, 4.36 µmol, 6% yield).

LCMS (ES-API, m/z): [M+H]⁺= 642.7; ¹H NMR (400 MHz, MeOD) δ 7.04 (s, 1H), 6.85 (d, *J* = 5.3 Hz, 2H), 5.67 (s, 0.5H), 5.54 (s, 0.5H), 4.92 (s, 1H), 4.82 - 4.62 (m, 3H), 4.53 (d, *J* = 14.1 Hz, 2H), 4.17 (s, 1H), 4.15 - 4.03 (m, 1H), 3.94 (t, *J* = 14.7 Hz, 3H), 3.54 (dd, J = 10.3, 5.6 Hz, 1H), 2.81 - 2.62 (m, 2H), 2.58 (s, 1H), 2.49 (q, *J* = 3.2 Hz, 2H), 2.40 (dt, *J* = 11.3, 6.3 Hz, 1H), 2.17 (s, 1H), 2.15 - 1.93 (m, 4H).

### EXAMPLE 57: Synthesis of 4-(1-((1R,5S)-3,8-diazabicyclo[3.2.1]octan-3-yl)-5-fluoro-3-(((2R,7aS)-2-fluorotetrahydro-1H-pyrrolizin-7a(5H)-yl)methoxy)-8-methylfuro[3,2-f]quinazolin-6-yl)-5-ethynyl-6-fluoronaphthalen-2-amine.

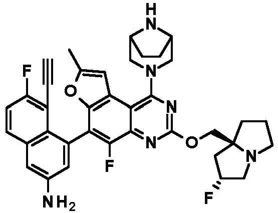

The compound was synthesized in the same manner as in EXAMPLE 44 using Intermediate 18. Purification by Prep-HPLC and lyophilization was performed to obtain 4-(1-((1*R*,5*S*)-3,8-diazabicyclo[3.2.1]octan-3-yl)-5-fluoro-3-(((2*R*,7a*S*)-2-fluorotetrahydro-1*H*-pyrrolizin-7a(5*H*)-yl)methoxy)-8-methylfuro[3,2-*f*]quinazolin-6-yl)-5-ethynyl-6-fluoronaphthalen-2-amine (26 mg, 39.8 µmol, 26% yield).

LCMS (ES-API, m/z): [M+H]⁺= 653.8; ¹H NMR (400 MHz, DMSO-*d*₆, ppm) δ 7.73 (dd, *J* = 9.2, 6.0 Hz, 1H), 7.28 (t, *J* = 9.0 Hz, 1H), 7.09 - 6.94 (m, 2H), 6.76 (s, 1H), 5.58 (s, 2H), 5.29 (s, 0.5H), 5.15 (s, 0.5H), 4.11 - 3.84 (m, 3H), 3.37 (d, *J* = 22.7 Hz, 4H), 3.03 (d, *J =* 9.2 Hz, 1H), 2.95 (s, 1H), 2.76 (d, *J* = 7.1 Hz, 1H), 2.31 (s, 6H), 2.14 - 1.91 (m, 4H), 1.90 - 1.64 (m, 4H), 1.51 (s, 3H).

### EXAMPLE 58: Synthesis of 4-(1-((1R,5S)-3,8-diazabicyclo[3.2.1]octan-3-yl)-3-((1-((1,1-difluoro-6-azaspiro[2.5]octan-6-yl)methyl)cyclopropyl)methoxy)-5-fluoro-8-methylfuro[3,2-f]quinazolin-6-yl)-5-ethynyl-6-fluoronaphthalen-2-amine.

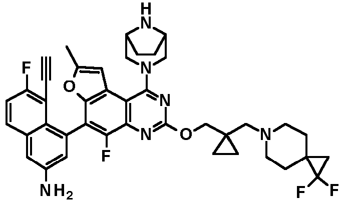

The compound was synthesized in the same manner as in EXAMPLE 44 using Intermediate 18 and intermediate 8. Lyophilization was performed to obtain 4-(1-((1*R*,5*S*)-3,8-diazabicyclo[3.2.1]octan-3-yl)-3-((1-((1,1-difluoro-6-azaspiro[2.5]octan-6-yl)methyl)cyclopropyl)methoxy)-5-fluoro-8-methylfuro[3,2-*f*]quinazolin-6-yl)-5-ethynyl-6-fluoronaphthalen-2-amine (70 mg, 96.6 µmol, 58% yield).

LCMS (ES-API, m/z): [M+H]⁺= 724.8; ¹H NMR (400 MHz, MeOD, ppm) δ 7.79 (dd, *J =* 9.2, 5.8 Hz, 1H), 7.32 - 7.23 (m, 2H), 7.15 (d, *J* = 2.4 Hz, 1H), 6.87 (s, 1H), 4.60 (dd, *J =* 44.9, 13.1 Hz, 2H), 4.50 - 4.32 (m, 2H), 4.20 (d, *J* = 21.3 Hz, 2H), 3.96 (dd, *J* = 32.0, 14.2 Hz, 3H), 2.91 (s, 1H), 2.46 (s, 3H), 2.28 (s, 2H), 2.10 (s, 4H), 1.33 (dt, J = 16.9, 8.3 Hz, 3H), 1.01 (s, 2H), 0.90 (d, *J* = 4.5 Hz, 2H).

### EXAMPLE 59: Synthesis of 4-(1-((1R,5S)-3,8-diazabicyclo[3.2.1]octan-3-yl)-3-((1-((1,1-difluoro-6-azaspiro[2.5]octan-6-yl)methyl)cyclopropyl)methoxy)-5-fluoro-8-methylfuro[3,2-f]quinazolin-6-yl)-5-ethynyl-6-fluoronaphthalen-2-ol.

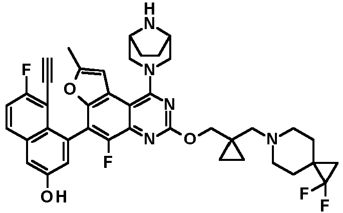

The compound was synthesized in the same manner as in EXAMPLE 44 using Intermediate 8. 4-(1-((1*R*,5*S*)-3,8-diazabicyclo[3.2.1]octan-3-yl)-3-((1-((1,1-difluoro-6-azaspiro[2.5]octan-6-yl)methyl)cyclopropyl)methoxy)-5-fluoro-8-methylfuro[3,2-*f*]quinazolin-6-yl)-5-ethynyl-6-fluoronaphthalen-2-ol (16 mg, 22.1 mmol, 58% yield) was obtained as a white solid.

LCMS (ES-API, m/z): [M+H]⁺= 726.8; ¹H NMR (400 MHz, CD₃OD, ppm): δ 10.26 (d, *J =* 13.3 Hz, 1H), 8.06 - 7.98 (m, 1H), 7.54 - 7.43 (m, 1H), 7.21 (s, 1H), 6.79 (s, 1H), 4.31 (s, 1H), 4.15 (s, 4H), 3.77 (s, 2H), 3.52 (s, 1H), 2.41 (s, 3H), 1.57 (s, 1H), 1.47 (s, 2H), 1.18 (s, 1H), 0.89 (s, 1H), 0.80 (s, 1H), 0.66 (s, 1H), 0.43 (s, 1H).

### EXAMPLE 60: Synthesis of 6-(1-((1R,5S)-3,8-diazabicyclo[3.2.1]octan-3-yl)-5-fluoro-3-(((2R,7aS)-2-fluorotetrahydro-1H-pyrrolizin-7a(5H)-yl)methoxy)-8-methylfuro[3,2-f]quinazolin-6-yl)-4-methyl-5-(trifluoromethyl)pyridin-2-amine.

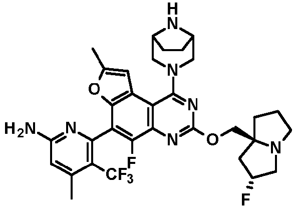

The compound was synthesized in the same manner as in EXAMPLE 20 using Intermediate 3. 6-(1-((1*R*,5*S*)-3,8-diazabicyclo[3.2.1]octan-3-yl)-5-fluoro-3-(((2*R*,7a*S*)-2-fluorotetrahydro-1*H*-pyrrolizin-7a(5*H*)-yl)methoxy)-8-methylfuro[3,2-*f*]quinazolin-6-yl)-4-methyl-5-(trifluoromethyl)pyridin-2-amine (9 mg, 13.9 mmol, 46% yield) was obtained. LCMS (ES-API, m/z): [M+H]⁺= 644.3; ¹H NMR (400 MHz, DMSO-*d*₆, ppm): δ 8.49 (br d, *J* = 1.2 Hz, 1H), 7.98 (br s, 1H), 7.75 - 7.46 (m, 1H), 7.27 - 7.07 (m, 1H), 6.70 (br s, 2H), 6.50 (s, 1H), 5.39 - 5.14 (m, 1H), 4.16 - 3.93 (m, 2H), 3.76 - 3.44 (m, 4H), 3.25 - 3.17 (m, 1H), 3.14 - 2.94 (m, 4H), 2.90 - 2.75 (m, 1H), 2.37 (br d, *J* = 1.6 Hz, 3H), 2.21 - 1.94 (m, 4H), 1.90 - 1.69 (m, 4H), 1.63 - 1.18 (m, 3H).

### EXAMPLE 61: Synthesis of 4-(1-((1R,5S)-3,8-diazabicyclo[3.2.1]octan-3-yl)-5-fluoro-8-methyl-3-((S)-1-((S)-1-methylpyrrolidin-2-yl)ethoxy)furo[3,2-f]quinazolin-6-yl)-5-ethynyl-6-fluoronaphthalen-2-ol.

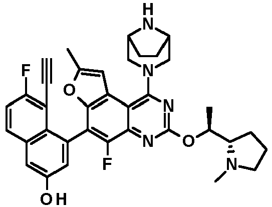

The compound was synthesized in the same manner as in EXAMPLE 44 using (*S*)-1-((*S*)-1-Methylpyrrolidin-2-yl)ethanol. 4-(1-((1*R*,5*S*)-3,8-Diazabicyclo[3.2.1]octan-3-yl)-5-fluoro-8-methyl-3-((*S*)-1-((*S*)-1-methylpyrrolidin-2-yl)ethoxy)furo[3,2-*f*]quinazolin-6-yl)-5-ethynyl-6-fluoronaphthalen-2-ol (5 mg, 0.008 mmol, 36% yield) was obtained.

LCMS (ES-API, m/z): [M+H]⁺= 738.7; ¹H NMR (400 MHz, CD₃OD, ppm): δ 7.90 (ddd, *J* = 8.2, 5.8, 2.0 Hz, 1H), 7.43 - 7.30 (m, 1H), 7.22 (d, *J* = 2.5 Hz, 1H), 6.85 (d, *J* = 5.1 Hz, 1H), 5.50 (s, 1H), 4.25 (s, 1H), 4.19 (s, 2H), 4.12 - 3.96 (m, 2H), 3.83 (d, *J* = 8.8 Hz, 2H), 3.72 (s, 2H), 3.64 (q, *J =* 7.2 Hz, 3H), 3.15 (s, 2H), 2.46 (s, 3H), 2.28 - 2.01 (m, 2H), 1.58 (d, *J =* 6.1 Hz, 3H).

### EXAMPLE 62: Synthesis of 4-(1-((1R,5S)-3,8-diazabicyclo[3.2.1]octan-3-yl)-5-fluoro-3-(((2R,7aS)-2-fluorotetrahydro-1H-pyrrolizin-7a(5H)-yl)methoxy)-8-methylfuro[3,2-f]quinazolin-6-yl)-2-amino-7-fluorobenzo[b]thiophene-3-carbonitrile.

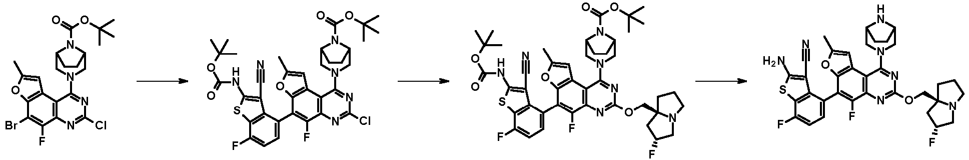

Step 1: *tert*-Butyl (1*R*,5*S*)-3-(6-bromo-3-chloro-5-fluoro-8-methylfuro[3,2-*f*]quinazolin-1-yl)-3,8-diazabicyclo[3.2.1]octane-8-carboxylate (300 mg, 571 µmol), *tert*-butyl (3-cyano-7-fluoro-4-(4,4,5,5-tetramethyl-1,3,2-dioxaborolan-2-yl)benzo[b]thiophen-2-yl)carbamate (358 mg, 285 µmol), dichloro[bis(2-(diphenylphosphino)phenyl)ether]palladium(II) (81.6 mg, 393 µmol) and Cs₂CO₃ (558 mg, 1710 µmol) were added to 1,4-dioxane (4 mL) and water (1 mL), and then degassing and nitrogen purging were repeated three times. Then, the reaction mixture was stirred at 120 °C under nitrogen for 6 h. After the starting material was completely consumed, the palladium catalyst was removed using a Celite filter. The reaction mixture was diluted with water, and the compound was extracted with ethyl acetate. The organic layer was dried over anhydrous Na₂SO₄ and concentrated under reduced pressure. The residue was purified by silica gel chromatography to obtain *tert-*butyl (1*R*,5*S*)-3-(6-(2-((*tert*-butoxycarbonyl)amino)-3-cyano-7-fluorobenzo[*b*]thiophen-4-yl)-3-chloro-5-fluoro-8-methylfuro[3,2-*f*]quinazolin-1-yl)-3,8-diazabicyclo[3.2.1]octane-8-carboxylate (290 mg, 393 µmol, 69% yield). LCMS (ES-API, m/z): [M+H]⁺= 738.2.

Step 2: ((2*R*,7a*S*)-2-fluorotetrahydro-1*H*-pyrrolizin-7a(5*H*)-yl)methanol (111.5 mg, 700 µmol) and NaH (28 mg, 700 µmol) were added to THF (2 mL) and stirred at room temperature for 30 min. After 30 min, *tert-*butyl (1*R*,5*S*)-3-(6-(2-((*tert-*butoxycarbonyl)amino)-3-cyano-7-fluorobenzo[b]thiophen-4-yl)-3-chloro-5-fluoro-8-methylfuro[3,2-*f*]quinazolin-1-yl)-3,8-diazabicyclo[3.2.1]octane-8-carboxylate (100 mg, 140 µmol) was added to the reaction mixture at room temperature, and the mixture was stirred at 50 °C for 2 h. After the starting material was completely consumed, water was slowly added at room temperature to quench the reaction, and the organic layer was extracted with ethyl acetate. After drying over anhydrous Na₂SO₄, concentration under reduced pressure was performed. The residue was purified by silica gel chromatography to obtain *tert*-butyl (1*R*,5*S*)-3-(6-(2-((*tert*-butoxycarbonyl)amino)-3-cyano-7-fluorobenzo[b]thiophen-4-yl)-5-fluoro-3-(((2*R*,7a*S*)-2-fluorotetrahydro-1*H*-pyrrolizin-7a(5*H*)-yl)methoxy)-8-methylfuro[3,2-*f*]quinazolin-1-yl)-3,8-diazabicyclo[3.2.1]octane-8-carboxylate (50 mg, 59.8 µmol, 43% yield). LCMS (ES-API, m/z): [M+H]⁺= 836.9.

Step 3: The compound was synthesized in the same manner as in Step 3 of EXAMPLE 54 to obtain 4-(1-((1*R*,5*S*)-3,8-diazabicyclo[3.2.1]octan-3-yl)-5-fluoro-3-(((2*R*,7a*S*)-2-fluorotetrahydro-1*H*-pyrrolizin-7a(5*H*)-yl)methoxy)-8-methylfuro[3,2-*f*]quinazolin-6-yl)-2-amino-7-fluorobenzo[b]thiophene-3-carbonitrile (17 mg, 25.8 µmol, 43 % yield).

LCMS (ES-API, m/z): [M+H]⁺= 660.7; ¹H NMR (400 MHz, MeOD) δ 7.40 (dd, *J* = 8.4, 5.1 Hz, 1H), 7.09 (t, *J =* 8.9 Hz, 1H), 6.79 (s, 1H), 5.41 (s, 0.5H), 5.28 (s, 0.5H), 4.55 - 4.06 (m, 4H), 3.80 - 3.46 (m, 3H), 3.28 (dd, *J* = 29.1, 19.7 Hz, 39H), 3.05 (d, *J* = 5.5 Hz, 1H), 2.54 - 1.62 (m, 11H), 1.31 (s, 1H).

### EXAMPLE 63: Synthesis of 4-(1-((1R,5S)-3,8-diazabicyclo[3.2.1]octan-3-yl)-5-fluoro-3-(((S,Z)-2-(fluoromethylene)tetrahydro-1H-pyrrolizin-7a(5H)-yl)methoxy)-8-methylfuro[3,2-f]quinazolin-6-yl)-2-amino-7-fluorobenzo[b]thiophene-3-carbonitrile.

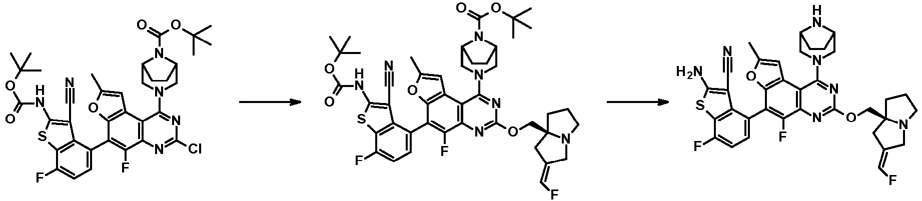

Step 1: ((2*R*,7a*S*)-2-Fluorotetrahydro-1*H*-pyrrolizin-7a(5*H*)-yl)methanol (139 mg, 814 µmol) and sodium *tert*-butoxide (156 mg, 1,630 µmol) were added to THF (1 mL) and DMF (3 mL), and stirred at room temperature for 30 min. After 30 min, *tert*-butyl (1*R*,5*S*)-3-(6-(2-((*tert*-butoxycarbonyl)amino)-3-cyano-7-fluorobenzo[b]thiophen-4-yl)-3-chloro-5-fluoro-8-methylfuro[3,2-*f*]quinazolin-1-yl)-3,8-diazabicyclo[3.2.1]octane-8-carboxylate (200 mg, 271 µmol) obtained in Step 2 of EXAMPLE 62 was added to the reaction mixture at room temperature, and stirred for 2 h. After the starting material was completely consumed, water was slowly added at room temperature to quench the reaction and the organic layer was extracted with ethyl acetate. After drying over anhydrous Na₂SO₄, concentration under reduced pressure was performed. The residue was purified by silica gel chromatography to obtain *tert*-butyl (1*R*,5*S*)-3-(6-(2-((*tert*-butoxycarbonyl)amino)-3-cyano-7-fluorobenzo[b]thiophen-4-yl)-5-fluoro-3-(((*S*,*Z*)-2-(fluoromethylene) tetrahydro-1*H*-pyrrolizin-7a(5*H*)-yl)methoxy)-8-methylfuro[3,2-*f*]quinazolin-1-yl)-3,8-diazabicyclo[3.2.1]octane-8-carboxylate (110 mg, 126 µmol, 46% yield). LCMS (ES-API, m/z): [M+H]⁺= 873.3.

Step 2: The compound was synthesized in the same manner as in Step 3 of EXAMPLE 54. 4-(1-((1*R*,5*S*)-3,8-Diazabicyclo[3.2.1]octan-3-yl)-5-fluoro-3-(((*S*,*Z*)-2-(fluoromethylene)tetrahydro-1*H*-pyrrolizin-7a(5*H*)-yl)methoxy)-8-methylfuro[3,2-*f*]quinazolin-6-yl)-2-amino-7-fluorobenzo[b]thiophene-3-carbonitrile (32 mg, 45.2 µmol, 39% yield) was obtained.

LCMS (ES-API, m/z): [M+H]⁺= 672.7; ¹H NMR (400 MHz, MeOD) δ 7.41 (dd, *J* = 8.4, 5.1 Hz, 1H), 7.11 (t, *J =* 8.9 Hz, 1H), 6.84 (d, *J* = 14.4 Hz, 1.5H), 6.65 (s, 0.5H), 4.68 - 4.39 (m, 2H), 4.21 (d, *J* = 64.5 Hz, 2H), 3.99 - 3.60 (m, 3H), 3.22 (q, *J* = 7.3 Hz, 2H), 3.14 - 2.79 (m, 3H), 2.62 (d, *J=* 15.9 Hz, 1H), 2.49 (s, 3H), 2.35 - 1.70 (m, 7H), 1.44 - 1.14 (m, 3H).

### EXAMPLE 64: Synthesis of 4-(1-((1R,5S)-3,8-diazabicyclo[3.2.1]octan-3-yl)-5-fluoro-3-(((S,Z)-2-(fluoromethylene)tetrahydro-1H-pyrrolizin-7a(5H)-yl)methoxy)-8-methylfuro[3,2-f]quinazolin-6-yl)-5-ethynyl-6-fluoronaphthalen-2-ol.

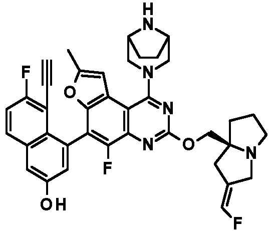

The compound was synthesized in the same manner as in EXAMPLE 44 using (*S*,*Z*)-(2-(fluoromethylene)tetrahydro-1*H*-pyrrolizin-7a(5*H*)-yl)methanol. 4-(1-((1*R*,5*S*)-3,8-diazabicyclo[3.2.1]octan-3-yl)-5-fluoro-3-(((*S*,*Z*)-2-(fluoromethylene)tetrahydro-1*H-*pyrrolizin-7a(5*H*)-yl)methoxy)-8-methylfuro[3,2-*f*]quinazolin-6-yl)-5-ethynyl-6-fluoronaphthalen-2-ol (32 mg, 0.048 mmol, 39% yield) was obtained.

LCMS (ES-API, m/z): [M+H]⁺= 666.7; ¹H NMR (400 MHz, CD₃OD, ppm): δ 8.03 - 7.99(m, 1H), 7.51-7.44 (m, 1H), 7.22 (s, 1H), 6.87 (d, *J* = 2.6 Hz, 1H), 6.65 (s, 1H), 4.82 - 4.66 (m, 3H), 4.44 - 4.35 (m, 1H), 4.27 - 4.22 (m, 1H), 4.18 - 4.14 (m, 1H), 4.09 - 3.97 (m, 2H), 3.97 - 3.84 (m, 3H), 3.54 - 3.44 (m, 1H), 2.77 - 2.58 (m, 3H), 2.47 (s, 3H), 2.43 - 2.22 (m, 3H), 2.20 - 1.95 (m, 4H).

### EXAMPLE 65: Synthesis of 4-(1-((1R,5S)-3,8-diazabicyclo[3.2.1]octan-3-yl)-5-fluoro-3-(((2S,7aR)-2-fluoro-6-methylenetetrahydro-1H-pyrrolizin-7a(5H)-yl)methoxy)-8-methylfuro[3,2-f]quinazolin-6-yl)-5-ethynyl-6-fluoronaphthalen-2-ol.

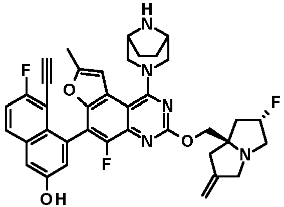

The compound was synthesized in the same manner as in EXAMPLE 44 using Intermediate 16. 4-(1-((1*R*,5*S*)-3,8-diazabicyclo[3.2.1]octan-3-yl)-5-fluoro-3-(((2*S*,7a*R*)-2-fluoro-6-methylenetetrahydro-1*H*-pyrrolizin-7a(5*H*)-yl)methoxy)-8-methylfuro[3,2-*f*]quinazolin-6-yl)-5-ethynyl-6-fluoronaphthalen-2-ol (20 mg, 70% yield) was obtained as a white solid.

LCMS (ES-API, m/z): [M+H]⁺= 666.7; ¹H NMR (400 MHz, CD₃OD, ppm): δ 7.90 - 7.87(m, 1H), 7.38-7.31 (m, 1H), 7.21 (s, 1H), 6.78 (z, 1H), 6.65 (s, 1H), 4.82 - 4.66 (m, 3H), 4.44 - 4.35 (m, 1H), 4.27 - 4.22 (m, 1H), 4.18 - 4.14 (m, 1H), 4.09 - 3.97 (m, 2H), 3.97 - 3.84 (m, 3H), 3.54 - 3.44 (m, 1H), 2.77 - 2.58 (m, 3H), 2.43 (s, 3H), 2.43 - 2.22 (m, 3H), 2.20 - 1.95 (m, 4H).

### EXAMPLE 66: Synthesis of 4-(1-((1R,5S)-3,8-diazabicyclo[3.2.1]octan-3-yl)-3-(((S)-2-(difluoromethylene)tetrahydro-1H-pyrrolizin-7a(5H)-yl)methoxy)-5-fluoro-8-methylfuro[3,2-f]quinazolin-6-yl)-5-ethynyl-6-fluoronaphthalen-2-ol.

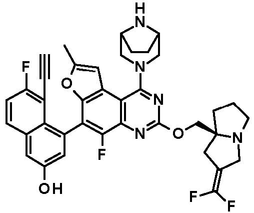

The compound was synthesized in the same manner as in EXAMPLE 44 using (7a*R*)-2-(difluoromethylene)tetrahydro-1*H*-pyrrolizine-7a(5*H*)-methanol. 4-(1-((1*R*,5*S*)-3,8-diazabicyclo[3.2.1]octan-3-yl)-3-(((*S*)-2-(difluoromethylene)tetrahydro-1*H*-pyrrolizin-7a(5*H*)-yl)methoxy)-5-fluoro-8-methylfuro[3,2-*f*]quinazolin-6-yl)-5-ethynyl-6-fluoronaphthalen-2-ol (27 mg, 47% yield) was obtained as an orange solid.

LCMS (ES-API, m/z): [M+H]⁺= 684.2; ¹H NMR (400 MHz, DMSO-*d*₆, ppm): δ 10.28 (s, 1H), 8.01 (m, 1H), 7.48 (t, *J =* 8.4 Hz, 1H), 7.44 (s, 1H), 7.21 (s, 1H), 6.84 (s, 1H), 4.16 - 3.95 (m, 3H), 3.66 (m, 1H), 3.55 - 3.46 (m, 5H), 3.00 (m, 1H), 2.68 (s, 1H), 2.38 (s, 3H), 2.33 (s, 1H), 2.00 - 1.58 (m, 9H).

### EXAMPLE 67: Synthesis of 4-(1-((1R,5S)-3,8-diazabicyclo[3.2.1]octan-8-yl)-5-fluoro-3-(((2R,7aS)-2-fluorotetrahydro-1H-pyrrolizin-7a(5H)-yl)methoxy)-8-methylfuro[3,2-f]quinazolin-6-yl)-5-ethynyl-6-fluoronaphthalen-2-amine.

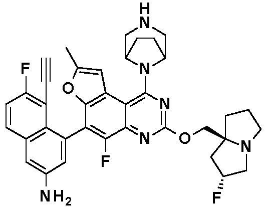

The compound was synthesized in the same manner as in EXAMPLE 57 using *tert*-Butyl (1*R*,5*S*)-3,8-diazabicyclo[3.2.1]octane-3-carboxylate. 4-(1-((1*R*,5*S*)*-*3,8-diazabicyclo[3.2.]octan-8-yl)-5-fluoro-3-(((2*R*,7a*S*)-2-fluorotetrahydro-1*H*-pyrrolizin-7a(5*H*)-yl)methoxy)-8-methylfuro[3,2-*f*]quinazolin-6-yl)-5-ethynyl-6-fluoronaphthalen-2-amine (50 mg, 62% yield) was obtained as a brown solid.

LCMS (ES-API, m/z): [M+H]⁺= 653.2; ¹H NMR (400 MHz, DMSO-*d*₆, ppm): δ 7.80 (m, 1H), 7.35 (t, J = 9.0 Hz, 1H), 7.08 (m, 2H), 6.85 (s, 1H), 5.65 (s, 2H), 5.36-5.22 (m, 1H), 4.43 (s, 2H), 4.06 (m, 2H), 3.41 (s, 1H), 3.21-3.03 (m, 5H), 2.84 (m, 1H), 2.68(m, 2H), 2.41 (s, 3H), 2.16 - 1.77 (m, 14H).

### EXAMPLE 68: Synthesis of 4-(9-((1R,5S)-3,8-diazabicyclo[3.2.1]octan-3-yl)-7-(((2R,7aS)-2-fluorotetrahydro-1H-pyrrolizin-7a(5H)-yl)methoxy)-2-methyl-2H-pyrazolo[4,3-f]quinazolin-4-yl)naphthalen-2-ol.

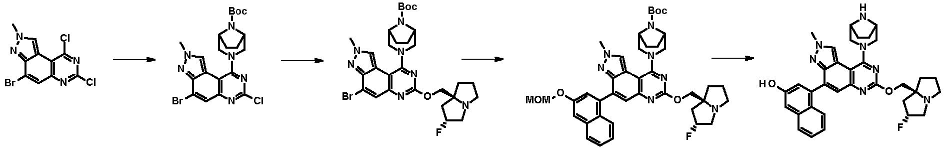

Step 1: 4-bromo-7,9-dichloro-2-methyl-2*H*-pyrazolo[4,3-*f*]quinazoline (300 mg, 0.904 mmol) was dissolved in DCM (48 mL), and DIPEA (350 mg, 2.71 mmol, 0.501 mL) was added, then *tert*-butyl (1*R*,5*S*)-3,8-diazabicyclo[3.2.1]octane-8-carboxylate (230 mg, 1.08 mmol) was added. The reaction mixture was stirred at room temperature for 1 h. The reaction mixture was diluted with water (80 mL) and extracted with DCM (50 mL*3). The organic matter was washed with saturated NaCl aqueous solution (50 mL*2), dried over NaSO₄, filtered, and concentrated to obtain the residue. The product was precipitated in hexane, filtered, and the filtered solid was dried. *tert*-Butyl (1*R*,5*S*)-3-(4-bromo-7-chloro-2-methyl-2*H*-pyrazolo[4,3-*f*]quinazolin-9-yl)-3,8-diazabicyclo[3.2.1]octane-8-carboxylate (437 mg, 0.861 mmol, 95% yield) was obtained as a pale beige solid. LCMS (ES-API, m/z): [M+H]⁺= 508.8.

Step 2: ((2*R*,7a*S*)-2-Fluorotetrahydro-1*H*-pyrrolizin-7a(5*H*)-yl)methanol (480 mg, 3.02 mmol) was dissolved in THF (15 mL), and NaH (120.6 mg, 3.02 mmol) was added at 0 °C. The mixture was stirred at room temperature for 20 minutes, and *tert*-butyl (1*R*,5*S*)-3-(4-bromo-7-chloro-2-methyl-2*H*-pyrazolo[4,3-*f*]quinazolin-9-yl)-3,8-diazabicyclo[3.2.1]octane-8-carboxylate (123 mg, 0.302 mmol) obtained in Step 1 was slowly added to a solution of THF (5 mL) and reacted at room temperature for 3 hours. The reaction mixture was poured into water (20 mL) and extracted with ethyl acetate (50 mL*2). The combined organic layers were washed with NaCl aqueous solution (40 mL*2) and dried over Na₂SO₄. The filtered solution was concentrated under reduced pressure to obtain a residue. The residue was purified by silica gel chromatography (5% DCM/MeOH). *tert-*butyl (1*R*,5*S*)-3-(4-bromo-7-(((2*R*,7a*S*)-2-fluorotetrahydro-1*H-*pyrrolizin-7a(5*H*)-yl)methoxy)-2-methyl-2*H*-pyrazolo[4,3-*f*]quinazolin-9-yl)-3,8-diazabicyclo[3.2.1]octane-8-carboxylate (150 mg, 0.283 mmol, 94% yield) was obtained. LCMS (ES-API, m/z): [M+H]⁺= 531.4.

Step 3: *tert*-butyl (1*R*,5*S*)-3-(4-bromo-7-(((2*R*,7a*S*)-2-fluorotetrahydro-1*H*-pyrrolizin-7a(5*H*)-yl)methoxy)-2-methyl-2*H*-pyrazolo[4,3-*f*]quinazolin-9-yl)-3,8-diazabicyclo[3.2.1]octane-8-carboxylate (53.5 mg, 84.8 µmol) obtained in Step 2, 4-(4,4,5,5-Tetramethyl-1,3,2-dioxaborolan-2-yl)naphthalen-2-ol (32 mg, 102 µmol), cataCXium^{®} A Pd G3 (9.2 mg, 12.7 µmol) and Cs₂CO₃ (54 mg, 255 µmol) were dissolved in toluene (1.5 mL) and water (0.15 mL), and then degassed and purged with nitrogen three times. Then, the reaction mixture was stirred at 80 °C for 1 h under nitrogen. After the starting material was completely consumed, the reaction mixture was concentrated under reduced pressure, the residue was diluted with water (10 mL), and the compound was extracted with ethyl acetate (5 mL*2). The organic layer was washed with saturated NaCl aqueous solution (5 mL*2), dried over anhydrous Na₂SO₄, and concentrated under reduced pressure. The residue was purified by silica gel chromatography (5% MeOH/DCM). *tert*-Butyl (1*R*,5*S*)-3-(7-(((2*R*,7a*S*)-2-fluorotetrahydro-1*H*-pyrrolizin-7a(5*H*)-yl)methoxy)-4-(3-(methoxymethoxy)naphthalen-1-yl)-2-methyl-2*H*-pyrazolo[4,3-*f*]quinazolin-9-yl)-3,8-diazabicyclo[3.2.1]octane-8-carboxylate (40 mg, 84.8 µmol, 64% yield) was obtained. LCMS (ES-API, m/z): [M+H]⁺= 738.9.

Step 4: *tert*-butyl (1*R*,5*S*)-3-(7-(((2*R*,7a*S*)-2-fluorotetrahydro-1*H*-pyrrolizin-7a(5*H*)-yl)methoxy)-4-(3-(methoxymethoxy)naphthalen-1-yl)-2-methyl-2*H*-pyrazolo[4,3-*f*]quinazolin-9-yl)-3,8-diazabicyclo[3.2.1]octane-8-carboxylate (40 mg, 54.2 µmol) obtained in Step 3 was dissolved in DCM (1 mL), and HCI in dioxane (4 N, 0.3 mL) was added. The reaction mixture was stirred at room temperature for 1 h. After the starting material was completely consumed, the reaction mixture was concentrated under reduced pressure to obtain the residue. The residue was purified by Prep-HPLC (Column: Waters Xbridge C18 150*50mm* 10um; Mobile phase: [water(0.1% TFA)-ACN]; B%: 40%). 4-(9-((1*R*,5*S*)-3,8-Diazabicyclo[3.2.1]octan-3-yl)-7-(((2*R*,7a*S*)-2-fluorotetrahydro-1*H*-pyrrolizin-7a(5*H*)-yl)methoxy)-2-methyl-2*H*-pyrazolo[4,3-*f*]quinazolin-4-yl)naphthalen-2-ol (25 mg, 54.2 µmol, 78% yield) was obtained as a yellow solid.

LCMS (ES-API, m/z): [M+H]⁺= 594.7; ¹H NMR (400 MHz, DMSO-*d₆*, ppm): δ 7.84 (d, *J* = 8.3 Hz, 1H), 7.50 - 7.43 (m, 2H), 7.40 (d, *J* = 8.5 Hz, 1H), 7.32 (d, *J =* 2.4 Hz, 1H), 7.24 - 7.17 (m, 2H), 6.88 (s, 1H), 5.66 (s, 0.5H), 5.53 (s, 0.5H), 4.61 (s, 2H), 4.37 - 4.23 (m, 3H), 4.15 (s, 2H), 3.95 - 3.88 (m, 4H), 3.37 - 3.27 (m, 2H), 2.43 (s, 3H), 2.25 - 2.13 (m, 4H), 2.11 - 1.86 (m, 6H).

### EXAMPLE 69: Synthesis of 4-(9-((1R,5S)-3,8-diazabicyclo[3.2.1]octan-3-yl)-7-(((2R,7aS)-2-fluorotetrahydro-1H-pyrrolizin-7a(5H)-yl)methoxy)-2-methyl-2H-pyrazolo[4,3-f]quinazolin-4-yl)-5-ethynyl-6-fluoronaphthalen-2-ol.

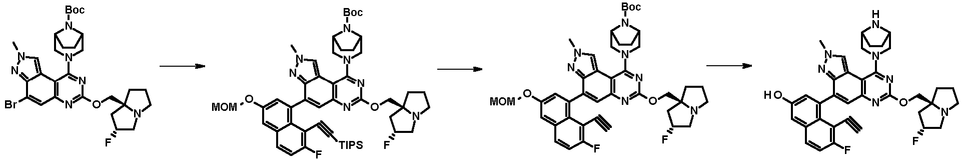

Step 1: *tert*-Butyl (1*R*,5*S*)-3-(4-bromo-7-(((2*R*,7a*S*)-2-fluorotetrahydro-1*H*-pyrrolizin-7a(5*H*)-yl)methoxy)-2-methyl-2*H*-pyrazolo[4,3-*f*]quinazolin-9-yl)-3,8-diazabicyclo[3.2.1]octane-8-carboxylate (107 mg, 170 µmol), (2-Fluoro-6-(methoxymethoxy)-8-(4,4,5,5-tetramethyl-1,3,2-dioxaborolan-2-yl)naphthalen-1-yl)ethynyl)triisopropylsilane (87 mg, 170 µmol), cataCXium A Pd G3 (18.5 mg, 25.5 µmol) and Cs₂CO₃ (108 mg, 509 µmol) were added to toluene (1 mL) and water (0.1 mL), and degassing and nitrogen purging were repeated three times. Then, the reaction mixture was stirred at 80 °C for 3 h under nitrogen. After the starting material was completely consumed, the reaction mixture was concentrated under reduced pressure, and the residue was diluted with purified water (10 mL), and the compound was extracted with ethyl acetate (5 mL*2). The organic layer was washed with saturated NaCl aqueous solution (5 mL*2), dried over anhydrous Na₂SO₄, and concentrated under reduced pressure. The residue was purified by silica gel chromatography (5% MeOH/DCM). *tert-*Butyl (1*R*,5*S*)-3-(4-(7-fluoro-3-(methoxymethoxy)-8-((triisopropylsilyl)ethynyl)naphthalen-1-yl)-7-(((2*R*,7a*S*)-2-fluorotetrahydro-1*H*-pyrrolizin-7a(5*H*)-yl)methoxy)-2-methyl-2*H*-pyrazolo[4,3-*f*]quinazolin-9-yl)-3,8-diazabicyclo[3.2.1]octane-8-carboxylate (60 mg, 64.1 µmol, 38% yield) was obtained. LCMS (ES-API, m/z): [M+H]⁺= 937.2.

Step 2: *tert*-butyl (1*R*,5*S*)-3-(4-(7-fluoro-3-(methoxymethoxy)-8-((triisopropylsilyl)ethynyl)naphthalen-1-yl)-7-(((2*R*,7a*S*)-2-fluorotetrahydro-1*H*-pyrrolizin-7a(5*H*)-yl)methoxy)-2-methyl-2*H*-pyrazolo[4,3-*f*]quinazolin-9-yl)-3,8-diazabicyclo[3.2.1]octane-8-carboxylate (60 mg, 64.1 µmol) obtained in Step 1 was dissolved in THF (0.5 mL), and TBAF (20.1 mg, 76.9 µmol) was added dropwise at 0°C. After the starting material was completely consumed, the reaction mixture was concentrated under reduced pressure, the residue was diluted with purified water (10 mL), and the compound was extracted with ethyl acetate (5 mL*2). The organic layer was washed with saturated NaCl aqueous solution (5 mL*2), dried over anhydrous Na₂SO₄, and concentrated under reduced pressure. The residue was purified by silica gel chromatography (MC: MeOH = 20:1) to obtain *tert*-butyl (1*R*,5*S*)-3-(4-(8-ethynyl-7-fluoro-3-(methoxymethoxy)naphthalen-1-yl)-7-(((2*R*,7a*S*)-2-fluorotetrahydro-1*H*-pyrrolizin-7a(5*H*)-yl)methoxy)-2-methyl-2*H*-pyrazolo[4,3-*f*]quinazolin-9-yl)-3,8-diazabicyclo[3.2.1]octane-8-carboxylate (50 mg, 64.1 µmol, 100% yield). LCMS (ES-API, m/z): [M+H]⁺= 780.9.

Step 3: *tert*-butyl (1*R*,5*S*)-3-(4-(8-ethynyl-7-fluoro-3-(methoxymethoxy)naphthalen-1-yl)-7-(((2*R*,7a*S*)-2-fluorotetrahydro-1*H*-pyrrolizin-7a(5*H*)-yl)methoxy)-2-methyl-2*H-*pyrazolo[4,3-*f*]quinazolin-9-yl)-3,8-diazabicyclo[3.2.1]octane-8-carboxylate (50 mg, 53.4 µmol) obtained in Step 2 was dissolved in DCM (0.5 mL), and 4 N HCI in dioxane (0.5 mL) was added. The reaction mixture was stirred at room temperature for 1 h. After the starting material was completely consumed, the reaction mixture was concentrated under reduced pressure to obtain the residue. The residue was purified by Prep-HPLC (Column: Waters Xbridge C18 150*50mm* 10um; Mobile phase: [water (0.1% TFA)-acetonitrile]; B%: 25%). 4-(9-((1*R*,5*S*)-3,8-Diazabicyclo[3.2.1]octan-3-yl)-7-(((2*R*,7a*S*)-2-fluorotetrahydro-1*H*-pyrrolizin-7a(5*H*)-yl)methoxy)-2-methyl-2*H*-pyrazolo[4,3-*f*]quinazolin-4-yl)-5-ethynyl-6-fluoronaphthalen-2-ol (25 mg, 39.3 µmol, 74% yield) was obtained as a yellow solid.

LCMS (ES-API, m/z): [M+H]⁺= 636.7; ¹H NMR (400 MHz, DMSO-*d*₆, ppm): δ 8.33 (s, 1H), 7.98 (dd, *J* = 9.2, 6.0 Hz, 1H), 7.45 (t, *J =* 9.0 Hz, 1H), 7.38 (d, *J* = 2.6 Hz, 1H), 7.32 (s, 1H), 7.13 (d, *J* = 2.6 Hz, 1H), 5.67 (s, 0.5H), 5.54 (s, 0.5H), 4.64 - 4.54 (m, 2H), 4.39 (s, 1H), 4.20 - 4.13 (m, 2H), 4.10 (s, 3H), 3.94 - 3.81 (m, 3H), 3.69 - 3.60 (m, 3H), 3.44 (d, *J* = 4.2 Hz, 1H), 3.37 - 3.27 (m, 2H), 2.36 - 2.32 (m, 2H), 2.24 - 2.14 (m, 3H), 2.10 - 2.01 (m, 2H), 1.98 - 1.89 (m, 3H).

### EXAMPLE 70: Synthesis of 4-(9-((1R,5S)-3,8-diazabicyclo[3.2.1]octan-3-yl)-7-(((2R,7aS)-2-fluorotetrahydro-1H-pyrrolizin-7a(5H)-yl)methoxy)-2-methyl-2H-pyrazolo[4,3-f]quinazolin-4-yl)-5,6-difluoronaphthalen-2-ol.

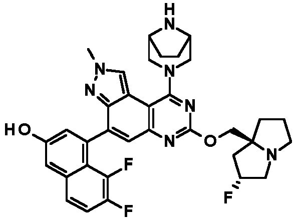

The compound was synthesized in the same manner as in EXAMPLE 68 using 2-(7,8-difluoro-3-(methoxymethoxy)naphthalen-1-yl)-4,4,5,5-tetramethyl-1,3,2-dioxaborolane. The residue was purified by Prep-HPLC to obtain 4-(9-((1*R*,5*S*)-3,8-diazabicyclo[3.2.1]octan-3-yl)-7-(((2*R*,7a*S*)-2-fluorotetrahydro-1*H*-pyrrolizin-7a(5*H*)-yl)methoxy)-2-methyl-2H-pyrazolo[4,3-*f*]quinazolin-4-yl)-5,6-difluoronaphthalen-2-ol (16 mg, 25.8 µmol, 100% yield) as a yellow solid.

LCMS (ES-API, m/z): [M+H]⁺= 630.7; ¹H NMR (400 MHz, DMSO-*d₆*, ppm): δ 8.37 (s, 1H), 7.76 - 7.70 (m, 1H), 7.55 (q, *J* = 9.0 Hz, 1H), 7.40 (s, 1H), 7.37 (s, 1H), 7.13 (d, *J* = 2.3 Hz, 1H), 5.67 (s, 0.5H), 5.54 (s, 0.5H), 4.60 (s, 2H), 4.33 - 4.25 (m, 1H), 4.16 (s, 2H), 4.11 (s, 3H), 3.94 - 3.85 (m, 3H), 3.81 - 3.76 (m, 3H), 3.37 - 3.28 (m, 2H), 2.26 - 2.14 (m, 3H), 2.13 - 2.01 (m, 3H), 2.01 - 1.88 (m, 4H).

### EXAMPLE 71: Synthesis of 4-(9-((1R,5S)-3,8-diazabicyclo[3.2.1]octan-3-yl)-7-(((2R,7aS)-2-fluorotetrahydro-1H-pyrrolizin-7a(5H)-yl)methoxy)-2-methyl-2H-pyrazolo[4,3-f]quinazolin-4-yl)-5-ethynyl-6-fluoronaphthalen-2-amine.

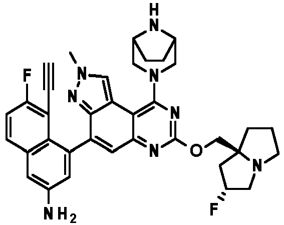

The compound was synthesized in the same manner as in EXAMPLE 69 using Intermediate 18. After Prep-HPLC purification, lyophilization was performed to obtain 4-(9-((1*R*,5*S*)-3,8-diazabicyclo[3.2.1]octan-3-yl)-7-(((2*R*,7a*S*)-2-fluorotetrahydro-1*H-*pyrrolizin-7a(5*H*)-yl)methoxy)-2-methyl-2*H*-pyrazolo[4,3-*f*]quinazolin-4-yl)-5-ethynyl-6-fluoronaphthalen-2-amine (60 mg, 94.5 µmol, 42% yield) as a yellow solid.

LCMS (ES-API, m/z): [M+H]⁺= 635.7; ¹H NMR (400 MHz, DMSO-*d*₆) δ 8.28 (s, 1H), 7.75 (dd, *J =* 9.2, 6.0 Hz, 1H), 7.30 (t, *J* = 9.0 Hz, 1H), 7.16 (s, 1H), 7.01 (d, *J* = 2.9 Hz, 2H), 5.57 (s, 2H), 5.36 (s, 0.5H), 5.22 (s, 0.5H), 4.29 - 3.87 (m, 5H), 3.78 - 3.35 (m, 7H), 3.31 (s, 3H), 3.22 - 2.96 (m, 3H), 2.84 (q, *J* = 8.4 Hz, 1H), 2.21 - 1.70 (m, 7H), 1.58 (s, 3H).

### EXAMPLE 72: Synthesis of 4-(9-((1R,5S)-3,8-diazabicyclo[3.2.1]octan-3-yl)-7-(3-(dimethylamino)azetidin-1-yl)-5-fluoro-2-methyl-2H-pyrazolo[4,3-f]quinazolin-4-yl)-5-ethynyl-6-fluoronaphthalen-2-ol.

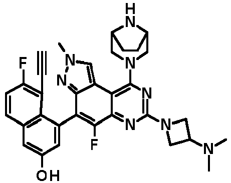

The compound was synthesized in the same manner as in EXAMPLE 68 using 4-Bromo-7,9-dichloro-5-fluoro-2-methyl-2*H*-pyrazolo[4,3-*f*]quinazoline and *N*,*N*-dimethylazetidin-3-amine. Prep-HPLC purification and lyophilization were performed to obtain 4-(9-((1*R*,5*S*)-3,8-diazabicyclo[3.2.1]octan-3-yl)-7-(3-(dimethylamino)azetidin-1-yl)-5-fluoro-2-methyl-2*H*-pyrazolo[4,3-*f*]quinazolin-4-yl)-5-ethynyl-6-fluoronaphthalen-2-ol (50.55 mg, 83.67 µmol, 43% yield) as a yellow solid.

LCMS (ES-API, m/z): [M+H]⁺= 595.3; ¹H NMR (400 MHz, DMSO-*d*₆, ppm) δ 11.30 - 10.30 (m, 2H), 9.71 - 9.51 (m, 2H), 9.41 - 9.23 (m, 1H), 8.20 - 8.10 (m, 1H), 8.08 - 8.03 (m, 1H), 7.74 - 7.66 (m, 1H), 7.58 - 7.53 (m, 1H), 6.18 - 6.06 (m, 1H), 5.72 - 5.61 (m, 1H), 4.98 - 4.83 (m, 1H), 4.41 - 4.36 (m, 4H), 4.29 - 4.18 (m, 3H), 4.03 - 3.93 (m, 1H), 2.86 (s, 6H), 2.38 - 2.23 (m, 1H), 2.05 - 1.74 (m, 3H), 1.52 - 1.36 (m, 1H), 1.30 - 1.18 (m, 1H).

### EXAMPLE 73: Synthesis of 4-(9-((1R,5S)-3,8-diazabicyclo[3.2.1]octan-3-yl)-7-((1-((1,1-difluoro-6-azaspiro[2.5]octan-6-yl)methyl)cyclopropyl)methoxy)-2-methyl-2H-pyrazolo[4,3-f]quinazolin-4-yl)-5-ethynyl-6-fluoronaphthalen-2-ol.

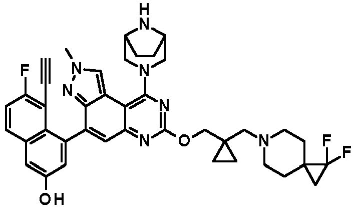

The compound was synthesized in the same manner as in EXAMPLE 69 using Intermediate 8. Prep-HPLC purification and lyophilization were performed to obtain 4-(9-((1*R*,5*S*)-3,8-diazabicyclo[3.2.1]octan-3-yl)-7-((1-((1,1-difluoro-6-azaspiro[2.5]octan-6-yl)methyl)cyclopropyl)methoxy)-2-methyl-2*H*-pyrazolo[4,3-*f*]quinazolin-4-yl)-5-ethynyl-6-fluoronaphthalen-2-ol (30 mg, 42.4 µmol, 45%).

LCMS (ES-API, m/z): [M+H]⁺= 707.8; ¹H NMR (400 MHz, DMSO-*d*₆, ppm) δ 10.05 (s, 1H), 8.28 (s, 1H), 7.96 (dd, *J* = 9.2, 6.0 Hz, 1H), 7.43 (t, *J* = 9.0 Hz, 1H), 7.35 (d, *J* = 2.6 Hz, 1H), 7.22 (s, 1H), 7.13 (d, *J* = 2.5 Hz, 1H), 4.27 (s, 2H), 4.06 (s, 2H), 3.85 - 3.55 (m, 3H), 3.42 (d, *J* = 14.8 Hz, 2H), 2.36 (q, *J* = 12.4 Hz, 7H), 1.99 (s, 1H), 1.83 - 1.28 (m, 7H), 1.29 - 1.04 (m, 3H), 0.64 (d, *J* = 4.9 Hz, 2H), 0.42 (s, 2H).

### EXAMPLE 74: Synthesis of 4-(9-((1R,5S)-3,8-diazabicyclo[3.2.1]octan-3-yl)-7-(((S,Z)-2-(fluoromethylene)tetrahydro-1H-pyrrolizin-7a(5H)-yl)methoxy)-2-methyl-2H-pyrazolo[4,3-f]quinazolin-4-yl)-5-ethynyl-6-fluoronaphthalen-2-amine.

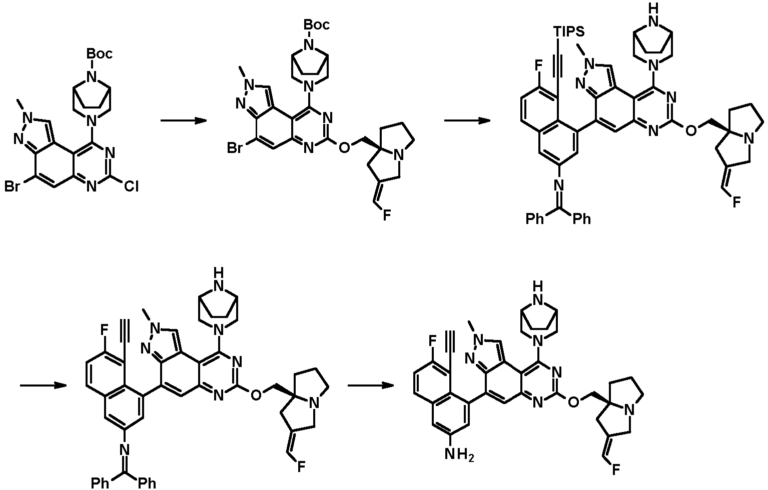

Step 1: (*S*,*Z*)-(2-(Fluoromethylene)tetrahydro-1*H*-pyrrolizin-7a(5*H*)-yl)methanol (405 mg, 2.36 mmol), and sodium *tert*-butoxide (454 mg, 4.73 mmol) were added to THF (3 mL) and DMF (9 mL), and stirred at room temperature for 30 min. After 30 min, *tert*-butyl (1*R*,5*S*)-3-(4-bromo-2,7-dimethyl-2*H*-pyrazolo[4,3-*f*]quinazolin-9-yl)-3,8-diazabicyclo[3.2.1]octane-8-carboxylate (400 mg, 778 µmol) was added to the reaction mixture at room temperature, and the mixture was stirred for 2 h. After the starting material was completely consumed, the reaction was quenched by slowly adding water at room temperature and the organic layer was extracted with ethyl acetate. After drying over anhydrous Na₂SO₄, concentration under reduced pressure was performed. The residue was purified by silica gel chromatography (5% MeOH/DCM) to obtain *tert-*butyl (1*R*,5*S*)-3-(4-bromo-7-(((*S*,*Z*)-2-(fluoromethylene)tetrahydro-1*H*-pyrrolizin-7a(5*H*)-yl)methoxy)-2-methyl-2*H*-pyrazolo[4,3-*f*]quinazolin-9-yl)-3,8-diazabicyclo[3.2.1]octane-8-carboxylate (470 mg, 731 µmol, 93% yield). LCMS (ES-API, m/z): [M+H]⁺= 643.6.

Step 2: *tert*-Butyl (1*R*,5*S*)-3-(4-bromo-7-(((*S*,*Z*)-2-(fluoromethylene)tetrahydro-1*H-*pyrrolizin-7a(5*H*)-yl)methoxy)-2-methyl-2*H*-pyrazolo[4,3-*f*]quinazolin-9-yl)-3,8-diazabicyclo[3.2.1]octane-8-carboxylate (200 mg, 311 µmol), N-(6-fluoro-4-(4,4,5,5-tetramethyl-1,3,2-dioxaborolan-2-yl)-5-((triisopropylsilyl)ethynyl)naphthalen-2-yl)-1,1-diphenylmethanimine (295 mg, 467 µmol), cataCXium A Pd G3 (33.9 mg, 46.6 µmol) and Cesium carbonate (304 mg, 934 µmol) were added to toluene (4 mL) and water (1 mL), and degassing and nitrogen purging were repeated three times. Then, the reaction mixture was stirred at 110 °C for 24 h under nitrogen. After the starting material was completely consumed, the Palladium catalyst was removed through a Celite filter. The residue was diluted with water, and the compound was extracted with ethyl acetate. The organic layer was dried over anhydrous Na₂SO₄ and concentrated under reduced pressure. The residue was purified by silica gel chromatography (5% MeOH/DCM) to obtain *tert-*butyl (1*R*,5*S*)-3-(4-(3-((diphenylmethylene)amino)-7-fluoro-8-((triisopropylsilyl)ethynyl)naphthalen-1-yl)-7-(((*S*,*Z*)-2-(fluoromethylene)tetrahydro-1*H-*pyrrolizin-7a(5*H*)-yl)methoxy)-2-methyl-2*H*-pyrazolo[4,3-*f*]quinazolin-9-yl)-3,8-diazabicyclo[3.2.1]octane-8-carboxylate (90 mg, 84.3 µmol, 27% yield). LCMS (ES-API, m/z): [M+H]⁺= 1068.

Step 3: *tert*-Butyl (1*R*,5*S*)-3-(4-(3-((diphenylmethylene)amino)-7-fluoro-8-((triisopropylsilyl)ethynyl)naphthalen-1-yl)-7-(((*S*,*Z*)-2-(fluoromethylene)tetrahydro-1*H-*pyrrolizin-7a(5*H*)-yl)methoxy)-2-methyl-2*H*-pyrazolo[4,3-*f*]quinazolin-9-yl)-3,8-diazabicyclo[3.2.1]octane-8-carboxylate (90 mg, 84.3 µmol) was dissolved in THF (2 mL), and tetrabutylammonium fluoride in tetrahydrofuran (1.0 M, 126 µL, 126 µmol) was added dropwise at 0°C. After completion of the reaction, the reaction mixture was diluted with water, extracted with ethyl acetate, and concentrated under reduced pressure. The residue was purified by silica gel chromatography (5% MeOH/DCM) to obtain *tert*-butyl (1*R*,5*S*)-3-(4-(3-((diphenylmethylene)amino)-8-ethynyl-7-fluoronaphthalen-1-yl)-7-(((*S*,*Z*)-2-(fluoromethylene)tetrahydro-1*H*-pyrrolizin-7a(5*H*)-yl)methoxy)-2-methyl-2*H-*pyrazolo[4,3-*f*]quinazolin-9-yl)-3,8-diazabicyclo[3.2.1]octane-8-carboxylate (40 mg, 43.9 µmol, 52% yield). LCMS (ES-API, m/z): [M+H]⁺= 912.1.

Step 4: *tert*-Butyl (1*R*,5*S*)-3-(4-(3-((diphenylmethylene)amino)-8-ethynyl-7-fluoronaphthalen-1-yl)-7-(((*S*,*Z*)-2-(fluoromethylene)tetrahydro-1*H*-pyrrolizin-7a(5*H*)-yl)methoxy)-2-methyl-2*H*-pyrazolo[4,3-*f*]quinazolin-9-yl)-3,8-diazabicyclo[3.2.1]octane-8-carboxylate (40 mg, 43.9 µmol) was dissolved in DCM (0.5 mL), and 4 N HCI in dioxane (1 mL) was added. The reaction mixture was stirred at room temperature for 10 min. After the starting material was completely consumed, the reaction mixture was concentrated under reduced pressure to obtain the residue. The residue was purified by silica gel chromatography (5% Methanol/DCM, 5% NH4OH) to obtain 4-(9-((1*R*,5*S*)-3,8-diazabicyclo[3.2.1]octan-3-yl)-7-(((*S*,*Z*)-2-(fluoromethylene)tetrahydro-1*H*-pyrrolizin-7a(5*H*)-yl)methoxy)-2-methyl-2*H*-pyrazolo[4,3-t]quinazolin-4-yl)-5-ethynyl-6-fluoronaphthalen-2-amine (18 mg, 27.8 µmol, 63% yield).

LCMS (ES-API, m/z): [M+H]⁺= 647.7; ¹H NMR (400 MHz, MeOD) δ 8.31 (s, 1H), 7.76 (dd, *J =* 9.2, 5.8 Hz, 1H), 7.42 (s, 1H), 7.30 - 7.09 (m, 3H), 6.92 (s, 0.5H), 6.71 (s, 0.5H), 4.68 - 4.37 (m, 3H), 4.33 - 4.08 (m, 6H), 3.90 (d, *J* = 13.8 Hz, 2H), 3.78 (d, *J* = 14.1 Hz, 1H), 3.57 (s, 1H), 3.11 (s, 1H), 2.96 (d, *J* = 15.7 Hz, 1H), 2.87 (s, 1H), 2.70 (d, *J* = 15.7 Hz, 1H), 2.42 - 1.93 (m, 8H).

### EXAMPLE 75: 4-(9-((1R,5S)-3,8-diazabicyclo[3.2.1]octan-3-yl)-7-(((S,Z)-2-(fluoromethylene)tetrahydro-1H-pyrrolizin-7a(5H)-yl)methoxy)-2-methyl-2H-pyrazolo[4,3-f]quinazolin-4-yl)-5-ethynyl-6-fluoronaphthalen-2-ol.

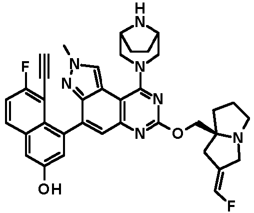

The compound was synthesized in the same manner as in EXAMPLE 69 using (*S*,*Z*)-(2-(fluoromethylene)tetrahydro-1*H*-pyrrolizin-7a(5*H*)-yl)methanol. After Prep-HPLC purification, lyophilization was performed to obtain 4-(9-((1*R*,5*S*)-3,8-diazabicyclo[3.2.1]octan-3-yl)-7-(((*S*,*Z*)-2-(fluoromethylene)tetrahydro-1*H*-pyrrolizin-7a(5*H*)-yl)methoxy)-2-methyl-2*H*-pyrazolo[4,3-*f*]quinazolin-4-yl)-5-ethynyl-6-fluoronaphthalen-2-ol (62 mg, 95.7 µmol, 84% yield) as a white solid.

LCMS (ES-API, m/z): [M+H]⁺= 648.7; ¹H NMR (400 MHz, MeOD, ppm) δ 8.31 (s, 1H), 7.87 (dd, *J* = 9.2, 5.7 Hz, 1H), 7.41 (s, 1H), 7.38 - 7.24 (m, 2H), 7.20 (d, *J* = 2.6 Hz, 1H), 6.85 (s, 0.5H), 6.64 (s, 0.5H), 4.54 - 4.36 (m, 2H), 4.08 (d, *J* = 53.9 Hz, 6H), 3.87 (d, *J =* 13.7 Hz, 1H), 3.73 (t, *J* = 14.1 Hz, 2H), 3.48 - 3.30 (m, 16H), 3.02 - 2.77 (m, 3H), 2.59 (d, *J =* 15.6 Hz, 1H), 2.33 - 2.17 (m, 2H), 2.18 - 1.89 (m, 5H).

### EXAMPLE 76: 4-(9-((1R,5S)-3,8-diazabicyclo[3.2.1]octan-3-yl)-7-((1-((1,1-difluoro-6-azaspiro[2.5]octan-6-yl)methyl)cyclopropyl)methoxy)-2-methyl-2H-pyrazolo[4,3-f]quinazolin-4-yl)-5-ethynyl-6-fluoronaphthalen-2-amine.

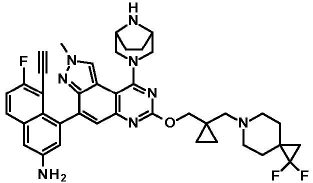

The compound was synthesized in the same manner as in EXAMPLE 74 using Intermediate 8. 4-(9-((1*R*,5*S*)-3,8-Diazabicyclo[3.2.1]octan-3-yl)-7-((1-((1,1-difluoro-6-azaspiro[2.5]octan-6-yl)methyl)cyclopropyl)methoxy)-2-methyl-2*H*-pyrazolo[4,3-*f*]quinazolin-4-yl)-5-ethynyl-6-fluoronaphthalen-2-amine (30 mg, 42.4 µmol, 41% yield) was obtained as a yellow solid.

LCMS (ES-API, m/z): [M+H]⁺= 707.8; ¹H NMR (400 MHz, MeOD, ppm) δ 8.33 (s, 1H), 7.81 (dd, *J*= 9.2, 5.8 Hz, 1H), 7.44 (s, 1H), 7.34 - 7.21 (m, 1H), 7.17 (d, *J* = 2.4 Hz, 1H), 4.66 - 4.42 (m, 2H), 4.19 (d, *J*= 20.4 Hz, 3H), 3.89 (dd, *J* = 41.4, 14.1 Hz, 2H), 2.91 (s, 1H), 2.22 (d, *J* = 67.0 Hz, 4H), 1.83 (d, *J* = 55.1 Hz, 2H), 1.51 - 1.26 (m, 2H), 0.99 (dd, *J* = 44.0, 5.8 Hz, 3H).

### EXAMPLE 77: Synthesis of 4-(9-((1R,5S)-3,8-diazabicyclo[3.2.1]octan-3-yl)-7-(((2R,7aS)-2-fluorotetrahydro-1H-pyrrolizin-7a(5H)-yl)methoxy)-2-methyl-2H-pyrazolo[4,3-f]quinazolin-4-yl)-2-amino-7-fluorobenzo[b]thiophene-3-carbonitrile.

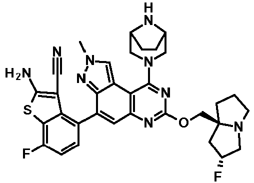

The compound was synthesized in the same manner as in EXAMPLE 62 using Intermediate 4. Lyophilization was performed to obtain 4-(9-((1*R*,5*S*)-3,8-diazabicyclo[3.2.1]octan-3-yl)-7-(((2*R*,7a*S*)-2-fluorotetrahydro-1*H*-pyrrolizin-7a(5*H*)-yl)methoxy)-2-methyl-2*H*-pyrazolo[4,3-*f*]quinazolin-4-yl)-2-amino-7-fluorobenzo[*b*]thiophene-3-carbonitrile (5.8 mg, 9.04 µmol, 11% yield) as a white solid. LCMS (ES-API, m/z): [M+H]⁺= 707.8; ¹H NMR (400 MHz, MeOD, ppm) δ 8.37 (s, 1H), 7.47 (s, 1H), 7.41 (dd, *J =* 8.3, 5.2 Hz, 1H), 7.08 (t, *J* = 8.9 Hz, 1H), 5.69 (s, 0.5H), 5.57 (s, 0.5H), 4.70 (d, *J* = 11.6 Hz, 3H), 4.38 - 3.74 (m, 9H), 3.50 (dq, *J* = 11.2, 5.9 Hz, 1H), 2.95 - 2.27 (m, 6H), 2.05 (d, *J* = 9.9 Hz, 5H), 1.86 (s, 2H), 1.57 (s, 2H), 1.46 - 1.25 (m, 3H).

### EXAMPLE 78: Synthesis of 9-((1R,5S)-3,8-diazabicyclo[3.2.1]octan-3-yl)-4-(8-ethynyl-7-fluoronaphthalen-1-yl)-7-(((S,Z)-2-(fluoromethylene)tetrahydro-1H-pyrrolizin-7a(5H)-yl)methoxy)-2-methyl-2H-pyrazolo[4,3-f]quinazoline.

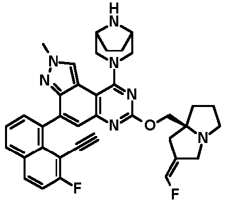

The compound was synthesized in the same manner as in EXAMPLE 27 using Intermediate 4. 9-((1*R*,5*S*)-3,8-Diazabicyclo[3.2.1]octan-3-yl)-4-(8-ethynyl-7-fluoronaphthalen-1-yl)-7-(((*S*,*Z*)-2-(fluoromethylene)tetrahydro-1*H*-pyrrolizin-7a(5*H*)-yl)methoxy)-2-methyl-2*H*-pyrazolo[4,3-*f*]quinazoline (23 mg, 53% yield) was obtained as a yellow solid.

LCMS (ES-API, m/z): [M+H]⁺= 632.3; ¹H NMR (400 MHz, MeOD, ppm): δ 8.27 (S, 1H), 8.13 - 8.09 (m, 1H), 7.67 - 7.61 (m, 1H), 7.44 - 7.39 (m, 1H), 7.38 (s, 1H), 6.79 (s, 0.5H), 6.58 (s, 0.5H), 4.34 - 4.12 (m, 3H), 4.09 (s, 3H), 4.06 (m, 1H), 3.92 - 3.88 (d, 1H), 3.69 - 3.50 (m, 5H), 3.21 - 3.19 (m, 1H), 2.96 (s, 1H), 2.81 - 2.74 (m, 2H), 2.51 - 2.47 (d, 1H), 2.20 - 2.17 (m, 1H), 2.06 - 1.84 (m, 8H).

### EXAMPLE 79: Synthesis of 4-(9-((1R,5S)-3,8-diazabicyclo[3.2.1]octan-3-yl)-7-(((2S,7aR)-2-fluoro-6-methylenetetrahydro-1H-pyrrolizin-7a(5H)-yl)methoxy)-2-methyl-2H-pyrazolo[4,3-f]quinazolin-4-yl)-5-ethynyl-6-fluoronaphthalen-2-ol.

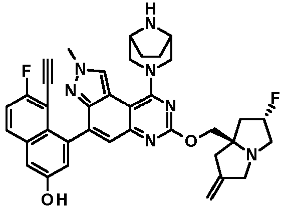

The compound was synthesized in the same manner as in EXAMPLE 32 using Intermediate 16. 4-(9-((1*R*,5*S)*-3,8-Diazabicyclo[3.2.1]octan-3-yl)-7-(((2*S*,7a*R*)-2-fluoro-6-methylenetetrahydro-1*H*-pyrrolizin-7a(5*H*)-yl)methoxy)-2-methyl-2*H*-pyrazolo[4,3-*f*]quinazolin-4-yl)-5-ethynyl-6-fluoronaphthalen-2-ol (68 mg, 83% yield) was obtained as a yellow solid.

LCMS (ES-API, m/z): [M+H]⁺= 650.7; ¹H NMR (400 MHz, DMSO-*d*₆, ppm): δ 8.27 (s, 1H), 8.13 - 8.09 (m, 1H), 7.67 - 7.61 (m, 1H), 7.44 - 7.38 (m, 1H), 6.79 (s, 0.5H), 6.58 (s, 0.5H), 4.34 - 4.29 (m, 3H), 4.12 (s, 3H), 4.09 (m, 2H), 3.92 - 3.88 (d, 1H), 3.58 - 3.19 (m, 5H), 2.77 - 2.74 (m, 2H), 2.51 (d, 1H), 2.20 - 2.17 (m, 1H), 2.06 - 1.31 (m, 8H).

### EXAMPLE 80: Synthesis of 4-(9-((1R,5S)-3,8-diazabicyclo[3.2.1]octan-3-yl)-5-fluoro-7-(((2R,7aS)-2-fluorotetrahydro-1H-pyrrolizin-7a(5H)-yl)methoxy)-2-methyl-2H-pyrazolo[4,3-f]quinazolin-4-yl)-5-ethynyl-6-fluoronaphthalen-2-ol.

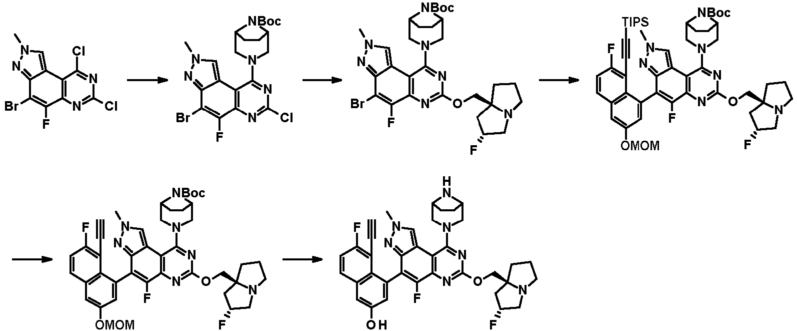

Step 1: 4-Bromo-7,9-dichloro-5-fluoro-2-methyl-2*H*-pyrazolo[4,3-*f*]quinazoline (2 g, 5.71 mmol) was dissolved in ACN (30 mL), and DIPEA (1.11 g, 8.57 mmol, 1.49 mL) and *tert-*butyl (1*R*,5*S*)-3,8-diazabicyclo[3.2.1]octane-8-carboxylate (230 mg, 1.46 g, 6.86 mmol) were added. The reaction mixture was stirred at room temperature for 1 h. The reaction mixture was diluted with water (40 mL) and extracted with ethyl acetate (30 mL*4). The organic matter was washed with saturated NaCl aqueous solution (50 mL*2), dried over NaSO₄, filtered, and concentrated to obtain the residue. The product was stirred in PE: EtOAc = 2:1 at room temperature for 30 min to obtain *tert*-butyl (1*R*,5*S*)-3-(4-bromo-7-chloro-5-fluoro-2-methyl-2*H*-pyrazolo[4,3-*f*]quinazolin-9-yl)-3,8-diazabicyclo[3.2.1]octane-8-carboxylate (3.1 g, 5.13 mmol, 90% yield) as a pink solid. LCMS (ES-API, m/z): [M+H]⁺= 527.1; ¹H NMR (400 MHz, DMSO-*d*₆, ppm): *δ* 8.69 (s, 1H), 4.24 (s, 3H), 4.21 (br d, *J* = 4.0 Hz, 2H), 4.13 - 4.02 (m, 2H), 3.59 (br d, *J* = 11.6 Hz, 2H), 1.69 - 1.54 (m, 4H), 1.46 (s, 9H).

Step 2: *tert*-Butyl (1*R*,5*S*)-3-(4-bromo-7-chloro-5-fluoro-2-methyl-2*H*-pyrazolo[4,3-*f*]quinazolin-9-yl)-3,8-diazabicyclo[3.2.1]octane-8-carboxylate (1.5 g, 2.48 mmol) was dissolved in DMSO (30 mL), and KF (865.15 mg, 14.89 mmol), ((2*R*,7a*S*)-2-fluorotetrahydro-1*H*-pyrrolizin-7a(5*H*)-yl)methanol (1.19 g, 7.45 mmol) and 18-crown-6 (2.30 g, 8.69 mmol) were added. The reaction mixture was reacted at 120 °C for 16 h. After confirming the disappearance of the starting material, the reaction mixture was diluted with water (100 mL) and extracted with ethyl acetate (80 mL*4). The organic matter was washed with saturated NaCl aqueous solution (50 mL*2), dried over Na₂SO₄, filtered, and concentrated to obtain a residue. The reaction mixture was purified by silica gel chromatography (Eluent of 0-50% Ethyl acetate/Petroleum ether gradient) to obtain tert-butyl (1*R*,5*S*)-3-(4-bromo-5-fluoro-7-(((2*R*,7a*S*)-2-fluorotetrahydro-1*H*-pyrrolizin-7a(5*H*)-yl)methoxy)-2-methyl-2*H*-pyrazolo[4,3-*f*]quinazolin-9-yl)-3,8-diazabicyclo[3.2.1]octane-8-carboxylate (545 mg, 689.09 µmol, 28% yield) as a yellow solid.

LCMS (ES-API, m/z): [M+H]⁺= 650.3; ¹H NMR (400 MHz, DMSO-*d₆*, ppm): δ 8.54 (s, 1H), 5.40 - 5.11 (m, 1H), 4.20 (s, 5H), 4.15 - 4.10 (m, 1H), 4.06 - 4.00 (m, 2H), 3.58 - 3.50 (m, 2H), 3.12 - 3.06 (m, 2H), 3.01 (s, 1H), 2.87 - 2.78 (m, 1H), 2.16 - 2.10 (m, 1H), 2.08 - 2.04 (m, 1H), 2.03 - 1.99 (m, 1H), 1.90 - 1.71 (m, 4H), 1.65 (br s, 4H), 1.45 (s, 9H).

Step 3: tert-butyl (1*R*,5*S*)-3-(4-bromo-5-fluoro-7-(((2*R*,7a*S*)-2-fluorotetrahydro-1*H-*pyrrolizin-7a(5*H*)-yl)methoxy)-2-methyl-2*H*-pyrazolo[4,3-*f*]quinazolin-9-yl)-3,8-diazabicyclo[3.2.1]octane-8-carboxylate (300 mg, 379.31 µmol), ((2-fluoro-6-(methoxymethoxy)-8-(4,4,5,5-tetramethyl-1,3,2-dioxaborolan-2-yl)naphthalen-1-yl)ethynyl)triisopropylsilane (233.29 mg, 455.18 µmol), Pd(dtbpf)Cl₂ (24.72 mg, 37.93 µmol), and K₃PO₄ (241.55 mg, 1.14 mmol) were dissolved in 1,4-dioxane (3 mL) and water (0.75 mL), and then degassed and nitrogen purged three times. The reaction mixture was reacted at 80 °C for 3 hours. After the reaction was completed, water (10 mL) was added to the reaction mixture, and extracted with ethyl acetate (10 mL*3). Purification by silica gel chromatography was performed to obtain *tert*-butyl (1*R*,5*S*)-3-(5-fluoro-4-(7-fluoro-3-(methoxymethoxy)-8-((triisopropylsilyl)ethynyl)naphthalen-1-yl)-7-(((2*R*,7a*S*)-2-fluorotetrahydro-1*H*-pyrrolizin-7a(5*H*)-yl)methoxy)-2-methyl-2*H*-pyrazolo[4,3-*f*]quinazolin-9-yl)-3,8-diazabicyclo[3.2.1]octane-8-carboxylate (229 mg, 225.59 µmol, 59% yield) as a yellow solid.

LCMS (ES-API, m/z): [M+H]⁺= 954.5; ¹H NMR (400 MHz, DMSO-*d*₆, ppm): δ 8.42 (br s, 1H), 8.14 - 8.02 (m, 1H), 7.72 (d, *J =* 2.4 Hz, 1H), 7.54 (br t, *J =* 8.8 Hz, 1H), 7.28 (br d, *J* = 1.6 Hz, 1H), 5.35 (s, 2H), 5.22 (br s, 1H), 4.66 - 4.41 (m, 1H), 4.34 - 4.28 (m, 1H), 4.21 (br d, *J =* 4.8 Hz, 1H), 4.15 - 4.08 (m, 1H), 4.03 - 3.92 (m, 2H), 3.83 - 3.75 (m, 1H), 3.58 - 3.48 (m, 3H), 3.43 (s, 3H), 3.10 (br d, *J* = 6.8 Hz, 2H), 3.02 (br s, 1H), 2.88 - 2.80 (m, 1H), 2.28 - 2.15 (m, 2H), 2.10 - 2.00 (m, 2H), 1.86 - 1.73 (m, 4H), 1.47 (s, 9H), 1.28 - 1.20 (m, 2H), 0.88 - 0.80 (m, 4H), 0.69 - 0.60 (m, 18H).

Step 4: *tert*-Butyl (1*R*,5*S*)-3-(5-fluoro-4-(7-fluoro-3-(methoxymethoxy)-8-((triisopropylsilyl)ethynyl)naphthalen-1-yl)-7-(((2*R*,7a*S*)-2-fluorotetrahydro-1*H*-pyrrolizin-7a(5*H*)-yl)methoxy)-2-methyl-2*H*-pyrazolo[4,3-*f*]quinazolin-9-yl)-3,8-diazabicyclo[3.2.1]octane-8-carboxylate (229 mg, 225.59 µmol) was dissolved in DMF (4 mL), CsF (514.02 mg, 3.38 mmol) was added, and the mixture was reacted at room temperature for 40 minutes. After the reaction was completed, extraction was performed using ethyl acetate (10 mL*4) and saturated NaCl aqueous solution. Seperation was performed by Prep-TLC to obtain *tert*-butyl (1*R*,5*S*)-3-(4-(8-ethynyl-7-fluoro-3-(methoxymethoxy)naphthalen-1-yl)-5-fluoro-7-(((2*R*,7a*S*)-2-fluorotetrahydro-1*H-*pyrrolizin-7a(5*H*)-yl)methoxy)-2-methyl-2*H*-pyrazolo[4,3-*f*]quinazolin-9-yl)-3,8-diazabicyclo[3.2.1]octane-8-carboxylate (174 mg, 209.36 µmol, 93% yield) as an orange solid.

LCMS (ES-API, m/z): [M+H]⁺= 798.4; ¹H NMR (400 MHz, DMSO-*d*₆, ppm): δ 8.41 (s, 1H), 8.10 (dd, *J* = 6.0, 9.2 Hz, 1H), 7.74 (d, *J =* 2.8 Hz, 1H), 7.53 (t, *J* = 9.2 Hz, 1H), 7.35 (d, *J* = 2.0 Hz, 1H), 5.22 (br s, 3H), 4.27 (br s, 2H), 4.13 (br dd, *J* = 6.0, 10.4 Hz, 2H), 4.05 (s, 4H), 3.98 - 3.90 (m, 1H), 3.64 - 3.50 (m, 3H), 3.45 (s, 3H), 3.14 - 3.06 (m, 2H), 3.02 (br s, 1H), 2.88 - 2.80 (m, 1H), 2.15 (br dd, *J =* 4.4, 6.8 Hz, 1H), 2.09 - 2.00 (m, 2H), 1.89 - 1.67 (m, 7H), 1.47 (s, 9H).

Step 5: To *tert*-Butyl (1*R*,5*S*)-3-(4-(8-ethynyl-7-fluoro-3-(methoxymethoxy)naphthalen-1-yl)-5-fluoro-7-(((2*R*,7a*S*)-2-fluorotetrahydro-1*H*-pyrrolizin-7a(5*H*)-yl)methoxy)-2-methyl-2*H*-pyrazolo[4,3-*f*]quinazolin-9-yl)-3,8-diazabicyclo[3.2.1]octane-8-carboxylate (174 mg, 209.36 µmol), HCI in 1.4-dioxane (2 M, 2.5 mL) was added and reacted at 0 °C for 1 h. After completion of the reaction, the product was purified by prep-HPLC to obtain 4-(9-((1*R*,5*S*)-3,8-diazabicyclo[3.2.1]octan-3-yl)-5-fluoro-7-(((2*R*,7a*S*)-2-fluorotetrahydro-1*H-*pyrrolizin-7a(5*H*)-yl)methoxy)-2-methyl-2*H*-pyrazolo[4,3-*f*]quinazolin-4-yl)-5-ethynyl-6-fluoronaphthalen-2-ol (47.05 mg, 71.26 µmol, 34% yield) as a yellow solid.

LCMS (ES-API, m/z): [M+H]⁺= 654.3; ¹H NMR (400 MHz, DMSO-*d*₆, ppm): *δ* 10.28 - 9.91 (m, 1H), 8.32 (s, 1H), 7.97 (dd, *J* = 6.0, 9.2 Hz, 1H), 7.44 (t, *J* = 9.2 Hz, 1H), 7.38 (d, *J =* 2.4 Hz, 1H), 7.16 (d, *J* = 2.4 Hz, 1H), 5.39 - 5.18 (m, 1H), 4.11 (dd, *J* = 5.6, 10.4 Hz, 1H), 4.05 (s, 3H), 4.04 - 3.98 (m, 2H), 3.89 - 3.80 (m, 1H), 3.55 (br d, *J* = 13.2 Hz, 1H), 3.45 (br d, *J =* 8.0 Hz, 5H), 3.15 - 3.07 (m, 2H), 3.05 - 3.00 (m, 1H), 2.87 - 2.79 (m, 1H), 2.18 - 2.11 (m, 1H), 2.09 - 1.98 (m, 2H), 1.88 - 1.71 (m, 4H), 1.69 - 1.52 (m, 3H).

### EXAMPLE 81: Synthesis of 4-(9-((1R,5S)-3,8-diazabicyclo[3.2.1]octan-3-yl)-5-fluoro-7-(((2R,7aS)-2-fluorotetrahydro-1H-pyrrolizin-7a(5H)-yl)methoxy)-2-methyl-2H-pyrazolo[4,3-f]quinazolin-4-yl)-5-ethynyl-6-fluoronaphthalen-2-amine.

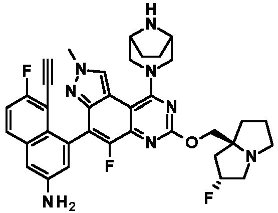

The compound was synthesized in the same manner as in EXAMPLE 57 using Intermediate 4. The obtained compound was purified by prep-HPLC and then lyophilized to obtain 4-(9-((1*R*,5*S*)-3,8-diazabicyclo[3.2.1]octan-3-yl)-5-fluoro-7-(((2*R*,7a*S*)-2-fluorotetrahydro-1*H*-pyrrolizin-7a(5*H*)-yl)methoxy)-2-methyl-2*H*-pyrazolo[4,3-*f*]quinazolin-4-yl)-5-ethynyl-6-fluoronaphthalen-2-amine (75.99 mg, 115.06 µmol, 49% yield) as a yellow solid.

LCMS (ES-API, m/z): [M+H]⁺= 653.4; ¹H NMR (400 MHz, DMSO-*d*₆, ppm): δ 8.35 - 8.25 (m, 1H), 7.81 - 7.72 (m, 1H), 7.37 - 7.25 (m, 1H), 7.09 - 6.96 (m, 2H), 5.61 - 5.50 (m, 2H), 5.41 - 5.18 (m, 1H), 4.15 - 3.95 (m, 6H), 3.87 - 3.77 (m, 1H), 3.59 - 3.50 (m, 1H), 3.49 - 3.41 (m, 3H), 3.36 - 3.34 (m, 2H), 3.16 - 3.06 (m, 2H), 3.05 - 2.99 (m, 1H), 2.88 - 2.78 (m, 1H), 2.20 - 2.11 (m, 1H), 2.10 - 1.96 (m, 2H), 1.91 - 1.70 (m, 4H), 1.70 - 1.52 (m, 3H).

### EXAMPLE 82: Synthesis of 9-((1R,5S)-3,8-diazabicyclo[3.2.1]octan-3-yl)-4-(8-ethynyl-7-fluoronaphthalen-1-yl)-5-fluoro-7-(((2R,7aS)-2-fluorotetrahydro-1H-pyrrolizin-7a(5H)-yl)methoxy)-2-methyl-2H-pyrazolo[4,3-f]quinazoline.

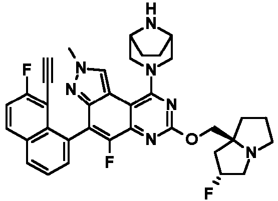

The compound was synthesized in the same manner as in EXAMPLE 80 using ((2-Fluoro-8-(4,4,5,5-tetramethyl-1,3,2-dioxaborolan-2-yl)naphthalen-1-yl)ethynyl)triisopropylsilane. The obtained compound was purified by prep-HPLC and then lyophilized to obtain 9-((1*R*,5*S*)-3,8-diazabicyclo[3.2.1]octan-3-yl)-4-(8-ethynyl-7-fluoronaphthalen-1-yl)-5-fluoro-7-(((2*R*,7a*S*)-2-fluorotetrahydro-1*H*-pyrrolizin-7a(5*H*)-yl)methoxy)-2-methyl-2*H-*pyrazolo[4,3-*f*]quinazoline (32 mg, 49.38 µmol, 24% yield) as a pink solid.

LCMS (ES-API, m/z): [M+H]⁺= 638.3; ¹H NMR (400 MHz, DMSO-*d*₆, ppm): δ 8.32 (s, 1H), 8.26 - 8.13 (m, 2H), 7.74 - 7.53 (m, 3H), 5.39 - 5.18 (m, 1H), 4.12 (dd, *J* = 6.0, 10.4 Hz, 1H), 4.07 - 3.98 (m, 5H), 3.87 (br d, *J* = 6.0 Hz, 1H), 3.59 - 3.43 (m, 5H), 3.21 - 2.96 (m, 4H), 2.89 - 2.78 (m, 1H), 2.22 - 2.12 (m, 1H), 1.99 (br s, 2H), 1.92 - 1.66 (m, 5H), 1.57 (br s, 2H).

### EXAMPLE 83: Synthesis of 4-(9-((1R,5S)-3,8-diazabicyclo[3.2.1]octan-3-yl)-7-(((2R,7aS)-2-fluorotetrahydro-1H-pyrrolizin-7a(5H)-yl)methoxy)-2-methyl-2H-pyrazolo[4,3-f]quinazolin-4-yl)-5-ethyl-6-fluoronaphthalen-2-ol.

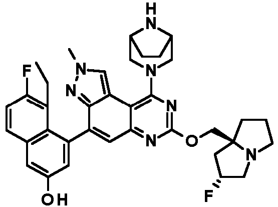

The compound was synthesized in the same manner as in EXAMPLE 68 using 2-(8-Ethyl-7-fluoro-3-(methoxymethoxy)naphthalen-1-yl)-4,4,5,5-tetramethyl-1,3,2-dioxaborolane. 4-(9-((1*R*,5*S*)-3,8-Diazabicyclo[3.2.1]octan-3-yl)-7-(((2*R*,7a*S*)-2-fluorotetrahydro-1*H-*pyrrolizin-7a(5*H*)-yl)methoxy)-2-methyl-2*H*-pyrazolo[4,3-*f*]quinazolin-4-yl)-5-ethyl-6-fluoronaphthalen-2-ol (35 mg, 54.7 mmol, 86% yield) was obtained.

LCMS (ES-API, m/z): [M+H]⁺= 640.7; ¹H NMR (400 MHz, MeOD, ppm): δ 8.31 (s, 1H), 7.66 (dd, *J* = 9.2, 6.0 Hz, 1H), 7.39 (s, 1H), 7.28 - 7.16 (m, 1H), 7.05 (d, *J* = 2.7 Hz, 1H), 5.40 (s, 0.5H), 5.27 (s, 0.5H), 4.32 (d, *J* = 10.2 Hz, 2H), 4.23 (d, *J* = 10.3 Hz, 2H), 4.13 (s, 3H), 4.09 (s, 1H), 3.73 - 3.60 (m, 1H), 3.57 (s, 3H), 3.26 (s, 1H), 3.21 (s, 1H), 3.04 (s, 2H), 2.46 (s, 2H), 2.30 (s, 1H), 2.17 (s, 1H), 2.03 (s, 3H), 1.92 (d, *J* = 7.5 Hz, 1H), 1.79 (s, 2H), 0.64 (t, *J* = 7.2 Hz, 3H).

### EXAMPLE 84: Synthesis of 4-(9-((1R,5S)-3,8-diazabicyclo[3.2.1]octan-3-yl)-7-(((S,Z)-2-(fluoromethylene)tetrahydro-1H-pyrrolizin-7a(5H)-yl)methoxy)-2-methyl-2H-pyrazolo[4,3-f]quinazolin-4-yl)-5,6-difluoronaphthalen-2-ol.

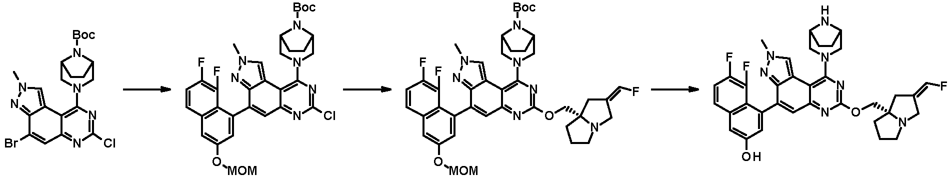

Step 1: *tert*-Butyl (1*R*,5*S*)-3-(4-bromo-7-chloro-2-methyl-2*H*-pyrazolo[4,3-*f*]quinazolin-9-yl)-3,8-diazabicyclo[3.2.1]octane-8-carboxylate (300 mg, 0.591 mmol), 2-(7,8-difluoro-3-(methoxymethoxy)naphthalen-1-yl)-4,4,5,5-tetramethyl-1,3,2-dioxaborolane (310 mg, 0.886 mmol), cataCXium A Pd G3 (44 mg, 0.0591 mmol), and Cs₂CO₃ (577 mg, 1.77 mmol) were dissolved in toluene (12 mL) and purified water (3 mL), and then degassed and purged with nitrogen three times. After stirring at 100 °C for 3 hours, the reaction was quenched by adding water. Ethyl acetate was added, the organic layer was extracted, and dried over anhydrous MgSO₄. Purification was performed by silica gel chromatography to obtain *tert*-butyl (1*R*,5*S*)-3-(7-chloro-4-(7,8-difluoro-3-(methoxymethoxy)naphthalen-1-yl)-2-methyl-2*H*-pyrazolo[4,3-*f*]quinazolin-9-yl)-3,8-diazabicyclo[3.2.1]octane-8-carboxylate (286 mg, 0.439 mmol, 74% yield) as a brown oil. LCMS (ES-API, m/z): [M+H]⁺= 651.2.

Step 2: *tert*-Butyl (1*R*,5*S*)-3-(7-chloro-4-(7,8-difluoro-3-(methoxymethoxy)naphthalen-1-yl)-2-methyl-2*H*-pyrazolo[4,3-*f*]quinazolin-9-yl)-3,8-diazabicyclo[3.2.1]octane-8-carboxylate (286 mg, 0.439 mmol) obtained in Step 1 was dissolved in THF (3 mL) and DMF (3 mL), and (*S*,*Z*)-(2-(fluoromethylene)tetrahydro-1*H*-pyrrolizin-7a(5*H*)-yl)methanol (376 mg, 2.2 mmol) and sodium tert-butoxide (211 mg, 2.2 mmol) were added sequentially and stirred at 40 °C for 3 h. After completion of the reaction, purified water was added to the reaction mixture to quench the reaction, and ethyl acetate was added to extract the organic layer, which was then dried over anhydrous MgSO₄. Purification was performed by silica gel chromatography to obtain *tert*-butyl (1*R*,5*S*)-3-(4-(7,8-difluoro-3-(methoxymethoxy)naphthalen-1-yl)-7-(((*S*,*Z*)-2-(fluoromethylene)tetrahydro-1*H*-pyrrolizin-7a(5*H*)-yl)methoxy)-2-methyl-2*H*-pyrazolo[4,3-*f*]quinazolin-9-yl)-3,8-diazabicyclo[3.2.1]octane-8-carboxylate (150 mg, 0.191 mmol, 43% yield). LCMS (ES-API, m/z): [M+H]⁺= 785.9.

Step 3: To *tert*-Butyl (1*R*,5*S*)-3-(4-(7,8-difluoro-3-(methoxymethoxy)naphthalen-1-yl)-7-(((S,Z)-2-(fluoromethylene)tetrahydro-1*H*-pyrrolizin-7a(5*H*)-yl)methoxy)-2-methyl-2*H-*pyrazolo[4,3-*f*]quinazolin-9-yl)-3,8-diazabicyclo[3.2.1]octane-8-carboxylate (150 mg, 0.191 mmol), HCI in 1,4-dioxane (4 N, 2.5 mL) was added and stirred at 0 °C for 1 h. After completion of the reaction, the product was purified by prep-HPLC to obtain 4-(9-((1*R*,5*S*)-3,8-diazabicyclo[3.2.1]octan-3-yl)-7-(((*S*,*Z*)-2-(fluoromethylene)tetrahydro-1*H-*pyrrolizin-7a(5*H*)-yl)methoxy)-2-methyl-2*H*-pyrazolo[4,3-*f*]quinazolin-4-yl)-5,6-difluoronaphthalen-2-ol (100 mg, 0.16 mmol, 82% yield) as a white solid.

LCMS (ES-API, m/z): [M+H]⁺= 642.7; ¹H NMR (400 MHz, MeOD, ppm): δ 8.29 (s, 1H), 7.63 - 7.59 (m, 1H), 7.39 - 7.31 (m, 2H), 7.31 (s, 1H), 7.19 (s, 1H), 6.78 (s, 0.5H), 6.57 (s, 0.5H), 4.34 - 4.12 (m, 3H), 4.09 (s, 3H), 4.06 (m, 1H), 3.92 - 3.88 (d, 1H), 3.69 - 3.50 (m, 5H), 3.21 - 3.19 (m, 1H), 2.96 (s, 1H), 2.81 - 2.74 (m, 2H), 2.51 - 2.47 (d, 1H), 2.18 - 1.87 (m, 9H).

### EXAMPLE 85: Synthesis of 4-(9-((1R,5S)-3,8-diazabicyclo[3.2.1]octan-3-yl)-5-fluoro-7-(((S,Z)-2-(fluoromethylene)tetrahydro-1H-pyrrolizin-7a(5H)-yl)methoxy)-2-methyl-2H-pyrazolo[4,3-f]quinazolin-4-yl)-5-ethyl-6-fluoronaphthalen-2-ol.

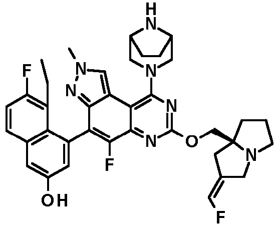

The compound was synthesized in the same manner as in EXAMPLE 84 using 2-(8-Ethyl-7-fluoro-3-(methoxymethoxy)naphthalen-1-yl)-4,4,5,5-tetramethyl-1,3,2-dioxaborolane. 4-(9-((1*R*,5*S*)-3,8-Diazabicyclo[3.2.1]octan-3-yl)-5-fluoro-7-(((*S*,*Z*)-2-(fluoromethylene)tetrahydro-1*H*-pyrrolizin-7a(5*H*)-yl)methoxy)-2-methyl-2*H-*pyrazolo[4,3-*f*]quinazolin-4-yl)-5-ethyl-6-fluoronaphthalen-2-ol (33 mg, 0.049 mmol, 72% yield) was obtained as a white solid.

LCMS (ES-API, m/z): [M+H]⁺= 670.8; ¹H NMR (400 MHz, MeOD, ppm): δ 8.33 (s, 1H), 7.70 (dd, *J* = 9.1, 6.0 Hz, 1H), 7.32 (d, *J =* 2.7 Hz, 1H), 7.24 (t, *J* = 9.3 Hz, 1H), 7.07 (d, *J* = 2.7 Hz, 1H), 6.78 (s, 0.5H), 6.56 (s, 0.5H), 4.35 - 4.26 (m, 3H), 4.13 (s, 5H), 3.89 (d, J = 15.1 Hz, 2H), 3.67 (d, J = 16.5 Hz, 2H), 3.59 (s, 4H), 3.47 (s, 2H), 3.32 (s, 2H), 2.50 (s, 2H), 1.92 (s, 2H), 1.80 (s, 3H), 1.31 (s, 2H), 0.68 (t, J = 7.3 Hz, 3H).

### EXAMPLE 86: Synthesis of 4-(9-((1R,5S)-3,8-diazabicyclo[3.2.1]octan-3-yl)-5-fluoro-7-(((S,Z)-2-(fluoromethylene)tetrahydro-1H-pyrrolizin-7a(5H)-yl)methoxy)-2-methyl-2H-pyrazolo[4,3-f]quinazolin-4-yl)-5,6-difluoronaphthalen-2-ol.

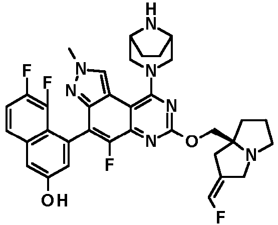

The compound was synthesized in the same manner as in EXAMPLE 84 using 2-(7,8-difluoro-3-(methoxymethoxy)naphthalen-1-yl)-4,4,5,5-tetramethyl-1,3,2-dioxaborolane. 4-(9-((1*R*,5*S*)-3,8-diazabicyclo[3.2.1]octan-3-yl)-5-fluoro-7-(((*S*,*Z*)-2-(fluoromethylene)tetrahydro-1*H*-pyrrolizin-7a(5H)-yl)methoxy)-2-methyl-2*H-*pyrazolo[4,3-*f*]quinazolin-4-yl)-5,6-difluoronaphthalen-2-ol (46 mg, 0.069 mmol, 56% yield) was obtained as a white solid.

LCMS (ES-API, m/z): [M+H]⁺= 660.7; ¹H NMR (400 MHz, MeOD, ppm): δ 8.31 (s, 1H), 7.62 (dd, *J* = 9.2, 4.8 Hz, 1H), 7.44 - 7.35 (m, 1H), 7.34 (d, *J* = 2.2 Hz, 1H), 7.23 (d, *J =* 2.3 Hz, 1H), 6.78 (s, 0.5H), 6.57 (s, 0.5H), 4.38 - 4.27 (m, 2H), 4.22 (s, 2H), 4.14 (s, 3H), 3.89 (d, *J =* 15.0 Hz, 1H), 3.65 (dd, *J* = 12.9, 6.5 Hz, 2H), 3.59 (s, 2H), 3.49 (d, *J* = 14.2 Hz, 1H), 3.19 (dd, *J* = 10.4, 5.8 Hz, 1H), 2.85 - 2.69 (m, 2H), 2.48 (d, *J* = 15.2 Hz, 1H), 2.21 (s, 1H), 2.08 - 1.84 (m, 5H), 1.80 (s, 3H), 1.31 (s, 3H).

### EXAMPLE 87: Synthesis of 4-(9-((1R,5S)-3,8-diazabicyclo[3.2.1]octan-3-yl)-7-((1-((1,1-difluoro-6-azaspiro[2.5]octan-6-yl)methyl)cyclopropyl)methoxy)-5-fluoro-2-methyl-2H-pyrazolo[4,3-f]quinazolin-4-yl)-5-ethynyl-6-fluoronaphthalen-2-ol.

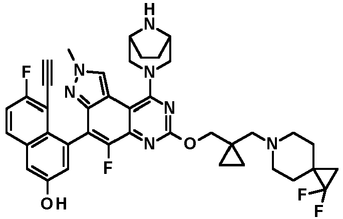

The compound was synthesized in the same manner as in EXAMPLE 80 using intermediate 8. 4-(9-((1*R*,5*S*)-3,8-diazabicyclo[3.2.1]octan-3-yl)-7-((1-((1,1-difluoro-6-azaspiro[2.5]octan-6-yl)methyl)cyclopropyl)methoxy)-5-fluoro-2-methyl-2*H*-pyrazolo[4,3-*f*]quinazolin-4-yl)-5-ethynyl-6-fluoronaphthalen-2-ol (26.26 mg, 35.82 µmol, 20% yield) was obtained as a yellow solid.

LCMS (ES-API, m/z): [M+H]⁺= 726.3; ¹H NMR (400 MHz, DMSO-*d*₆, ppm): δ 10.28 - 9.90 (m, 1H), 8.31 (s, 1H), 7.97 (dd, *J* = 6.0, 9.2 Hz, 1H), 7.44 (t, *J =* 9.2 Hz, 1H), 7.38 (d, *J =* 2.4 Hz, 1H), 7.16 (d, *J* = 2.4 Hz, 1H), 4.30 (s, 2H), 4.10 (br d, *J* = 11.2 Hz, 1H), 4.05 (s, 3H), 3.83 (br d, *J* = 12.0 Hz, 1H), 3.57 (br d, *J* = 11.6 Hz, 2H), 3.48 (br s, 3H), 3.42 (s, 3H), 2.44 (br d, *J* = 1.2 Hz, 2H), 2.36 (br d, *J* = 9.6 Hz, 2H), 1.82 - 1.66 (m, 2H), 1.62 - 1.53 (m, 4H), 1.49 (br s, 2H), 1.17 (br t, *J* = 8.4 Hz, 2H), 0.64 (s, 2H), 0.41 (s, 2H).

### EXAMPLE 88: Synthesis of 4-(9-(2,5-diazabicyclo[2.2.2]octan-2-yl)-5-fluoro-7-(((2R,7aS)-2-fluorotetrahydro-1H-pyrrolizin-7a(5H)-yl)methoxy)-2-methyl-2H-pyrazolo[4,3-f]quinazolin-4-yl)-5-ethynyl-6-fluoronaphthalen-2-ol.

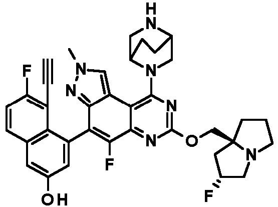

The compound was synthesized in the same manner as in EXAMPLE 80 using *tert*-Butyl 2,5-diazabicyclo[2.2.2]octane-2-carboxylate. 4-(9-(2,5-Diazabicyclo[2.2.2]octan-2-yl)-5-fluoro-7-(((2*R*,7a*S*)-2-fluorotetrahydro-1*H*-pyrrolizin-7a(5*H*)-yl)methoxy)-2-methyl-2*H-*pyrazolo[4,3-*f*]quinazolin-4-yl)-5-ethynyl-6-fluoronaphthalen-2-ol (23.80 mg, 35.68 µmol, 18% yield) was obtained as a white solid.

LCMS (ES-API, m/z): [M+H]⁺= 654.3; ¹H NMR (400 MHz, DMSO-*d*₆, ppm): δ 10.72 - 9.56 (m, 1H), 8.33 (d, *J* = 9.2 Hz, 1H), 7.97 (dd, *J =* 6.0, 9.2 Hz, 1H), 7.44 (t, *J* = 8.8 Hz, 1H), 7.39 (d, *J* = 2.4 Hz, 1H), 7.20 - 7.07 (m, 1H), 5.40 - 5.17 (m, 1H), 4.34 (br d, J = 13.2 Hz, 1H), 4.16 - 4.08 (m, 1H), 4.06 (d, *J* = 4.4 Hz, 3H), 3.99 (br d, *J =* 10.4 Hz, 1H), 3.80 (br d, *J =* 10.4 Hz, 1H), 3.58 (br d, *J* = 11.2 Hz, 2H), 3.53 - 3.48 (m, 1H), 3.20 (br d, *J =* 9.2 Hz, 1H), 3.09 (br d, *J =* 9.2 Hz, 2H), 3.03 - 2.98 (m, 2H), 2.86 - 2.79 (m, 1H), 2.43 - 2.31 (m, 1H), 2.30 - 2.18 (m, 1H), 2.14 (br d, *J* = 5.6 Hz, 1H), 2.08 - 1.94 (m, 3H), 1.92 - 1.72 (m, 6H).

### EXAMPLE 89: Synthesis of 9-((1R,5S)-3,8-diazabicyclo[3.2.1]octan-3-yl)-4-(5-chloro-3,6-dimethyl-1H-indazol-4-yl)-5-fluoro-7-(((2R,7aS)-2-fluorotetrahydro-1H-pyrrolizin-7a(5H)-yl)methoxy)-2-methyl-2H-pyrazolo[4,3-f]quinazoline.

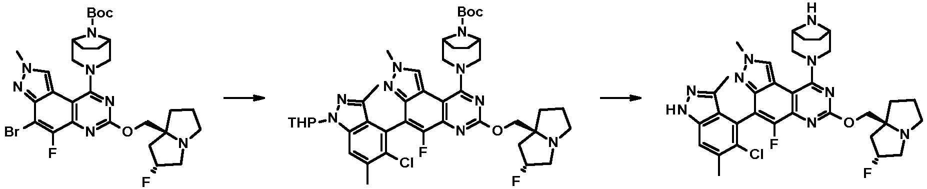

Step 1: Intermediate 22 (100 mg, 324.08 µmol), *tert*-butyl (1*R*,5*S*)-3-(4-bromo-5-fluoro-7-(((2*R*,7a*S*)-2-fluorotetrahydro-1*H*-pyrrolizin-7a(5*H*)-yl)methoxy)-2-methyl-2*H-*pyrazolo[4,3-*f*]quinazolin-9-yl)-3,8-diazabicyclo[3.2.1]octane-8-carboxylate (199.67 mg, 307.87 µmol), K₃PO₄ (206.37 mg, 972.23 µmol), and cataCXium A Pd G4 (21.12 mg, 32.41 µmol) were dissolved in 1,4-dioxane (1 mL) and water (0.2 mL), degassing and nitrogen purging were performed three times, and the mixture was stirred at 90°C for 2 hours. After completion of the reaction, purified water was added to the reaction mixture to quench the reaction, and ethyl acetate was added to extract the organic layer, which was then dried over anhydrous MgSO₄. The reaction residue was purified by prep-TLC to obtain *tert*-butyl (1*R*,5*S*)-3-(4-(5-chloro-3,6-dimethyl-1-(tetrahydro-2*H*-pyran-2-yl)-1*H-*indazol-4-yl)-5-fluoro-7-(((2*R*,7a*S*)-2-fluorotetrahydro-1*H*-pyrrolizin-7a(5*H*)-yl)methoxy)-2-methyl-2*H*-pyrazolo[4,3-*f*]quinazolin-9-yl)-3,8-diazabicyclo[3.2.1]octane-8-carboxylate (87 mg, 99.19 µmol, 31% yield). LCMS (ES-API, m/z): [M+H]⁺= 832.3.

Step 2: *tert*-Butyl (1*R*,5*S*)-3-(4-(5-chloro-3,6-dimethyl-1-(tetrahydro-2*H*-pyran-2-yl)-1*H-*indazol-4-yl)-5-fluoro-7-(((2*R*,7a*S*)-2-fluorotetrahydro-1*H*-pyrrolizin-7a(5*H*)-yl)methoxy)-2-methyl-2*H*-pyrazolo[4,3-*f*]quinazolin-9-yl)-3,8-diazabicyclo[3.2.1]octane-8-carboxylate (77 mg, 92.51 µmol) was dissolved in DCM (1 mL), TFA (3.07 g, 26.92 mmol) was added dropwise, and the mixture was stirred at room temperature for 1 h. After completion of the reaction, purified water was added to the reaction mixture to quench the reaction, ethyl acetate was added to extract the organic layer, and dried over anhydrous MgSO₄. The reaction residue was purified by prep-HPLC to obtain 9-((1*R*,5*S*)-3,8-diazabicyclo[3.2.1]octan-3-yl)-4-(5-chloro-3,6-dimethyl-1*H*-indazol-4-yl)-5-fluoro-7-(((2*R*,7a*S*)-2-fluorotetrahydro-1*H*-pyrrolizin-7a(5*H*)-yl)methoxy)-2-methyl-2*H-*pyrazolo[4,3-*f*]quinazoline (15.36 mg, 23.63 µmol, 26% yield) as a white solid.

LCMS (ES-API, m/z): [M+H]⁺= 832.3; ¹H NMR (400 MHz, DMSO-*d₆*, ppm): δ 12.84 (br s, 1H), 8.39 (s, 1H), 7.58 (s, 1H), 5.38 - 5.19 (m, 1H), 4.17 - 4.10 (m, 1H), 4.06 (s, 2H), 4.09 - 4.00 (m, 1H), 4.05 - 4.00 (m, 1H), 3.92 - 3.84 (m, 1H), 3.59 (br d, *J* = 11.6 Hz, 1H), 3.53 - 3.43 (m, 3H), 3.16 - 3.05 (m, 2H), 3.04 - 2.98 (m, 1H), 2.87 - 2.79 (m, 1H), 2.52 (s, 4H), 2.22 - 2.12 (m, 1H), 2.09 - 1.97 (m, 2H), 1.89 - 1.72 (m, 4H), 1.58 (s, 6H).

### EXAMPLE 90: Synthesis of 4-(9-((1R,5S)-3,8-diazabicyclo[3.2.1]octan-3-yl)-7-(((S)-2-(difluoromethylene)tetrahydro-1H-pyrrolizin-7a(5H)-yl)methoxy)-5-fluoro-2-methyl-2H-pyrazolo[4,3-f]quinazolin-4-yl)-5-ethynyl-6-fluoronaphthalen-2-ol.

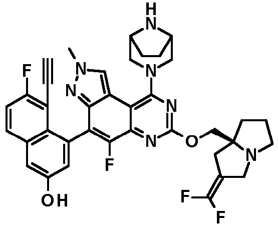

The compound was synthesized in the same manner as in EXAMPLE 80 using (*S*)-(2-(difluoromethylene)tetrahydro-1*H*-pyrrolizin-7a(5*H*)-yl)methanol. 4-(9-((1*R*,5*S*)-3,8-diazabicyclo[3.2.1]octan-3-yl)-7-(((*S*)-2-(difluoromethylene)tetrahydro-1*H*-pyrrolizin-7a(5H)-yl)methoxy)-5-fluoro-2-methyl-2*H*-pyrazolo[4,3-*f*]quinazolin-4-yl)-5-ethynyl-6-fluoronaphthalen-2-ol (115.52 mg, 166.43 µmol, 53% yield) was obtained as a yellow solid.

LCMS (ES-API, m/z): [M+H]⁺= 684.3; ¹H NMR (400 MHz, DMSO-*d*₆, ppm): δ 10.22 - 9.95 (m, 1H), 8.32 (s, 1H), 7.97 (s, 1H), 7.48 - 7.40 (m, 1H), 7.39 (d, *J* = 2.0 Hz, 1H), 7.16 (d, *J* = 2.0 Hz, 1H), 4.21 - 4.06 (m, 3H), 4.05 (s, 3H), 3.91 - 3.78 (m, 1H), 3.72 - 3.61 (m, 1H), 3.59 - 3.51 (m, 1H), 3.44 (m, 4H), 3.06 - 2.95 (m, 1H), 2.66 (br d, *J* = 15.6 Hz, 1H), 2.62 - 2.52 (m, 2H), 2.48 - 2.34 (m, 2H), 1.99 (m, 1H), 1.91 - 1.70 (m, 4H), 1.69 - 1.50 (m, 3H).

### EXAMPLE 91: Synthesis of one isomer of 4-(9-((1R,5S)-3,8-diazabicyclo[3.2.1]octan-3-yl)-7-(((S)-2-(difluoromethylene)tetrahydro-1H-pyrrolizin-7a(5H)-yl)methoxy)-5-fluoro-2-methyl-2H-pyrazolo[4,3-f]quinazolin-4-yl)-5-ethynyl-6-fluoronaphthalen-2-ol.

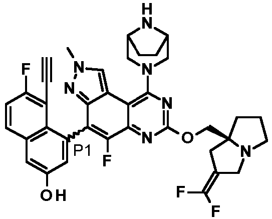

The compound of EXAMPLE 90 was separated under isocratic elution conditions (retention time: 1.160 min) using DAICEL CHIRALPAK AD (250 mm * 30 mm, 10 µm); Mobile phase: [CO₂-ACN/i-PrOH (0.1% NH3 H2O)]; B%: 55%, to obtain a single atropisomer (ee% = 98.5%) (126.94 mg, 182.88 µmol, 44% yield) as a yellow solid.

LCMS (ES-API, m/z): [M+H]⁺= 684.3; ¹H NMR (400 MHz, DMSO-*d*₆, ppm): δ 10.36 - 9.85 (m, 1H), 8.32 (s, 1H), 7.97 (dd, *J* = 6.0, 8.8 Hz, 1H), 7.44 (s, 1H), 7.40 - 7.36 (m, 1H), 7.16 (d, *J =* 2.4 Hz, 1H), 4.19 - 4.02 (m, 6H), 3.90 - 3.79 (m, 1H), 3.71 - 3.61 (m, 1H), 3.59 - 3.52 (m, 1H), 3.44 (br d, *J* = 12.0 Hz, 5H), 3.04 - 2.96 (m, 1H), 2.69 - 2.52 (m, 3H), 2.45 - 2.37 (m, 1H), 2.04 - 1.93 (m, 1H), 1.78 (br s, 4H), 1.56 (br s, 3H).

### EXAMPLE 92: Synthesis of one isomer of 4-(9-((1R,5S)-3,8-diazabicyclo[3.2.1]octan-3-yl)-7-(((S)-2-(difluoromethylene)tetrahydro-1H-pyrrolizin-7a(5H)-yl)methoxy)-5-fluoro-2-methyl-2H-pyrazolo[4,3-f]quinazolin-4-yl)-5-ethynyl-6-fluoronaphthalen-2-ol.

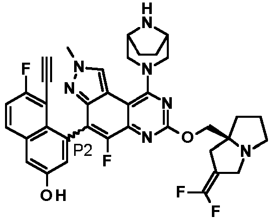

The compound of EXAMPLE 90 was separated under isocratic elution conditions (retention time: 1.605 min) using DAICEL CHIRALPAK AD (250 mm * 30 mm, 10 µm); Mobile phase: [CO₂-ACN/i-PrOH (0.1% NH3 H2O)]; B%: 55%, to obtain a single atropisomer (ee% = 96.5%) (98.73 mg, 141.7 µmol, 34% yield) as a yellow solid. LCMS (ES-API, m/z): [M+H]⁺= 684.3; ¹H NMR (400 MHz, DMSO-*d*₆, ppm): δ 10.37 - 9.83 (m, 1H), 8.32 (s, 1H), 7.97 (dd, *J* = 6.0, 8.8 Hz, 1H), 7.51 - 7.35 (m, 2H), 7.16 (d, *J =* 2.4 Hz, 1H), 4.17 - 4.01 (m, 6H), 3.90 - 3.79 (m, 1H), 3.71 - 3.62 (m, 1H), 3.59 - 3.52 (m, 1H), 3.51 - 3.41 (m, 5H), 3.06 - 2.95 (m, 1H), 2.53 (br s, 3H), 2.43 (br s, 1H), 2.04 - 1.93 (m, 1H), 1.92 - 1.71 (m, 4H), 1.70 - 1.51 (m, 3H).

### EXAMPLE 93: Synthesis of 4-(9-((1R,5S)-3,8-diazabicyclo[3.2.1]octan-3-yl)-5-fluoro-7-(((2R,7aS)-2-fluorotetrahydro-1H-pyrrolizin-7a(5H)-yl)methoxy)-2-methyl-2H-pyrazolo[4,3-f]quinazolin-4-yl)-5-ethyl-6-fluoronaphthalen-2-ol.

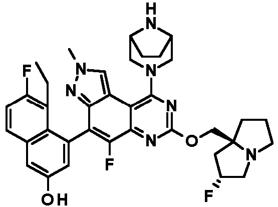

The compound was synthesized in the same manner as in EXAMPLE 83 using 4-Bromo-7,9-dichloro-5-fluoro-2-methyl-2*H*-pyrazolo[4,3-*f*]quinazoline. The reaction residue was purified by Prep-HPLC and lyophilized to obtain 4-(9-((1*R*,5*S*)-3,8-diazabicyclo[3.2.1]octan-3-yl)-5-fluoro-7-(((2*R*,7a*S*)-2-fluorotetrahydro-1*H*-pyrrolizin-7a(5*H*)-yl)methoxy)-2-methyl-2*H*-pyrazolo[4,3-*f*]quinazolin-4-yl)-5-ethyl-6-fluoronaphthalen-2-ol (40 mg, 0.061 mmol, 49% yield) as a yellow solid.

LCMS (ES-API, m/z): [M+H]⁺= 658.7; ¹H NMR (400 MHz, MeOD, ppm): δ 8.33 (s, 1H), 7.72 (dd, *J =* 9.2, 5.8 Hz, 1H), 7.31 (s, 1H), 7.27 (t, *J* = 8.9 Hz, 1H), 7.06 (d, *J* = 2.6 Hz, 1H), 5.41 (s, 0.5H), 5.27 (s, 0.5H), 4.36 - 4.18 (m, 4H), 4.13 (s, 3H), 3.71 - 3.59 (m, 4H), 3.27 - 3.22 (m, 2H), 3.05 - 3.04 (m, 1H), 2.31 - 2.21 (m, 4H), 2.05 - 2.03 (m, 5H), 1.99 - 1.80 (m, 4H) 1.31 (m, 2H), 0.68 (m, 3H).

### EXAMPLE 94: Synthesis of 4-(9-((1R,5S)-3,8-diazabicyclo[3.2.1]octan-3-yl)-5-fluoro-7-(((S,Z)-2-(fluoromethylene)tetrahydro-1H-pyrrolizin-7a(5H)-yl)methoxy)-2-methyl-2H-pyrazolo[4,3-f]quinazolin-4-yl)-5-ethynyl-6-fluoronaphthalen-2-ol.

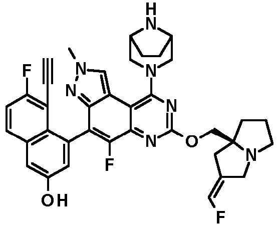

The compound was synthesized in the same manner as in EXAMPLE 80 using (*S*,*Z*)-(2-(fluoromethylene)tetrahydro-1*H*-pyrrolizin-7a(5*H*)-yl)methanol. The compound was purified by Prep-HPLC to obtain 4-(9-((1*R*,5*S*)-3,8-diazabicyclo[3.2.1]octan-3-yl)-5-fluoro-7-(((*S*,*Z*)-2-(fluoromethylene)tetrahydro-1*H*-pyrrolizin-7a(5*H*)-yl)methoxy)-2-methyl-2*H*-pyrazolo[4,3-*f*]quinazolin-4-yl)-5-ethynyl-6-fluoronaphthalen-2-ol (12 mg, 0.617 mmol, 29% yield) as a white solid.

LCMS (ES-API, m/z): [M+H]⁺= 666.7; ¹H NMR (400 MHz, MeOD, ppm): δ 8.29 (s, 1H), 7.88 (dd, *J* = 9.2, 5.8 Hz, 1H), 7.36 (d, *J* = 2.6 Hz, 1H), 7.31 (t, *J* = 8.9 Hz, 1H), 7.22 (d, *J* = 2.6 Hz, 1H), 6.78 (s, 0.5H), 6.57 (s, 0.5H), 4.39 - 4.25 (m, 2H), 4.21 (t, *J* = 14.2 Hz, 2H), 4.13 (s, 3H), 3.90 (d, *J* = 15.1 Hz, 4H), 3.67 (d, *J* = 7.3 Hz, 2H), 3.52 (s, 2H), 2.85 - 2.70 (m, 2H), 2.48 (d, *J* = 15.2 Hz, 1H), 1.94 - 1.85 (m, 3H).

### EXAMPLE 95: Synthesis of 4-(9-((1R,5S)-3,8-diazabicyclo[3.2.1]octan-3-yl)-5-fluoro-7-(((2R,7aS)-2-fluorotetrahydro-1H-pyrrolizin-7a(5H)-yl)methoxy)-2-methyl-2H-pyrazolo[4,3-f]quinazolin-4-yl)-5,6-difluoronaphthalen-2-ol.

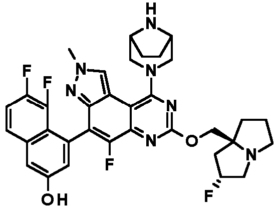

The compound was synthesized in the same manner as in EXAMPLE 83 using 2-(7,8-Difluoro-3-(methoxymethoxy)naphthalen-1-yl)-4,4,5,5-tetramethyl-1,3,2-dioxaborolane. The reaction residue was purified by prep-HPLC to obtain 4-(9-((1*R*,5*S*)-3,8-diazabicyclo[3.2.1]octan-3-yl)-5-fluoro-7-(((2*R*,7a*S*)-2-fluorotetrahydro-1*H*-pyrrolizin-7a(5*H*)-yl)methoxy)-2-methyl-2*H*-pyrazolo[4,3-*f*]quinazolin-4-yl)-5,6-difluoronaphthalen-2-ol (56 mg, 0.086 mmol, 62% yield) as a white solid.

LCMS (ES-API, m/z): [M+H]⁺= 648.7; ¹H NMR (400 MHz, MeOD, ppm): δ 8.31 (s, 1H), 7.62 (dd, *J* = 9.3, 4.8 Hz, 1H), 7.44 - 7.35 (m, 1H), 7.33 (d, *J* = 2.3 Hz, 1H), 7.23 (d, *J =* 2.4 Hz, 1H), 5.41 (s, 0.5H), 5.28 (s, 0.5H), 4.35 (d, *J* = 10.4 Hz, 1H), 4.30 - 4.20 (m, 3H), 4.18 (s, 3H), 3.67 (s, 3H), 3.60 (s, 2H), 3.05 (q, *J* = 9.0 Hz, 1H), 2.42 (dd, *J* = 15.0, 4.7 Hz, 3H), 2.37 - 2.25 (m, 2H), 2.21 (dd, *J* = 18.9, 11.4 Hz, 2H), 2.03 (m, 2H), 1.94 - 1.84 (m, 4H).

### EXAMPLE 96: Synthesis of 4-(9-((1R,5S)-3,8-diazabicyclo[3.2.1]octan-3-yl)-5-fluoro-7-(((5S,7aS)-5-(methoxymethyl)-2-methylenetetrahydro-1H-pyrrolizin-7a(5H)-yl)methoxy)-2-methyl-2H-pyrazolo[4,3-f]quinazolin-4-yl)-5-ethynyl-6-fluoronaphthalen-2-ol.

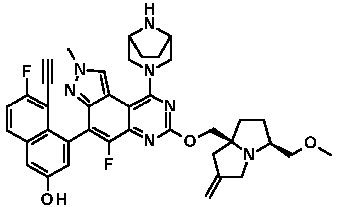

The compound was synthesized in the same manner as in EXAMPLE 80 using Intermediate 17. 4-(9-((1*R*,5*S*)-3,8-Diazabicyclo[3.2.1]octan-3-yl)-5-fluoro-7-(((5*S*,7a*S*)-5-(methoxymethyl)-2-methylenetetrahydro-1*H*-pyrrolizin-7a(5*H*)-yl)methoxy)-2-methyl-2*H*-pyrazolo[4,3-*f*]quinazolin-4-yl)-5-ethynyl-6-fluoronaphthalen-2-ol (28.22 mg, 40.06 µmol, 34% yield) was obtained as a white solid.

LCMS (ES-API, m/z): [M+H]⁺= 692.3; ¹H NMR (400 MHz, DMSO-*d*₆, ppm): δ 10.20 - 9.99 (m, 1H), 8.32 (s, 1H), 7.97 (dd, *J* = 6.0, 9.2 Hz, 1H), 7.44 (t, *J* = 9.2 Hz, 1H), 7.39 (d, *J* = 2.4 Hz, 1H), 7.19 - 7.12 (m, 1H), 4.89 (br s, 2H), 4.12 - 4.00 (m, 5H), 3.93 (br dd, *J =* 6.0, 10.0 Hz, 1H), 3.89 - 3.81 (m, 1H), 3.60 - 3.48 (m, 5H), 3.48 - 3.42 (m, 2H), 3.28 - 3.19 (m, 6H), 2.94 - 2.84 (m, 1H), 2.62 - 2.54 (m, 1H), 2.33 (br d, *J =* 15.6 Hz, 1H), 2.12 - 2.04 (m, 1H), 1.96 (td, *J =* 2.8, 5.6 Hz, 1H), 1.82 - 1.73 (m, 1H), 1.73 - 1.55 (m, 5H).

### EXAMPLE 97: Synthesis of 4-(9-((1R,5S)-3,8-diazabicyclo[3.2.1]octan-3-yl)-7-(((S)-2-(difluoromethylene)tetrahydro-1H-pyrrolizin-7a(5H)-yl)methoxy)-5-fluoro-2-methyl-2H-pyrazolo[4,3-f]quinazolin-4-yl)-5-ethyl-6-fluoronaphthalen-2-ol.

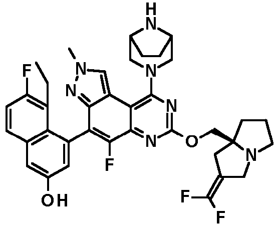

The compound was synthesized in the same manner as in EXAMPLE 83 using (*S*)-(2-(difluoromethylene)tetrahydro-1*H*-pyrrolizin-7a(5*H*)-yl)methanol. 4-(9-((1*R*,5*S*)-3,8-Diazabicyclo[3.2.1]octan-3-yl)-7-(((*S*)-2-(difluoromethylene)tetrahydro-1*H*-pyrrolizin-7a(5*H*)-yl)methoxy)-5-fluoro-2-methyl-2*H*-pyrazolo[4,3-*f*]quinazolin-4-yl)-5-ethyl-6-fluoronaphthalen-2-ol (42 mg, 0.998 mmol, 61% yield) was obtained as a yellow solid. LCMS (ES-API, m/z): [M+H]⁺= 688.7; ¹H NMR (400 MHz, MeOD,_{,} ppm): δ 8.33 (s, 1H), 7.70 (dd, *J* = 9.0, 5.9 Hz, 1H), 7.32 (d, *J* = 2.7 Hz, 1H), 7.24 (t, *J* = 9.4 Hz, 1H), 7.07 (d, *J* = 2.6 Hz, 1H), 4.40 (dd, *J* = 10.5, 5.1 Hz, 1H), 4.31 (dd, *J* = 10.5, 3.1 Hz, 2H), 4.25 (s, 1H), 4.13 (s, 3H), 3.84 (d, *J* = 14.4 Hz, 1H), 3.72 - 3.63 (m, 2H), 3.60 (s, 3H), 3.45 (d, *J* = 14.5 Hz, 1H), 3.22 - 3.15 (m, 1H), 2.86 (d, *J* = 16.2 Hz, 1H), 2.73 (d, *J* = 8.3 Hz, 1H), 2.54 (d, *J* = 16.0 Hz, 3H), 2.26 (s, 1H), 2.17 (d, *J* = 5.5 Hz, 1H), 2.00 (d, *J* = 17.7 Hz, 1H), 1.94 (s, 3H), 1.99 - 1.88 (m, 1H), 1.81 (s, 4H), 0.68 (t, *J* = 7.3 Hz, 3H).

### EXAMPLE 98: Synthesis of 4-(9-((1R,5S)-3,8-diazabicyclo[3.2.1]octan-3-yl)-5-fluoro-7-((1-(((R)-3-fluoropyrrolidin-1-yl)methyl)cyclobutyl)methoxy)-2-methyl-2H-pyrazolo[4,3-f]quinazolin-4-yl)-5-ethynyl-6-fluoronaphthalen-2-ol.

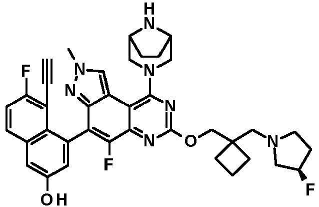

The compuond was synthesized in the same manner as in EXAMPLE 80 using Intermediate 15. 4-(9-((1*R*,5*S*)-3,8-Diazabicyclo[3.2.1]octan-3-yl)-5-fluoro-7-((1-(((*R*)-3-fluoropyrrolidin-1-yl)methyl)cyclobutyl)methoxy)-2-methyl-2*H*-pyrazolo[4,3-*f*]quinazolin-4-yl)-5-ethynyl-6-fluoronaphthalen-2-ol (5.24 mg, 7.46 µmol, 25% yield) was obtained as an off-white solid.

LCMS (ES-API, m/z): [M+H]⁺= 682.4; ¹H NMR (400 MHz, DMSO-*d*₆, ppm): δ 10.23 - 9.97 (m, 1H), 7.44 (t, *J =* 9.2 Hz, 1H), 7.39 (d, *J =* 2.4 Hz, 1H), 5.24 - 5.01 (m, 1H), 4.48 - 4.37 (m, 2H), 4.14 - 4.01 (m, 4H), 3.86 (br d, *J* = 10.8 Hz, 1H), 3.56 (br d, *J* = 12.0 Hz, 1H), 3.47 (br d, *J =* 17.6 Hz, 4H), 2.81 - 2.61 (m, 5H), 2.42 - 2.29 (m, 1H), 2.05 - 1.83 (m, 7H), 1.80 - 1.52 (m, 5H), 1.23 (s, 1H).

### EXAMPLE 99: Synthesis of 4-(9-((1R,5S)-3,8-diazabicyclo[3.2.1]octan-3-yl)-7-(((S)-2-(difluoromethylene)tetrahydro-1H-pyrrolizin-7a(5H)-yl)methoxy)-5-fluoro-2-methyl-2H-pyrazolo[4,3-f]quinazolin-4-yl)-5-ethynyl-6-fluoronaphthalen-2-amine.

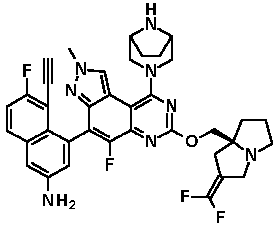

The compound was synthesized in the same manner as in EXAMPLE 81 using (*S*)-(2-(difluoromethylene)tetrahydro-1*H*-pyrrolizin-7a(5*H*)-yl)methanol. 4-(9-((1*R*,5*S*)-3,8-Diazabicyclo[3.2.1]octan-3-yl)-7-(((*S*)-2-(difluoromethylene)tetrahydro-1*H*-pyrrolizin-7a(5*H*)-yl)methoxy)-5-fluoro-2-methyl-2*H*-pyrazolo[4,3-*f*]quinazolin-4-yl)-5-ethynyl-6-fluoronaphthalen-2-amine (45 mg, 65.91 µmol, 26% yield) was obtained as a white solid. LCMS (ES-API, m/z): [M+H]⁺= 683.3; ¹H NMR (400 MHz, DMSO-*d*₆, ppm): δ 8.32 (s, 1H), 7.77 (dd, *J* = 6.0, 9.2 Hz, 1H), 7.31 (t, *J* = 9.2 Hz, 1H), 7.06 - 7.00 (m, 2H), 5.57 (s, 2H), 4.17 -4.03 (m, 7H), 3.87 - 3.77 (m, 1H), 3.70 - 3.62 (m, 1H), 3.56 (br d, *J =* 12.0 Hz, 1H), 3.49 - 3.44 (m, 3H), 3.06 - 2.97 (m, 1H), 2.70 - 2.56 (m, 3H), 2.41 (br d, *J* = 15.6 Hz, 1H), 2.04 - 1.67 (m, 6H), 1.66 - 1.52 (m, 3H).

### EXAMPLE 100: Synthesis of 4-(9-((1R,5S)-3,8-diazabicyclo[3.2.1]octan-3-yl)-5-fluoro-7-(((2S,7aR)-2-fluoro-6-methylenetetrahydro-1H-pyrrolizin-7a(5H)-yl)methoxy)-2-methyl-2H-pyrazolo[4,3-f]quinazolin-4-yl)-5-ethynyl-6-fluoronaphthalen-2-ol.

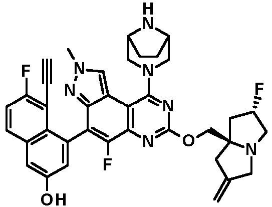

The compound was synthesized in the same manner as in EXAMPLE 80 using Intermediate 16. 4-(9-((1*R*,5*S*)-3,8-Diazabicyclo[3.2.1]octan-3-yl)-5-fluoro-7-(((2*S*,7a*R*)-2-fluoro-6-methylenetetrahydro-1*H*-pyrrolizin-7a(5*H*)-yl)methoxy)-2-methyl-2*H-*pyrazolo[4,3-*f*]quinazolin-4-yl)-5-ethynyl-6-fluoronaphthalen-2-ol (18 mg, 46% yield) was obtained as a yellow solid.

LCMS (ES-API, m/z): [M+H]⁺= 666.7; ¹H NMR (400 MHz, MeOD, ppm): δ 8.29 (s, 1H), 7.88 (dd, *J =* 9.2, 5.7 Hz, 1H), 7.37 (d, *J* = 2.6 Hz, 1H), 7.31 (t, *J* = 8.9 Hz, 1H), 7.22 (d, *J* = 2.6 Hz, 1H), 5.45 (s, 0.5H), 5.31 (s, 0.5H), 5.01 (s, 2H), 4.36 - 4.18 (m, 3H), 4.13 (s, 3H), 3.82 (d, *J* = 13.5 Hz, 1H), 3.68 (t, *J* = 11.9 Hz, 3H), 3.45 (d, *J* = 15.1 Hz, 1H), 3.27 - 3.17 (m, 1H), 2.91 (d, *J* = 11.4 Hz, 1H), 2.80 (s, 2H), 2.32 (dd, *J* = 24.0, 14.7 Hz, 1H), 2.05 (d, *J* = 9.3 Hz, 1H), 1.92 (s, 1H), 1.86 (s, 3H), 0.91 (s, 3H).

### EXAMPLE 101: Synthesis of 9-((1R,5S)-3,8-diazabicyclo[3.2.1]octan-3-yl)-7-(((S)-2-(difluoromethylene)tetrahydro-1H-pyrrolizin-7a(5H)-yl)methoxy)-4-(8-ethynyl-7-fluoronaphthalen-1-yl)-5-fluoro-2-methyl-2H-pyrazolo[4,3-f]quinazoline.

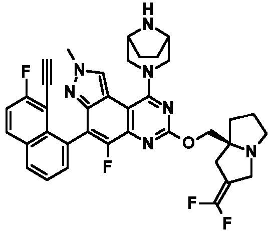

The compound was synthesized in the same manner as in EXAMPLE 80 using ((2-Fluoro-8-(4,4,5,5-tetramethyl-1,3,2-dioxaborolan-2-yl)naphthalen-1-yl)ethynyl)triisopropylsilane and (*S*)-(2-(difluoromethylene)tetrahydro-1*H*-pyrrolizin-7a(5*H*)-yl)methanol. 9-((1*R*,5*S*)-3,8-Diazabicyclo[3.2.1]octan-3-yl)-7-(((*S*)-2-(difluoromethylene)tetrahydro-1*H*-pyrrolizin-7a(5*H*)-yl)methoxy)-4-(8-ethynyl-7-fluoronaphthalen-1-yl)-5-fluoro-2-methyl-2*H-*pyrazolo[4,3-*f*]quinazoline (22 mg, 56% yield) was obtained as a white solid.

LCMS (ES-API, m/z): [M+H]⁺= 666.7; ¹H NMR (400 MHz, MeOD, ppm): δ 8.31 (s, 1H), 8.17 - 8.09 (m, 1H), 7.73 - 7.61 (m, 1H), 7.43 (t, *J* = 8.9 Hz, 1H), 4.40 (dd, *J* = 10.6, 7.7 Hz, 1H), 4.32 (dd, *J* = 10.6, 6.8 Hz, 1H), 4.24 (d, *J* = 13.0 Hz, 2H), 4.12 (s, 3H), 3.86 (d, *J* = 14.5 Hz, 1H), 3.74 - 3.67 (m, 4H), 3.46 (s, 1H), 3.23 - 3.15 (m, 1H), 2.99 (s, 1H), 2.87 (d, *J =* 15.8 Hz, 1H), 2.75 (q, *J* = 8.2 Hz, 1H), 2.55 (d, *J =* 16.1 Hz, 1H), 2.04 (d, *J =* 9.2 Hz, 1H), 2.01 - 1.92 (m, 1H), 1.95 (s, 4H), 1.87 (s, 2H), 1.31 (s, 2H).

### EXAMPLE 102: Synthesis of (4-(4-(8-ethynyl-7-fluoro-3-hydroxynaphthalen-1-yl)-5-fluoro-7-(((2R,7aS)-2-fluorotetrahydro-1H-pyrrolizin-7a(5H)-yl)methoxy)-2-methyl-2H-pyrazolo[4,3-f]quinazolin-9-yl)piperazin-1-yl)(oxiran-2-yl)methanone.

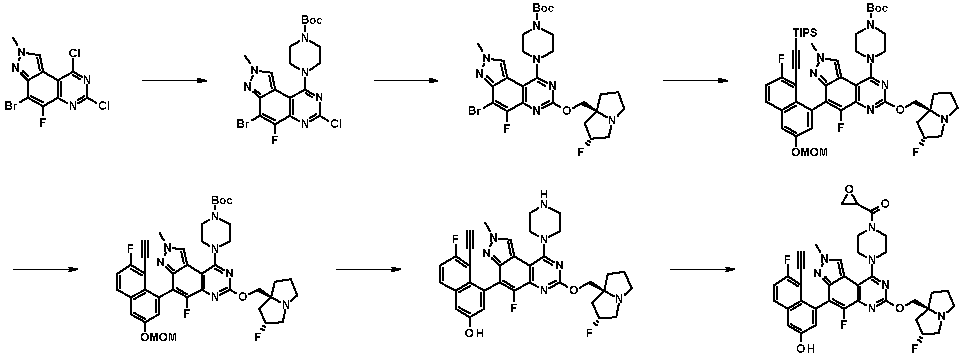

Step 1: 4-bromo-7,9-dichloro-5-fluoro-2-methyl-2*H*-pyrazolo[4,3-*f*]quinazoline (500 mg, 1.43 mmol, 1 equiv) was dissolved in ACN (8 mL), and DIPEA (369.29 mg, 2.86 mmol, 497.70 µL, 2 equiv) and *tert*-butyl piperazine-1-carboxylate (319.31 mg, 1.71 mmol, 1.2 equiv) were added dropwise. The reaction mixture was stirred at room temperature for 1 h, filtered, washed with PE, and dried under vacuum. *tert*-butyl 4-(4-bromo-7-chloro-5-fluoro-2-methyl-2*H*-pyrazolo[4,3-*f*]quinazolin-9-yl)piperazine-1-carboxylate (570 mg, crude mixture) was obtained as a yellow solid.

LCMS (ES-API, m/z): [M+H]⁺=501.0 ; HNMR (400 MHz, DMSO-*d*₆, ppm): δ 8.73 (s, 1H), 4.28 (s, 3H), 3.56 - 3.54 (m, 8H), 1.45 (s, 9H).

Step 2: The compound was synthesized in the same manner as in Step 2 of EXAMPLE 80 using *tert*-butyl 4-(4-bromo-7-chloro-5-fluoro-2-methyl-2*H*-pyrazolo[4,3-*f*]quinazolin-9-yl)piperazine-1-carboxylate. *tert*-butyl 4-(4-bromo-5-fluoro-7-(((2*R*,7a*S*)-2-fluorotetrahydro-1*H*-pyrrolizin-7a(5*H*)-yl)methoxy)-2-methyl-2*H*-pyrazolo[4,3-*f*]quinazolin-9-yl)piperazine-1-carboxylate (384 mg, 561.96 µmol, 74% yield) was obtained as a yellow solid.

LCMS (ES-API, m/z): [M+H]⁺=624.0 ; HNMR (400 MHz, DMSO-*d*₆, ppm): δ 8.64 (s, 1H), 5.33 (d, *J* = 53.6 Hz, 1H) 4.29 (s, 3H), 4.20 - 4.07 (m, 2H), 3.63 (s, 3H), 3.55 - 3.50 (m, 4H), 3.15 - 3.06 (m, 3H), 2.93 - 2.77 (m, 2H), 2.20 - 2.03 (m, 3H), 1.93 - 1.83 (m, 3H), 1.48 (s, 9H).

Step 3: The compound was synthesized in the same manner as in Step 3 of EXAMPLE 80 using *tert*-butyl 4-(4-bromo-5-fluoro-7-(((2*R*,7a*S*)-2-fluorotetrahydro-1*H*-pyrrolizin-7a(5*H*)-yl)methoxy)-2-methyl-2*H*-pyrazolo[4,3-*f*]quinazolin-9-yl)piperazine-1-carboxylate. tert-butyl 4-(5-fluoro-4-(7-fluoro-3-(methoxymethoxy)-8-((triisopropylsilyl)ethynyl)naphthalen-1-yl)-7-(((2*R*,7a*S*)-2-fluoro tetrahydro-1*H*-pyrrolizin-7a(5*H*)-yl)methoxy)-2-methyl-2*H*-pyrazolo[4,3-*f*]quinazolin-9-yl)piperazine-1-carboxylate (300 mg, 323.22 µmol, 59% yield) was obtained as a yellow solid. LCMS (ES-API, m/z): [M+H]⁺=928.5.

Step 4: The compound was synthesized in the same manner as in Step 4 of EXAMPLE 80 using tert-butyl 4-(5-fluoro-4-(7-fluoro-3-(methoxymethoxy)-8-((triisopropylsilyl)ethynyl)naphthalen-1-yl)-7-(((2*R*,7a*S*)-2-fluorotetrahydro-1*H*-pyrrolizin-7a(5*H*)-yl)methoxy)-2-methyl-2*H*-pyrazolo[4,3-*f*]quinazolin-9-yl)piperazine-1-carboxylate. *tert*-butyl 4-(4-(8-ethynyl-7-fluoro-3-(methoxymethoxy)naphthalen-1-yl)-5-fluoro-7-(((2*R*,7a*S*)-2-fluorotetrahydro-1*H*-pyrrolizin-7a(5*H*)-yl)methoxy)-2-methyl-2*H-*pyrazolo[4,3-*f*]quinazolin-9-yl)piperazine-1-carboxylate (310 mg, crude mixture) was obtained as a yellow solid. LCMS (ES-API, m/z): [M+H]⁺=772.4.

Step 5: The compound was synthesized in the same manner as in Step 5 of EXAMPLE 80 using tert-butyl 4-(4-(8-ethynyl-7-fluoro-3-(methoxymethoxy)naphthalen-1-yl)-5-fluoro-7-(((2*R*,7a*S*)-2-fluorotetrahydro-1*H*-pyrrolizin-7a(5*H*)-yl)methoxy)-2-methyl-2*H-*pyrazolo[4,3-*f*]quinazolin-9-yl)piperazine-1-carboxylate. 5-ethynyl-6-fluoro-4-(5-fluoro-7-(((2*R*,7a*S*)-2-fluorotetrahydro-1*H*-pyrrolizin-7a(5*H*)-yl)methoxy)-2-methyl-9-(piperazin-1-yl)-2*H*-pyrazolo[4,3-*f*]quinazolin-4-yl)naphthalen-2-ol (64 mg, 101.35 µmol, 46% yield) was obtained as a yellow solid. LCMS (ES-API, m/z): [M+H]⁺=628.3.

Step 6: Potassium oxirane-2-carboxylate (19.29 mg, 152.95 µmol, 2.4 equiv) was dissolved in DMF (0.5 mL) and HATU (29.08 mg, 76.47 µmol, 1.2 equiv) was added dropwise. 5-ethynyl-6-fluoro-4-(5-fluoro-7-(((2*R*,7a*S*)-2-fluorotetrahydro-1*H*-pyrrolizin-7a(5*H*)-yl)methoxy)-2-methyl-9-(piperazin-1-yl)-2*H*-pyrazolo[4,3-*f*]quinazolin-4-yl)naphthalen-2-ol (40 mg, 63.73 µmol, 1 equiv) and DIPEA (24.71 mg, 191.19 µmol, 33.30 µL, 3 equiv) were added to the reaction mixture, and stirred at room temperature for 1 h. Prep-HPLC (neutral condition; column: Waters Xbridge 150*25mm* 5um; mobile phase: [water(10 mM NH4HCO3)-ACN]; gradient: 25%-55% B over 9 min) was performed to obtain (4-(4-(8-ethynyl-7-fluoro-3-hydroxynaphthalen-1-yl)-5-fluoro-7-(((2*R*,7a*S*)-2-fluorotetrahydro-1*H*-pyrrolizin-7a(5*H*)-yl)methoxy)-2-methyl-2*H*-pyrazolo[4,3-*f*]quinazolin-9-yl)piperazin-1-yl)(oxiran-2-yl)methanone (10.77 mg, 14.74 µmol, 12% yield) as a yellow solid.

LCMS (ES-API, m/z): [M+H]⁺=698.4 ; HNMR (400 MHz, DMSO-*d*₆, ppm): δ 10.51 - 9.83 (m, 1H), 8.63 - 8.39 (m, 1H), 8.07 - 7.87 (m, 1H), 7.49 - 7.31 (m, 2H), 7.22 - 7.12 (m, 1H), 5.42 - 5.14 (m, 1H), 4.18 - 4.02 (m, 5H), 4.01 - 3.88 (m, 3H), 3.85 - 3.74 (m, 2H), 3.65 - 3.41 (m, 5H), 3.13 - 3.06 (m, 2H), 3.04 - 2.99 (m, 1H), 2.98 - 2.94 (m, 1H), 2.87 - 2.80 (m, 2H), 2.19 - 2.13 (m, 1H), 2.10 - 1.99 (m, 2H), 1.90 - 1.72 (m, 3H).

### EXAMPLE 103: Synthesis of 4-(9-((1R,5S)-3,8-diazabicyclo[3.2.1]octan-8-yl)-5-fluoro-7-(((2R,7aS)-2-fluorotetrahydro-1H-pyrrolizin-7a(5H)-yl)methoxy)-2-methyl-2H-pyrazolo[4,3-f]quinazolin-4-yl)-5-ethynyl-6-fluoronaphthalen-2-ol.

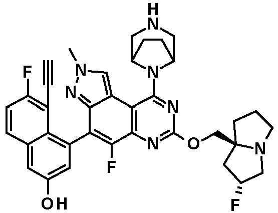

The compound was synthesized in the same manner as in EXAMPLE 80 using 3-Boc-3,8-Diazabicyclo[3.2.1]octane. 4-(9-((1*R*,5*S*)-3,8-Diazabicyclo[3.2.1]octan-8-yl)-5-fluoro-7-(((2*R*,7a*S*)-2-fluorotetrahydro-1*H*-pyrrolizin-7a(5*H*)-yl)methoxy)-2-methyl-2*H-*pyrazolo[4,3-*f*]quinazolin-4-yl)-5-ethynyl-6-fluoronaphthalen-2-ol (38 mg, 58.1 µmol, 71% yield) was obtained as a white solid.

LCMS (ES-API, m/z): [M+H]⁺= 654.3; ¹H NMR (400 MHz, CD₃OD, ppm): δ 8.28 (s, 1H), 7.88 (dd, *J* = 9.1, 5.8 Hz, 1H), 7.37 (d, *J* = 2.5 Hz, 1H), 7.31 (t, *J =* 8.9 Hz, 1H), 7.24 (d, *J* = 2.6 Hz, 1H), 5.42 (m, 0.5H), 5.29(m, 0.5H), 4.62 (m, 3H), 4.41-4.25 (m, 1H), 4.31(m, 1H), 4.15(s, 3H), 2.90-2.82 (m, 3H), 2.05-1.99 (m, 7H), 1.92 (m, 3H), 1.32(m, 6H).

### EXAMPLE 104: Synthesis of 4-(9-((1R,5S)-3,8-diazabicyclo[3.2.1]octan-3-yl)-5-fluoro-7-(((2R,7aS)-2-fluorotetrahydro-1H-pyrrolizin-7a(5H)-yl)methoxy)-2-methyl-2H-pyrazolo[4,3-f]quinazolin-4-yl)-2-amino-7-fluorobenzo[b]thiophene-3-carbonitrile.

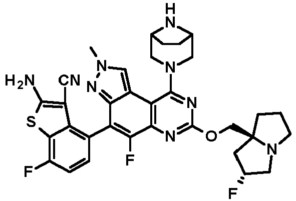

The compound was synthesized in the same manner as in EXAMPLE 62 using Intermediate 5. 4-(9-((1*R*,5*S*)-3,8-diazabicyclo[3.2.1]octan-3-yl)-5-fluoro-7-(((2*R*,7a*S*)-2-fluorotetrahydro-1*H*-pyrrolizin-7a(5*H*)-yl)methoxy)-2-methyl-2*H*-pyrazolo[4,3-*f*]quinazolin-4-yl)-2-amino-7-fluorobenzo[*b*]thiophene-3-carbonitrile (82 mg, 124.29 µmol, 76% yield) was obtained as a white solid.

LCMS (ES-API, m/z): [M+H]⁺= 660.3; ¹H NMR (400 MHz, DMSO-*d*₆, ppm): δ 8.34 (s, 1H), 7.99 - 7.88 (m, 2H), 7.38 (dd, *J* = 5.2, 8.4 Hz, 1H), 7.20 - 7.09 (m, 1H), 5.38 - 5.18 (m, 1H), 4.16 - 3.99 (m, 6H), 3.90 - 3.78 (m, 1H), 3.56 - 3.36 (m, 4H), 3.14 - 3.00 (m, 3H), 2.90 - 2.75 (m, 1H), 2.18 - 2.00 (m, 3H), 1.94 - 1.59 (m, 5H), 1.51 (br s, 3H).

### EXAMPLE 105: Synthesis of 4-(9-((1R,4R)-2,5-diazabicyclo[2.2.1]heptan-2-yl)-5-fluoro-7-(((2R,7aS)-2-fluorotetrahydro-1H-pyrrolizin-7a(5H)-yl)methoxy)-2-methyl-2H-pyrazolo[4,3-f]quinazolin-4-yl)-5-ethynyl-6-fluoronaphthalen-2-ol.

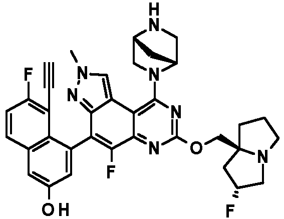

The compound was synthesized in the same manner as in EXAMPLE 80 using *tert*-Butyl (1R,4R)-2,5-diazabicyclo[2.2.1]heptane-2-carboxylate. 4-(9-((1*R*,4*R*)-2,5-Diazabicyclo[2.2.1]heptan-2-yl)-5-fluoro-7-(((2*R*,7a*S*)-2-fluorotetrahydro-1*H*-pyrrolizin-7a(5*H*)-yl)methoxy)-2-methyl-2*H*-pyrazolo[4,3-*f*]quinazolin-4-yl)-5-ethynyl-6-fluoronaphthalen-2-ol (78 mg, 122.0 µmol, 77% yield) was obtained as an off-white solid. LCMS (ES-API, m/z): [M+H]⁺= 640.3; ¹H NMR (400 MHz, DMSO-*d*₆, ppm): δ 10.16 (s, 1H), 8.32 (d, *J* = 28.0 Hz, 1H), 7.99 - 7.96 (m, 1H), 7.44 (t, *J* = 8.0 Hz, 1H), 7.39 (m, 1H), 5.29 (d, *J* = 54.0 Hz, 1H), 4.86 (d, *J =* 55.2 Hz, 1H), 4.13-3.99 (m, 5H), 3.67-3.48 (m, 2H), 3.14-2.80 (m, 5H), 2.16-1.72 (m, 9H).

### EXAMPLE 106: Synthesis of 4-(1-((1R,5S)-3,8-diazabicyclo[3.2.1]octan-8-yl)-3-(((S)-2-(difluoromethylene)tetrahydro-1H-pyrrolizin-7a(5H)-yl)methoxy)imidazo[1',2':1,6]pyrido[3,2-d]pyrimidin-6-yl)-5-ethynyl-6-fluoronaphthalen-2-ol.

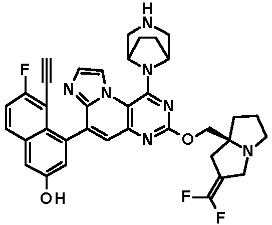

The compound was synthesized in the same manner as in EXAMPLE 2 using (*S*)-(2-(difluoromethylene)tetrahydro-1*H*-pyrrolizin-7a(5H)-yl)methanol. 4-(1-((1*R*,5*S*)-3,8-diazabicyclo[3.2.1]octan-8-yl)-3-(((*S*)-2-(difluoromethylene)tetrahydro-1*H*-pyrrolizin-7a(5*H*)-yl)methoxy)imidazo[1',2':1,6]pyrido[3,2-*d*]pyrimidin-6-yl)-5-ethynyl-6-fluoronaphthalen-2-ol (20 mg, 29.5 µmol, 15% yield) was obtained as a white solid. LCMS (ES-API, m/z): [M+H]⁺= 652.4; ¹H NMR (400 MHz, DMSO-*d*₆, ppm): **δ** 10.12 (br d, *J* = 0.8 Hz, 1H), 8.81 - 8.55 (m, 1H), 7.97 (dd, *J* = 6.0, 9.1 Hz, 1H), 7.53 - 7.33 (m, 4H), 7.22 (s, 1H), 4.45 - 4.06 (m, 4H), 3.98 - 3.87 (m, 1H), 3.72 - 3.46 (m, 2H), 3.26 (br s, 1H), 3.05 - 2.97 (m, 1H), 2.84 - 2.55 (m, 4H), 2.43 (br d, *J* = 15.6 Hz, 2H), 2.18 - 1.73 (m, 8H).

### EXAMPLE 107: Synthesis of 4-(1-((1R,5S)-3,8-diazabicyclo[3.2.1]octan-8-yl)-3-(((S)-2-(difluoromethylene)tetrahydro-1H-pyrrolizin-7a(5H)-yl)methoxy)imidazo[1',2':1,6]pyrido[3,2-d]pyrimidin-6-yl)-5-ethyl-6-fluoronaphthalen-2-ol.

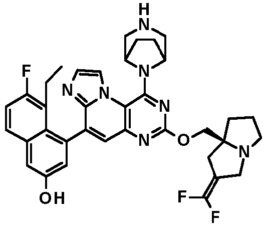

The compound was synthesized in the same manner as in EXAMPLE 1 using (*S*)-(2-(difluoromethylene)tetrahydro-1*H*-pyrrolizin-7a(5*H*)-yl)methanol. 4-(1-((1*R*,5*S*)-3,8-diazabicyclo[3.2.1]octan-8-yl)-3-(((*S*)-2-(difluoromethylene)tetrahydro-1*H*-pyrrolizin-7a(5*H*)-yl)methoxy)imidazo[1',2':1,6]pyrido[3,2-*d*]pyrimidin-6-yl)-5-ethyl-6-fluoronaphthalen-2-ol (15 mg, 22.12 µmol, 16% yield) was obtained as a white solid. LCMS (ES-API, m/z): [M+H]⁺= 656.4; ¹H NMR (400 MHz, DMSO-*d*₆, ppm): **δ** 8.79 - 8.59 (m, 1H), 7.70 - 7.64 (m, 1H), 7.60 (s, 1H), 7.56 - 7.53 (m, 1H), 7.52 - 7.47 (m, 3H), 7.22 (br d, *J* = 3.6 Hz, 5H), 5.27 (s, 3H), 4.26 (br d, *J* = 2.4 Hz, 6H), 3.99 - 3.78 (m, 3H), 3.50 (s, 6H), 3.44 - 3.24 (m, 4H), 2.97 - 2.63 (m, 3H), 1.53 - 1.47 (m, 9H), 0.67 - 0.56 (m, 5H).

### EXAMPLE 108: Synthesis of 4-(1-((1R,5S)-3,8-diazabicyclo[3.2.1]octan-8-yl)-3-(((S)-2-(difluoromethylene)tetrahydro-1H-pyrrolizin-7a(5H)-yl)methoxy)imidazo[1',2':1,6]pyrido[3,2-d]pyrimidin-6-yl)-5-ethynyl-6-fluoronaphthalen-2-amine.

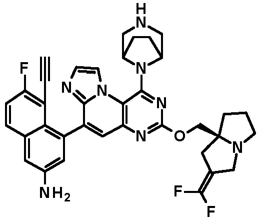

The compound was synthesized in the same manner as in EXAMPLE 14 using (*S*)-(2-(Difluoromethylene)tetrahydro-1*H*-pyrrolizin-7a(5*H*)-yl)methanol. 4-(1-((1*R,*5*S*)-3,8-Diazabicyclo[3.2.1]octan-8-yl)-3-(((*S*)-2-(difluoromethylene)tetrahydro-1*H*-pyrrolizin-7a(5*H*)-yl)methoxy)imidazo[1',2':1,6]pyrido[3,2-*d*]pyrimidin-6-yl)-5-ethynyl-6-fluoronaphthalen-2-amine (17.17 mg, 26.29 µmol, 17% yield) was obtained as a yellow solid.

LCMS (ES-API, m/z): [M+H]⁺= 651.3; ¹H NMR (400 MHz, DMSO-*d*₆, ppm): **δ**8.71 (br s, 1H), 7.81 - 7.71 (m, 1H), 7.53 - 7.46 (m, 1H), 7.37 - 7.26 (m, 2H), 7.11 - 7.02 (m, 2H), 5.75 - 5.48 (m, 2H), 4.49 - 4.05 (m, 4H), 4.02 - 3.86 (m, 1H), 3.74 - 3.61 (m, 1H), 3.56 - 3.44 (m, 1H), 3.38 - 3.34 (m, 2H), 3.04 - 2.54 (m, 6H), 2.47 - 2.38 (m, 1H), 2.34 - 1.65 (m, 9H).

### EXAMPLE 109: Synthesis of 4-(1-((1R,5S)-3,8-diazabicyclo[3.2.1]octan-3-yl)-3-(((S)-2-(difluoromethylene)tetrahydro-1H-pyrrolizin-7a(5H)-yl)methoxy)imidazo[1',2':1,6]pyrido[3,2-d]pyrimidin-6-yl)-5-ethyl-6-fluoronaphthalen-2-ol.

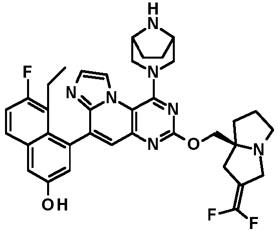

The compound was synthesized in the same manner as in EXAMPLE 1 using (*S*)-(2-(difluoromethylene)tetrahydro-1*H*-pyrrolizin-7a(5*H*)-yl)methanol. 4-(1-((1*R*,5*S*)-3,8-Diazabicyclo[3.2.1]octan-3-yl)-3-(((*S*)-2-(difluoromethylene)tetrahydro-1*H*-pyrrolizin-7a(5H)-yl)methoxy)imidazo[1',2':1,6]pyrido[3,2-*d*]pyrimidin-6-yl)-5-ethyl-6-fluoronaphthalen-2-ol (11.65 mg, 17.61 µmol, 31% yield) was obtained as a white solid. LCMS (ES-API, m/z): [M+H]⁺= 656.4; ¹H NMR (400 MHz, DMSO-*d*₆, ppm): **δ** 9.83 (s, 1H), 8.69 - 8.40 (m, 1H), 8.13 - 7.95 (m, 1H), 7.75 (dd, *J* = 6.2, 8.8 Hz, 1H), 7.65 - 7.36 (m, 2H), 7.34 - 7.26 (m, 2H), 7.06 (s, 1H), 4.21 - 4.07 (m, 2H), 3.75 - 3.59 (m, 2H), 3.53 - 3.39 (m, 2H), 3.33 (br s, 2H), 3.03 - 2.97 (m, 1H), 2.68 - 2.54 (m, 3H), 2.48 - 2.37 (m, 3H), 2.10 - 1.92 (m, 3H), 1.88 - 1.72 (m, 4H), 1.58 - 1.48 (m, 1H), 1.38 - 1.17 (m, 2H), 0.51 (br t, *J* = 7.2 Hz, 3H).

### EXAMPLE 110: Synthesis of 4-(9-((1R,5S)-3,8-diazabicyclo[3.2.1]octan-3-yl)-5-fluoro-7-(((2R,7aS)-2-fluorotetrahydro-1H-pyrrolizin-7a(5H)-yl)methoxy)-2-methyl-2H-pyrazolo[4,3-f]quinazolin-4-yl)-2-amino-5-fluorobenzo[b]thiophene-3-carbonitrile.

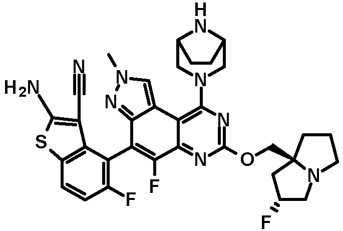

The compound is synthesized in the same manner as in the synthetic method of EXAMPLE 62.

### EXAMPLE 111. Synthesis of 4-(9-((1R,5S)-3,8-diazabicyclo[3.2.1]octan-3-yl)-5-fluoro-7-(((2R,7aS)-2-fluorotetrahydro-1H-pyrrolizin-7a(5H)-yl)methoxy)-2-methyl-2H-pyrazolo[4,3-f]quinazolin-4-yl)-2-amino-5,7-difluorobenzo[b]thiophene-3-carbonitrile.

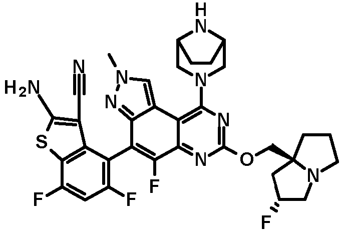

The compound is synthesized in the same manner as in the synthetic method of EXAMPLE 62.

### EXAMPLE 112. Synthesis of 4-(9-((1R,5S)-3,8-diazabicyclo[3.2.1]octan-3-yl)-5-fluoro-7-(((2R,7aS)-2-fluorotetrahydro-1H-pyrrolizin-7a(5H)-yl)methoxy)-2-methyl-2H-pyrazolo[4,3-f]quinazolin-4-yl)-2-amino-7-fluorothieno[3,2-c]pyridine-3-carbonitrile.

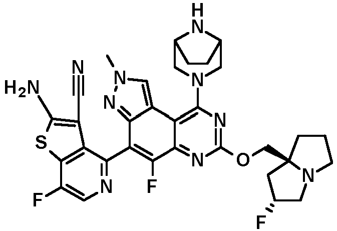

The compound is synthesized in the same manner as in the synthetic method of EXAMPLE 62.

### EXAMPLE 113. Synthesis of 4-(9-((1R,5S)-3,8-diazabicyclo[3.2.1]octan-3-yl)-5-fluoro-7-(((2R,7aS)-2-fluorotetrahydro-1H-pyrrolizin-7a(5H)-yl)methoxy)-2-methyl-2H-pyrazolo[4,3-f]quinazolin-4-yl)-2-aminobenzofuran-3-carbonitrile.

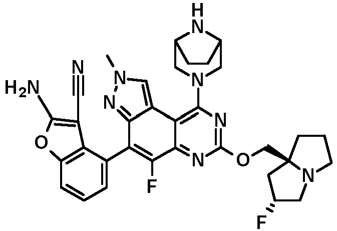

The compound is synthesized in the same manner as in the synthetic method of EXAMPLE 62.

### EXAMPLE 114. Synthesis of 4-(9-((1R,5S)-3,8-diazabicyclo[3.2.1]octan-3-yl)-5-fluoro-7-(((2R,7aS)-2-fluorotetrahydro-1H-pyrrolizin-7a(5H)-yl)methoxy)-2-methyl-2H-pyrazolo[4,3-f]quinazolin-4-yl)-2-amino-7-fluorobenzofuran-3-carbonitrile.

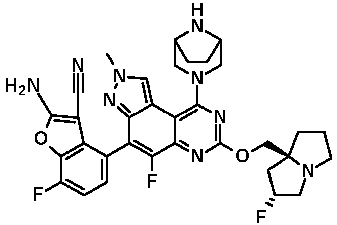

The compound is synthesized in the same manner as in the synthetic method of EXAMPLE 62.

### EXAMPLE 115. Synthesis of 4-(9-((1R,5S)-3,8-diazabicyclo[3.2.1]octan-3-yl)-5-fluoro-7-(((2R,7aS)-2-fluorotetrahydro-1H-pyrrolizin-7a(5H)-yl)methoxy)-2-methyl-2H-pyrazolo[4,3-f]quinazolin-4-yl)-2-amino-5-fluorobenzofuran-3-carbonitrile.

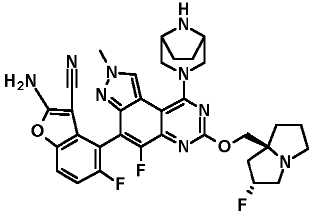

The compound is synthesized in the same manner as in the synthetic method of EXAMPLE 62.

### EXAMPLE 116. Synthesis of 4-(9-((1R,5S)-3,8-diazabicyclo[3.2.1]octan-8-yl)-5-fluoro-7-(((2R,7aS)-2-fluorotetrahydro-1H-pyrrolizin-7a(5H)-yl)methoxy)-2-methyl-2H-pyrazolo[4,3-f]quinazolin-4-yl)-5-ethynyl-6-fluoronaphthalen-2-amine

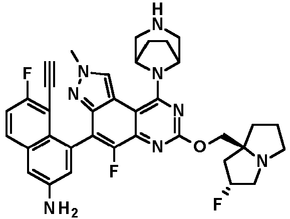

The compound is synthesized in the same manner as in the synthetic method of EXAMPLE 80.

### EXAMPLE 117. Synthesis of 4-(9-((1R,5S)-3,8-diazabicyclo[3.2.1]octan-3-yl)-5-fluoro-7-(((2R,7aS)-2-methoxytetrahydro-1H-pyrrolizin-7a(5H)-yl)methoxy)-2-methyl-2H-pyrazolo[4,3-f]quinazolin-4-yl)-5-ethynyl-6-fluoronaphthalen-2-ol.

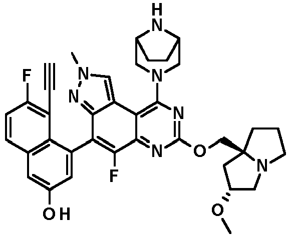

The compound is synthesized in the same manner as in the synthetic method of EXAMPLE 80.

### EXAMPLE 118. Synthesis of 9-((1R,5S)-3,8-diazabicyclo[3.2.1]octan-3-yl)-4-(5-ethynyl-6-fluoroisoquinolin-4-yl)-5-fluoro-7-(((2R,7aS)-2-fluorotetrahydro-1H-pyrrolizin-7a(5H)-yl)methoxy)-2-methyl-2H-pyrazolo[4,3-f]quinazoline

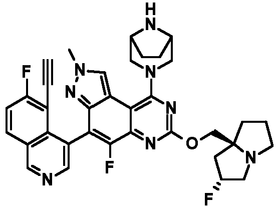

The compound is synthesized in the same manner as in the synthetic method of EXAMPLE 80.

### EXAMPLE 119. Synthesis of 4-(9-((1R,5S)-3,8-diazabicyclo[3.2.1]octan-3-yl)-7-(((R)-1-((dimethylamino)methyl)-2,2-difluorocyclopropyl)methoxy)-5-fluoro-2-methyl-2H-pyrazolo[4,3-f]quinazolin-4-yl)-5-ethynyl-6-fluoronaphthalen-2-ol.

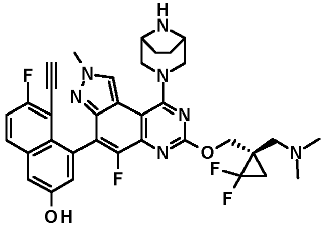

The compound is synthesized in the same manner as in the synthetic method of EXAMPLE 80.

### EXAMPLE 120. 4-(9-((1R,5S)-3,8-diazabicyclo[3.2.1]octan-3-yl)-7-(((1S,7a'S)-2,2-difluorodihydro-1'H,3'H-spiro[cyclopropane-1,2'-pyrrolizin]-7a'(5'H)-yl)methoxy)-5-fluoro-2-methyl-2H-pyrazolo[4,3-f]quinazolin-4-yl)-5-ethynyl-6-fluoronaphthalen-2-ol.

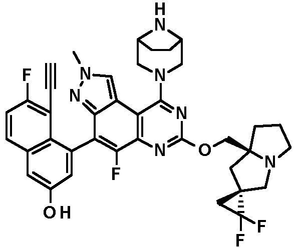

The compound is synthesized in the same manner as in the synthetic method of EXAMPLE 80.

### Test Example 1: Measurement of binding affinity to KRAS G12D mutant protein

The binding affinities of the compounds according to one embodiment of the present application to the KRAS G12D mutant protein were evaluated using differential scanning fluorimetry (DSF).

Differential scanning fluorimetry (DSF) is a method used to study the stability of a protein upon binding to a compound. The experiment can be performed using a real-time PCR (RT-PCR) instrument, and is commonly employed as a screening technique for small-molecule compounds targeting specific proteins. As the temperature increases, folded proteins begin to unfold, during which a hydrophobic dye such as SYPRO Orange binds to the exposed hydrophobic regions of the protein, resulting in a measurable increase in fluorescence intensity (maximum absorption at 470 nm and maximum emission at 570 nm).Protein-compound binding is determined by the difference in the melting temperature (Tm) of the protein in the presence of the compound compared to the Tm in the absence of the compound, expressed as **Δ**Tm.

The binding affinity to the KRAS G12D mutant protein was measured using the Protein Thermal Shift Dye kit (#4461146, Applied Biosystems). The GDP-KRAS G12D mutant protein and GppNHp-KRAS G12D mutant protein used in the experiment were recombinant proteins synthesized by the Daegu Gyeongbuk Medical Innovation Foundation (DGMIF) upon request. The analysis of binding between the compound and the KRAS G12D mutant protein was performed according to the following reaction recipe: each test sample contained 4 µg of KRAS protein, 10 µM of the compound, and 2X SYPRO Orange dye. For the GDP-KRAS G12D protein samples, the buffer provided with the Protein Thermal Shift Dye kit was used, while for the GppNHp-KRAS G12D protein samples, 50 mM HEPES (pH 7.5) buffer was used.

For the protein-compound binding reaction, the compound was added to the KRAS G12D mutant protein and reacted at room temperature for 30 minutes, followed by the addition of 2X SYPRO Orange dye. The temperature was increased to 25°C-99°C (0.05°C/s) using QuantStudio1 RT-PCR instrument (Applied Biosystems), and fluorescence was measured using a ROX reporter with filters of 580 ± 10 nm (excitation)/623 ± 14 nm (emission).

The measurements obtained at a compound concentration of 10 µM were analyzed using the Protein Thermal Shift Software program (version v1.4, Thermo Fisher Scientific), and the **Δ**Tm (°C) values, which indicate the binding affinity of the compound to the KRAS G12D mutant protein, were calculated. The DSF measurement results for the compounds according to one embodiment of the present application with respect to the KRAS G12D mutant protein are shown in Table 2.

**[Table 2]**

| EXAMPLE | GDP-KRAS G12D (ΔTmD, °C) | GppNHp-KRAS G12D (ΔTmD, °C) |
|---|---|---|
| 1 | 10.05 | 4.27 |
| 2 | 9.91 | 5.61 |
| 3 | 6.05 | 1.90 |
| 4 | 1.90 | 0.57 |
| 5 | 4.27 | 1.60 |
| 6 | 1.01 | 1.31 |
| 7 | 8.57 | 3.24 |
| 8 | 9.02 | 3.39 |
| 9 | 8.87 | 3.39 |
| 10 | 9.00 | 2.79 |
| 11 | 9.74 | 2.49 |
| 12 | 9.61 | 2.65 |
| 13 | 0.28 | -0.61 |
| 14 | 7.98 | 4.73 |
| 15 | -0.01 | 0.28 |
| 16 | 9.01 | 4.27 |
| 17 | 9.45 | 4.71 |
| 18 | 2.35 | 1.01 |
| 19 | 7.68 | 3.82 |
| 20 | -0.17 | 0.27 |
| 21 | 8.13 | 4.72 |
| 22 | 4.28 | 1.02 |
| 23 | 9.90 | 6.94 |
| 24 | 5.48 | 2.07 |
| 25 | 0.27 | -0.17 |
| 26 | 4.12 | 1.01 |
| 27 | 4.27 | 0.71 |
| 28 | 3.53 | 0.27 |
| 29 | 12.01 | 6.82 |
| 30 | 8.12 | 2.81 |
| 31 | 7.82 | 2.22 |
| 32 | 9.05 | 4.04 |
| 33 | 7.42 | 3.59 |
| 34 | 0.13 | -0.32 |
| 35 | 8.91 | 2.68 |
| 36 | 7.57 | 3.42 |
| 37 | 11.53 | 5.76 |
| 38 | 9.61 | 6.65 |
| 39 | 8.87 | 5.61 |
| 40 | 5.95 | 0.61 |
| 41 | 5.46 | 0.87 |
| 42 | 10.96 | 5.75 |
| 43 | 9.30 | 4.27 |
| 44 | 14.33 | 9.89 |
| 45 | 11.82 | 7.68 |
| 46 | 11.68 | 6.49 |
| 47 | 2.65 | 1.90 |
| 48 | 8.41 | 6.78 |
| 49 | 1.92 | 0.73 |
| 50 | 0.58 | -0.31 |
| 51 | 4.73 | 1.32 |
| 52 | 0.30 | 0.00 |
| 53 | -0.30 | 0.15 |
| 54 | 2.95 | 1.61 |
| 55 | 3.24 | 1.91 |
| 56 | -0.75 | -2.53 |
| 57 | 8.57 | 5.76 |
| 58 | 6.81 | 1.92 |
| 59 | 12.88 | 6.06 |
| 60 | 0.87 | 1.61 |
| 61 | 3.54 | 0.58 |
| 62 | 9.01 | 8.57 |
| 63 | 8.72 | 9.02 |
| 64 | 15.24 | 11.39 |
| 65 | 14.23 | 8.60 |
| 66 | 14.49 | 9.45 |
| 67 | 10.06 | 8.28 |
| 68 | 5.75 | 1.90 |
| 69 | 11.68 | 7.24 |
| 70 | 6.34 | 3.38 |
| 71 | 8.14 | 4.88 |
| 72 | -0.29 | 0.00 |
| 73 | 11.97 | 5.60 |
| 74 | 7.82 | 5.30 |
| 75 | 13.74 | 9.60 |
| 76 | 5.17 | 1.47 |
| 77 | 1.76 | 2.35 |
| 78 | 5.46 | 1.17 |
| 79 | 10.98 | 7.13 |
| 80 | 14.81 | 11.13 |
| 81 | 10.04 | 6.66 |
| 82 | 7.52 | 3.26 |
| 83 | 10.06 | 5.76 |
| 84 | 8.13 | 4.13 |
| 85 | 15.24 | 12.43 |
| 86 | 13.07 | 10.69 |
| 87 | 14.94 | 8.86 |
| 88 | 6.35 | 5.31 |
| 89 | 2.80 | 0.42 |
| 90 | 16.27 | 11.38 |
| 91 | -0.16 | -0.45 |
| 92 | 17.61 | 13.02 |
| 93 | 14.20 | 8.72 |
| 94 | 17.46 | 12.28 |
| 95 | 11.83 | 7.54 |
| 96 | 15.09 | 10.64 |
| 97 | 14.20 | 8.42 |
| 98 | 11.43 | 5.79 |
| 99 | 11.13 | 7.27 |
| 100 | 14.50 | 8.92 |
| 101 | 7.99 | 2.55 |
| 103 | 14.80 | 11.59 |
| 106 | 10.21 | 6.65 |
| 107 | 8.91 | 4.32 |
| 108 | 8.47 | 5.36 |
| 109 | 10.51 | 4.28 |

As shown in Table 2, it was verified that the compounds according to the present application exhibited high binding ability to GDP-/GppNHp-KRAS G12D mutant protein.

### Test Example 2: Measurement of inhibitory activity against ERK phosphorylation (pERK) Induced by KRAS G12D mutation (Immunofluorescence Assay)

Immunofluorescence is a method used to detect specific substances within cells or tissues through antigen-antibody interactions, and allows for the identification of the presence or absence of specific proteins by using antibodies labeled with various fluorescent dyes. After a primary antibody that recognizes a specific protein selectively binds to the epitope, a fluorescent antibody (secondary antibody) is bound to the primary antibody.

It was confirmed as follows that the compounds according to an embodiment of the present application inhibit phosphorylation of extracellular signal-regulated kinases (Phospho-ERK, pERK) induced by KRAS G12D mutation.

AsPC-1 cells were seeded at a density of 10,000 cells/well in RPMI-1640 medium containing 10% FBS and 1% Penicillin-Streptomycin in 384-well plates (Perkinelmer, #6057300) and allowed to attach for 16 h. After the cells attached to the plate, the cells were treated with drugs at a maximum concentration of 10 µM with a 3-fold serial dilution. After 3 h of drug treatment, the medium was removed and the cells were treated with 50 µL of 4% formaldehyde (Biosesang, #PC2031-050-00) for 20 min and washed three times with 50 µL of phosphate buffered saline (PBS).

50 µL of PBS containing 0.3% Triton X-100 (Sigma, #T8787-250mL) was added and reacted for 20 minutes to allow antibody penetration into cells, and then the plate was washed three times with 50 µL of PBS. To block nonspecific binding of antibodies to the plate, 50 µL of PBS containing 2% BSA (sigma, #A7906-500G) and 0.01% Tween-20 (Sigma, #P1379-500mL) (hereinafter referred to as PBS-T) was added, and reacted at room temperature for 2 hours.

To detect pERK, the primary antibody (cell signaling, #9101) was diluted 1:500 in PBS-T containing 2% BSA, 50 µL was added, and the plate was stored in a refrigerator at 4°C for 16 hours to react, followed by washing three times with 50 µL of PBS-T. To visualize the primary antibody, the secondary antibody, i.e., anti-rabbit-Alexa Fluor 488 (ThermoFisher, #A11008), was diluted 1:1000 in PBS-T containing 2% BSA, 50 µL was added, and after reaction at room temperature for 1 hour, the plate was washed three times with 50 µL of PBS-T. Next, to visualize the nuclei of cells, DAPI (Thermofisher, #62248, 1 mg/mL) was diluted 1:1000 in PBS-T, 50 µL each was added, reacted at room temperature for 10 minutes, and washed three times with 50 µL of PBS-T. 50 µL of PBS-T was added to each well, and the plate was read for fluorescence value using HCS equipment (MOLECULAR DEVICES / ImageXpress Micro).

Data analysis was performed by normalizing the pERK (Thr202/Tyr204) signal to the DAPI signal and calculating the percentage relative to the DMSO as a control. The IC₅₀ value was determined by fitting a four-parameter dose-response curve using the GraphPad Prism software (version 8.4.3), and interpolating the concentration at which 50% inhibition occurred. The results of calculating the inhibitory efficacy of the compounds according to one embodiment of the present application against KRAS G12D mutation-induced pERK activity are shown in Table 3.

**[Table 3]**

| EXAMPLE | pERK IC₅₀ (nM) |
|---|---|
| 1 | 322 |
| 2 | 169 |
| 3 | 1419 |
| 5 | 953 |
| 6 | 2323 |
| 7 | 269 |
| 8 | 149 |
| 9 | 165 |
| 10 | 2104 |
| 11 | 174 |
| 12 | 1064 |
| 14 | 75 |
| 16 | 176 |
| 17 | 1169 |
| 21 | 196 |
| 23 | 25 |
| 29 | 15 |
| 37 | 12 |
| 38 | 15 |
| 39 | 76 |
| 41 | 899 |
| 42 | 64 |
| 43 | 953 |
| 44 | 12 |
| 45 | 70 |
| 46 | 45 |
| 47 | 7980 |
| 48 | 333 |
| 57 | 167 |
| 59 | 54 |
| 62 | 631 |
| 63 | 532 |
| 64 | 62 |
| 65 | 4 |
| 68 | 9016 |
| 69 | 169 |
| 70 | 735 |
| 71 | 512 |
| 73 | 49 |
| 74 | 143 |
| 75 | 20 |
| 79 | 22 |
| 80 | 35 |
| 81 | 81 |
| 83 | 100 |
| 85 | 7 |
| 86 | 29 |
| 87 | 4 |
| 88 | 484 |
| 90 | 5 |
| 93 | 16 |
| 94 | 18 |
| 95 | 82 |
| 96 | 20 |

As shown in Table 3, it was verified that the compounds according to one embodiment of the present application exhibited high inhibitory ability against pERK induced by KRAS G12D mutation.

### Test Example 3: Cell Viability Assay using KRAS G12D mutant cells

The KRAS G12D mutant pancreatic cancer cell line AsPC-1 was obtained from the Korean Cell Line Bank (KCLB) and maintained in 100-mm dishes or T75 (75 cm²) flasks using RPMI1640 + 10% fetal bovine serum (FBS) + 1% Penicillin-Streptomycin medium. The cells were cultured at 37°C with 5% CO₂. The number of viable cells was counted using Countess (Invitrogen).

When AsPC-1 cells reached 85-100% confluence in the flask, the cells were harvested using 1X trypsin/EDTA solution, and seeded into a 96-well plate for 3D culture at 1000 cells/100 µL/well. The next day, the compounds were diluted 1/5 with DMSO from 10 µM or 50 µM to 0.0001 µM or 0.0006 µM and treated 1:1 (v/v) in the plate where the cells were seeded.

Cell culture was terminated on the 5th day after compound treatment, and cell viability at each concentration was measured using the celltiter-glo 3D cell viability assay kit.

Cell viability assays were normalized to 100% for the DMSO-treated group and expressed as a percentage of the number of viable cells when treated with the compound. Cell viability % = (average RLU of group treated with test substance - average RLU of untreated group) / (average RLU of group treated with DMSO - average RLU of untreated group) X 100

The data were modeled using GraphPad Prism 9 (GraphPad Software) by applying a nonlinear regression curve fit with a four-parameter sigmoidal dose-response curve to determine cell viability as a function of compound concentration. The 50% inhibitory concentration (IC₅₀) corresponds to the concentration at which the curve intersects the midpoint of cell viability. The results of the inhibitory effect of the compounds according to one embodiment of the present application on the viability of KRAS G12D mutant cells are presented in Table 4.

**[Table 4]**

| EXAMPLE | AsPC-1 IC₅₀ (nM) |
|---|---|
| 1 | 364 |
| 2 | 226 |
| 7 | 109 |
| 8 | 50 |
| 9 | 50 |
| 11 | 140 |
| 12 | 975 |
| 14 | 53 |
| 16 | 94 |
| 23 | 24 |
| 29 | 19 |
| 37 | 29 |
| 38 | 12 |
| 39 | 91 |
| 41 | 1020 |
| 42 | 105 |
| 44 | 7 |
| 45 | 37 |
| 46 | 25 |
| 48 | 136 |
| 57 | 101 |
| 59 | 69 |
| 65 | 5 |
| 69 | 190 |
| 73 | 37 |
| 74 | 110 |
| 75 | 34 |
| 79 | 29 |
| 80 | 46 |
| 81 | 280 |
| 85 | 11 |
| 86 | 48 |
| 87 | 11 |
| 88 | 734 |
| 90 | 4 |
| 93 | 38 |
| 94 | 14 |
| 95 | 263 |
| 96 | 11 |
| 97 | 7 |
| 98 | 110 |
| 99 | 27 |
| 100 | 29 |

As shown in Table 4, the compounds according to one embodiment of the present application were demonstrated to suppress the viability of KRAS G12D mutant cells.

These results indicate that the compounds according to one embodiment of the present application can effectively inhibit the survival of cancer cells driven by the KRAS G12D mutation and can be advantageously used as a pharmaceutical composition for the prevention or treatment of cancer.

## Claims

1. A compound represented by the following Chemical Formula 1, or an optical isomer, stereoisomer, racemate, isotopic variant, solvate, hydrate, or a pharmaceutically acceptable salt thereof: 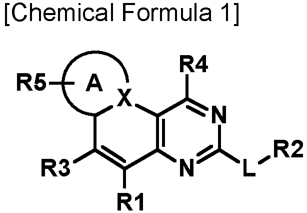 wherein,
A is 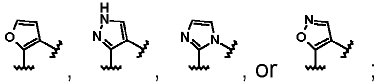
X is C or N;
R₁ is hydrogen, halogen, C₁₋₃ alkyl, or C₁₋₃ alkoxy;
L is a direct bond, O, or NR₆;
R₂ is C₁₋₆ alkyl, C₁₋₆ alkoxy, C₁₋₆ hydroxyalkyl, C₂₋₆ alkoxyalkyl, C₁₋₆ haloalkyl, Rₓ, - Z₁-Rₓ, -Z₁-R_{y}-Rₓ, -Z₁-R_{y}-Z₂-N(R₁₅)₂, -Z₁-R_{y}-Z₂-Rₓ, -N(R₁₅)₂, -Z₁-N(R₁₅)₂, -Z₁-C(O)N(R₁₅)₂, or -Z₁-OR₁₅;
Rₓ and R_{y} are each independently 3 to 10-membered cycloalkyl, 3 to 10-membered heterocyclyl, 6 to 20-membered aryl, or 6 to 20-membered heteroaryl, and may optionally be substituted with one or more of R₇;
R₇ is independently H, halogen, hydroxy, C₁₋₄ alkyl, C₂₋₄ alkenyl, C₂₋₄ alkynyl, C₁₋₄ hydroxyalkyl, C₁₋₄ haloalkyl, C₂₋₄ haloalkenyl, C₂₋₄ haloalkynyl, C₁₋₄ alkoxy, C₁₋₄ alkyl-N(R₁₆)₂, =C-(R₁₆)₂, cyano, C(O)R₁₆, C(=O)OR₁₆, C(=O)N(R₁₆)₂, -NHC(O)-6 to 20-membered aryl, -N(R₁₆)₂, (C₁₋₄ alkoxy)C₁₋₄ alkyl-, oxo, -OR₁₆, -SR₁₆, -(C₁₋₄alkyl)C(O)R₁₆, 3 to 10-membered cycloalkyl, 3 to 10-membered heterocycloalkyl, 6 to 20-membered aryl, 6 to 20-membered heteroaryl, -Z₃-3 to 10-membered cycloalkyl, -Z₃-3 to 10-membered heterocycloalkyl, -Z₃-6 to 20-membered aryl, -Z₃-6 to 20-membered heteroaryl, -Z₃-OC(O)N(R₁₆)₂, or -Z₃-OC(O)-3 to 10-membered heterocyclyl; and R₇ may be substituted with 1 to 3 of cyano, halogen, haloalkyl, amino, -OR₁₇, -SR₁₇, or -N(R₁₇)₂;
Z₁ to Z₃ are each independently C₁₋₄ alkyl; and optinally may be substituted with hydroxy, C₁₋₄ hydroxyalkyl, or 6 to 20-membered heteroaryl;
R₆, R₁₅, R₁₆, R₁₇, R₁₈, R₁₉, R₂₀ and R₂₁ are each independently H or C₁₋₃ alkyl;
R₃ is 3 to 10-membered cycloalkyl, 3 to 10-membered heterocycloalkyl, 6 to 20-membered aryl, or 6 to 20-membered heteroaryl, and may optionally be substituted with one or more of R₈;
R₈ is H, halogen, hydroxy, -N(R₁₈)₂, OR₁₈, SH, S(C₁₋₃ alkyl), S(=O)(C₁₋₆ alkyl), S(=O)₂(C₁₋₆ alkyl), C(=O)(C₁₋₆ alkyl), C(=O)OH, C(=O)N(R₁₈)₂, C₁₋₄ alkyl, C₂₋₄ alkenyl, C₂₋₄ alkynyl, C₁₋₃ haloalkyl, C₁₋₃ hydroxyalkyl, C₁₋₃ alkoxyalkyl, C₁₋₄ alkyl-N(R₁₈)₂, C₁₋₃ alkoxy, C₁₋₃ haloalkoxy, cyanoalkyl, cyano, oxo, 3 to 10-membered cycloalkyl, 3 to 10-membered heterocycloalkyl, 6 to 20-membered aryl, or 6 to 20-membered heteroaryl;
R₄ is 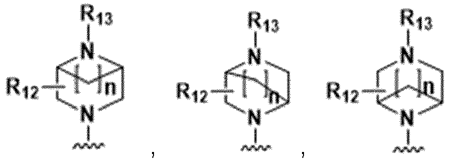 or -NH-R₉-R₁₀;
R₉ is a direct bond or C₁₋₄ alkyl;
R₁₀ is 3 to 10-membered cycloalkyl or 3 to 10-membered heterocycloalkyl, and may optionally be substituted with one or more of R₁₁;
R₁₁ is C₁₋₃ alkyl, hydroxy, N(R₁₉)₂, C₁₋₃ alkyl-N(R₁₉)₂, C₁₋₃ cyanoalkyl, or 3 to 10-membered heterocyclyl;
R₁₂ is H, C₁₋₃ alkyl, OH, -N(R₂₀)₂, -CH₂N(R₂₀)₂, cyano, cyanomethyl, or 3 to 10-membered heterocyclyl;
R₁₃ is H, or -C(=O)R₁₄;
R₁₄ is C₁₋₄ alkyl, C₂₋₄ alkenyl, C₂₋₄ alkynyl, -N(R₆)₂, -OR₆, -SR₆, 3 to 10-membered cycloalkyl, 3 to 10-membered cycloalkenyl, 3 to 10-membered cycloalkynyl, 3 to 10-membered heterocyclyl, 6 to 20-membered aryl, or 6 to 20-membered heteroaryl;
n is an integer of 1 to 4; and
R₅ is H, halogen, C₁₋₆ alkyl, C₁₋₃ halo alkyl, 3 to 6-membered cycloalkyl, 3 to 6-membered heterocycloalkyl, cyano, cyano C₁₋₃ alkyl, hydroxy, C₁₋₃ hydroxyalkyl, C(O)(NR₂₁)₂, 6 to 20-membered aryl, C₁₋₃ alkyl-3 to 6-membered cycloalkyl, C₁₋₃ alkyl-3 to 6-membered heterocycloalkyl, C₁₋₃ alkyl-6 to 20-membered aryl, or C₁₋₃ alkyl-6 to 20-membered heteroaryl.

2. The compound, or the optical isomer, stereoisomer, racemate, isotopic variant, solvate, hydrate, or the pharmaceutically acceptable salt thereof according to claim 1,
wherein A is

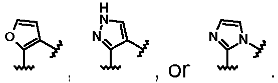

3. The compound, or the optical isomer, stereoisomer, racemate, isotopic variant, solvate, hydrate, or the pharmaceutically acceptable salt thereof according to claim 1,
wherein R₁ is hydrogen, halogen, or C₁₋₃ alkyl.

4. The compound, or the optical isomer, stereoisomer, racemate, isotopic variant, solvate, hydrate, or the pharmaceutically acceptable salt thereof according to claim 1,
wherein L is a direct bond or O.

5. The compound, or the optical isomer, stereoisomer, racemate, isotopic variant, solvate, hydrate, or the pharmaceutically acceptable salt thereof according to claim 1,
wherein Rₓ and R_{y} are each independently 3 to 10-membered cycloalkyl or 3 to 10-membered heterocyclyl, and may optionally be substituted with one or more of R₇.

6. The compound, or the optical isomer, stereoisomer, racemate, isotopic variant, solvate, hydrate, or the pharmaceutically acceptable salt thereof according to claim 1,
wherein Rₓ is 3 to 10-membered heterocyclyl, R_{y} is 3 to 10-membered cycloalkyl, and Rₓ and R_{y} may optionally be substituted with one or more of R₇.

7. The compound, or the optical isomer, stereoisomer, racemate, isotopic variant, solvate, hydrate, or the pharmaceutically acceptable salt thereof according to claim 1,
wherein R₇ is independently H, halogen, hydroxy, C₁₋₄ alkyl, C₂₋₄ alkenyl, C₂₋₄ alkynyl, C₂₋₄ hydroxyalkyl, C₁₋₄ haloalkyl, C₂₋₄ haloalkenyl, C₁₋₄ alkoxy, C₁₋₄ alkyl-N(R₁₆)₂, =C-(R₁₆)₂, C(O)R₁₆, C(=O)OR₁₆, C(=O)N(R₁₆)₂, -NHC(O)aryl, -N(R₁₆)₂, (C₁₋₄ alkoxy)C₁₋₄ alkyl-, oxo, -OR₁₆, -SR₁₆, -(C₁₋₄ alkyl)C(O)R₁₆, 3 to 10-membered cycloalkyl, 3 to 10-membered heterocycloalkyl, 6 to 20-membered aryl, 6 to 20-membered heteroaryl, -Z₃-3 to 10-membered cycloalkyl, -Z₃-3 to 10-membered heterocycloalkyl, -Z₃-6 to 20-membered aryl, -Z₃-6 to 20-membered heteroaryl, -Z₃-OC(O)N(R₁₆)₂, or -Z₃-OC(O)-3 to 10-membered heterocyclyl; and may be substituted with 1 to 3 of cyano, halogen, haloalkyl, amino, -OR₁₇, -SR₁₇, or -N(R₁₇)₂.

8. The compound, or the optical isomer, stereoisomer, racemate, isotopic variant, solvate, hydrate, or the pharmaceutically acceptable salt thereof according to claim 1,
wherein R₃ is 6 to 20-membered aryl, or 6 to 20-membered heteroaryl, and may optionally be substituted with one or more of R₈; and
wherein R₈ is H, halogen, hydroxy, -N(R₁₈)₂, OR₆, SH, S(C₁₋₃ alkyl), S(=O)(C₁₋₆ alkyl), S(=O)₂(C₁₋₆ alkyl), C(=O)(C₁₋₆ alkyl), C(=O)OH, C(=O)N(R₁₈)₂, C₁₋₄ alkyl, C₂₋₄ alkenyl, C₂₋₄ alkynyl, C₁₋₃ haloalkyl, C₁₋₃ hydroxyalkyl, C₁₋₃ alkoxyalkyl, C₁₋₄ alkyl-N(R₁₈)₂, cyano, oxo, or 3 to 10-membered cycloalkyl.

9. The compound, or the optical isomer, stereoisomer, racemate, isotopic variant, solvate, hydrate, or the pharmaceutically acceptable salt thereof according to claim 1,
wherein R₁₀ is 3 to 10-membered cycloalkyl, and may optionally be substituted with one or more of R₁₁; and
wherein R₁₁ is C₁₋₃ alkyl, hydroxy, N(R₁₉)₂, C₁₋₃ alkyl-N(R₁₉)₂, or cyano C₁₋₃ alkyl.

10. The compound, or the optical isomer, stereoisomer, racemate, isotopic variant, solvate, hydrate, or the pharmaceutically acceptable salt thereof according to claim 8,
wherein R₃ is phenyl, biphenyl, naphthyl, toluyl, naphthalenyl, pyridinyl, anthracenyl, indenyl, indanyl, quinolinyl, isoquinolinyl, benzimidazolyl, benzothiazolyl, benzothiophenyl, benzofuranyl, or indazoly, and may optionally be substituted with one or more of R₈.

11. The compound, or the optical isomer, stereoisomer, racemate, isotopic variant, solvate, hydrate, or the pharmaceutically acceptable salt thereof according to claim 8,
wherein R₃ is phenyl, naphthyl, naphthalenyl, pyridinyl, quinolinyl, isoquinolinyl, benzothiazolyl, benzothiophenyl, benzofuranyl, or indazolyl, and may optionally be substituted with one or more of R₈, and
wherein R₈ is H, halogen, hydroxy, -N(R₁₈)₂, C₁₋₄ alkyl, C₂₋₄ alkenyl, C₂₋₄ alkynyl, C₁₋₃ haloalkyl, C₁₋₃ hydroxyalkyl, or cyano.

12. The compound, or the optical isomer, stereoisomer, racemate, isotopic variant, solvate, hydrate, or the pharmaceutically acceptable salt thereof according to claim 1,
wherein R₉ is C₁₋₃ alkyl,
wherein R₁₀ is 3 to 5-membered cycloalkyl, and
wherein R₁₁ is N(R₁₉)₂.

13. The compound, or the optical isomer, stereoisomer, racemate, isotopic variant, solvate, hydrate, or the pharmaceutically acceptable salt thereof according to claim 1,
wherein R₁₂ is H.

14. The compound, or the optical isomer, stereoisomer, racemate, isotopic variant, solvate, hydrate, or the pharmaceutically acceptable salt thereof according to claim 1,
wherein R₅ is H or C₁₋₃ alkyl.

15. The compound, or the optical isomer, stereoisomer, racemate, isotopic variant, solvate, hydrate, or the pharmaceutically acceptable salt thereof according to claim 5,
wherein Rₓ and R_{y} are each independently 3 to 5-membered cycloalkyl, azetidinyl, pyrrolidinyl, piperidinyl, morpholinyl, 2-oxa-5-azabicyclo[2.2.1]heptanyl, pyrrolizidinyl, methylenepyrrolizidinyl, tetrahydro-1'*H*,3'*H*-spiro[cyclopropane-1,2'-pyrrolizidinyl], 6-azaspiro[2.5]octanyl, quinolizidinyl, indolinyl, benzimidazolyl, azaspirooctanyl, benztriazolyl, thioxanthinyl, carbazolyl, carbolinyl, or acridinyl, and may optionally be substituted with one or more of R₇.

16. The compound, or the optical isomer, stereoisomer, racemate, isotopic variant, solvate, hydrate, or the pharmaceutically acceptable salt thereof according to claim 6,
wherein R₂ is Rₓ, -Z1-Rₓ, -Z1-R_{y}-Rₓ, -Z₁-R_{y}-Z₂-N(R₁₅)₂, or -Z₁-R_{y}-Z₂-Rₓ,
wherein Rₓ is azetidinyl, pyrrolidinyl, piperidinyl, morpholinyl, 2-oxa-5-azabicyclo[2.2.1]heptanyl, pyrrolizidinyl, methylenepyrrolizidinyl, tetrahydro-1'*H*,3'*H-*spiro[cyclopropane-1,2'-pyrrolizidinyl], or 6-azaspiro[2.5]octanyl,
wherein R_{y} is 3 to 5-membered cycloalkyl,
wherein Rₓ and R_{y} may optionally be substituted with one or more of R₇, and
wherein R₇ is independently H, halogen, C₁₋₄ alkyl, C₂₋₄ alkenyl, C₂₋₄ alkynyl, C₁₋₄ haloalkyl, C₂₋₄ haloalkenyl, C₂₋₄ haloalkynyl, C₁₋₄ alkoxy, =C-(R₁₆)₂, -N(R₁₆)₂, or (C₁₋₄ alkoxy)C₁₋₄ alkyl-, and may be substituted with 1 to 3 of halogen.

17. The compound, or the optical isomer, stereoisomer, racemate, isotopic variant, solvate, hydrate, or the pharmaceutically acceptable salt thereof according to claim 1,
wherein the compound of Chemical Formula 1 is selected from the group consisting of the following (1) to (120) compounds:
**[Table 5]**
| | |
|---|---|
| (1) | 4-(1-((1*R*,5*S*)-3,8-diazabicyclo[3.2.1]octan-3-yl)-3-(((2*R*,7a*S*)-2-fluorotetrahydro-1*H*-pyrrolizin-7a(5*H*)-yl)methoxy)imidazo[1',2':1,6]pyrido[3,2-*d*]pyrimidin-6-yl)-5-ethyl-6-fluoronaphthalen-2-ol |
| (2) | 4-(1-((1*R*,5*S*)-3,8-diazabicyclo[3.2.1]octan-3-yl)-3-(((2*R*,7a*S*)-2-fluorotetrahydro-1*H*-pyrrolizin-7a(5*H*)-yl)methoxy)imidazo[1',2':1,6]pyrido[3,2-*d*\|pyrimidin-6-yl)-5-ethynyl-6-fluoronaphthalen-2-ol |
| (3) | 4-(1-((1*R*,5*S*)-3,8-diazabicyclo[3.2.1]octan-3-yl)-3-(((2*R*,7a*S*)-2-fluorotetrahydro-1*H*-pyrrolizin-7a(5*H*)-yl)methoxy)im idazo[1',2':1,6]pyrido[3,2-*d*]pyrimidin-6-yl)-5,6-difluoronaphthalen-2-ol |
| (4) | 4 (1-(((1-(dimethylamino)cyclobutyl)methyl)amino)-3-(((2*R*,7a*S*)-2-fluorotetrahydro-1*H*-pyrrolizin-7a(5*H*)-yl)methoxy)im idazo[1',2':1,6]pyrido[3,2-*d*]pyrimidin-6-yl)-5,6-difluoronaphthalen-2-ol. |
| (5) | 4-(1-(((1-(dimethylamino)cyclobutyl)methyl)amino)-3-(((2*R*,7a*S*)-2-fluorotetrahydro-1*H*-pyrrolizin-7a(5*H*)-yl)methoxy)im idazo[1',2':1,6]pyrido[3,2-*d*\|pyrimidin-6-yl)-5-ethyl-6-fluoronaphthalen-2-ol. |
| (6) | 4-(1-(((1-(dimethylamino)cyclobutyl)methyl)amino)-3-(((2*R*,7a*S*)-2-fluorotetrahydro-1*H*-pyrrolizin-7a(5*H*)-yl)methoxy)im idazo[1',2':1,6]pyrido[3,2-*d*]pyrimidin-6-yl)-5-ethynyl-6-fluoronaphthalen-2-ol. |
| (7) | 4-(1-((1*R*,5*S*)-3,8-diazabicyclo[3.2.1]octan-3-yl)-3-((1-(morpholinomethyl)cyclopropyl)methoxy)imidazo[1',2':1,6]pyrido[3,2-*d*\|pyrimidin-6-yl)-5-ethynyl-6-fluoronaphthalen-2-ol. |
| (8) | 4-(1-((1*R*,5*S*)-3,8-diazabicyclo[3.2.1]octan-3-yl)-3-((1-(((*R*)-3-methylmorpholino)methyl)cyclopropyl)methoxy)imidazo[1',2':1,6]pyrido[3,2-*d*]pyrimidin-6-yl)-5-ethynyl-6-fluoronaphthalen-2-ol. |
| (9) | 4 (1-((1R,5S)-3,8-diazabicyclo[3.2.1]octan-3-yl)-3-((1-((1,1-difluoro-6-azaspiro[2.5]octan-6-yl)methyl)cyclopropyl)methoxy)imidazo[1',2':1,6]pyrido[3,2-*d*]pyrimidin-6-yl)-5-ethynyl-6-fluoronaphthalen-2-ol. |
| (10) | 4-(3-((1-(((1*R*,4*R*)-2-oxa-5-azabicyclo[2.2.1]heptan-5-yl)methyl)cyclopropyl)methoxy)-1-((1*R*,5*S*)-3,8-diazabicyclo[3.2.1]octan-3-yl)imidazo[1',2':1,6]pyrido[3,2-*d*]pyrimidin-6-yl)-5-ethynyl-6-fluoronaphthalen-2-ol. |
| (11) | 4-(1-((1*R*,5*S*)-3,8-diazabicyclo[3.2.1]octan-3-yl)-3-((1-(((*S*)-3-methylmorpholino)methyl)cyclopropyl)methoxy)imidazo[1',2':1,6]pyrido[3,2-*d*]pyrimidin-6-yl)-5-ethynyl-6-fluoronaphthalen-2-ol. |
| (12) | 4-(3-((1-(((1*S*,4*S*)-2-oxa-5-azabicyclo[2.2.1]heptan-5-yl)imidazo[1',2':1,6]pyrido[3,2-*d*]pyrimidin-6-yl)-5-ethynyl-6-fluoronaphthalen-2-ol. |
| (13) | 3-(1-((1*R*,5*S*)-3,8-diazabicyclo[3.2.1]octan-3-yl)-3-(((2*R*,7a*S*)-2-fluorotetrahydro-1*H*-pyrrolizin-7a(5*H*)-yl)methoxy)imidazo[1',2':1,6]pyrido[3,2-*d*]pyrimidin-6-yl)-2,4,5,6-tetrafluoroaniline |
| (14) | 4-(1-((1*R*,5*S*)-3,8-diazabicyclo[3.2.1]octan-8-yl)-3-(((2*R*,7a*S*)-2-fluorotetrahydro-1*H*-pyrrolizin-7a(5*H*)-yl)methoxy)imidazo[1',2':1,6]pyrido[3,2-*d*]pyrimidin-6-yl)-5-ethynyl-6-fluoronaphthalen-2-amine. |
| (15) | 4-(1-((1*R*,5*S*)-3,8-diazabicyclo[3.2.1]octan-8-yl)-3-(((2*R*,7a*S*)-2-fluorotetrahydro-1*H*-pyrrolizin-7a(5*H*)-yl)methoxy)imidazo[1',2':1,6]pyrido[3,2-*d*]pyrimidin-6-yl)-7-fluorobenzo[*d*]thiazol-2-amine. |
| (16) | 4-(1-((1*R*,5*S*)-3,8-diazabicyclo[3.2.1]octan-8-yl)-3-((1-(((*R*)-3-fluoropyrrolidin-1-yl)methyl)cyclopropyl)methoxy)imidazo[1',2':1,6]pyrido[3,2-*d*]pyrimidin-6-yl)-5-ethynyl-6-fluoronaphthalen-2-ol. |
| (17) | 4-(1-((1*R*,5*S*)-3,8-diazabicyclo[3.2.1]octan-8-yl)-3-((1-(pyrrolidin-1-ylmethyl)cyclopropyl)methoxy)imidazo[1',2':1,6]pyrido[3,2-*d*]pyrimidin-6-yl)-5-ethynyl-6-fluoronaphthalen-2-ol. |
| (18) | 1-((1*R*,5*S*)-3,8-diazabicyclo[3.2.1]octan-8-yl)-3-(((2*R*,7a*S*)-2-fluorotetrahydro-1*H*-pyrrolizin-7a(5*H*)-yl)methoxy)-6-(6-methyl-5-(trifluoromethyl)-1*H*-indazol-4-vl)imidazo[1',2':1,6]pyrido[3,2-*d*]pyrimidine. |
| (19) | 4-(1-((1*R*,5*S*)-3,8-diazabicyclo[3.2.1]octan-8-yl)-3-((1-(((*S*)-3-fluoropiperidin-1-yl)methyl)cyclopropyl)methoxy)imidazo[1',2':1,6]pyrido[3,2-d]pyrimidin-6-yl)-5-ethynyl-6-fluoronaphthalen-2-ol. |
| (20) | 6-(1-((1*R*,5*S*)-3,8-diazabicyclo[3.2.1]octan-8-yl)-3-(((2*R*,7a*S*)-2-fluorotetrahydro-1*H*-pyrrolizin-7a(5*H*)-yl)methoxy)im idazo[1',2':1,6]pyrido[3,2-*d*]pyrimidin-6-yl)-4-methyl-5-(trifluoromethyl)pyridin-2-amine. |
| (21) | 4-(1-((1*R*,5*S*)-3,8-diazabicyclo[3.2.1]octan-8-yl)-3-(((R)-2-methylenetetrahydro-1*H*-pyrrolizin-7a(5*H*)-yl)methoxy)imidazo[1',2'-1,6]pyrido[3,2-*d*]pyrimidin-6-yl)-5-ethynyl-6-fluoronaphthalen-2-ol. |
| (22) | 1-((1*R*,5*S*)-3,8-diazabicyclo[3.2.1]octan-8-yl)-6-(8-ethynyl-7-fluoronaphthalen-1-yl)-3-(((*S*,*Z*)-2-(fluoromethylene)tetrahydro-1*H*-pyrrolizin-7a(5*H*)-yl)methoxy)imidazo[1',2':1,6]pyrido[3,2-*d*]pyrimidine. |
| (23) | 4-(1-((1*R*,5*S*)-3,8-diazabicyclo[3.2.1]octan-8-yl)-3-(((*S*,*Z*)-2-(fluoromethylene)tetrahydro-1*H*-pyrrolizin-7a(5*H*)-yl)methoxy)imidazo[1',2':1,6]pyrido[3,2-*d*]pyrimidin-6-yl)-5-ethynyl-6-fluoronaphthalen-2-ol. |
| (24) | 4-(1-((1*R*,5*S*)-3,8-diazabicyclo[3.2.1]octan-3-yl)-3-(((2*R*,7a*S*)-2-fluorotetrahydro-1*H*-pyrrolizin-7a(5*H*)-yl)methoxy)imidazo[1',2':1,6]pyrido[3,2-*d*]pyrimidin-6-yl)-5-ethynyl-6-fluoronaphthalen-2-amine. |
| (25) | fluorotetrahydro-1*H*-pyrrolizin-7a(5*H*)-yl)methoxy)imidazo[1',2':1,6]pyridor3,2-*d*]pyrimidin-6-yl)-7-fluorobenzo[*d*]thiazol-2-amine. |
| (26) | 1-((1*R*,5*S*)-3,8-diazabicyclo[3.2.1]octan-3-yl)-3-(((2*R*,7a*S*)-2-fluorotetrahydro-1*H*-pyrrolizin-7a(5*H*)-yl)methoxy)-6-(6-methyl-5-(trifluoromethyl)-1*H*-indazol-4-yl)imidazo[1',2':1,6]pyrido[3,2-*d*]pyrimidine. |
| (27) | 1-((1*R*,5*S*)-3,8-diazabicyclo[3.2.1]octan-3-yl)-6-(8-ethynyl-7-fluoronaphthalen-1-yl)-3-(((*S*,*Z*)-2-(fluoromethylene)tetrahydro-1*H*-pyrrolizin-7a(5*H*)-yl)methoxy)imidazo[1',2':1,6]pyrido[3,2-*d*]pyrimidine. |
| (28) | 1-((1*R*,5*S*)-3,8-diazabicyclo[3.2.1]octan-3-yl)-6-(8-ethynyl-7-fluoronaphthalen-1-yl)-3-(((2*R*,7a*S*)-2-fluorotetrahydro-1*H*-pyrrolizin-7a(5*H*)-yl)methoxy)imidazo[1',2":1,6]pyrido[3,2-*d*]\|pyrimidine. |
| (29) | 4-(1-((1*R*,5*S*)-3,8-diazabicyclo[3.2.1]octan-3-yl)-3-(((*S*,*Z*)-2-(fluoromethylene)tetrahydro-1*H*-pyrrolizin-7a(5*H*)-yl)methoxy)imidazo[1',2':1,6]pyrido[3,2-*d*]pyrimidin-6-yl)-5-ethynyl-6-fluoronaphthalen-2-ol. |
| (30) | 4-(1-((1*R*,5*S*)-3,8-diazabicyclo[3.2.1]octan-3-yl)-3-((1-(((*R*)-3-fluoropyrrolidin-1-yl)methyl)cyclopropyl)methoxy)imidazo[1',2':1,6]pyrido[3,2-*d*]pyrimidin-6-yl)-5-ethynyl-6-fluoronaphthalen-2-ol. |
| (31) | 4-(1-((1*R*,5*S*)-3,8-diazabicyclo[3.2.1]octan-3-yl)-3-((1-(((*S*)-3-fluoropiperidin-1-yl)methyl)cyclopropyl)methoxy)imidazo[1',2':1,6]pyrido[3,2-*d*]pyrimidin-6-yl)-5-ethynyl-6-fluoronaphthalen-2-ol. |
| (32) | 4-(1-((1*R*,5*S*)-3,8-diazabicyclo[3.2.1]octan-3-yl)-3-(((2*S*,7a*R*)-2-fluoro-6-methylenetetrahydro-1*H*-pyrrolizin-7a(5*H*)-yl)methoxy)imidazo[1',2':1,6]pyrido[3,2-*d*]pyrimidin-6-yl)-5-ethynyl-6-fluoronaphthalen-2-ol. |
| (33) | 4-(1-((1*R*,5*S*)-3,8-diazabicyclo[3.2.1]octan-3-yl)-3-(((*R*)-2-methylenetetrahydro-1*H*-pyrrolizin-7a(5*H*)-yl)methoxy)imidazo[1',2':1,6]pyrido[3,2-*d*]pyrimidin-6-yl)-5-ethynyl-6-fluoronaphthalen-2-ol. |
| (34) | 6-(1-((1*R*,5*S*)-3,8-diazabicyclo[3.2.1]octan-3-yl)-3-(((2*R*,7a*S*)-2-fluorotetrahydro-1*H*-pyrrolizin-7a(5*H*)-yl)methoxy)im idazo[1',2':1,6]pyrido[3,2-*d*]pyrimidin-6-yl)-4-methyl-5-(trifluoromethyl)pyridin-2-amine. |
| (35) | 4-(1-((1*R*,5*S*)-3,8-diazabicyclo[3.2.1]octan-3-yl)-3-((1-(pyrrolidin-1-ylmethyl)cyclopropyl)methoxy)imidazo[1',2':1,6]pyrido[3,2-*d*]pyrimidin-6-yl)-5-ethynyl-6-fluoronaphthalen-2-ol. |
| (36) | 4-(1-((1*R*,5*S*)-3,8-diazabicyclo[3.2.1]octan-3-yl)-3-(((*S*,*Z*)-2-(fluoromethylene)tetrahydro-1*H*-pyrrolizin-7a(5*H*)-yl)methoxy)imidazo[1',2':1,6]pyrido[3,2-*d*]pyrimidin-6-yl)-5-ethynyl-6-fluoronaphthalen-2-amine. |
| (37) | 4-(1-((1*R*,5*S*)-3,8-diazabicyclo[3.2.1]octan-3-yl)-3-(((*S*)-2-yl)methoxy)imidazo[1',2'-1,6]pyrido[3,2-*d*]pyrimidin-6-yl)-5-ethynyl-6-fluoronaphthalen-2-ol. |
| (38) | 4-(1-((1*R*,5*S*)-3,8-diazabicyclo[3.2.1]octan-8-yl)-3-(((*S,Z*)-2-(fluoromethylene)tetrahydro-1*H*-pyrrolizin-7a(5*H*)-yl)methoxy)imidazo[1',2':1,6]pyrido[3,2-*d*]pyrimidin-6-yl)-5-ethynyl-6-fluoronaphthalen-2-amine. |
| (39) | 4-(1-((1*R*,5*S*)-3,8-diazabicyclo[3.2.1]octan-8-yl)-3-(((2*S*,7a*R*)-2-fluoro-6-methylenetetrahydro-1*H*-pyrrolizin-7a(5*H*)-yl)methoxy)imidazo[1',2':1,6]pyrido[3,2-*d*]pyrimidin-6-yl)-5-ethynyl-6-fluoronaphthalen-2-ol. |
| (40) | 4-(1-((1*R*,5*S*)-3,8-diazabicyclo[3.2.1]octan-3-yl)-3-((1-(((*R*)-3-fluoropyrrolidin-1-yl)methyl)cyclobutyl)methoxy)imidazo[1',2':1,6]pyrido[3,2-*d*]pyrimidin-6-yl)-5-ethynyl-6-fluoronaphthalen-2-ol. |
| (41) | 4-(1-((1*R*,5*S*)-3,8-diazabicyclo[3.2.1]octan-3-yl)-3-(((2*R*,7a*S*)-2-fluorotetrahydro-1*H*-pyrrolizin-7a(5*H*)-yl)methoxy)-8-methylfuro[3,2-*f*]quinazolin-6-yl)naphthalen-2-ol. |
| (42) | 4-(1-((1*R*,5*S*)-3,8-diazabicyclo[3.2.1]octan-3-yl)-3-(((2*R*,7a*S*)-2-fluorotetrahydro-1*H*-pyrrolizin-7a(5*H*)-yl)methoxy)-8-methylfuro[3,2-*f*]quinazolin-6-yl)-5-ethynyl-6-fluoronaphthalen-2-ol. |
| (43) | 4-(1-((1*R*,5*S*)-3,8-diazabicyclo[3.2.1]octan-3-yl)-5-fluoro-3-(((2*R*,7a*S*)-2-fluorotetrahydro-1*H*-pyrrolizin-7a(5*H*)-yl)methoxy)-8-methylfuro[3,2-*f*]quinazolin-6-yl)naphthalen-2-ol. |
| (44) | 4-(1-((1*R*,5*S*)-3,8-diazabicyclo[3.2.1]octan-3-yl)-5-fluoro-3-(((2*R*,7a*S*)-2-fluorotetrahydro-1*H*-pyrrolizin-7a(5*H*)-yl)methoxy)-8-methylfuro[3,2-*f*]quinazolin-6-yl)-5-ethynyl-6-fluoronaphthalen-2-ol. |
| (45) | 4-(1-((1*R*,5*S*)-3,8-diazabicyclo[3.2.1]octan-3-yl)-5-fluoro-3-(((2*R*,7a*S*)-2-fluorotetrahydro-1*H*-pyrrolizin-7a(5*H*)-yl)methoxy)-8-methylfuro[3,2-*f*]quinazolin-6-yl)-5-ethyl-6-fluoronaphthalen-2-ol. |
| (46) | 4-(1-((1*R*,5*S*)-3,8-diazabicyclo[3.2.1]octan-3-yl)-5-fluoro-3-(((2R,7aS)-2-fluorotetrahydro-1*H*-pyrrolizin-7a(5*H*)-yl)methoxy)-8-methylfuro[3,2-*f*]quinazolin-6-yl)-5,6-difluoronaphthalen-2-ol. |
| (47) | 4-(1-(((1-(dimethylamino)cyclobutyl)methyl)amino)-5-fluoro-3-(((2*R*,7a*S*)-2-fluorotetrahydro-1*H*-pyrrolizin-7a(5*H*)-yl)methoxy)-8-methylfuro[3,2-*f*]quinazolin-6-yl)naphthalen-2-ol. |
| (48) | 4-(1-(((1-(dimethylamino)cyclobutyl)methyl)amino)-5-fluoro-3-(((2*R*,7a*S*)-2-fluorotetrahydro-1*H*-pyrrolizin-7a(5*H*)-yl)methoxy)-8-methylfuro[3,2-*f*]quinazolin-6-yl)-5-ethynyl-6-fluoronaphthalen-2-ol. |
| (49) | 3-(1-((1*R*,5*S*)-3,8-diazabicyclo[3.2.1]octan-3-yl)-5-fluoro-3-(((2*R*,7a*S*)-2-fluorotetrahydro-1*H*-pyrrolizin-7a(5*H*)-yl)methoxy)-8-methylfuro[3,2-*f*]quinazolin-6-yl)-4-(trifluoromethyl)aniline. |
| (50) | 6-(1-((1*R*,5*S*)-3,8-diazabicyclo[3.2.1]octan-3-yl)-5-fluoro-3-(((2*R*,7a*S*)-2-fluorotetrahydro-1*H*-pyrrolizin-7a(5*H*)-yl)methoxy)-8-methylfuro[3,2-*f*]quinazolin-6-yl)-5-(trifluoromethyl)pyridin-2-amine. |
| (51) | 1-((1*R*,5*S*)-3,8-diazabicyclo[3.2.1]octan-3-yl)-6-(5-chloro-6-methyl-1*H*-indazol-4-yl)-5-fluoro-3-(((2*R*,7a*S*)-2-fluorotetrahydro-1*H*-pyrrolizin-7a(5*H*)-yl)methoxy)-8-methylfuro[3,2-*f*]quinazoline. |
| (52) | 6(1-((1*R*,5*S*)-3,8-diazabicyclo[3.2.1]octan-3-yl)-5-fluoro-3-(((2*R*,7a*S*)-2-fluorotetrahydro-1*H*-pyrrolizin-7a(5*H*)-yl)methoxy)-8-methylfuro[3,2-*f*]quinazolin-6-yl)-4-methylpyridin-2-amine. |
| (53) | 6-(1-((1*R*,5*S*)-3,8-diazabicyclo[3.2.1]octan-3-yl)-5-fluoro-3-(((2*R*,7a*S*)-2-fluorotetrahydro-1*H*-pyrrolizin-7a(5*H*)-yl)methoxy)-8-methylfuro[3,2-*f*]quinazolin-6-yl)-5-iodo-4-methylpyridin-2-amine. |
| (54) | 4-(1-((1*R*,5*S*)-3,8-diazabicyclo[3.2.1]octan-3-yl)-5-fluoro-3-(((2*R*,7a*S*)-2-fluorotetrahydro-1*H*-pyrrolizin-7a(5*H*)-yl)methoxy)-8-methylfuro[3,2-*f*]quinazolin-6-yl)benzo[*d*]thiazol-2-amine. |
| (55) | 4-(1-((1*R*,5*S*)-3,8-diazabicyclo[3.2.1]octan-3-yl)-5-fluoro-3-(((2*R*,7a*S*)-2-fluorotetrahydro-1*H*-pyrrolizin-7a(5*H*)-yl)methoxy)-8-methylfuro[3,2-*f*]quinazolin-6-yl)-7-fluorobenzo[*d*]thiazol-2-amine. |
| (56) | 3-(1-((1*R*,5*S*)-3,8-diazabicyclo[3.2.1]octan-3-yl)-5-fluoro-3-(((2*R*,7a*S*)-2-fluorotetrahydro-1*H*-pyrrolizin-7a(5*H*)-yl)methoxy)-8-methylfuro[3,2-*f*]quinazolin-6-yl)-5-methyl-4-(trifluoromethyl)aniline. |
| (57) | 4-(1-((1*R*,5*S*)-3,8-diazabicyclo[3.2.1]octan-3-yl)-5-fluoro-3-(((2*R*,7a*S*)-2-fluorotetrahydro-1*H*-pyrrolizin-7a(5*H*)-yl)methoxy)-8-methylfuro[3,2-*f*]quinazolin-6-yl)-5-ethynyl-6-fluoronaphthalen-2-amine. |
| (58) | 4-(1-((1*R*,5*S*)-3,8-diazabicyclo[3.2.1]octan-3-yl)-3-((1-((1,1-difluoro-6-azaspiro[2.5]octan-6-yl)methyl)cyclopropyl)methoxy)-5-fluoro-8-methylfuro[3,2-*f*]quinazolin-6-yl)-5-ethynyl-6-fluoronaphthalen-2-amine. |
| (59) | 4-(1-((1*R*,5*S*)-3,8-diazabicyclo[3.2.1]octan-3-yl)-3-((1-((1,1-difluoro-6-azaspiro[2.5]octan-6-yl)methyl)cyclopropyl)methoxy)-5-fluoro-8-methylfuro[3,2-*f*]quinazolin-6-yl)-5-ethynyl-6-fluoronaphthalen-2-ol. |
| (60) | 6-(1-((1*R*,5*S*)-3,8-diazabicyclo[3.2.1]octan-3-yl)-5-fluoro-3-(((2*R*,7a*S*)-2-fluorotetrahydro-1*H*-pyrrolizin-7a(5*H*)-yl)methoxy)-8-methylfuro[3,2-*f*]quinazolin-6-yl)-4-methyl-5-(trifluoromethyl)pyridin-2-amine. |
| (61) | 4-(1-((1R,5S)-3,8-diazabicyclo[3.2.1]octan-3-yl)-5-fluoro-8-methyl-3-((*S*)-1-((*S*)-1-methylpyrrolidin-2-yl)ethoxy)furo[3,2-*f*]quinazolin-6-yl)-5-ethynyl-6-fluoronaphthalen-2-ol. |
| (62) | 4-(1-((1*R*,5*S*)-3,8-diazabicyclo[3.2.1]octan-3-yl)-5-fluoro-3-(((2*R*,7a*S*)-2-fluorotetrahydro-1*H*-pyrrolizin-7a(5*H*)-yl)methoxy)-8-methylfuro[3,2-*f*]quinazolin-6-yl)-2-amino-7-fluorobenzo[*b*]thiophene-3-carbonitrile. |
| (63) | 4-(1-((1*R*,5*S*)-3,8-diazabicyclo[3.2.1]octan-3-yl)-5-fluoro-3-(((*S,Z*)-2-(fluoromethylene)tetrahydro-1*H*-pyrrolizin-7a(5*H*)-yl)methoxy)-8-methylfuro[3,2-*f*]quinazolin-6-yl)-2-amino-7-fluorobenzo[*b*]thiophene-3-carbonitrile. |
| (64) | 4-(1-((1*R*,5*S*)-3,8-diazabicyclo[3.2.1]octan-3-yl)-5-fluoro-3-(((*S,Z*)-2-(fluoromethylene)tetrahydro-1*H*-pyrrolizin-7a(5*H*)-yl)methoxy)-8-methylfuro[3,2-*f*]quinazolin-6-yl)-5-ethynyl-6-fluoronaphthalen-2-ol. |
| (65) | 4-(1-((1*R*,5*S*)-3,8-diazabicyclo[3.2.1]octan-3-yl)-5-fluoro-3-(((2*S*,7a*R*)-2-fluoro-6-methylenetetrahydro-1*H*-pyrrolizin-7a(5*H*)-yl)methoxy)-8-methylfuro[3,2-*f*]quinazolin-6-yl)-5-ethynyl-6-fluoronaphthalen-2-ol. |
| (66) | 4-(1-((1*R*,5*S*)-3,8-diazabicyclo[3.2.1]octan-3-yl)-3-(((*S*)-2-(difluoromethylene)tetrahydro-1*H*-pyrrolizin-7a(5*H*)-yl)methoxy)-5-fluoro-8-methylfuro[3,2-*f*]quinazolin-6-yl)-5-ethynyl-6-fluoronaphthalen-2-ol. |
| (67) | 4-(1-((1*R*,5*S*)-3,8-diazabicyclo[3.2.1]octan-8-yl)-5-fluoro-3-(((2*R*,7a*S*)-2-fluorotetrahydro-1*H*-pyrrolizin-7a(5*H*)-yl)methoxy)-8-methylfuro[3,2-*f*]quinazolin-6-yl)-5-ethynyl-6-fluoronaphthalen-2-amine. |
| (68) | 4-(9-((1*R*,5*S*)-3,8-diazabicyclo[3.2.1]octan-3-yl)-7-(((2*R*,7a*S*)-2-fluorotetrahydro-1*H*-pyrrolizin-7a(5*H*)-yl)methoxy)-2-methyl-2*H*-pyrazolo[4,3-*f*]quinazolin-4-yl)naphthalen-2-ol. |
| (69) | 4-(9-((1*R*,5*S*)-3,8-diazabicyclo[3.2.1]octan-3-yl)-7-(((2*R*,7a*S*)-2-fluorotetrahydro-1*H*-pyrrolizin-7a(5*H*)-yl)methoxy)-2-methyl-2*H*-pyrazolo[4,3-*f*]quinazolin-4-yl)-5-ethynyl-6-fluoronaphthalen-2-ol. |
| (70) | 4-(9-((1*R*,5*S*)-3,8-diazabicyclo[3.2.1]octan-3-yl)-7-(((2*R*,7a*S*)-2-fluorotetrahydro-1*H*-pyrrolizin-7a(5*H*)-yl)methoxy)-2-methyl-2*H*-pyrazolo[4,3-*f*]quinazolin-4-yl)-5,6-difluoronaphthalen-2-ol. |
| (71) | 4-(9-((1*R*,5*S*)-3,8-diazabicyclo[3.2.1]octan-3-yl)-7-(((2*R*,7a*S*)-2-fluorotetrahydro-1*H*-pyrrolizin-7a(5*H*)-yl)methoxy)-2-methyl-2*H*-pyrazolo[4,3-*f*]quinazolin-4-yl)-5-ethynyl-6-fluoronaphthalen-2-amine. |
| (72) | 4-(9-((1*R*,5*S*)-3,8-diazabicyclo[3.2.1]octan-3-yl)-7-(3-(dimethylamino)azetidin-1-yl)-5-fluoro-2-methyl-2*H*-pyrazolo[4,3-*f*]quinazolin-4-yl)-5-ethynyl-6-fluoronaphthalen-2-ol. |
| (73) | 4-(9-((1*R*,5*S*)-3,8-diazabicyclo[3.2.1]octan-3-yl)-7-((1-((1,1-difluoro-6-azaspiro[2.5]octan-6-yl)methyl)cyclopropyl)methoxy)-2-methyl-2*H-*pyrazolo[4,3-*f*]quinazolin-4-yl)-5-ethynyl-6-fluoronaphthalen-2-ol. |
| (74) | 4-(9-((1*R*,5*S*)-3,8-diazabicyclo[3.2.1]octan-3-yl)-7-(((*S,Z*)-2-(fluoromethylene)tetrahydro-1*H*-pyrrolizin-7a(5*H*)-yl)methoxy)-2-methyl-2*H-*pyrazolo[4,3-*f*]quinazolin-4-yl)-5-ethynyl-6-fluoronaphthalen-2-amine. |
| (75) | 4-(9-((1*R*,5*S*)-3,8-diazabicyclo[3.2.1]octan-3-yl)-7-(((*S,Z*)-2-(fluoromethylene)tetrahydro-1*H*-pyrrolizin-7a(5*H*)-yl)methoxy)-2-methyl-2*H-*pyrazolo[4,3-*f*]quinazolin-4-yl)-5-ethynyl-6-fluoronaphthalen-2-ol. |
| (76) | 4-(9-((1*R*,5*S*)-3,8-diazabicyclo[3.2.1]octan-3-yl)-7-((1-((1,1-difluoro-6-azaspiro[2.5]octan-6-yl)methyl)cyclopropyl)methoxy)-2-methyl-2*H-*pyrazolo[4,3-*f*]quinazolin-4-yl)-5-ethynyl-6-fluoronaphthalen-2-amine. |
| (77) | 4-(9-((1*R*,5*S*)-3,8-diazabicyclo[3.2.1]octan-3-yl)-7-(((2*R*,7a*S*)-2-fluorotetrahydro-1*H*-pyrrolizin-7a(5*H*)-yl)methoxy)-2-methyl-2*H*-pyrazolo[4,3-*f*]quinazolin-4-yl)-2-amino-7-fluorobenzo[*b*]thiophene-3-carbonitrile. |
| (78) | 9-((1*R*,5*S*)-3,8-diazabicyclo[3.2.1]octan-3-yl)-4-(8-ethynyl-7-fluoronaphthalen-1-yl)-7-(((*S*,*Z*)-2-(fluoromethylene)tetrahydro-1*H*-pyrrolizin-7a(5*H*)-yl)methoxy)-2-methyl-2*H*-pyrazolo[4,3-*f*]quinazoline |
| (79) | 4-(9-((1*R*,5*S*)-3,8-diazabicyclo[3.2.1]octan-3-yl)-7-(((2*S*,7a*R*)-2-fluoro-6-methylenetetrahydro-1*H*-pyrrolizin-7a(5*H*)-yl)methoxy)-2-methyl-2*H-*pyrazolo[4,3-*f*]quinazolin-4-yl)-5-ethynyl-6-fluoronaphthalen-2-ol. |
| (80) | 4-(9-((1*R*,5*S*)-3,8-diazabicyclo[3.2.1]octan-3-yl)-5-fluoro-7-(((2*R*,7a*S*)-2-fluorotetrahydro-1*H*-pyrrolizin-7a(5*H*)-yl)methoxy)-2-methyl-2*H*-pyrazolo[4,3-*f*]quinazolin-4-yl)-5-ethynyl-6-fluoronaphthalen-2-ol. |
| (81) | 4-(9-((1*R*,5*S*)-3,8-diazabicyclo[3.2.1]octan-3-yl)-5-fluoro-7-(((2*R*,7a*S*)-2-fluorotetrahydro-1*H*-pyrrolizin-7a(5*H*)-yl)methoxy)-2-methyl-2*H*-pyrazolo[4,3-*f*]quinazolin-4-yl)-5-ethynyl-6-fluoronaphthalen-2-amine. |
| (82) | 9-((1*R*,5*S*)-3,8-diazabicyclo[3.2.1]octan-3-yl)-4-(8-ethynyl-7-fluoronaphthalen-1-yl)-5-fluoro-7-(((2*R*,7a*S*)-2-fluorotetrahydro-1*H*-pyrrolizin-7a(5*H*)-yl)methoxy)-2-methyl-2*H*-pyrazolo[4,3-*f*]quinazoline. |
| (83) | 4-(9-((1*R*,5*S*)-3,8-diazabicyclo[3.2.1]octan-3-yl)-7-(((2*R*,7a*S*)-2-fluorotetrahydro-1*H*-pyrrolizin-7a(5*H*)-yl)methoxy)-2-methyl-2*H*-pyrazolo[4,3-*f*]quinazolin-4-yl)-5-ethyl-6-fluoronaphthalen-2-ol. |
| (84) | 4-(9-((1*R*,5*S*)-3,8-diazabicyclo[3.2.1]octan-3-yl)-7-(((*S,Z*)-2-(fluoromethylene)tetrahydro-1*H*-pyrrolizin-7a(5*H*)-yl)methoxy)-2-methyl-2H-pyrazolo[4,3-*f*]quinazolin-4-yl)-5,6-difluoronaphthalen-2-ol. |
| (85) | 4-(9-((1*R*,5*S*)-3,8-diazabicyclo[3.2.1]octan-3-yl)-5-fluoro-7-(((*S,Z*)-2-(fluoromethylene)tetrahydro-1*H*-pyrrolizin-7a(5*H*)-yl)methoxy)-2-methyl-2*H-*pyrazolo[4,3-*f*]quinazolin-4-yl)-5-ethyl-6-fluoronaphthalen-2-ol. |
| (86) | 4-(9-((1*R*,5*S*)-3,8-diazabicyclo[3.2.1]octan-3-yl)-5-fluoro-7-(((*S,Z*)-2-(fluoromethylene)tetrahydro-1*H*-pyrrolizin-7a(5*H*)-yl)methoxy)-2-methyl-2*H-*pyrazolo[4,3-*f*]quinazolin-4-yl)-5,6-difluoronaphthalen-2-ol. |
| (87) | 4-(9-((1*R*,5*S*)-3,8-diazabicyclo[3.2.1]octan-3-yl)-7-((1-((1,1-difluoro-6-azaspiro[2.5]octan-6-yl)methyl)cyclopropyl)methoxy)-5-fluoro-2-methyl-2*H-*pyrazolo[4,3-*f*]quinazolin-4-yl)-5-ethynyl-6-fluoronaphthalen-2-ol. |
| (88) | 4-(9-(2,5-diazabicyclo[2.2.2]octan-2-yl)-5-fluoro-7-(((2*R*,7a*S*)-2-fluorotetrahydro-1*H*-pyrrolizin-7a(5*H*)-yl)methoxy)-2-methyl-2*H*-pyrazolo[4,3-*f*]quinazolin-4-yl)-5-ethynyl-6-fluoronaphthalen-2-ol. |
| (89) | 9-((1*R*,5*S*)-3,8-diazabicyclo[3.2.1]octan-3-yl)-4-(5-chloro-3,6-dimethyl-1*H-*indazol-4-yl)-5-fluoro-7-(((2*R*,7a*S*)-2-fluorotetrahydro-1*H*-pyrrolizin-7a(5*H*)-yl)methoxy)-2-methyl-2*H*-pyrazolo[4,3-*f*]quinazoline. |
| (90) | 4-(9-((1*R*,5*S*)-3,8-diazabicyclo[3.2.1]octan-3-yl)-7-(((*S*)-2-(difluoromethylene)tetrahydro-1*H*-pyrrolizin-7a(5*H*)-yl)methoxy)-5-fluoro-2-methyl-2*H*-pyrazolo[4,3-*f*]quinazolin-4-yl)-5-ethynyl-6-fluoronaphthalen-2-ol. |
| (91) | 4-((R)-9-((1*R*,5*S*)-3,8-diazabicyclo[3.2.1]octan-3-yl)-7-(((*S*)-2-(difluoromethylene)tetrahydro-1*H*-pyrrolizin-7a(5*H*)-yl)methoxy)-5-fluoro-2-methyl-2*H*-pyrazolo[4,3-*f*]quinazolin-4-yl)-5-ethynyl-6-fluoronaphthalen-2-ol. |
| (92) | 4-((*S*)-9-((1*R*,5*S*)-3,8-diazabicyclo[3.2.1]octan-3-yl)-7-(((*S*)-2-(difluoromethylene)tetrahydro-1*H*-pyrrolizin-7a(5*H*)-yl)methoxy)-5-fluoro-2-methyl-2*H*-pyrazolo[4,3-*f*]quinazolin-4-yl)-5-ethynyl-6-fluoronaphthalen-2-ol. |
| (93) | 4-(9-((1*R*,5*S*)-3,8-diazabicyclo[3.2.1]octan-3-yl)-5-fluoro-7-(((2*R*,7a*S*)-2-fluorotetrahydro-1*H*-pyrrolizin-7a(5*H*)-yl)methoxy)-2-methyl-2*H*-pyrazolo[4,3-*f*]quinazolin-4-yl)-5-ethyl-6-fluoronaphthalen-2-ol. |
| (94) | 4-(9-((1*R*,5*S*)-3,8-diazabicyclo[3.2.1]octan-3-yl)-5-fluoro-7-(((*S,Z*)-2-(fluoromethylene)tetrahydro-1*H*-pyrrolizin-7a(5*H*)-yl)methoxy)-2-methyl-2*H-*pyrazolo[4,3-*f*]quinazolin-4-yl)-5-ethynyl-6-fluoronaphthalen-2-ol. |
| (95) | 4-(9-((1*R*,5*S*)-3,8-diazabicyclo[3.2.1]octan-3-yl)-5-fluoro-7-(((2*R*,7a*S*)-2-fluorotetrahydro-1*H*-pyrrolizin-7a(5*H*)-yl)methoxy)-2-methyl-2*H*-pyrazolo[4,3-*f*]quinazolin-4-yl)-5,6-difluoronaphthalen-2-ol. |
| (96) | 4-(9-((1*R*,5*S*)-3,8-diazabicyclo[3.2.1]octan-3-yl)-5-fluoro-7-(((5*S*,7a*S*)-5-(methoxymethyl)-2-methylenetetrahydro-1*H*-pyrrolizin-7a(5*H*)-yl)methoxy)-2-methyl-2H-pyrazolo[4,3-*f*]quinazolin-4-yl)-5-ethynyl-6-fluoronaphthalen-2-ol. |
| (97) | 4-(9-((1*R*,5*S*)-3,8-diazabicyclo[3.2.1]octan-3-yl)-7-(((*S*)-2-(difluoromethylene)tetrahydro-1*H*-pyrrolizin-7a(5*H*)-yl)methoxy)-5-fluoro-2-methyl-2*H*-pyrazolo[4,3-*f*]quinazolin-4-yl)-5-ethyl-6-fluoronaphthalen-2-ol. |
| (98) | 4-(9-((1*R*,5*S*)-3,8-diazabicyclo[3.2.1]octan-3-yl)-5-fluoro-7-((1-(((*R*)-3-fluoropyrrolidin-1-yl)methyl)cyclobutyl)methoxy)-2-methyl-2*H*-pyrazolo[4,3-*f*]quinazolin-4-yl)-5-ethynyl-6-fluoronaphthalen-2-ol. |
| (99) | 4-(9-((1*R*,5*S*)-3,8-diazabicyclo[3.2.1]octan-3-yl)-7-(((*S*)-2-(difluoromethylene)tetrahydro-1*H*-pyrrolizin-7a(5*H*)-yl)methoxy)-5-fluoro-2-amine. |
| (100) | 4-(9-((1*R*,5*S*)-3,8-diazabicyclo[3.2.1]octan-3-yl)-5-fluoro-7-(((2*S*,7a*R*)-2-fluoro-6-methylenetetrahydro-1*H*-pyrrolizin-7a(5*H*)-yl)methoxy)-2-methyl-2*H-*pyrazolo[4,3-*f*]quinazolin-4-yl)-5-ethynyl-6-fluoronaphthalen-2-ol. |
| (101) | 9-((1*R*,5*S*)-3,8-diazabicyclo[3.2.1]octan-3-yl)-7-(((*S*)-2-(difluoromethylene)tetrahydro-1*H*-pyrrolizin-7a(5*H*)-yl)methoxy)-4-(8-ethynyl-7-fluoronaphthalen-1-yl)-5-fluoro-2-methyl-2*H*-pyrazolo[4,3-*f*]quinazoline. |
| (102) | (4-(4-(8-ethynyl-7-fluoro-3-hydroxynaphthalen-1-yl)-5-fluoro-7-(((2*R*,7a*S*)-2-fluorotetrahydro-1*H*-pyrrolizin-7a(5*H*)-yl)methoxy)-2-methyl-2*H*-pyrazolo[4,3-*f*]guinazolin-9-yl)piperazin-1-yl)(oxiran-2-yl)methanone. |
| (103) | 4-(9-((1*R*,5*S*)-3,8-diazabicyclo[3.2.1]octan-8-yl)-5-fluoro-7-(((2*R*,7a*S*)-2-fluorotetrahydro-1*H*-pyrrolizin-7a(5*H*)-yl)methoxy)-2-methyl-2*H*-pyrazolo[4,3-*f*]quinazolin-4-yl)-5-ethynyl-6-fluoronaphthalen-2-ol. |
| (104) | 4-(9-((1*R*,5*S*)-3,8-diazabicyclo[3.2.1]octan-3-yl)-5-fluoro-7-(((2*R*,7a*S*)-2-fluorotetrahydro-1*H*-pyrrolizin-7a(5*H*)-yl)methoxy)-2-methyl-2*H*-pyrazolo[4,3-*f*]quinazolin-4-yl)-2-amino-7-fluorobenzo[*b*]thiophene-3-carbonitrile |
| (105) | 4-(9-((1*R*,4*R*)-2,5-diazabicyclo[2.2.1]heptan-2-yl)-5-fluoro-7-(((2*R*,7a*S*)-2-fluorotetrahydro-1*H*-pyrrolizin-7a(5*H*)-yl)methoxy)-2-methyl-2*H*-pyrazolo[4,3-*f*]quinazolin-4-yl)-5-ethynyl-6-fluoronaphthalen-2-ol |
| (106) | 4-(1-((1*R*,5*S*)-3,8-diazabicyclo[3.2.1]octan-8-yl)-3-(((*S*)-2-(difluoromethylene)tetrahydro-1*H*-pyrrolizin-7a(5*H*)-yl)methoxy)imidazo[1',2':1,6]pyrido[3,2-*d*]pyrimidin-6-yl)-5-ethynyl-6-fluoronaphthalen-2-ol |
| (107) | 4-(1-((1*R*,5*S*)-3,8-diazabicyclo[3.2.1]octan-8-yl)-3-(((*S*)-2-(difluoromethylene)tetrahydro-1*H*-pyrrolizin-7a(5*H*)-yl)methoxy)imidazo[1',2':1,6]pyrido[3,2-*d*]pyrimidin-6-yl)-5-ethyl-6-fluoronaphthalen-2-ol |
| (108) | 4-(1-((1*R*,5*S*)-3,8-diazabicyclo[3.2.1]octan-8-yl)-3-(((*S*)-2-(difluoromethylene)tetrahydro-1*H*-pyrrolizin-7a(5*H*)-yl)methoxy)imidazo[1',2':1,6]pyrido[3,2-*d*]pyrimidin-6-yl)-5-ethynyl-6-fluoronaphthalen-2-amine |
| (109) | 4-(1-((1*R*,5*S*)-3,8-diazabicyclo[3.2.1]octan-3-yl)-3-(((*S*)-2-(difluoromethylene)tetrahydro-1*H*-pyrrolizin-7a(5*H*)-yl)methoxy)imidazo[1',2':1,6]pyrido[3,2-*d*]pyrimidin-6-yl)-5-ethyl-6-fluoronaphthalen-2-ol |
| (110) | 4-(9-((1*R*,5*S*)-3,8-diazabicyclo[3.2.1]octan-3-yl)-5-fluoro-7-(((2*R*,7a*S*)-2-fluorotetrahydro-1*H*-pyrrolizin-7a(5*H*)-yl)methoxy)-2-methyl-2*H*-pyrazolo[4,3-*f*]quinazolin-4-yl)-2-amino-5-fluorobenzo[*b*]thiophene-3-carbonitrile. |
| (111) | 4-(9-((1*R*,5*S*)-3,8-diazabicyclo[3.2.1]octan-3-yl)-5-fluoro-7-(((2*R*,7a*S*)-2-fluorotetrahydro-1*H*-pyrrolizin-7a(5*H*)-yl)methoxy)-2-methyl-2*H*-pyrazolo[4,3-*f*]quinazolin-4-yl)-2-amino-5,7-difluorobenzo[*b*]thiophene-3-carbonitrile. |
| (112) | 4-(9-((1*R*,5*S*)-3,8-diazabicyclo[3.2.1]octan-3-yl)-5-fluoro-7-(((2*R*,7a*S*)-2-fluorotetrahydro-1*H*-pyrrolizin-7a(5*H*)-yl)methoxy)-2-methyl-2*H*-pyrazolo[4,3-*f*]quinazolin-4-yl)-2-amino-7-fluorothieno[3,2-*c*]pyridine-3-carbonitrile. |
| (113) | 4-(9-((1*R*,5*S*)-3,8-diazabicyclo[3.2.1]octan-3-yl)-5-fluoro-7-(((2*R*,7a*S*)-2-fluorotetrahydro-1*H*-pyrrolizin-7a(5*H*)-yl)methoxy)-2-methyl-2*H*-pyrazolo[4,3-*f*]quinazolin-4-yl)-2-aminobenzofuran-3-carbonitrile. |
| (114) | 4-(9-((1*R*,5*S*)-3,8-diazabicyclo[3.2.1]octan-3-yl)-5-fluoro-7-(((2*R*,7a*S*)-2-fluorotetrahydro-1*H*-pyrrolizin-7a(5*H*)-yl)methoxy)-2-methyl-2*H*-pyrazolo[4,3-*f*]quinazolin-4-yl)-2-amino-7-fluorobenzofuran-3-carbonitrile. |
| (115) | 4-(9-((1*R*,5*S*)-3,8-diazabicyclo[3.2.1]octan-3-yl)-5-fluoro-7-(((2*R*,7a*S*)-2-fluorotetrahydro-1*H*-pyrrolizin-7a(5*H*)-yl)methoxy)-2-methyl-2*H*-pyrazolo[4,3-*f*]quinazolin-4-yl)-2-amino-5-fluorobenzofuran-3-carbonitrile. |
| (116) | 4-(9-((1*R*,5*S*)-3,8-diazabicyclo[3.2.1]octan-8-yl)-5-fluoro-7-(((2*R*,7a*S*)-2-fluorotetrahydro-1*H*-pyrrolizin-7a(5*H*)-yl)methoxy)-2-methyl-2*H*-pyrazolo[4,3-*f*]quinazolin-4-yl)-5-ethynyl-6-fluoronaphthalen-2-amine |
| (117) | 4-(9-((1*R*,5*S*)-3,8-diazabicyclo[3.2.1]octan-3-yl)-5-fluoro-7-(((2*R*,7a*S*)-2-methoxytetrahydro-1H-pyrrolizin-7a(5*H*)-yl)methoxy)-2-methyl-2*H-*pyrazolo[4,3-*f*]quinazolin-4-yl)-5-ethynyl-6-fluoronaphthalen-2-ol. |
| (118) | 9-((1*R*,5*S*)-3,8-diazabicyclo[3.2.1]octan-3-yl)-4-(5-ethynyl-6-fluoroisoquinolin-4-yl)-5-fluoro-7-(((2*R*,7a*S*)-2-fluorotetrahydro-1*H*-pyrrolizin-7a(5*H*)-yl)methoxy)-2-methyl-2*H*-pyrazolo[4,3-*f*]quinazoline |
| (119) | 4-(9-((1*R*,5*S*)-3,8-diazabicyclo[3.2.1]octan-3-yl)-7-(((*R*)-1-((dimethylamino)methyl)-2,2-difluorocyclopropyl)methoxy)-5-fluoro-2-methyl-2*H*-pyrazolo[4,3-*f*]quinazolin-4-yl)-5-ethynyl-6-fluoronaphthalen-2-ol. |
| (120) | 4-(9-((1*R*,5*S*)-3,8-diazabicyclo[3.2.1]octan-3-yl)-7-(((1*S*,7a'*S*)-2,2-difluorodihydro-1'*H*,3'*H*-spiro[cyclopropane-1,2'-pyrrolizin]-7a'(5'*H*)-yl)methoxy)-5-fluoro-2-methyl-2*H*-pyrazolo[4,3-*f*]quinazolin-4-yl)-5-ethynyl-6-fluoronaphthalen-2-ol. |

18. A pharmaceutical composition comprising the compound, or the optical isomer, stereoisomer, racemate, isotopic variant, solvate, hydrate, or the pharmaceutically acceptable salt thereof according to any one of claims 1 to 17.

19. The pharmaceutical composition according to claim 18, further comprising a pharmaceutically acceptable carrier.

20. The pharmaceutical composition according to claim 18, wherein the pharmaceutical composition is an antagonist of KRAS G12D mutant protein.

21. The pharmaceutical composition according to claim 18, wherein the pharmaceutical composition is for the prevention or treatment of a disease associated with KRAS G12D mutant protein.

22. The pharmaceutical composition according to claim 18, wherein the pharmaceutical composition is for the prevention or treatment of cancer.

23. The pharmaceutical composition according to claim 18, wherein the composition is administered by one or more routes selected from the group consisting of oral administration, intravenous injection, subcutaneous injection, intramuscular injection, intraperitoneal injection, intradermal administration, topical administration, intranasal administration, intrapulmonary administration, and rectal administration.

24. The pharmaceutical composition according to claim 18, wherein the composition is formulated in one or more dosage forms selected from the group consisting of tablets, pills, hard or soft capsules, liquids, suspensions, emulsions, syrups, granules, and elixirs.

25. The pharmaceutical composition according to claim 18, further comprising one or more selected from the group consisting of RTK/Ras-MAPK pathway-related protein inhibitor, DNA-damaging agent, EGFR antibody, and an immune-oncology therapeutic agent.

26. The pharmaceutical composition according to claim 25, wherein the RTK/Ras-MAPK pathway-related protein inhibitor is one or more selected from the group consisting of EGFR inhibitor, FGFR inhibitor, ALK inhibitor, ROS inhibitor, MET inhibitor, RAF inhibitor, ERK inhibitor, MEK inhibitor, SHP-2 inhibitor, PI3K inhibitor, KRAS inhibitor, KRAS-G12C inhibitor, and SOS1 inhibitor.

27. The pharmaceutical composition according to claim 22, wherein the cancer is one or more selected from the group consisting of hemangiosarcoma, fibrosarcoma, rhabdomyosarcoma, liposarcoma, myxoma, rhabdomyoma, fibroma, lipoma, teratoma, squamous cell carcinoma, undifferentiated small cell carcinoma, undifferentiated multicellular carcinoma, adenocarcinoma, alveolar (bronchiolar) cancer, bronchiolar adenoma, sarcoma, lymphoma, chondromatosis, mesothelioma, esophageal cancer, stomach cancer, pancreatic cancer, small intestine cancer, colon cancer, kidney cancer, bladder cancer, urethral cancer, prostate cancer, testicular cancer, liver cancer, biliary tract cancer, hepatoblastoma, hepatocellular adenoma, hemangioma, gallbladder cancer, ampullary cancer, osteosarcoma, malignant fibrous histiocytoma, chondrosarcoma, Ewing's sarcoma, malignant lymphoma (reticulocyte cell sarcoma), multiple myeloma, malignant giant cell tumor, osteochondroma, benign chondroma, chondromyxoid fibroma, chondroostoma, giant cell tumor, cranioma, cranial hemangioma, cranial granuloma, cranial xanthoma, cranial osteitis deformans, meningioma, meningiosarcoma, glioblastoma, astrocytoma, medulloblastoma, ependymoma, germinoma, oligodendroglioma, schwannoma, retinoblastoma, spinal neurofibroma, endometrial cancer, cervical cancer, ovarian cancer, blood cancer, acute lymphoblastic leukemia, chronic lymphocytic leukemia, acute myeloid leukemia, monocytic leukemia, chronic myeloid leukemia, chronic lymphocytic leukemia, mixed lineage leukemia, Hodgkin's lymphoma, non-Hodgkin's lymphoma, malignant melanoma, basal cell carcinoma, adenocarcinoma, and neuroblastoma.

28. A composition for binding to KRAS G12D mutant protein, comprising the compound, or the optical isomer, stereoisomer, racemate, isotopic variant, solvate, hydrate, or the pharmaceutically acceptable salt thereof according to any one of claims 1 to 17.
